# EUROPEAN PATENT SPECIFICATION

(11) **EP 2 646 425 B1**
(45) Date of publication and mention of the grant of the patent: **10.06.2015**
(21) Application number: 11788845.3
(22) Date of filing: 30.11.2011
(51) Int. Cl.: C07D 307/80, C07D 405/12, C07D 407/12, C07D 413/12, A61K 31/343, A61P 3/10

(54) **Indanyloxydihydrobenzofuranylacetic acids useful for the treatment of metabolic syndrome**
Zur Behandlung des metabolischen Syndroms geeignete Indanyloxydihydrobenzofuranylessigsäuren
Acides indanyloxydihydrobenzofurannylacétiques utiles dans le traitement du syndrome métabolique

(30) Priority: 01.12.2010 EP 10193382; 18.02.2011 EP 11154999; 27.07.2011 EP 11175646
(43) Date of publication of application: 09.10.2013
(73) Proprietor: BOEHRINGER INGELHEIM INTERNATIONAL GMBH, 55216 Ingelheim am Rhein (DE)
(72) Inventor: HIMMELSBACH, Frank, 55216 Ingelheim Am Rhein (DE); BAKKER, Remko, 55216 Ingelheim Am Rhein (DE); ECKHARDT, Matthias, 55216 Ingelheim Am Rhein (DE); HAMPRECHT, Dieter, 55216 Ingelheim Am Rhein (DE); LANGKOPF, Elke, 55216 Ingelheim Am Rhein (DE); WAGNER, Holger, 55216 Ingelheim Am Rhein (DE)
(74) Representative: Simon, Elke Anna Maria
(86) International application number: PCT/EP2011/071410
(87) International publication number: WO 2012/072691

(56) References cited:
- EP-A2- 2 006 271
- WO-A2-2010/045258

## Description

### Field of the Invention

The present invention relates to novel indanyloxydihydrobenzofuranylacetic acids, that are agonists of the G-protein coupled receptor 40 (GPR40, also known as free fatty acid receptor FFAR 1), to processes for their preparation, to pharmaceutical compositions containing these compounds and to their medical use for the prophylaxis and/or treatment of diseases which can be influenced by the modulation of the function of GPR40. Particularly, the pharmaceutical compositions of the invention are suitable for the prophylaxis and/or therapy of metabolic diseases, such as diabetes, more specifically type 2 diabetes mellitus, and conditions associated with the disease, including insulin resistance, obesity, cardiovascular disease and dyslipidemia.

### Background of the Invention

Metabolic diseases are diseases caused by an abnormal metabolic process and may either be congenital due to an inherited enzyme abnormality or acquired due to a disease of an endocrine organ or failure of a metabolically important organ such as the liver or the pancreas.

Diabetes mellitus is a disease state or process derived from multiple causative factors and is defined as a chronic hyperglycemia associated with resulting damages to organs and dysfunctions of metabolic processes. Depending on its etiology, one differentiates between several forms of diabetes, which are either due to an absolute (lacking or decreased insulin secretion) or to a relative lack of insulin. Diabetes mellitus Type I (IDDM, insulin-dependent diabetes mellitus) generally occurs in adolescents under 20 years of age. It is assumed to be of auto-immune etiology, leading to an insulitis with the subsequent destruction of the beta cells of the islets of Langerhans which are responsible for the insulin synthesis. In addition, in latent autoimmune diabetes in adults (LADA; Diabetes Care. 8: 1460-1467, 2001) beta cells are being destroyed due to autoimmune attack. The amount of insulin produced by the remaining pancreatic islet cells is too low, resulting in elevated blood glucose levels (hyperglycemia). Diabetes mellitus Type II generally occurs at an older age. It is above all associated with a resistance to insulin in the liver and the skeletal muscles, but also with a defect of the islets of Langerhans. High blood glucose levels (and also high blood lipid levels) in turn lead to an impairment of beta cell function and to an increase in beta cell apoptosis.

Persistent or inadequately controlled hyperglycemia is associated with a wide range of pathologies. Diabetes is a very disabling disease, because today's common antidiabetic drugs do not control blood sugar levels well enough to completely prevent the occurrence of high and low blood sugar levels. Out of range blood sugar levels are toxic and cause long-term complications for example retinopathy, renopathy, neuropathy and peripheral vascular disease. There is also a host of related conditions, such as obesity, hypertension, stroke, heart disease and hyperlipidemia, for which persons with diabetes are substantially at risk.

Obesity is associated with an increased risk of follow-up diseases such as cardiovascular diseases, hypertension, diabetes, hyperlipidemia and an increased mortality. Diabetes (insulin resistance) and obesity are part of the "metabolic syndrome" which is defined as the linkage between several diseases (also referred to as syndrome X, insulin-resistance syndrome, or deadly quartet). These often occur in the same patients and are major risk factors for development of diabetes type II and cardiovascular disease. It has been suggested that the control of lipid levels and glucose levels is required to treat diabetes type II, heart disease, and other occurrences of metabolic syndrome (see e.g., Diabetes 48: 1836-1841, 1999; JAMA 288: 2209-2716, 2002).

The free fatty acid receptor GPR40 (also referred to as either FFAR, FFAR1, or FFA1) is a cell-surface receptor and a member of the gene superfamily of G-protein coupled receptors, which was first identified as a so-called orphan receptor, i.e. a receptor without a known ligand, based on the predicted prescence of seven putative transmembrane regions in the corresponding protein (Sawzdargo et al. (1997) Biochem. Biophys. Res. Commun. 239: 543-547). GPR40 is found to be highly expressed in several particular cell types: the pancreatic β cells and insulin-secreting cell lines, as well as in enteroendocrine cells, taste cells, and is reported to be expressed in immune cells, splenocytes, and in the human and monkey brain. Meanwhile, fatty acids of varying chain lengths are thought to represent the endogenous ligands for GPR40, activation of which is linked primarily to the modulation of the Gq family of intra-cellular signaling G proteins and concomitant induction of elevated calcium levels, although activation of Gs- and Gi-proteins to modulate intracellular levels of cAMP have also been reported. GPR40 is activated especially by long-chain FFA, particularly oleate, as well as the PPAR-gamma agonist rosiglitazone.

It has been recognized that the fatty acids that serve as activators for GPR40 augment the elevated plasma glucose-induced secretion of insulin through GPR40 receptors that are expressed in the insulin secreting cells (Itoh et al. (2003) Nature 422: 173-176; Briscoe et al. (2003) J. Biol. Chem. 278: 11303-11311; Kotarsky et al. (2003) Biochem. Biophys. Res. Commun. 301: 406-410). Despite initial controversy, the use of GPR40 agonist appears to be the appropriate for increasing insulin release for the treatment of diabetes (see e.g. Diabetes 2008, 57, 2211; J. Med. Chem. 2007, 50, 2807). Typically, long term diabetes therapy leads to the gradual diminution of islet activity, so that after extended periods of treatment Type 2 diabetic patients need treatment with daily insulin injections instead. GPR40 agonists may have the potential to restore or preserve islet function, therefore, GPR40 agonists may be beneficial also in that that they may delay or prevent the diminution and loss of islet function in a Type 2 diabetic patient.

It is well established that the incretins GLP-1 (glucagon-like peptide- 1) and GIP (glucose-dependent insulinotropic peptide; also known as gastric inhibitory peptide) stimulate insulin secretion and are rapidly inactivated in vivo by DPP-4. These peptidyl hormones are secreted by endocrine cells that are located in the epithelium of the small intestine. When these endocrine cells sense an increase in the concentration of glucose in the lumen of the digestive tract, they act as the trigger for incretin release. Incretins are carried through the circulation to beta cells in the pancreas and cause the beta cells to secrete more insulin in anticipation of an increase of blood glucose resulting from the digesting meal. Further studies indicating that the GPR40 modulatory role on the release of incretins from the enteroendocrine cells, including CCK, GLP-1, GIP, PYY, and possibly others, suggest that GPR40 modulators may contribute to enhanced insulin release from the pancreatic beta cells also indirectly by e.g. a synergistic effect of GLP-1 and possibly GIP on the insulin release, and the other release incretins may also contribute to an overall beneficial contribution of GPR40 modulation on metabolic diseases. The indirect contributions of GPR40 modulation on insulin release through the elevation of plasma levels of incretins may be further augmented by the coadministration of inhibitors of the enzymes responsible for the incretin degradation, such as inhibitors of DPP-4.

Insulin imbalances lead to conditions such as type II diabetes mellitus, a serious metabolic disease. The modulation of the function of GPR40 in modulating insulin secretion indicates the therapeutic agents capable of modulating GPR40 function could be useful for the treatment of disorders such as diabetes and conditions associated with the disease, including insulin resistance, obesity, cardiovascular disease and dyslipidemia.

### Object of the invention

The problem underlying the present invention is to provide potent and selective GPR40 agonists, hereinafter described as compounds of formula (I), for use in prophylaxis and/or treatment of metabolic diseases, such as diabetes, more specifically type 2 diabetes mellitus, and conditions associated with the disease, including insulin resistance, obesity, cardiovascular disease and dyslipidemia, and their consecutive complications and disorders,

GPR40 modulators are known in the art, for example, the compounds disclosed in WO 2004041266 (EP 1559422), WO 2007033002 and WO 2009157418. The indanyloxydihydrobenzofuranylacetic acids of the present invention may provide several advantages, such as enhanced potency, high metabolic and/or chemical stability, high selectivity and tolerability, enhanced solubility, and the possibility to form stable salts.

### Summary of the Invention

In one aspect the invention provides compounds of formula (I): 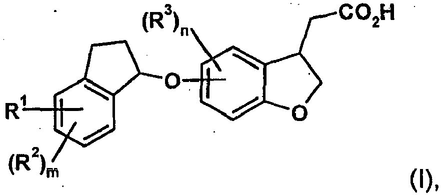 as defined hereinafter, the isoforms, tautomers, stereoisomers, solvates, hydrates, and the salts thereof, particularly the physiologically acceptable salts thereof with inorganic or organic acid or bases, or the combinations thereof.

The present invention further provides a pharmaceutical composition comprising a compound of formula (I) and a pharmaceutically acceptable carrier.

In some embodiments, a compound of formula I is administered in combination with other therapeutic agents. In some such embodiments, the other therapeutic agent is metformin and/or is a PPAR agonist (i.e. a thiazolidinedione such as pioglitazone) and/or is a glinide and/or is a. DPP-IV inhibitor (i.e. linagliptin) and/or is a SGLT2-inhibitor and/or is a GLP-1 receptor agonist (i.e. a GLP-1 analogue or mimetic (i.e. glutides)) and/or is insulin or an insulin analogue and/or is an 11β-HSD1 inhibitor and/or is a glucokinase activator and/or is a GPR119 agonist. The other therapeutic agent(s) may be administered before, during, or after administration of the compound of formula I.

The present invention further provides a compound of formula (I), as described herein, for use in a therapeutic method of treatment of the human or animal body.

The present invention further provides compounds of formula (I) for use in the treatment and/or prophylaxis of diseases responsive to the modulation of GPR40.

The present invention further provides compounds of formula (I) for use in the treatment and/or prophylaxis of an indication selected from diseases related to the regulation of metabolic disorders, such as of the carbohydrate metabolism and their consecutive complications or the lipid metabolism (i.e. lipid disorders) and their consecutive complications.

The present invention further provides compounds of formula (I) for use in the treatment and/or prophylaxis of an indication selected from type II diabetes, obesity, hyperglycemia, glucose intolerance, insulin resistance, hyperinsulinemia, hypercholesterolemia, hypertension, hyperlipoproteinemia, hyperlipidemia, hypertriglylceridemia, dyslipidemia, metabolic syndrome, syndrome X3 cardiovascular disease, atherosclerosis, kidney disease, ketoacidosis, thrombotic disorders, nephropathy, diabetic neuropathy, diabetic retinopathy, sexual dysfunction, dermatopathy, dyspepsia, hypoglycemia, cancer or edema.

The present invention further provides the use of a compound of formula (I) for preparing a pharmaceutical composition for the treatment and/or prophylaxis of diseases responsive to the modulation of GPR40.

The present invention further provides the use of a compound of formula (I) for preparing a pharmaceutical composition for the treatment and/or prophylaxis of an indication selected from diseases related to the regulation of metabolic disorders, such as of the carbohydrate metabolism and their consecutive complications or the lipid metabolism (i.e. lipid disorders) and their consecutive complications.

The present invention further provides the use of a compound of formula (I) for preparing a pharmaceutical composition for the treatment and/or prophylaxis of an indication selected from type II diabetes, obesity, hyperglycemia, glucose intolerance, insulin resistance, hyperinsulinemia, hypercholesterolemia, hypertension, hyperlipoproteinemia, hyperlipidemia, hypertriglylceridemia, dyslipidemia, metabolic syndrome, syndrome X3 cardiovascular disease, atherosclerosis, kidney disease, ketoacidosis, thrombotic disorders, nephropathy, diabetic neuropathy, diabetic retinopathy, sexual dysfunction, dermatopathy, dyspepsia, hypoglycemia, cancer or edema.

In another aspect, the invention provides a therapeutic composition that includes a compound of formula (I) and (a) other therapeutic agent(s) such as those described herein, for example metformin and/or a thiazolidinedione and/or a glinide and/or a DPP-IV inhibitor (e.g. linagliptin) and/or a SGLT2-inhibitor (e.g. dapagliflozin or 1-chloro-4-(β-D-glucopyranos-1-yl)-2-{4-[*(S)*-tetrahydrofuran-3-yloxy]-benzyl}-benzene) and/or a PPAR agonist (e.g. pioglitazone) and/or a GLP-1 receptor agonist (i.e. a GLP-1 analogue or mimetic (e.g. glutides)) and/or insulin or an insulin analogue and/or a 11β-HSD1 inhibitor and/or a glucokinase activator and/or a GPR119 agonist, as a combined preparation for simultaneous, separate, or sequential use in the treatment of a disease or condition mediated by GPR40. In some such embodiments, the disease or condition is type II diabetes. In some embodiments, the compound of formula (I) and the second therapeutic agent are provided as a single composition, whereas in other embodiments they are provided separately as parts of a kit.

### Detailed Description of the Invention

The present invention provides in its broadest/first embodiment E-0 a compound of formula (I): 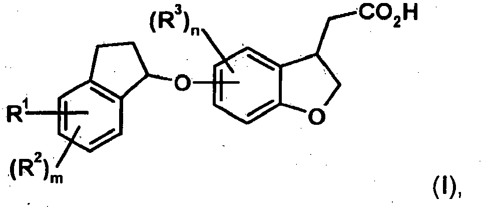 wherein:
- R¹: is selected from the group consisting of H, F, Cl, Br, I,
C₁₋₈-alkyl, C₂₋₆-alkenyl, C₂₋₆-alkynyl, C₃₋₁₂-cycloalkyl, C₃₋₁₂-cycloalkyl-C₁₋₄-alkyl, C₅₋₁₂-bicycloalkyl-, C₅₋₁₂-bicycloalkyl-C₁₋₆-alkyl-, C₅₋₆-cycloalkenyl, C₅₋₆-cycloalkenyl-C₁₋₄-alkyl, C₁₋₈-alkyloxy, C₃₋₆-cycloalkyl-oxy, and C₃₋₆-cycloalkyl-C₁₋₄-alkyloxy, wherein any of these groups optionally and independently is substituted with 1 to 3 fluorine atoms and/or 1 to 3 R⁴ groups,
C₃₋₁₂-heterocyclyl, C₃₋₁₂-heterocyclyl-C₁₋₄-alkyl, bicycloheterocyclyl-, bicycloheterocyclyl-C₁₋₆-alkyl- heterocyclyl-C₁₋₄-alkyl-oxy, and C₃₋₁₂-heterocyclyloxy, wherein any of these groups optionally and independently is substituted with 1 to 3 R⁴ groups,
aryl, aryl-C₁₋₄-alkyl, aryl-C₁₋₄-alkyl-oxy, aryloxy, heteroaryl, heteroaryl-C₁₋₄-alkyl, heteroaryl-C₁₋₄-alkyl-oxy, and heteroaryloxy, wherein any of these groups optionally and independently is substituted with 1 to 5 R⁵ groups,
C₁₋₄-alkyl-sulfanyl, C₁₋₄-alkyl-sulfinyl, and C₁₋₄-alkyl-sulfonyl, wherein any of these groups optionally is substituted with 1 to 3 fluorine atoms, and
cyano, nitro, amino, C₁₋₄-alkylamino, and di-(C₁₋₄-alkyl)-amino;
wherein the aforementioned heterocyclyl groups and submoieties may be partially unsaturated and comprise 1 to 3 heteroatoms or groups selected from N, NR^{N}, O and S, with the proviso that only up to two of the heteroatoms are O and S and no O-O, S=S, and S-O bond is formed, while a methylene group bound to a heteroatom may be replaced by a carbonyl group, whilst the heterocyclyl groups are bound to the respective residue via a carbon or nitrogen atom; R^{N} denotes H, C₁₋₄-alkyl-, C₁₋₄-alkyl-C(O)- or C₁₋₄-alkyl-O-C(O)-,
wherein the aforementioned bicycloheterocyclyl groups and submoieties may be partially unsaturated and comprise 6 to 12 ring members and 1 to 3 heteroatoms or groups selected from N, NR^{N}, O and S, with the proviso that only up to two of the heteroatoms are O and S and no O-O, S-S, and S-O
bond is formed, while a methylene group bound to a heteroatom may be replaced by a carbonyl group, whilst the heterocyclyl groups are bound to the respective residue via a carbon or nitrogen atom; R^{N} denotes H, C₁₋₄-alkyl-, C₁₋₄-alkyl-C(O)- or C₁₋₄-alkyl-O-C(O)-,
wherein the aforementioned bicycloalkyl and bicycloheterocyclyl groups and submoieties comprise fused, bridged and spiro ring systems,
wherein the aforementioned aryl groups and submoieties comprise 6 to 10 carbon atoms, wherein in bicyclic aromatic groups the ring not attached to the respective residue may be partially saturated, and
wherein the aforementioned heteroaryl groups and submoieties consist of 5 to 14 atoms containing 1 to 3 heteroatoms selected from N, O or S(O)ᵣ, wherein r = 0, 1 or 2, wherein in polycyclic heteroaromatic groups the rings not attached to the respective residue may be partially or fully saturated, while at least one aromatic ring includes one or more hetereoatoms,
- R²: is selected from the group consisting of F, Cl, Br, I, cyano, C₁₋₄-alkyl, C₃₋₆-cycloalkyl and C₁₋₄-alkyloxy, wherein any alkyl and cycloalkyl group or submoiety is optionally substituted with 1 to 3 fluorine atoms,
- R³: is selected from the group consisting of F, Cl, Br, I, C₁₋₄-alkyl and C₁₋₄-alkyloxy, wherein any alkyl group or submoiety is optionally substituted with 1 to 3 fluorine atoms,
- R⁴: is selected from the group consisting of F, Cl, Br, I, cyano, C₁₋₄-alkyl, hydroxy, hydroxy-C₁₋₄-alkyl, C₁₋₄-alkyl-oxy, C₁₋₄-alkyloxy-C₁₋₄-alkyl, C₁₋₄-alkyl-sulfanyl, C₁₋₄-alkyl-sulfinyl, C₁₋₄-alkyl-sulfonyl, C₃₋₆-cycloalkyl-, C₃₋₆-cycloalkyl-oxy-, wherein any alkyl and cycloalkyl group or submoiety is optionally substituted with 1 to 3 fluorine atoms, and
- R⁵: is selected from the group consisting of F, Cl, Br, I, cyano, nitro, amino, C₁₋₄-alkyl-amino, di-(C₁₋₄-alkyl)-amino, C₁₋₄-alkyl, C₂₋₄-alkenyl, C₂₋₄-alkynyl, hydroxy, hydroxy-C₁₋₄-alkyl, C₁₋₄-alkyl-oxy, C₁₋₄-alkyloxy-C₁₋₄-alkyl, C₁₋₄-alkyl-sulfanyl, C₁₋₄-alkyl-sulfinyl, C₁₋₄-alkyl-sulfonyl, C₃₋₆-cycloalkyl-, C₃₋₆-cycloalkyl-oxy-, wherein any alkyl and cycloalkyl group or submoiety is optionally substituted with 1 to 5 fluorine atoms, preferably with 1 to 3 fluorine atoms,
- m: is selected from the numbers 0, 1, 2 and 3, preferably from the numbers 0, 1 and 2, or, more preferably, from the numbers 0 and 1, whereas the number 0 is most preferred and
- n: is selected from the numbers 0, 1, 2 and 3, preferably from the numbers 0 and 1, whereas the number 0 is most preferred,
wherein in any definition mentioned hereinbefore and if not specified otherwise, any alkyl moiety mentioned hereinbefore or hereinafter may be straight-chained or branched,
the tautomers, stereoisomers, hydrates, and the physiologically acceptable salts thereof with inorganic or organic acids or bases, or the combinations thereof.

### Terms and definitions

Terms not specifically defined herein should be given the meanings that would be given to them by one of skill in the art in light of the disclosure and the context. As used in the specification, however, unless specified to the contrary the following terms have the meaning indicated and the following conventions are adhered to.
In the groups, radicals, or moieties defined below, the number of carbon atoms is often specified preceding the group, for example, C₁₋₆-alkyl means an alkyl group or radical having 1 to 6 carbon atoms. In general, for groups comprising two or more subgroups, the last named subgroup is the radical attachment point; for example, the substituent "aryl-C₁₋₃-alkyl-" means an aryl group which is bound to a C₁₋₃-alkyl group, the latter of which is bound to the core or to the group to which the substituent is attached.
In general, the attachment site of a given residue to another group shall be variable, i.e. any capable atom, bearing hydrogens to be replaced, within this residue may be the linking spot to the group being attached, unless otherwise indicated.

Unless specifically indicated, throughout the specification and the appended claims, a given chemical formula or name shall encompass all conceivable constitutional isomers and stereoisomers, including tautomers, enantiomers, diastereomers, cis/trans isomers, E/Z isomers, etc., and mixtures thereof, for example, 1:1 mixtures of enantiomers (termed racemates), mixtures of different proportions of separate enantiomers, mixtures of diastereomers, or mixtures of any of the foregoing forms where such isomers exist, as well as salts, including pharmaceutically acceptable salts thereof and solvates thereof such as, for instance, hydrates, including solvates of the free compounds or solvates of a salt of the compound.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, and commensurate with a reasonable benefit/risk ratio.

The term "partially unsaturated" as used herein means that in the designated group or moiety 1, 2, or more, preferably 1 or 2, double bonds are present. Preferably, as used herein, the term "partially unsaturated" does not cover fully unsaturated groups or moieties.

The term halogen denotes an atom selected from the group consisting of F, Cl, Br, and I.

The term C₁₋ₙ-alkyl, wherein n may have a value of 1 to 18, denotes a saturated, branched or unbranched hydrocarbon group with 1 to n C atoms. Examples of such groups include methyl, ethyl, n-propyl, iso-propyl, butyl, iso-butyl, sec-butyl, tert-butyl, n-pentyl, iso-pentyl, neo-pentyl, tert-pentyl, n-hexyl, iso-hexyl, etc..

The term "C₁₋ₙ-alkylene" wherein n is an integer of 1 to n, either alone or in combination with another radical, denotes an acyclic, straight or branched chain divalent alkyl radical containing 1 to n carbon atoms. For example, the term C₁₋₄-alkylene includes -(CH₂)-, -(CH₂-CH₂)-, -(CH(CH₃))-, -(CH₂-CH₂-CH₂)-, -(C(CH₃)₂)-, - (CH(CH₂CH₃))-, -(CH(CH₃)-CH₂)-, -(CH₂-CH(CH₃))₋, -(CH₂-CH₂-CH₂-CH₂)-, -(CH₂-CH₂-CH(CH₃))-, -(CH(CH₃)-CH₂-CH₂)-, -(CH₂-CH(CH₃)-CH₂)-, -(CH₂-C(CH₃)₂)-,-(C(CH₃)₂-CH₂)-, -(CH(CH₃)-CH(CH₃))-, -(CH₂-CH(CH₂CH₃))-, -(CH(CH₂CH₃)-CH₂)-,-(CH(CH₂CH₂CH₃))-, -(CHCH(CH₃)₂)-, and -C(CH₃)(CH₂CH₃)-.

The term C₂₋ₙ-alkenyl, wherein n has a value of 3 to 10, denotes a branched or unbranched hydrocarbon group with 2 to n C atoms and a C=C double bond. Examples of such groups include ethenyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, etc..

The term C₂₋ₙ-alkynyl, wherein n has a value of 3 to 10, denotes a branched or unbranched hydrocarbon group with 2 to n C atoms and a C=C triple bond. Examples of such groups include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, etc.

The term C₁₋ₙ-alkoxy denotes a C₁₋ₙ-alkyl-O group, wherein C₁₋ₙ-alkyl is as hereinbefore defined. Examples of such groups include methoxy, ethoxy, n-propoxy, iso-propoxy, n-butoxy, iso-butoxy, sec-butoxy, tert-butoxy, n-pentoxy, iso-pentoxy, neo-pentoxy, tert-pentoxy, n-hexoxy, iso-hexoxy, etc..

The term C₁₋ₙ-alkylcarbonyl denotes a C₁₋ₙ-alkyl-C(=O) group, wherein C₁₋ₙ-alkyl is as hereinbefore defined. Examples of such groups include methylcarbonyl, ethylcarbonyl, n-propylcarbonyl, iso-propylcarbonyl, n-butylcarbonyl, isobutylcarbonyl, sec-butylcarbonyl, tert-butylcarbonyl, n-pentylcarbonyl, isopentylcarbonyl, neo-pentylcarbonyl, tert-pentyl-carbonyl, n-hexylcarbonyl, isohexylcarbonyl, etc.

The term C₃₋ₙ-cycloalkyl denotes a saturated mono-, bi-, tri- or spirocarbocyclic group with 3 to n C atoms. Examples of such groups include cyclopropyl, cyclobutyl, cyclopentyl, cyclohexyl, cycloheptyl, cyclooctyl, cyclononyl, cyclododecyl, bicyclo[3.2.1]octyl, spiro[4.5]decyl, norpinyl, norbonyl, norcaryl, adamantyl, etc.. Preferably the term C₃₋₇-cycloalkyl denotes saturated monocyclic groups.

The term C₅₋ₙ-cycloalkenyl denotes a C₅₋ₙ-cycloalkyl group which is as hereinbefore defined and additionally has at least one C=C double bond.

The term C₃₋ₙ-cycloalkylcarbonyl denotes a C₃₋ₙ-cycloalkyl-C(=O) group wherein C₃₋ₙ-cyclo-alkyl is as hereinbefore defined.

The term C₃₋ₙ-heterocycloalkyl denotes a saturated mono-, bi-, tri- or spirocarbocyclic group, which is as hereinbefore defined, with 3-m to n-m C atoms, wherein m carbon atoms are replaced with m heteroatoms independently selected from N, NR^{N}, O, S, SO, and SO₂. Examples of such groups include aziridinyl, oxiranyl, azetidinyl, oxetanyl, pyrrolidinyl, tetrahydrofuranyl, tetrahydrothiophenyl, piperidinyl, tetrahydropyranyl, tetrahydrothiopyranyl, piperazinyl, morpholinyl, 1,3-dioxanyl, 1,4-dioxanyl, thiomorpholinyl, azepanyl, oxepanyl, thiepanyl, 1-aza-bicyclo[2.2.2]octane, 1,4-diaza-bicyclo[2.2.2]octane, etc.. Preferably, the term heterocycloalkyl denotes saturated monocyclic C₅-₆-cycloalkyl groups wherein one or two carbon atoms are replaced with N and/or O.

If a C₃₋ₙ-heterocycloalkyl group is optionally unsaturated the designated group has 1, 2, or more, preferably 1 or 2, double bonds, but does not form a heteroaromatic system. If in the optionally unsaturated C₃₋ₙ-heterocyclyl group a methylene group next to a heteroatom is optionally replaced by a carbonyl group, tautomeric unsaturated lactams of hydroxy substituted heteroaromatic groups can be formed and are included in the definition; examples are 1H-pyridin-2-one and 1 H-pyrimidin-2-one.

The term tri-(C₁₋₄-alkyl)silyl comprises silyl groups which have identical or two or three different alkyl groups.

The term di-(C₁₋₃-alkyl)amino comprises amino groups which have two identical or different alkyl groups.

If groups or residues are optionally substituted, this applies to any form of the group or residue. For instance, if an alkyl group is optionally mono- or polyfluorinated this comprises also alkyl residues which are part of larger groups, e.g. alkyloxy, alkylcarbonyl, alkoxyalkyl, etc., or if a (het)aryl group is optionally mono- or polysubstituted with a certain substituent or a set of substituents this also includes (het)aryl groups which are part of larger groups, e.g. (het)aryl-C₁₋ₙ-alkyl, (het)aryloxy, (het)aryloxy-C₁₋ₙ-alkyl, (het)aryl-C₁₋ₙ-alkyloxy, etc.. Accordingly, in cases where e.g. R¹ has, for example, the meaning (het)aryloxy, while (het)aryl residues are optionally mono- or polyfluorinated and (het)aryl denotes inter alia phenyl, the meanings mono-, di-, tri-, tetra-, and pentafluorophenoxy are also comprised. The same applies to groups or residues in which a part of the group or residue is replaced by another group, e.g. a CH₂ group is optionally replaced by O, S, NR^{N}, CO, or SO₂. For instance, a residue having inter alia the meaning hydroxy-C₁₋₃-alkyl in which a CH₂ group is optionally replaced by CO (= carbonyl), this also comprises carboxy, carboxymethyl, hydroxymethylcarbonyl, 1-hydroxy-2-oxo-ethyl, carboxyethyl, 2-carboxyethyl, 1-carboxyethyl, hydroxymethylcarbonylmethyl, 1-hydroxy-2-oxo-propyl, hydroxyethylcarbonyl, (2-hydroxyethyl)carbonyl, hydroxy-3-oxo-propyl, 1-hydroxy-3-oxo-propyl, 2-hydroxy-3-oxo-propyl, (1-hydroxyethyl)-carbonyl, 2-hydroxy-1-oxo-prop-2-yl, hydroxy-2-oxo-prop-2-yl and 3-hydroxy-1-oxo-prop-2-yl. Analogously, a definition such as C₁₋ₙ-alkyl wherein one or more CH₂ groups are optionally replaced by, for example, carbonyl and which is optionally substituted with e.g. hydroxy or amino also comprises explicit residues having no CH and/or CH₂ group, e.g. carboxy and aminocarbonyl.

All atoms/elements described herein, including atoms that are part of a group, comprise all stable isotopic forms of the respective element. For instance, whenever hydrogen is mentioned, either explicitly or as part of a group such as methyl, this includes hydrogen and deuterium as stable isotopic forms of the element hydrogen.

The term "aryl" as used herein, either alone or in combination (e.g. as a sub-moiety) with another substituent, if not otherwise specified means either an aromatic monocyclic system or aromatic multicyclic system containing carbon atoms, for example, a phenyl or a naphthyl group. Any of the aryl groups mentioned hereinbefore is optionally substituted, if not otherwise specified.

The term "heteroaryl" as used herein, either alone or in combination (e.g. as a sub-moiety) with another substituent, if not otherwise specified, denotes five- or six-membered heterocyclic aromatic groups or 8-10 membered, bicyclic heteroaromatic rings which contain one, two or three heteroatoms, selected from among O, S, S=O, S(=O)₂, and N. The ring may be linked to the rest of the molecule through a carbon atom or, if present, through a nitrogen atom. Any of the heteroaryl groups mentioned hereinbefore is optionally substituted, if not otherwise specified, including substitution at carbon and nitrogen atoms. The following are examples of five- or six-membered heterocyclic aromatic groups:

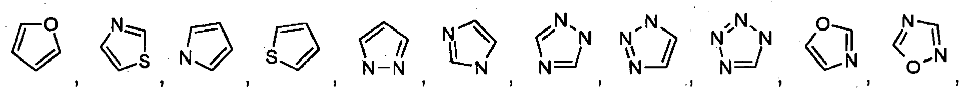

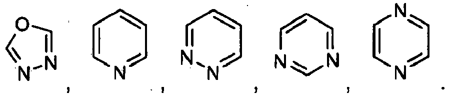

Examples of 8-10 membered bicyclic heteroaryl rings include pyrrolizine, indole, indolizine, isoindole, indazole, purine, quinoline, isoquinoline, benzimidazole, benzofuran, benzopyrane, benzothiazole, benzoisothiazole, pyridopyrimidine, pteridine, pyrimidopyrimidine.

The term "(het)aryl" as used herein, either alone or in combination (e.g. as a sub-moiety) with another substituent, if not otherwise specified means aryl and heteroaryl, wherein aryl and heteroaryl are defined as hereinbefore.

The term "substituted" as used herein means that any one or more hydrogens on the designated atom is replaced with a selection from the indicated group, provided that the designated atom's viable valence number is not exceeded, and that the substitution results in a stable compound. More specifically, the expression "substituted" or "optionally substituted" as used herein, if not otherwise specified, means substitution with a group selected from the indicated substituents or, if not otherwise specified, with 1, 2 or 3 substituents attached to carbon atoms selected from the group consisting of halogen atoms (fluorine, chlorine, bromine or iodine atoms), C₁₋₆-alkyl, C₂₋₆-alkenyl, cyclo-C₃₋₈-alkyl, cyclo-C₃₋₇-alkenyl, cyano, hydroxy, hydroxy-C₁₋₆-alkyl, hydroxy-C₃₋₆-alkenyl, C₁₋₆-alkoxy, C₁₋₆-alkoxy-C₁₋₆-alkyl, tetrahydrofuranyl, tetrahydropyranyl, pyrrolidino, piperidino, thiohydroxy, C₁₋₆-alkylthio, amino, C₁₋₆-alkyl-amino, C₃₋₆-alkenyl-amino, di-(C₁₋₆-alkyl)-amino, amino-C₁₋₆-alkyl, C₁₋₃-alkyl-amino-C₁₋₆-alkyl, di-(C₁₋₃-alkyl)-amino-C₁₋₆-alkyl, hydroxycarbonyl, phenyl, phenyl-C₁₋₃-alkyl, phenyloxy, phenyl-C₁₋₃-alkoxy, phenyloxy-C₁₋₃-alkyl, phenylcarbonyl, pyridyl, thiazolyl; pyridyl-carbonyl, C₁₋₆-alkyl-carbonyl, C₁₋₆-alkoxy-carbonyl, C₃₋₆-alkenoxy-carbonyl, aminocarbonyl, C₁₋₆-alkyl-aminocarbonyl, di-(C₁₋₆-alkyl)-aminocarbonyl, di-(C₃₋₆-alkenyl)-aminocarbonyl, C₁₋₆-alkyl-carbonylamino, C₁₋₆-alkyl-sulphonyl, C₁₋₆-alkyl-sulphinyl, C₁₋₆-alkyl-sulphonylamino, aminosulphonyl, C₁₋₆-alkylaminosulphonyl and di-(C₁₋₆-alkyl)-aminosulphonyl, while the substituents may be identical or different and wherein any alkyl groups or alkyl sub-moieties optionally are partially or fully fluorinated, e.g. a CH₃- substituent or methyl sub-moiety within the substituents mentioned herein is meant to include the corresponding fluoro-analogs such as the CFH₂-, CF₂H- and CF₃- group,
and wherein any phenyl, pyridyl and thiazolyl groups or phenyl-, pyridyl and thiazolyl-submoieties optionally are substituted with 1 or 2 substituents independently of each other selected from fluorine, chlorine, bromine, iodine, C₁₋₃-alkyl, C₁₋₃-alkoxy, amino, C₁₋₃-alkyl-amino, di-(C₁₋₃-alkyl)-amino C₁₋₃-alkylcarbonyl-amino, C₁₋₃-alkylcarbonyl-C₁₋₃-alkyl-amino, cyano or hydroxy,
and with substituents attached to a nitrogen atom selected from the group consisting of
C₁₋₆-alkyl, C₃₋₆-alkenyl, cyclo-C₃₋₇-alkyl, cyclo-C₃₋₇-alkyl-C₁₋₆-alkyl, pyrrolidino, piperidino, morpholino, tetrahydrofuranyl, tetrahydropyranyl, cyano, hydroxy, hydroxy-C₁₋₆-alkyl, C₁₋₆-alkoxy-C₁₋₆-alkyl, amino-C₁₋₆-alkyl, C₁₋₃-alkyl-amino-C₁₋₆-alkyl, di-(C₁₋₃-alkyl)-amino-C₁₋₆-alkyl, phenyl, phenyl-C₁₋₆-alkyl, phenylcarbonyl, pyridyl, pyridylcarbonyl, C₁₋₆-alkyl-carbonyl, C₁₋₆-alkoxy-carbonyl, aminocarbonyl, C₁₋₆-alkyl-aminocarbonyl, di-(C₁₋₆-alkyl)-aminocarbonyl, C₁₋₆-alkyl-sulphonyl, C₁₋₆-alkyl-sulphinyl, aminosulphonyl, C₁₋₆-alkylaminosulphonyl and di-(C₁₋₆-alkyl)-aminosulphonyl groups, while the substituents may be identical or different and wherein any alkyl groups or alkyl sub-moieties optionally are partially or fully fluorinated, e.g. a CH₃- substituent or methyl sub-moiety within the substituents mentioned herein is meant to include the corresponding fluoro-analogs such as the CFH₂-, CF₂H- and CF₃- group,
and wherein any of the di-(C₁₋₃-alkyl)-amino or di-(C₁₋₆-alkyl)-amino moieties may form optionally with the nitrogen atom a 4 to 8 membered ring system,
and wherein any phenyl and pyridyl groups or phenyl- and pyridyl-submoieties optionally are substituted with 1 or 2 substituents independently of each other selected from fluorine, chlorine, bromine, iodine, C₁₋₃-alkyl, C₁₋₃-alkoxy, amino, C₁₋₃-alkyl-amino, C₁₋₃-alkylcarbonyl-amino, cyano or hydroxy.

The expressions "prevention", "prophylaxis", "prophylactic treatment" or "preventive treatment" used herein should be understood synonymous and in the sense that the risk to develop a condition mentioned hereinbefore is reduced, especially in a patient having elevated risk for said conditions or a corresponding anamnesis, e.g. elevated risk of developing metabolic disorder such as diabetes or obesity or another disorder mentioned herein. Thus the expression "prevention of a disease" as used herein means the management and care of an individual at risk of developing the disease prior to the clinical onset of the disease. The purpose of prevention is to combat the development of the disease, condition or disorder, and includes the administration of the active compounds to prevent or delay the onset of the symptoms or complications and to prevent or delay the development of related diseases, conditions or disorders. Success of said preventive treatment is reflected statistically by reduced incidence of said condition within a patient population at risk for this condition in comparison to an equivalent patient population without preventive treatment.

The expression "treatment" or "therapy" means therapeutic treatment of patients having already developed one or more of said conditions in manifest, acute or chronic form, including symptomatic treatment in order to relieve symptoms of the specific indication or causal treatment in order to reverse or partially reverse the condition or to delay the progression of the indication as far as this may be possible, depending on the condition and the severity thereof. Thus the expression "treatment of a disease" as used herein means the management and care of a patient having developed the disease, condition or disorder. The purpose of treatment is to combat the disease, condition or disorder. Treatment includes the administration of the active compounds to eliminate or control the disease, condition or disorder as well as to alleviate the symptoms or complications associated with the disease, condition or disorder.

As used herein the term "metabolite" refers to (i) a product of metabolism, including intermediate and products, (ii) any substance involved in metabolism (either as a product of metabolism or as necessary for metabolism), or (iii) any substance produced or used during metabolism. In particular it refers to the end product that remains after metabolism.

As used herein the term "prodrug" refers to (i) an inactive form of a drug that exerts its effects after metabolic processes within the body converting it to a usable or active form, or (ii) a substance that gives rise to a pharmacologically active metabolite, although not itself active (i.e. an inactive precursor).

The terms "prodrug" or "prodrug derivative" mean a covalently-bonded derivative, carrier or precursor of the parent compound or active drug substance which undergoes at least some biotransformation prior to exhibiting its pharmacological effect(s). Such prodrugs either have metabolically cleavable or otherwise convertible groups and are rapidly transformed in vivo to yield the parent compound, for example, by hydrolysis in blood or by activation via oxidation as in case of thioether groups. Most common prodrugs include esters and amide analogs of the parent compounds. The prodrug is formulated with the objectives of improved chemical stability, improved patient acceptance and compliance, improved bioavailability, prolonged duration of action, improved organ selectivity, improved formulation (e.g., increased hydrosolubility), and/or decreased side effects (e.g., toxicity). In general, prodrugs themselves have weak or no biological activity and are stable under ordinary conditions. Prodrugs can be readily prepared from the parent compounds using methods known in the art, such as those described in A Textbook of Drug Design and Development, Krogsgaard-Larsen and H. Bundgaard (eds.), Gordon & Breach, 1991, particularly Chapter 5: "Design and Applications of Prodrugs"; Design of Prodrugs, H. Bundgaard (ed.), Elsevier, 1985; Prodrugs: Topical and Ocular Drug Delivery, K.B. Sloan (ed.), Marcel Dekker, 1998; Methods in Enzymology, K. Widder et al. (eds.), Vol. 42, Academic Press, 1985, particularly pp. 309-396; Burger's Medicinal Chemistry and Drug Discovery, 5th Ed., M. Wolff (ed.), John Wiley & Sons, 1995, particularly Vol. 1 and pp. 172-178 and pp. 949-982; Pro-Drugs as Novel Delivery Systems, T. Higuchi and V. Stella (eds.), Am. Chem. Soc., 1975; Bioreversible Carriers in Drug Design, E.B. Roche (ed.), Elsevier, 1987.

The term "pharmaceutically acceptable prodrug" as used herein means a prodrug of a compound of the invention which is, within the scope of sound medical judgment, suitable for use in contact with the tissues of humans and lower animals without undue toxicity, irritation, allergic response, and the like, commensurate with a reasonable benefit/risk ratio, and effective for their intended use, as well as the zwitterionic forms, where possible.

### Preferred embodiments of the invention

Further preferred embodiments of the invention are characterized by the following definitions:
***a)*** Definitions ***(aⁱ)*** for R¹ as alternative embodiments ***(a¹)*** to ***(a⁵)*:**
   ***(a¹)***: According to a first preferred embodiment, R¹ is defined as mentioned hereinbefore under the broadest/first embodiment of the invention **E-0.**
   ***(a²)*:** According to a second preferred embodiment
      - R¹: is selected from the group consisting of H, F, Cl, Br,
      C₁₋₆-alkyl, C₅₋₆-alkenyl, C₃₋₁₀-cycloalkyl, C₃₋₁₀-cycloalkyl-C₁₋₄-alkyl, C₅₋₆-cycloalkenyl, C₁₋₆-alkyloxy, C₃₋₆-cycloalkyl-oxy, and C₃₋₆-cycloalkyl-C₁₋₄-alkyloxy, wherein any of these groups optionally and independently is substituted with 1 to 3 fluorine atoms and/or 1 to 3 R⁴ groups,
      C₅₋₁₀-heterocyclyl, C₅₋₁₀-heterocyclyl-C₁₋₄-alkyl, bicycloheterocyclyl-, bicycloheterocyclyl-C₁₋₄-alkyl-, C₅₋₁₀-heterocyclyl-C₁₋₄-alkyl-oxy, and C₅₋₁₀-heterocyclyloxy, wherein any of these groups optionally and independently is substituted with 1 to 3 R⁴ groups,
      phenyl, phenyl-C₁₋₄-alkyl, phenyl-C₁₋₄-alkyl-oxy, phenyloxy, heteroaryl, heteroaryl-C₁₋₄-alkyl, heteroaryl-C₁₋₄-alkyl-oxy, and heteroaryloxy, wherein any of these groups optionally and independently is substituted with 1 to 3 R⁵ groups,
      C₁₋₂-alkyl-sulfanyl, optionally substituted with 1 to 3 fluorine atoms, and cyano,
      wherein the aforementioned heterocyclyl groups and submoieties may be partially unsaturated and comprise 1 to 3 heteroatoms or groups selected from N, NR^{N}, O and S, with the proviso that only up to two of the heteroatoms are O and S and no O-O, S-S; and S-O bond is formed, while a methylene group bound to a heteroatom may be replaced by a carbonyl group, whilst the heterocyclyl groups are bound to the respective residue via a carbon or nitrogen atom; R^{N} denotes H, C₁₋₄-alkyl-, C₁₋₄-alkyl-C(O)- or C₁₋₄-alkyl-O-C(O)-,
      wherein the aforementioned bicycloheterocyclyl groups and submoieties may be partially unsaturated and comprise 6 to 10 ring members and 1 to 3 heteroatoms or groups selected from N, NR^{N}, O and S, with the proviso that only up to two of the heteroatoms are O and S and no O-O, S-S, and S-O bond is formed, while a methylene group bound to a heteroatom may be replaced by a carbonyl group, whilst the heterocyclyl groups are bound to the respective residue via a carbon or nitrogen atom; R^{N} denotes H, C₁₋₄-alkyl-, C₁₋₄-alkyl-C(O)- or C₁₋₄-alkyl-O-C(O)-,
      wherein the aforementioned bicycloalkyl and bicycloheterocyclyl groups and submoieties comprise fused and bridged ring systems,
      wherein the aforementioned heteroaryl groups and submoieties consist of 5 to 10 atoms containing 1 to 3 heteroatoms selected from N, O or S(O)ᵣ, wherein r = 0, 1 or 2, wherein in polycyclic heteroaromatic groups the ring or rings not attached to the respective residue may be partially or fully saturated, while at least one aromatic ring includes one or more hetereoatoms.
   ***(a³):*** According to a third preferred embodiment
      - R¹: is selected from the group consisting of H, F, Cl, Br,
      C₁₋₆-alkyl, C₅₋₆-alkenyl, C₃₋₆-cycloalkyl, C₃₋₆-cycloalkyl-C₁₋₄-alkyl, C₅₋₆-cycloalkenyl, tetrahydropyranyl-C₁₋₂-alkyl, and C₁₋₆-alkyloxy, wherein any of these groups optionally and independently is substituted with 1 to 3 fluorine atoms and/or 1 to 3 R⁴ groups,
      oxetanyl, tetrahydrofuranyl, dihydropyranyl, tetrahydropyranyl, morpholinyl, tetrahydrofuran-3-yl-oxy, tetrahydropyran-3-yloxy, tetrahydropyran-4-yloxy, 8-oxa-spiro[4.5]decenyl, 3-oxa-spiro[5.5]undecenyl, 2-oxa-bicyclo[3.1.1]heptyl, 2-oxa-bicyclo[2.2.1]heptyl, 2-oxa-bicyclo[3.1.1]hept-4-yloxy, 2-oxa-bicyclo[3.1.1]hept-5-yloxy, 2-oxa-bicyclo[3.1.1]hept-6-yloxy, 2-oxa-bicyclo[2.2.1]hept-4-yloxy; 2-oxa-bicyclo[2.2.1]hept-5-yloxy, and 2-oxa-bicyclo[2.2.1]hept-6-yloxy, wherein any of these groups optionally and independently is substituted with 1 to 3 R⁴ groups,
      2-oxo-1,2-dihydro-pyridyl,, optionally substituted with 1 to 3 R⁴ groups,
      phenyl and heteroaryl, wherein any of these groups optionally and independently is substituted with 1 to 3 R⁵ groups,
      trifluoromethylsulfanyl and cyano,
      wherein the aforementioned heteroaryl groups and submoieties comprise 5-and 6-membered monocyclic ring systems containing 1 or 2 heteroatoms selected from N, NR^{N}, and O, wherein R^{N} denotes H and C₁₋₄-alkyl.
   ***(a⁴)*:** According to a fourth preferred embodiment
      - R¹: is selected from the group consisting of F, Cl, Br, CN,
      C₁₋₆-alkyl, C₅-alkenyl, C₃₋₆-cycloalkyl, C₃₋₆-cycloalkyl-C₁₋₄-alkyl, C₅₋₆-cycloalkenyl, , tetrahydropyranyl-C₁₋₂-alkyl, and C₁₋₆-alkyloxy, wherein any of these groups optionally and independently is substituted with 1 to 3 fluorine atoms and/or 1 to 3 R⁴ groups,
      oxetanyl, tetrahydrofuranyl, dihydropyranyl, tetrahydropyranyl, morpholinyl, 8-oxa-spiro[4.5]dec-1-enyl, 3-oxa-spiro[5.5]undec-7-enyl, and 2-oxa-bicyclo[3.1.1]heptyl, wherein any of these groups optionally and independently is substituted with 1 to 3 R⁴ groups,
      pyrazolyl, imidazolyl, isoxazolyl, phenyl, naphthyl, pyridyl, 2-oxo-1,2-dihydro-pyridyl, pyrimidyl, pyrazinyl, and isoquinolinyl, optionally substituted with 1 to 3 R⁵ groups.
   ***(a⁵)***: According to a fifth preferred embodiment
      - R¹: is selected from the group consisting of F, Cl, Br,
      C₁₋₆-alkyl, C₃₋₆-cycloalkyl, C₃₋₆-cycloalkyl-C₁₋₄-alkyl, cyclopentenyl and C₁₋₆-alkyloxy, wherein any of these groups optionally and independently is substituted with 1 to 3 fluorine atoms and/or 1 or 2 R⁴ groups,
      dihydropyranyl and tetrahydropyranyl.
   ***(a⁶)*:** According to a sixth preferred embodiment
      - R¹: is selected from the group consisting of F, Cl, Br, CN,
      C₁₋₆-alkyl, pentafluoroethyl, C₅-alkenyl, C₃₋₆-cycloalkyl, C₃₋₆-cycloalkyl-C₁₋₄-alkyl, C₅₋₆-cycloalkenyl, tetrahydropyranyl-C₁₋₂-alkyl, and C₁₋₆-alkyloxy, wherein any of these groups optionally and independently is substituted with 1 to 3 fluorine atoms and/or 1 to 3 groups independently selected from methyl, cyano, methoxy, and hydroxy,
      phenyl, optionally substituted with 1 to 3 groups independently selected from Cl, C₁₋₃-alkyl, trifluoromethyl, pentafluoroethyl, methoxy, and hydroxy,
      pyridyl, optionally substituted with 1 to 3 groups independently selected from F, methyl, trifluoromethyl, cyano, and methoxy,
      pyrazolyl, imidazolyl, isoxazolyl, pyrimidinyl, pyrazinyl, 2-oxo-1,2-dihydro-pyridyl, naphthyl, and isoquinolinyl, each of which is optionally substituted with 1 to 3 methyl groups,
      dihydropyranyl, morpholinyl,
      oxetanyl and tetrahydropyranyl, each of which is optionally substituted with 1 to 3 methyl groups,
      8-oxa-spiro[4.5]dec-1-enyl, and 3-oxa-spiro[5.5]undec-7-enyl.
***b)*** Definitions ***(bⁱ)*** for R² as alternative embodiments ***(b¹)*** to ***(b⁴)***:
   ***(b¹)***: According to a first preferred embodiment R² is defined as mentioned hereinbefore under the broadest/first embodiment of the invention **E-0.**
   ***(b²)***: According to a second preferred embodiment,
      - R²: is selected from the group consisting of F, Cl, Br, methyl, difluoromethyl, trifluoromethyl, cyclopropyl, methoxy, difluoromethoxy and trifluoromethoxy.
   ***(b³)***: According to a third preferred embodiment,
      - R²: is selected from the group consisting of F, Cl, methyl, difluoromethyl, trifluoromethyl, methoxy, difluoromethoxy and trifluoromethoxy.
   ***(b⁴)***: According to a fourth preferred embodiment,
      - R²: is selected from the group consisting of F, Cl, methyl and methoxy.
*c)* Definitions ***(cⁱ)*** for R³ as alternative embodiments ***(c¹)*** to ***(c³)***:
   ***(c¹):*** According to a first preferred embodiment R³ is defined as mentioned hereinbefore under the broadest/first embodiment of the invention **E-0.**
   ***(c²)***: According to a second preferred embodiment,
      - R³: is selected from the group consisting of F, Cl, Br, methyl, difluoromethyl, trifluoromethyl, methoxy, difluoromethoxy and trifluoromethoxy.
   ***(c³):*** According to a third preferred embodiment,
      - R³: is selected from the group consisting of F, methyl and methoxy.
*d)* Definitions ***(dⁱ)*** for R⁴ as alternative embodiments ***(d¹)*** to ***(d⁴)*:**
   ***(d¹):*** According to a first preferred embodiment R⁴ is defined as mentioned hereinbefore under the broadest/first embodiment of the invention **E-0.**
   ***(d²)***: According to a second preferred embodiment
      - R⁴: is selected from the group consisting of F, Cl, cyano, C₁₋₄-alkyl, hydroxy, hydroxy-C₁₋₄-alkyl, C₁₋₄-alkyl-oxy, C₁₋₄-alkyloxy-C₁₋₄-alkyl, C₃₋₆-cycloalkyl-, C₃₋₆-cycloalkyl-oxy-, wherein any of the aliphatic and cycloaliphatic groups or submoieties optionally are substituted by 1 to 3 fluorine atoms.
   ***(d³)***: According to a third preferred embodiment
      - R⁴: is selected from the group consisting of F, cyano, hydroxy, methyl, difluoromethyl, trifluoromethyl, methoxy, difluoromethoxy and trifluoromethoxy.
   ***(d⁴)*:** According to a fourth preferred embodiment
      - R⁴: is selected from the group consisting of F, cyano, methyl, hydroxy and methoxy.
*e)* Definitions ***eⁱ)*** for R⁵ as alternative embodiments ***(e¹)*** to ***(e³)*:**
   ***(e¹)***: According to a first preferred embodiment R⁵ is defined as mentioned hereinbefore under the broadest/first embodiment of the invention **E-0.**
   ***(e²)***: According to a second preferred embodiment,
      - R⁵: is selected from the group consisting of F, Cl, cyano, C₁₋₃-alkyl (preferably methyl), difluoromethyl, trifluoromethyl, pentafluoroethyl, hydroxy, methoxy, difluoromethoxy and trifluoromethoxy.
   ***(e³)***: According to a third preferred embodiment,
      - R⁵: is selected from the group consisting of F, Cl, cyano, C₁₋₃-alkyl (preferably methyl), trifluoromethyl, pentafluoroethyl, hydroxy, and methoxy.

Each **aⁱ, bⁱ, cⁱ, dⁱ, eⁱ** represents a characterised, individual embodiment for the corresponding substituent as described above. So given the above definitions, preferred individual embodiments of the first aspect of the invention are fully characterised by the term **(aⁱbⁱcⁱdⁱeⁱ)** if for each letter i in this term an individual figure is given. Indices i vary independently from each other. All individual embodiments described by the term in brackets with full permutation of indices i, referring to the above definitions, shall be comprised by the present invention.

The following table 1 shows such embodiments E-1 to E-20 of the invention that are considered specifically preferred:

**Table 1: Preferred embodiments E-1 to E-20 of the invention**

| | R¹ | R² | R³ | R⁴ | R⁵ | m | n |
|---|---|---|---|---|---|---|---|
| **E-1** | ***a¹*** | ***b¹*** | ***c¹*** | ***d¹*** | ***e¹*** | **0, 1, 2, 3** | **0, 1, 2, 3** |
| **E-2** | ***a²*** | ***b¹*** | ***c¹*** | ***d¹*** | ***e¹*** | **0, 1, 2, 3** | **0, 1, 2, 3** |
| **E-3** | ***a³*** | ***b¹*** | ***c¹*** | ***d¹*** | ***e¹*** | **0, 1** | **0, 1** |
| **E-4** | ***a²*** | ***b²*** | ***c²*** | ***d³*** | ***e²*** | **0,1,2** | **0, 1, 2** |
| **E-5** | ***a²*** | ***b²*** | **-*** | ***d³*** | ***e²*** | **1** | **0** |
| **E-6** | ***a³*** | ***b²*** | **-*** | ***d³*** | ***e²*** | **1** | **0** |
| **E-7** | ***a¹*** | **-*** | **-*** | ***d¹*** | ***e¹*** | **0** | **0** |
| **E-8** | ***a³*** | ***b³*** | ***c³*** | ***d⁴*** | ***e³*** | **1** | **1** |
| **E-9** | ***a³*** | **-*** | ***c³*** | ***d⁴*** | ***e³*** | **0** | **1** |
| **E-10** | ***a³*** | ***b³*** | **-*** | ***d⁴*** | ***e³*** | **1** | **0** |
| **E-11** | ***a¹*** | **-*** | **-*** | ***d²*** | ***e²*** | **0** | **0** |
| **E-12** | ***a²*** | **-*** | **-*** | ***d³*** | ***e²*** | **0** | **0** |
| **E-13** | ***a³*** | **-*** | **-*** | ***d³*** | ***e²*** | **0** | **0** |
| **E-14** | ***a¹*** | **-*** | **-*** | ***d⁴*** | ***e³*** | **0** | **0** |
| **E-15** | ***a⁴*** | **-*** | **-*** | ***d²*** | ***e²*** | **0** | **0** |
| **E-16** | ***a²*** | **-*** | **-*** | ***d⁴*** | ***e³*** | **0** | **0** |
| **E-17** | ***a³*** | **-*** | **-*** | ***d⁴*** | ***e³*** | **0** | **0** |
| **E-18** | ***a⁴*** | **-*** | **-*** | ***d³*** | ***e²*** | **0** | **0** |
| **E-19** | ***a⁴*** | **-*** | **-*** | ***d⁴*** | ***e³*** | **0** | **0** |
| **E-20** | ***a⁵*** | **-*** | **-*** | ***d⁴*** | **-*** | **0** | **0** |
| **E-21** | ***a⁶*** | **-*** | **-*** | **-*** | **-*** | **0** | **0** |

| | | | | | | | |
|---|---|---|---|---|---|---|---|
| -*means that the respective variable does not exist in the corresponding embodiment including the tautomers, the stereoisomers, the mixtures, and the salts thereof, in particular the pharmaceutically acceptable salts thereof. | | | | | | | |

The following preferred embodiments of compounds of the formula (I) are described using generic formulas (I.1) to (I.8), wherein any tautomers and stereoisomers (if not otherwise specified), solvates, hydrates and salts thereof; in particular the pharmaceutically acceptable salts thereof, are encompassed. 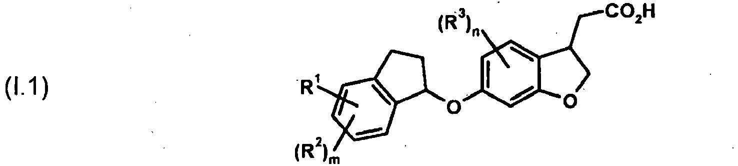 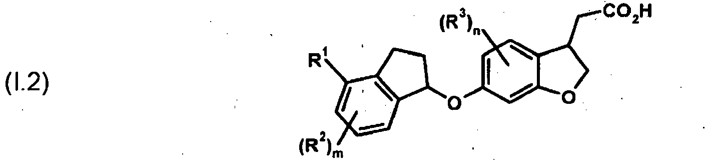 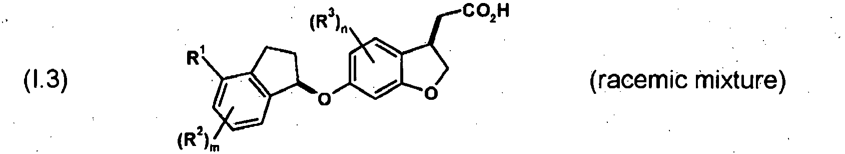 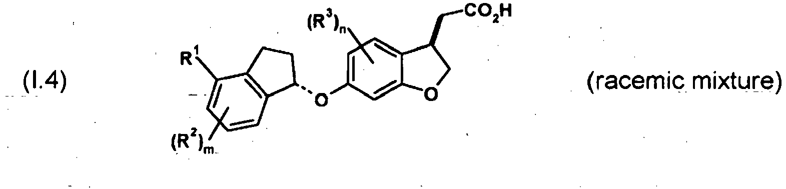 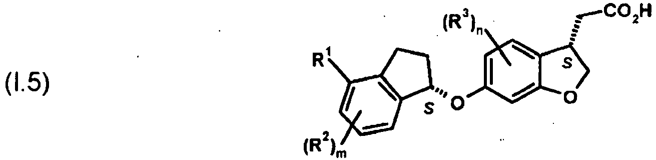 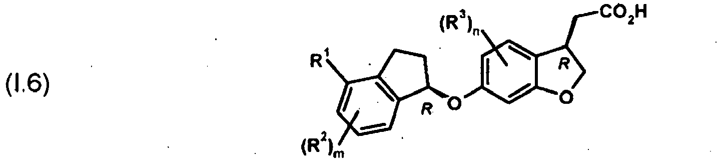 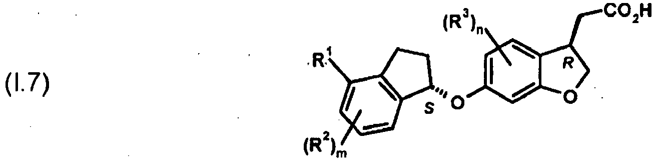 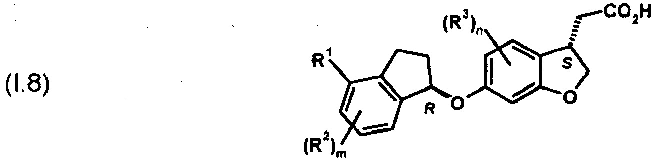 wherein in each of the above formulas (I.1) to (1.8), the groups R¹, R², R³, m, and n are defined as hereinbefore. Any preferred embodiment E-1 to E-20 as disclosed in table 1 applies to any of the generic formulas (I.1) to (1.8).

Particular preferred embodiments of the invention are described in the Examples.

The compounds according to the invention may be obtained using methods of synthesis known in principle. Preferably, the compounds are obtained by the following methods according to the invention which are described in more detail hereinafter.

The compounds of the invention (I) are preferably accessed from a precursor 1 that bears the carboxylic acid function in a protected or masked form as sketched in Scheme 1; R¹, R², R³, m, and n have the meanings as defined hereinbefore and hereinafter. Suited precursor groups for the carboxylic acid may be, e.g., a carboxylic ester, a carboxylic amide, cyano, an olefin, oxazole, or a thiazole All these groups have been transformed into the carboxylic acid function by different means which are described in the organic chemistry literature and are known to the one skilled in the art. The preferred precursor group is a C₁₋₄-alkyl or benzyl carboxylate group, each of which may be additionally mono- or polysubstituted with fluorine, methyl, and/or methoxy. These ester groups may be hydrolysed with an acid, such as hydrochloric acid or sulfuric acid, or an alkali metal hydroxide, such as lithium hydroxide, sodium hydroxide, or potassium hydroxide, to yield the carboxylic acid function; the hydrolysis is preferably conducted in aqueous solvents, such as water and tetrahydrofuran, 1,4-dioxane, alcohol, e.g. methanol, ethanol, and isopropanol, or dimethyl sulfoxide, at 0 to 120 °C. A tert-butyl ester is preferably cleaved under acidic conditions, e.g. trifluoroacetic acid or hydrochloric acid, in a solvent such as dichloromethane, 1,4-dioxane, isopropanol, or ethyl acetate. A benzyl ester is advantageously cleaved using hydrogen in the presence of a transition metal, preferably palladium on carbon. Benzyl esters bearing electron donating groups, such as methoxy groups, on the aromatic ring may also be removed under oxidative conditions; ceric ammonium nitrate (CAN) or 2,3-dichloro-5,6-dicyanoquinone (DDQ) are two commonly used reagents for this approach.

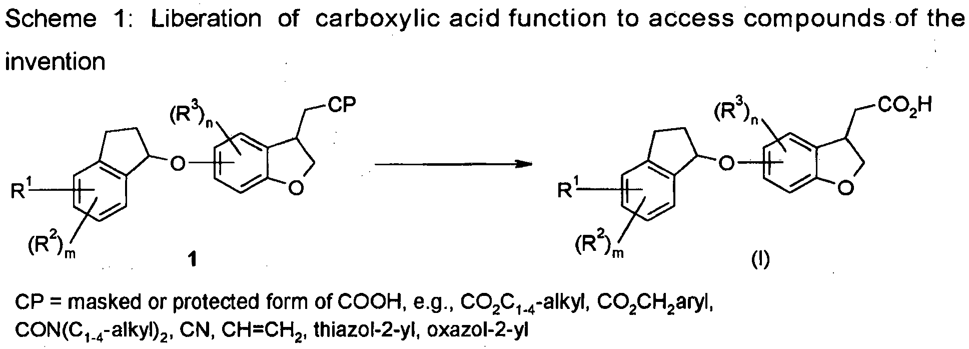

Compound 1, in turn, may be obtained from indane 2, which bears a leaving group, and indanol 3, which is decorated with the carboxylic acid precursor group (Scheme 2); R¹, R², R³, m, and n in Scheme 2 have the meanings as defined hereinbefore and hereinafter. The leaving group LG in **2** is replaced with the O in **3** via a nucleophilic substitution; suited LG may be Cl, Br, I, methylsulfonyloxy, phenylsulfonyloxy, p-tolylsulfonyloxy, and trifluoromethylsulfonyloxy. The reaction is usually carried out in the presence of a base, such as triethylamine, ethyldiisopropylamine, 1,8-diazabicyclo[5.4.0]undecene, carbonates, e.g. Li₂CO₃, Na₂CO₃, K₂CO₃, and Cs₂CO₃, hydroxides, e.g. LiOH, NaOH, and KOH, alcoholates, e.g. NaOMe, NaOEt, and KOtBu, hydrides, e.g. NaH and KH, amides, e.g. NaNH₂, KN(SiMe₃)₂, and LiN(iPr)₂, and oxides, e.g. CaO and Ag₂O. Additives, such as silver salts, e.g. AgNO₃, AgOSO₂CF₃, and Ag₂CO₃, crown ethers, e.g. 12-crown-4, 15-crown-5, and 18-crown-6, hexamethylphosphorus triamide (HMPT), and 1,3-dimethyl-3,4,5,6-dihydro-2-pyrimidinone (DMPU), may be beneficial or even essential for the reaction to proceed. Preferred solvents are dimethylsulfoxide, N,N-dimethylformamide, N,N-dimethylacetamide, N-methylpyrrolidinone, acetonitrile, acetone, 1,4-dioxane, tetrahydrofuran, alcohol, e.g. ethanol or isopropanol, water, or mixtures thereof, while not all solvents can be combined with each additive and base mentioned above. Suited reaction temperatures range from -20 to 140 °C.

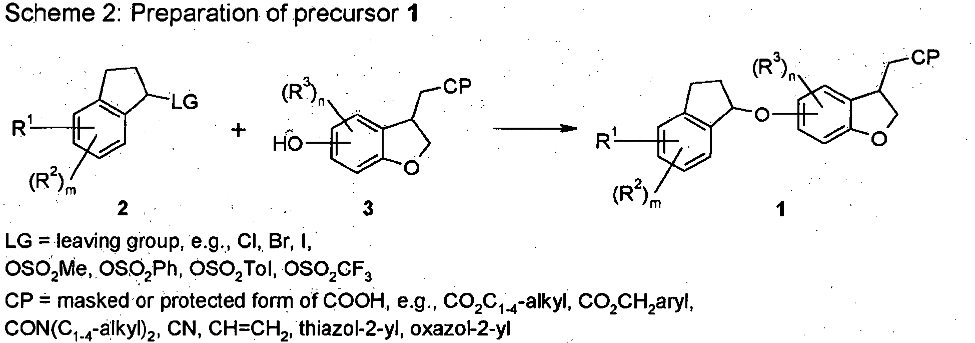

An alternative reaction to combine building blocks 2 and 3 is the Mitsunobu reaction or variations thereof (Scheme 3); R¹, R², R³, m, and n in Scheme 3 have the meanings as defined hereinbefore and hereinafter. The reaction is preferably conducted with a phosphine and an azodicarboxylic ester or amide in tetrahydrofuran, 1,4-dioxane, diethyl ether, toluene, benzene, dichloromethane, or mixtures thereof, at -30 to 100 °C. Phosphines often used are triphenylphosphine and tributylphosphine which are usually combined with dimethyl azodicarboxylate, diethyl azodicarboxylate, diisopropyl azodicarboxylate, di-(4-chlorobenzyl) azodicarboxylate, dibenzyl azodicarboxylate, di-tert-butyl azodicarboxylate, azodicarboxylic acid bis-(dimethylamide), azodicarboxylic acid dipiperidide, or azodicarboxylic acid dimorpholide.

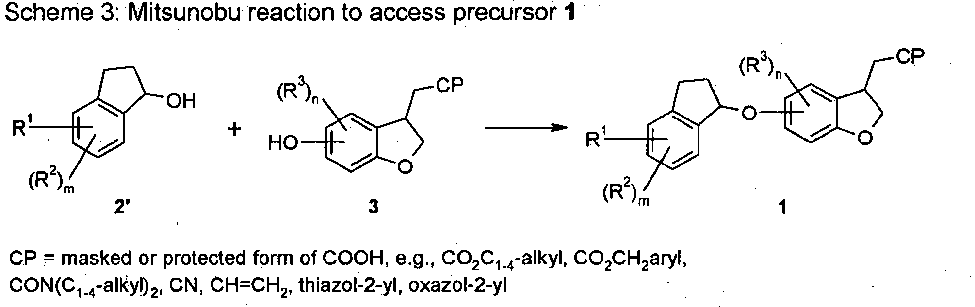

Intermediate **2/2'** is conveniently obtained from indanone 4 which, in turn, may be prepared from phenylpropionic acid derivative 5 (Scheme 4); R¹, R², and m in Scheme 4 have the meanings as defined hereinbefore and hereinafter. For the intramolecular acylation (Friedel-Crafts acylation), **5→4,** a considerable number of approaches has been reported. The reaction may be performed starting with a carboxylic acid, carboxylic ester, carboxylic anhydride, carboxylic chloride or fluoride, or a nitrile using a Lewis acid as catalyst. The following Lewis acids are some of the more often used ones: hydrobromic acid, hydroiodic acid, hydrochloric acid, sulfuric acid, phosphoric acid, P₄O₁₀, trifluoroacetic acid, methanesulfonic acid, toluenesulfonic acid, trifluoromethanesulfonic acid, ClSO₃H, Sc(OSO₂CF₃)₃, Tb(OSO₂CF₃)₃, SnCl₄, FeCl₃, AlBr₃, AlCl₃, SbCl₅, BCl₃, BF₃, ZnCl₂, montmorillonites, POCl₃, and PCl₅. The reaction may be conducted, e.g., in dichloromethane, 1,2-dichloroethane, nitrobenzene, chlorobenzene, carbon disulfide, mixtures thereof, or without an additonal solvent in an excess of the Lewis acid, at 0 to 180 °C. Carboxylic acids are preferably reacted in polyphosphoric acid at 0 to 120 °C, while carboxylic chlorides are preferably reacted with AlCl₃ in dichloromethane or 1,2-dichloroethane at 0 to 80 °C.

The subsequent reduction of the keto group in Scheme 4 is a standard transformation in organic synthesis, which is mainly accomplished with lithium borohydride, sodium borohydride, lithium aluminum hydride, and diisobutylaluminum hydride. While sodium borohydride is employed in aqueous or alcoholic solution at 0 to 60 °C, the other reducing agents mentioned are used in inert solvents, such as tetrahydrofuran, diethyl ether, dichloromethane, and toluene, at -80 to 60 °C. The reduction of the keto group may also be conducted in a stereoselective fashion providing the alcohol in enantiomerically enriched or pure form. Preferred chiral reducing agents are boranes combined with an enantiomerically pure [1,3,2]oxazaborol (Corey-Bakshi-Shibata reaction or Corey-Itsuno reaction) or formic acid, formates, hydrogen, or silanes in the presence of an enantiomerically pure transition metal catalyst. Typical reaction conditions for the former approach are borane (complexed with, e.g., dimethyl sulfide) and (R)- or (*S*)-3,3-diphenyl-1-methyltetrahydro-1H,3H-pyrrolo[1,2-c][1,3,2]oxazaborol in, e.g., dichloromethane, toluene, methanol, tetrahydrofuran, or mixtures thereof, at 0 to 60 °C. Using a chiral transition metal catalyst, such as a ruthenium complex, e.g. chloro{[(1S,2S)-(-)-2-amino-1,2-diphenylothyl](4-toluenesulfonyl)-amido}-(mesitylene)ruthenium(II), may deliver the hydroxy compound with high enantiomeric excess via reduction of the ketone with, e.g., formic acid in the presence of a base, e.g. triethylamine, in dichloromethane, at -20 to 60 °C.

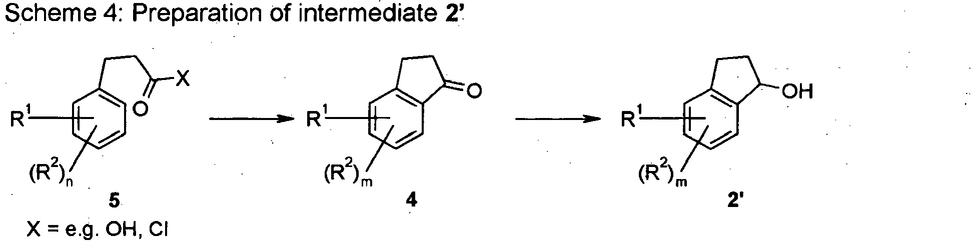

Alternatively, indanone **4** can be synthesized as described in Scheme 5; R¹, R², and m have the meanings as defined hereinbefore and hereinafter. Starting with benzene **6** and 3-halo-propionic acid or a derivative thereof of acrylic acid or a derivative thereof the required indanone **4** may be obtained via the combination of a Friedel-Crafts alkylation and acylation reaction in one pot or two separate reactions (eq. 1.)). These reactions are catalyzed by a Lewis acid, such as triflic acid, sulfuric acid, phosphoric acid, AlCl₃, ZnCl₂, and phosphorus pentoxide, and preferably conducted without additonal solvent in an excess of the Lewis acid or in dichloromethane, 1,2-dichloroethane, cyclohexane, or carbon disulfide, at 0 to 140 °C. A preferred combination comprises compound **6,** 3-chloro-propionyl chloride, and AlCl₃ in dichloromethane or 1,2-dichlorethane at 20 to 80 °C.

Starting with ethynylbenzene **7** indanone **4** is accessible by a transiton metal catalyzed reaction with carbon monoxide (eq. 2.)). Rhodium is a preferred catalyst basis which is combined with a phosphine, e.g. triphenylphosphine, and a base, e.g. triethylamine, and used in a solvent, preferably tetrahydrofuran, at high carbon monoxide pressure, preferably 50 to 150 bar, at 150 to 200 °C (see e.g. J. Org. Chem. 1993, 58, 5386-92).

Combination of 2-halo or pseudohalo substituted styrene **8** and carbon monoxide in the presence of a transiton metal also allows the preparation of indanone **4** (eq. 3.)). Palladium catalysts are preferred and used with carbon monoxide or molybdenum hexacarbonyl as carbon monoxide source. Preferred solvents are N,N-dimethylformamide, N,N-dimethylacetamide, N-methylpyrrolidone, and 1,4-dioxane which are preferably employed at 20 to 150 °C by conventional heating or microwave irradiation. Pyridine and tetrabutylmmonium chloride are preferred additives for this transformation (see e.g. J. Am. Chem. Soc. 2003, 125, 4804-7 and J. Org. Chem. 2005, 70, 346-9).

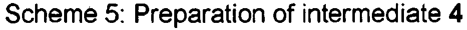

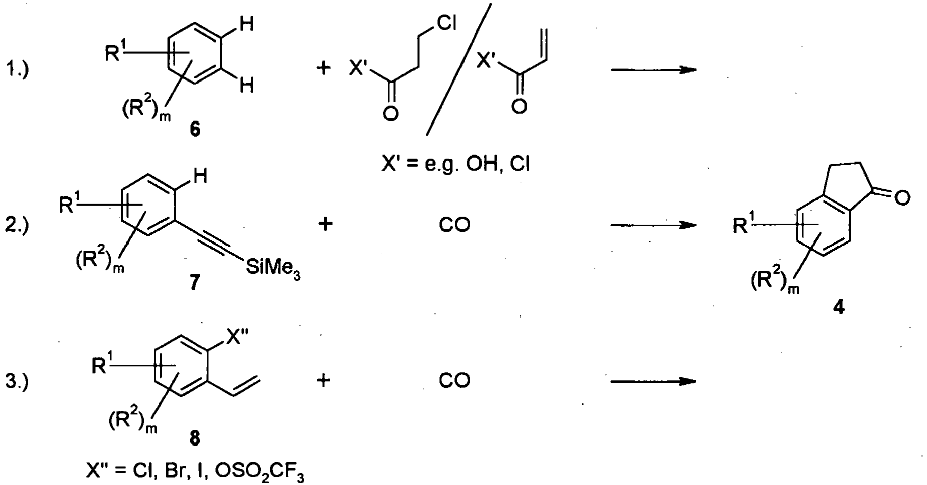

Common synthetic routes to building block **3** are summarized in Scheme 6; R³ and n have the meanings as defined hereinbefore and hereinafter. 2-lodo or bromo ether **9** can be transformed into indane **3** via addition of an *in situ* generated carbon anion or radical to the double bond and subsequent trapping of the cyclic anion by a proton and the cyclic radical by a hydride source (eq. 1.)). nBuLi, iPrMgCl, iPrMgCl*LiCl, Mg, Mg*LiCl, Zn, and Zn*LiCl are preferred reagents to convert the C-I or C-Br bond into a C-M bond (M = Li, MgI, Znl etc.) with sufficient nucleophilicity to add to the double bond. The reactions with lithium and magenesium reagents are preferably conducted in hexanes, tetrahydrofuran, diethyl ether, 1,2-dimethoxyethane, toluene, or mixtures thereof, at -100 to 60 °C. Zn is preferably used in tetrahydrofuran, dimethyl sulfoxide, N-methylpyrrolidinone, or mixtures thereof, at 0 to 100 °C. Often employed reaction conditions for the radical pathway are, e.g., tributyltin hydride, azobisisobutyronitrile, in benzene, at 60 to 100 °C (see e.g. J. Org. Chem. 1987, 52, 4072-8) and NaCNBH₃ in N,N-dimethylformamide under UV irradiation at 20 to 120 °C (see e.g. Synlett 2005, 2248-50).

In equation 2.) allyl ether **10** is combined with carbon monoxide to obtain indane **3.** This type of reaction is preferably conducted with a palladium catalyst in the presence of carbon monoxide or molybdenum hexacarbonyl as carbon monoxide source (see e.g. Tetrahedron Lett. 2010, 51, 2102-5).

Starting from benzofuran 11 or dihydrobenzofuran 12 indane 3 is yielded after reduction of the double bond. Hydrogen is the preferred reducing agent which is mainly employed in combination with a transition metal catalyst, such as palladium on carbon, Raney nickel, and PtO₂. N,N-dimethylformamide, tetrahydrofuran, ethyl acetate, alcohol, e.g. methanol and ethanol, acetic acid, water, or mixtures thereof are preferably used as solvents, at hydrogen pressures of 1 to 100 bar, and temperatures of 20 to 120 °C. This reaction may also be carried out stereoselectively providing compound 3 in enantiomerically enriched or pure form.

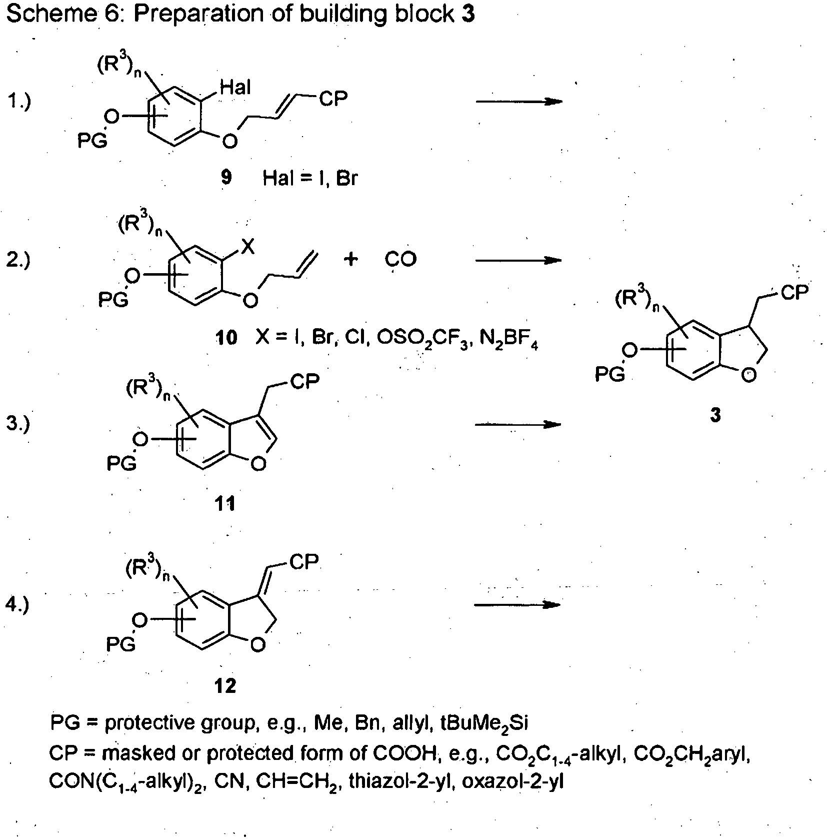

The syntheses of the starting compounds in Scheme 6 comprise standard procedures used in organic synthesis. Intermediate 11 can, for example, be prepared as described in Scheme 7; R³ and n have the meanings as defined hereinbefore and hereinafter. Compound 11 may thus be obtained from compound 13 which, in turn, may be assembled from phenol 14 and ester 15. The latter transformation may be achieved in the presence of a Lewis acid, e.g. sulfuric acid, ZrCl₄, InCl₃, methanesulfonic acid, p-toluenesulfonic acid, HI, or amberlyst, in toluene, dichloromethane, acetic acid, ethanol, water, or without a solvent in an excess of the Lewis acid, at 0 to 120 °C. Transformation of compound 13 into intermediate 11 is preferably accomplished under basic conditions with sodium hydroxide in an aqueous solution at 0 to 100 °C.

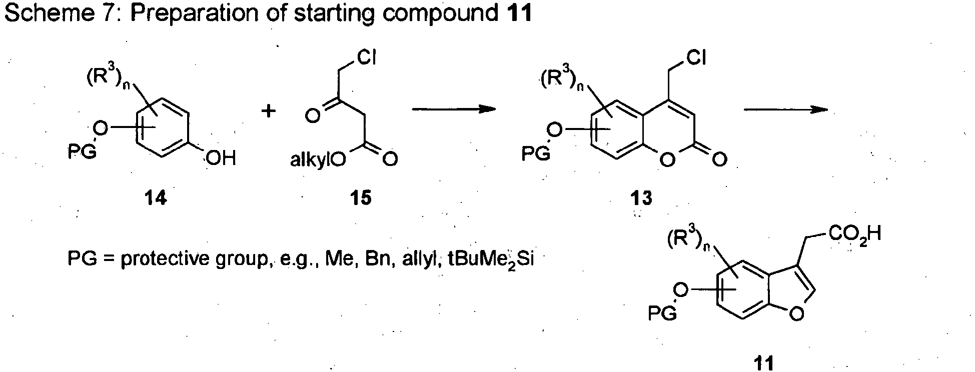

The synthetic routes presented may rely on the use of protecting groups. For example, potentially reactive groups present, such as hydroxy, carbonyl, carboxy, amino, alkylamino, or imino, may be protected during the reaction by conventional protecting groups which are cleaved again after the reaction. Suitable protecting groups for the respective functionalities and their removal are well known to the one skilled in the art and are described in the literature of organic synthesis.

The compounds of general formula I may be resolved into their enantiomers and/or diastereomers as mentioned before. Thus, for example, *cis*/*trans* mixtures may be resolved into their *cis* and *trans* isomers and racemic compounds may be separated into their enantiomers.

The *cis*/*trans* mixtures may be resolved, for example, by chromatography into the *cis* and *trans* isomers thereof. The compounds of general formula I which occur as racemates may be separated by methods known *per se* (cf. Allinger N. L. and Eliel E. L. in "Topics in Stereochemistry", Vol. 6, Wiley Interscience, 1971) into their optical antipodes and diastereomeric mixtures of compounds of general formula I may be resolved into their diastereomers by taking advantage of their different physico-chemical properties using methods known *per se,* e.g. chromatography and/or fractional crystallization; if the compounds obtained thereafter are racemates, they may be resolved into the enantiomers as mentioned above.

The racemates are preferably resolved by column chromatography on chiral phases or by crystallization from an optically active solvent or by reacting with an optically active substance which forms salts or derivatives such as esters or amides with the racemic compound. Salts may be formed with enantiomerically pure acids for basic compounds and with enantiomerically pure bases for acidic compounds. Diastereomeric derivatives are formed with enantiomerically pure auxiliary compounds, e.g. acids, their activated derivatives, or alcohols. Separation of the diastereomeric mixture of salts or derivatives thus obtained may be achieved by taking advantage of their different physico-chemical properties, e.g. differences in solubility; the free antipodes may be released from the pure diastereomeric salts or derivatives by the action of suitable agents. Optically active acids in common use for such a purpose are e.g. the D- and L-forms of tartaric acid, dibenzoyltartaric acid, ditoloyltartaric acid, malic acid, mandelic acid, camphorsulfonic acid, glutamic acid, aspartic acid, or quinic acid. Optically active alcohols applicable as auxiliary residues may be, for example, (+) or (-)-menthol and optically active acyl groups in amides may be, for example, (+)- or (-)-menthyloxycarbonyl.

As mentioned above, the compounds of formula I may be converted into salts, particularly for pharmaceutical use into the pharmaceutiically acceptable salts. As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. For example, such salts include acetates, ascorbates, benzenesulfonates (besylates), benzoates, bicarbonates, bitartrates, bromides/hydrobromides, Ca-edetates/edetates, camsylates, carbonates, chlorides/hydrochlorides, citrates, ethane disulfonates (edisylates), estolates, esylates, fumarates, gluceptates, gluconates, glutamates, glycolates, glycollylarsanilates, hexylresorcinates, hydrabamines, hydroxymaleates, hydroxynaphthoates, iodides, isothionates, lactates, lactobionates, malates, maleates, mandelatos, methanesulfonates, mucates, napsylates, nitrates, oxalates, pamoates, pantothenates, phenylacetates, phosphates/diphosphates, polygalacturonates, propionates, salicylates, stearates, subacetates, succinates, sulfamides, sulfates, tannates, tartrates, teoclates, toluenesulfonates (tosylates), triethiodides, ammonium, benzathines, chloroprocaines, cholines, diethanolamines, ethylenediamines, meglumines, and procaines. Further pharmaceutically acceptable salts can be formed with cations from metals like aluminum, calcium, lithium, magnesium, potassium, sodium, zinc, and the like (also see Pharmaceutical salts, Birge, S.M. et al., J. Pharm. Sci., (1977), 66, 1-19). Some of the salts mentioned above may also be useful for purifying or isolating the compounds of the invention.

The pharmaceutically acceptable salts of the present invention can be synthesized from the parent compound which contains a basic or acidic moiety by conventional chemical methods. Generally, such salts can be prepared by reacting the free acid or base forms of these compounds with a sufficient amount of the appropriate base or acid in water or in an organic diluent like ether, ethyl acetate, ethanol, isopropanol, or acetonitrile, or a mixture thereof.

Salts of other acids than those mentioned above which for example are useful for purifying or isolating the compounds of the present invention (e.g. trifluoroacetate salts), also comprise a part of the invention.

The compounds according to the invention are advantageously also obtainable using the methods described in the examples that follow, which may also be combined for this purpose with methods known to the skilled man from the literature.

As already mentioned, the compounds of general formula I according to the invention and the physiologically acceptable salts thereof have valuable pharmacological properties, particularly an activatory effect on the receptor GPR 40.

### Biological Examples

The potent stimulatory effect of the compounds of the invention may be determined by in vitro assays as follows:
(A) Cytosolic calcium measurement using the FLIPR system - 1321 N1 cells stably expressing human GPR40 receptor (Euroscreen, Belgium) were seeded 24 h before the assay in black clear-bottom collagen-coated 384-well plates in culture medium containing 10% FCS, 1% Na-Pyruvate and 400 µg/ml G418. On the assay day, wells were washed twice with 100 µL Krebs Ringer Buffer (KRB) containing 15 mM sodium hydrogencarbonate and 30 mM Hepes leaving 20 µl rest volume per well. Prior to stimulation the cells were loaded with the Ca²⁺-sensitive fluorescent dye from the Calcium4 Assay kit™ according to the Manufacturer's description (MDS Analytical technologies, Ismaning, Germany) for 80 min at room temperature in the dark, in the presence of 4 mM probenicid. Changes in cytosolic Ca²⁺ concentration upon compound addition were monitored using a FLIPR Tetra device (Fluorescence Imaging Plate Reader; MDS Analytical technologies). The peak height of the obtained fluorescence signal was then used to calculate the EC₅₀ values using GraphPad Prism 5 (Graphpad Software Inc, USA) using a sigmoidal curve fitting procedure allowing for a variable hill slope.
The compounds of general formula (I) according to the invention assayed as described above, for example, have EC₅₀ values below 10000 nM, particularly below 1000 nM, most preferably below 100 nM.

**Table 2: EC₅₀ values on GPR40 determined as described in (A) of Examples compiled in the experimental part**

| Example | EC₅₀ [nM] | Example | EC₅₀ [nM] | Example | EC₅₀ [nM] | Example | EC₅₀ [nM] |
|---|---|---|---|---|---|---|---|
| 1 | 50 | 2 | 27 | 3/4* | 186 | 5 | 2280 |
| 6 | 49 | 7 | 710 | 8 | 312 | 9 | 3247 |
| 10 | 333 | 11 | 628 | 12 | 459 | 13 | 583 |
| 14 | 364 | 15 | 3841 | 16 | 3192 | 17 | 111 |
| 18 | 21 | | | | | | |

| | | | | | | | |
|---|---|---|---|---|---|---|---|
| *mixture of two compounds; see corresponding entry in the experimental section | | | | | | | |

(B) IP₁ accumulation measurements using the IPOne assay system - 1321 N1 cells stably expressing human GPR40 receptor (Euroscreen, Belgium) were seeded 24 h before the assay in black clear-bottom collagen-coated 384-well plates in culture medium containing 10% FCS, 1% Na-Pyruvate and 400 µg/mL G418. IP₁ is assayed according to the Manufacturer's description (Cisbio Bioassays, France). In brief, the assay is started by substitution of the culture medium by stimulation buffer (Hepes 10 mM, CaCl₂ 1 mM, MgCl₂ 0.5 mM, KCl 4.2 mM, NaCl 146 mM and glucose 5.5 mM, pH 7.4) without LiCl. Cells are stimulated for 1 hour at 37 °C, 10% CO₂ by addition of the compounds that were diluted in stimulation buffer containing LiCl yielding a final LiCl concentration of 50mM. Assays are stopped by adding HTRF-conjugates (IP1-d2 and Anti-IP1 cryptate Tb) and lysis buffer, provided by the manufacturer. After an incubation time of 1 hour at room temperature plates are measured using an EnVision™, Perkin Elmer. The obtained fluorescence ratios at 665/615 nM are then used to calculate the pEC₅₀ values using GraphPad Prism 5 (Graphpad Software Inc, USA) by interpolation using an IP₁ reference curve and subsequent sigmoidal curve fitting allowing for a variable hill slope.

The compounds of general formula (I) according to the invention assayed as described above, for example, have EC₅₀ values below 10000 nM, particularly below 1000 nM, most preferably below 100 nM.

**Table 3: EC₅₀ values on GPR40 determined as described in (B) of Examples compiled in the experimental part**

| Example | EC₅₀ [nM] | Example | ECso [nM] | Example | EC₅₀ [nM] | Example | EC₅₀ [nM] |
|---|---|---|---|---|---|---|---|
| 1 | 69 | 2 | 20 | 3/4* | 501 | 5 | 8985 |
| 6 | 62 | 7 | 1995 | 8 | 1567 | 9 | >10000 |
| 10 | 178 | 11 | 2228 | 12 | 2692 | 13 | 43 |
| 14 | 125 | 15 | 30 | 16 | 47 | 17 | 139 |
| 18 | 162 | 19 | 5188 | 22 | 23 | 21 | 54 |
| 22 | 24 | 23 | 9120 | 24 | 35 | 25 | 18 |
| 26 | 19 | 27 | 23 | 28 | 25 | 29 | 14 |
| 30 | 302 | 31 | 168 | 32 | 98 | 33 | 4 |
| 34 | 13 | 35 | 4 | 36 | 19 | 37 | 78 |
| 38 | 131 | 39 | 262 | 40 | 13 | 41 | 323 |
| 42 | 131 | 43 | 420 | 44 | 25 | 46 | 982 |
| 47 | 71 | 48 | 16 | 49 | 1906 | 50 | 2972 |
| 51 | 9672 | 52 | 113 | 53 | 211 | 54 | 258 |
| 55 | 2914 | 56 | 124 | 57 | 8899 | 58 | 534 |
| 59 | 325 | 60 | 51 | 61 | 155 | 62 | 52 |
| 63 | 3151 | 64 | 213 | 65 | 1849 | 66 | 80 |
| 67 | 176 | 68 | 359 | 69 | 88 | 70 | 8 |
| 71 | 71 | 72 | 98 | 73 | 2724 | 74 | 327 |
| 75 | 39 | 76 | 95 | 77 | 57 | 78 | 76 |
| 79 | 288 | 80 | 20 | 81 | 1537 | 82 | 3353 |
| 83 | 392 | 84 | 117 | 85 | 3715 | 86 | 414 |
| 87 | 64 | 88 | 28 | 89 | 428 | 90 | 36 |
| 91 | 69 | 92 | 7 | 93 | 61 | 94 | 7 |
| 95 | 100 | 96 | 36 | 97 | 171 | 98 | 46 |
| 99 | 3266 | 100 | 26 | 101 | 25 | 105 | 4250 |
| 107 | 6053 | - | - | - | - | - | - |

| | | | | | | | |
|---|---|---|---|---|---|---|---|
| *mixture of two compounds; see corresponding entry in the experimental section | | | | | | | |

The pharmaceutical composition according to the present invention may further comprise an additional therapeutic agent. Particularly preferred are compositions, wherein the additional therapeutic agent is selected from antidiabetics like insulin, long and short acting insulin analogues, sulfonylureas, biguanides, DPP-IV inhibitors, SGLT2 inhibitors, 11β-HSD1 inhibitors, glucokinase activators, AMPK activators, Glp-1 receptor agonists, GIP receptor agonists, DGAT inhibitors, PPARgamma agonists, PPARdelta agonists, and other antidiabetics derived from thiazolidinediones, lipid lowering agents such as statines, fibrates, nicotinic acid derivatives, or HMG-CoA reductase inhibitors, cardiovascular therapeutics such as nitrates, antihypertensiva such as β-blockers, ACE inhibitors, Ca-channel blockers, angiotensin II receptor antagonists, diuretics, thrombocyte aggregation inhibitors, or antineoplastic agents such as alkaloids, alkylating agents, antibiotics, or antimetabolites, or anti-obesity agents.
More particularly preferred are compounds such as human NPH insulin, human lente or ultralente insulin, insulin Lispro, insulin Aspart, insulin Glulisine, insulin detemir or insulin Glargine, metformin, phenformin, acarbose, miglitol, voglibose, pioglitazone, rosiglizatone, rivoglitazone, aleglitazar, alogliptin, saxagliptin, sitagliptin, vildagliptin, linagliptin, dapagliflozin, remogliflozin etabonate, sergliflozin, canagliflozin, exenatide, liraglutide, albiglutide, pramlintide, carbutamide, chlorpropamide, glibenclamide (glyburide), gliclazide, glimepiride, glipizide, gliquidone, simvastatine, bezafibrate, fenofibrate, gemfibrozil, clofibrate, etofibrate, fluvastatine, lovastatine, pravastatin, colestyramide, acipimox, and niacin.

It will be appreciated by the person of ordinary skill in the art that the compounds of the invention and the additional therapeutic agent may be formulated in one single dosage form, or may be present in separate dosage forms and may be either administered concomitantly (i.e. at the same time) or sequentially.

The pharmaceutical compositions of the present invention may be in any form suitable for the intended method of administration.

The compounds of the present invention may be administered orally, parenterally, such as bronchopulmonary, subcutaneously, intravenously, intramuscularly, intraperitoneally, intrathecally, transdermally, transmucosally, subdurally, locally or topically via iontopheresis, sublingually, by inhalation spray, aerosol or rectally and the like in dosage unit formulations optionally comprising conventional pharmaceutically acceptable excipients.

Excipients that may be used in the formulation of the pharmaceutical compositions of the present invention comprise carriers, vehicles, diluents, solvents such as monohydric alcohols such as ethanol, isopropanol and polyhydric alcohols such as glycols and edible oils such as soybean oil, coconut oil, olive oil, safflower oil cottonseed oil, oily esters such as ethyl oleate, isopropyl myristate; binders, adjuvants, solubilizers, thickening agents, stabilizers, disintergrants, glidants, lubricating agents, buffering agents, emulsifiers, wetting agents, suspending agents, sweetening agents, colorants, flavors, coating agents, preservatives, antioxidants, processing agents, drug delivery modifiers and enhancers such as calcium phosphate, magnesium state, talc, monosaccharides, disaccharides, starch, gelatine, cellulose, methylcellulose, sodium carboxymethyl cellulose, dextrose, hydroxypropyl-ß-cyclodextrin, polyvinylpyrrolidone, low melting waxes, ion exchange resins.

Other suitable pharmaceutically acceptable excipients are described in Remington's Pharmaceutical Sciences, 15th Ed., Mack Publishing Co., New Jersey (1991).

Dosage forms for oral administration include tablets, capsules, lozenges, pills, wafers, granules, oral liquids such as syrups, suspensions, solutions, emulsions, powder for reconstitution.

Dosage forms for parenteral administration include aqueous or olageous solutions or emulsions for infusion, aqueous or olageous solutions, suspensions or emulsions for injection pre-filled syringes, and/or powders for reconstitution.

Dosage forms for local/topical administration comprise insufflations, aerosols, metered aerosols, transdermal therapeutic systems, medicated patches, rectal suppositories, and/or ovula.

The amount of the compound of the present invention that may be combined with the excipients to formulate a single dosage form will vary upon the host treated and the particular mode of administration.

The pharmaceutical compositions of the invention can be produced in a manner known per se to the skilled person as described, for example, in Remington's Pharmaceutical Sciences, 15th Ed., Mack Publishing Co., New Jersey (1991).

For the purpose of the present invention, a therapeutically effective dosage will generally be from about 1 to 2000 mg/day, preferably from about 10 to about 1000 mg/day, and most preferably from about 10 to about 500 mg/day, which may be administered in one or multiple doses, preferably by oral route of administration.

It will be appreciated, however, that specific dose level of the compounds of the invention for any particular patient will depend on a variety of factors such as age, sex, body weight, general health condition, diet, individual response of the patient to be treated, time of administration, severity of the disease to be treated, the activity of particular compound applied, dosage form, mode of application and concomitant medication. The therapeutically effective amount for a given situation will readily be determined by routine experimentation and is within the skills and judgment of the ordinary clinician or physician.

In another aspect, this invention relates to the use of a compound according to the invention or a physiologically acceptable salt of such a compound combined with at least one of the active substances described above as a combination partner, for preparing a pharmaceutical composition which is suitable for the treatment or prevention of diseases or conditions which can be affected by influencing the receptor GPR40. These are preferably metabolic diseases, particularly one of the diseases or conditions listed above, most particularly diabetes or diabetic complications.

The use of the compound according to the invention, or a physiologically acceptable salt thereof, in combination with another active substance may take place simultaneously or at staggered times, but particularly within a short space of time. If they are administered simultaneously, the two active substances are given to the patient together; while, if they are used at staggered times, the two active substances are given to the patient within a period of less than or equal to 12 hours, but particularly less than or equal to 6 hours.

Consequently, in another aspect, this invention relates to a pharmaceutical composition which comprises a compound according to the invention or a physiologically acceptable salt of such a compound and at least one of the active substances described above as combination partners, optionally together with one or more inert carriers and/or diluents.

The compound according to the invention, or a physiologically acceptable salt thereof, and the additional active substance to be combined therewith may both be present together in one formulation, for example, a tablet or capsule, or separately in two identical or different formulations, for example, as a so-called kit-of-parts.

The Examples that follow are intended to illustrate the present invention:
The terms "ambient temperature" and "room temperature" are used interchangeably and designate a temperature of about 20 °C.

As a rule, ¹H-NMR and/or mass spectra have been obtained for the compounds prepared. The r_{f} values are determined using Merck silica gel 60 F₂₅₄ plates and UV light at 254 nm or staining with a suited reagent such as molybdatophosphoric acid.

Analytical HPLC parameters employed for characterization of products:

| **method 1** | Waters, XBridge C18, 3x30 mm, 2.5 µm; 60 °C | | | **method 2** | Waters, XBridge C18, 4.6x30 mm, 3.5 µm; 60 °C | | |
|---|---|---|---|---|---|---|---|
| column | | | | column | | | |
| mobile phase | A: water + 0.2% F₃CCO₂H | | | mobile phase | A: water + 0.1% F₃CCO₂H | | |
| | B: methanol | | | | B: methanol | | |

| | TIME (min) | A% | B% | | TIME (min) | A% | B% |
|---|---|---|---|---|---|---|---|
| | 0.00 | 95 | 5 | | 0.00 | 95 | 5 |
| | 0.05 | 95 | 5 | | 0.20 | 95 | 5 |
| | 1.40 | 0 | 100 | | 1.50 | 0 | 100 |
| | 1.80 | 0 | 100 | | 1.75 | 0 | 100 |
| | | | | | 1.85 | 95 | 5 |
| flow rate | 2.2 mL/min | | | flow rate | 4 mL/min | | |
| wavelength | 210-400 nm | | | wavelength | 210-400 nm | | |
| | | | | | | | |

| **method 3** | Waters, XBridge C18, 4.6x30 mm, 3.5 µm; 60 °C | | | **method 4** | Waters, XBridge C18, 2.1x30 mm, 3.5 µm; 35 °C | | |
|---|---|---|---|---|---|---|---|
| column | | | | column | | | |
| mobile phase | A: water + 0.1% F₃CCO₂H | | | mobile phase | A: water + 0.1 % HCO₂H | | |
| | B: H₃COH + 0.1% F₃CCO₂H | | | | B: H₃CCN + 0.1% HCO₂H | | |

| | TIME (min) | A% | B% | | TIME (min) | A% | B% |
|---|---|---|---|---|---|---|---|
| | 0.00 | 95 | 5 | | 0.00 | 95 | 5 |
| | 0.15 | 95 | 5 | | 1.60 | 2 | 98 |
| | 1.70 | 0 | 100 | | 3.00 | 2 | 98 |
| | 2.25 | 0 | 100 | | | | |
| flow rate | 4 mL/min | | | flow rate | 1 mL/min | | |
| wavelength | 210-400 nm | | | wavelength | 220-320 nm | | |
| | | | | | | | |

| **method 5** | Waters, XBridge C18, 2.1x50 mm, 3.5 µm; 35 °C | | | **method 6** | Waters, XBridge C18, 4,6x30 mm, 3.5 µm; 60 °C | | |
|---|---|---|---|---|---|---|---|
| column | | | | column | | | |
| mobile phase | A: water + 0.1% HCO₂H | | | mobile phase | A: water + 0.1 % HCO₂H | | |
| | B: H₃CCN + 0.1 % HCO₂H | | | | B: H₃COH | | |

| | TIME (min) | A% | B% | | TIME (min) | A% | B% |
|---|---|---|---|---|---|---|---|
| | 0.00 | 95 | 5 | | 0.00 | 50 | 50 |
| | 3.50 | 2 | 98 | | 0.15 | 50 | 50 |
| | 6.00 | 2 | 98 | | 1.70 | 0 | 100 |
| | | | | | 2.25 | 0 | 100 |
| flow rate | 0.8 mL/min | | | flow rate | 4 mL/min | | |
| wavelength | 220-320 nm | | | wavelength | 210-400 nm | | |
| | | | | | | | |

| **method 7** | Waters, XBridge C18, 4,6x30 mm, 3.5 µm; 60 °C | | | **method 8** | Synergi Hydro RP100A, 3x50 mm, 2.5 µm | | |
|---|---|---|---|---|---|---|---|
| column | | | | column | | | |
| mobile phase | A: water + 0.1 % HCO₂H | | | mobile phase | A: 90% H₂O + 10% CH₃CN + 10 mM HCOONH₄ | | |
| | B: H₃COH + 0.1 % F₃CCO₂H | | | | B: 90% CH₃CN + 10% H₂O + 10 mM HCOONH₄ | | |

| | TIME (min) | A% | B% | | TIME (min) | A% | B% |
|---|---|---|---|---|---|---|---|
| | 0.00 | 95 | 5 | | 0.00 | 100 | 0 |
| | 0.15 | 95 | 5 | | 1.50 | 100 | 0 |
| | 1.70 | 0 | 100 | | 8.00 | 0 | 100 |
| | 2.25 | 0 | 100 | | 10.00 | 0 | 100 |
| | | | | | 11.00 | 100 | 0 |
| | | | | | 12.00 | 100 | 0 |
| flow rate | 4 mL/min | | | flow rate | 0.7 mL/min | | |
| wavelength | 210-400 nm | | | wavelength | 254 nm | | |
| | | | | | | | |

| **method 9** | Simmetry Shield RP8, 4.6x150 mm, 5 µm | | | | | | |
|---|---|---|---|---|---|---|---|
| column | | | | | | | |
| mobile phase | A: 90% H₂O + 10 % CH₃CN 0.1 % HCOOH | | | | | | |
| | B: 90% CH₃CN + 10 % H₂O + 0.1% HCOOH | | | | | | |

| | TIME (min) | A% | B% | | | | |
|---|---|---|---|---|---|---|---|
| | 0.00 | 70 | 30 | | | | |
| | 1.50 | 50 | 50 | | | | |
| | 8.50 | 0 | 100 | | | | |
| | 13.05 | 0 | 100 | | | | |
| | 14.00 | 70 | 30 | | | | |
| | 15.00 | 70 | 30 | | | | |
| flow rate | 0.85 mL/min | | | | | | |
| wavelength | 254 nm | | | | | | |

### Intermediate 1

### [6-(4-Trifluoromethyl-indan-1-yloxy)-2,3-dihydro-benzofuran-3-yl]-acetic acid methyl ester

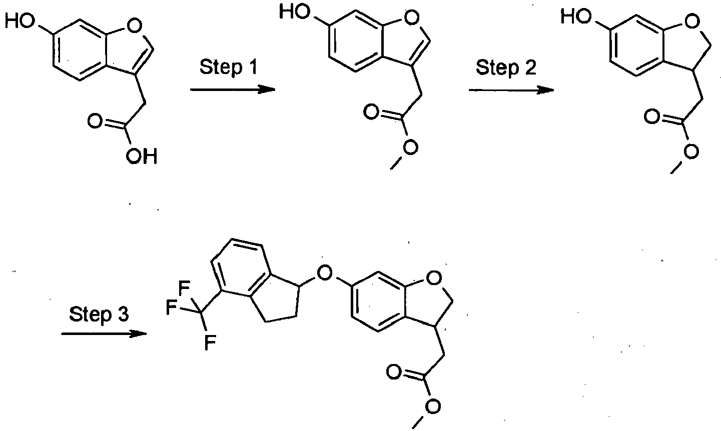

### Step 1: (6-hydroxy-benzofuran-3-yl)-acetic acid methyl ester

A mixture of (6-hydroxy-benzofuran-3-yl)-acetic acid (for preparation see WO 2008001931; 14.0 g), concentrated sulfuric acid (5 mL), and methanol (250 mL) is stirred at reflux temperature for 4 h. After cooling to room temperature, the mixture is concentrated. Ethyl acetate is added to the residue and the resulting mixture is washed with water, saturated aqueous NaHCO₃ solution, and brine and dried (MgSO₄). The solvent is evaporated and the residue is chromatographed on silica gel (cyclohexane/ethyl acetate 2:1→1:2) to give the title compound. Yield: 9:0 g (60% of theory); Mass spectrum (ESI⁺): m/z = 207 [M+H]⁺.

### Step 2: (6-hydroxy-2,3-dihydro-benzofuran-3-yl)-acetic acid methyl ester

A mixture of (6-hydroxy-benzofuran-3-yl)-acetic acid methyl ester (5.00 g), 10% palladium on carbon (0.50 g), and methanol (50 mL) is shaken under hydrogen atmosphere (3 bar) at room temperature for 3 h. The catalyst is separated by filtration and the filtrate is concentrated. The residue is chromatographed on silica gel (cyclohexane/ethyl acetate 4:1→1:1) to give the title compound as an oil that solidified upon standing. Yield: 2.60 g (51% of theory); Mass spectrum (ESI⁺): m/z = 209 [M+H]⁺.

The enantiomers may be separated by SFC on chiral phase (column: Daicel ADH, 5 µm, 250 mm x 20 mm; eluent: scCI₂/(isopropanol+0.2% diethylamine) 80:20, 70 mL/min):

### (S)-(6-Hydroxy-2,3-dihydro-benzofuran-3-yl)-acetic acid methyl ester: t_{R} = 2.33 min.

### (R)-(6-Hydroxy-2,3-dihydro-benzofuran-3-yl)-aceticacid methyl ester: t_{R} = 2.75 min. Alternatively, the pure enantiomers may be obtained as described in WO 2008001931.

### Step 3: [6-(4-trifluoromethyl-indan-1-yloxy)-2,3-dihydro-benzofuran-3-yl]-acetic acid methyl ester

Diethyl azodicarboxylate (40% in toluene, 1.13 mL; alternatively, di-*i*-propyl or di-*t-*butyl azodicarboxylate is used) is added to a solution of (6-hydroxy-2,3-dihydro-benzofuran-3-yl)-acetic acid methyl ester (0.52 g), 4-trifluoromethyl-indan-1-ol (for preparation see WO 2009157418; 0.50 g), and triphenylphosphine (0.65 g) in tetrahydrofuran (20 mL) at room temperature. The resulting solution is stirred at room temperature for 3 h and is then concentrated. The residue is chromatographed on silica gel (cyclohexane/ethyl acetate 9:1→2:1) to give the title compound as a mixture of four stereoisomers. Yield: 0.51 g (53% of theory); Mass spectrum (ESI⁺): m/z = 393 [M+H]⁺.

### Intermediate 2

### {6-[(R)-4-Bromo-indan-1-yloxy]-2,3-dihydro-benzofuran-3-yl}-acetic acid methyl ester

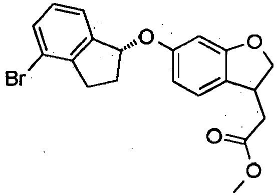

The title compound is prepared from (6-hydroxy-2,3-dihydro-benzofuran-3-yl)-acetic acid methyl ester and (S)-4-bromo-indan-1-ol (for preparation see WO 2009157418 and Agricultural and Biological Chemistry (1982), 46(10), 2579-85) following a procedure analogous to that described in Step 3 of Intermediate 1. Yield: 56% of theory; LC (method 1): t_{R} = 1.43 min; Mass spectrum (ESI⁺): m/z = 403/405 (Br) [M+H]⁺. The diastereomerically pure compounds are obtained by chromatographic separation of the title compound or by using [(*S*)-6-hydroxy-2,3-dihydro-benzofuran-3-yl]-acetic acid methyl ester in the above described procedure.

### Intermediate 3

### {6-[(R)-4-(1-Methyl-1H-pyrazol-4-yl)-indan-1-yloxyl-2,3-dihydro-benzofuran-3-yl}-acetic acid methyl ester

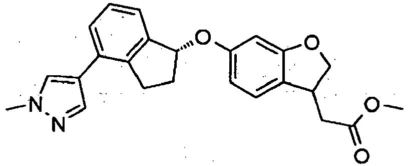

A flask charged with a stir bar, {6-[(*R*)-4-bromo-indan-1-yloxy]-2,3-dihydrobenzofuran-3-yl}-acetic acid methyl ester (100 mg), 1-methyl-4-(4,4,5,5-tetramethyl-[1,3,2]dioxaborolan-2-yl)-1H-pyrazole (52 mg), 2 M aqueous Na₂CO₃ solution (0.31 mL), and N,N-dimethylformamide, (1 mL) at room temperature is sparged with argon for 5 min. [1,1'-Bis(diphenylphosphino)-ferrocene]-dichloropalladium dichloromethane complex (20 mg) is added and the mixture is heated to 90 °C. After stirring at 90 °C for 3 h, the mixture is cooled to ambient temperature and diluted with water. The resulting mixture is extracted with ethyl acetate, and the combined extract is dried (MgSO₄). The solvent is evaporated and the residue is chromatographed on silica gel (cyclohexane/ethyl acetate 2:1→1:2) to give the title compound. Yield: 50 mg (50% of theory); LC (method 1): t_{R} = 1.25 min; Mass spectrum (ESI⁺): m/z = 405 [M+H]⁺.

### Intermediate 4

### {6-[(R)-4-(3,6-Dihydro-2H-pyran-4-yl)-indan-1-yloxy]-2,3-dihydro-benzofuran-3-yl}-acetic acid methyl ester

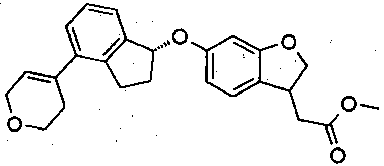

The title compound is prepared from {6-[(*R*)-4-bromo-indan-1-yloxy]-2,3-dihydro-benzofuran-3-yl}-acetic acid methyl ester and 4-(4,4,5,5-tetramethyl-[1,3,2]dioxaborolan-2-yl)-3,6-dihydro-2H-pyran following a procedure analogous to that described in Intermediate 3. Yield: 41% of theory; LC (method 1): t_{R} = 1.55 min; Mass spectrum (ESI⁺): m/z = 407 [M+H]⁺.

### Intermediate 5

### {6-[(R)-4-(1-Methyl-2-oxo-1,2-dihydro-pyridin-4-yl)-indan-1-yl)-indan-1-yloxy]-2,3-dihydrobenzofuran-3-yl}-acetic acid methyl ester

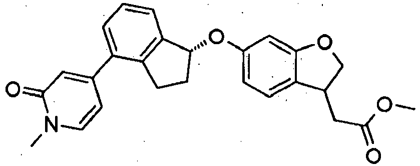

The title compound is prepared from {6-[(*R*)-4-bromo-indan-1-yloxy]-2,3-dihydro-benzofuran-3-yl}-acetic acid methyl ester and 1-methyl-2-oxo-1,2-dihydro-pyridine-4-boronic acid following a procedure analogous to that described in Intermediate 3. Yield: 62% of theory; LC (method 1): t_{R} = 1.20 min; Mass spectrum (ESI⁺): m/z = 432 [M+H]⁺.

### Intermediate 6

### {6-[(R)-4-(4-Methoxy-phenyl)-indan-1-yloxyl-2,3-dihydro-benzofuran-3-yl}-acetic acid methyl ester

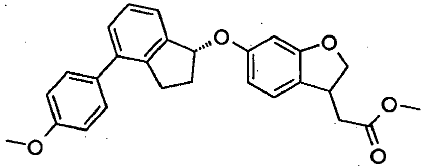

The title compound is prepared from {6-[(*R*)-4-bromo-indan-1-yloxy]-2,3-dihydrobenzofuran-3-yl}-acetic acid methyl ester and 4-methoxy-phenylboronic acid following a procedure analogous to that described in Intermediate 3. Yield: 62% of theory; LC (method 1): t_{R} = 1.46 min; Mass spectrum (ESI⁺): m/z = 431 [M+H]⁺.

### Intermediate 7

### {6-[4-(2,2-Difluoro-1-methoxy-ethyl)-indan-1-yloxy]-2,3-dihydro-benzofuran-3-yl}-acetic acid methyl ester

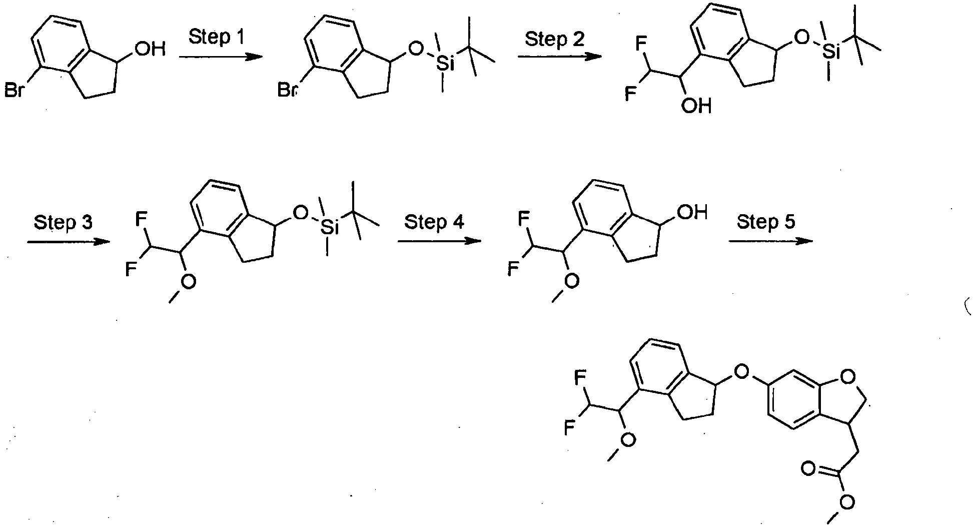

### Step 1: (4-bromo-indan-1-yloxy)-tert-butyl-dimethyl-silane

tert-Butyldimethylsilyl chloride (6.60 g) is added in three equal portions to a solution of 4-bromo-indan-1-ol (for preparation see J. Org. Chem. 1984, 49, 4226-37 and WO 2006/065809; 6.22 g) and imidazole (4.97 g) in N,N-dimethylformamide (40 mL) cooled to ca. 10 °C. The cooling bath is removed and the solution is stirred at room temperature for 6 h. Water (100 mL) is added and the resulting mixture is stirred for 30 min. The mixture is acidified using 1 M aqueous HCl solution (pH value ca. 5) and extracted with ethyl acetate. The combined extract is dried (MgSO₄) and the solvent is evaporated. The residue is chromatographed on silica gel (cyclohexane/ethyl acetate 20:1→4:1) to give the title compound as an oil. Yield: 6.3 g (66% of theory).

### Step 2: 1-[1-(tert-butyl-dimethyl-silanyloxy)-indan-4-yl)-2,2-difluoro-ethanol

n-Butyl lithium (1.6 mol/L in hexane, 0.96 mL) is added to a solution of (4-bromo-indan-1-yloxy)-tert-butyl-dimethyl-silane (0.50 g) in tetrahydrofuran (5 mL) at -78 °C. The resulting solution is stirred at -78 °C for 20 min prior to the addition of ethyl difluoroacetate (0.16 mL) dissolved in tetrahydrofuran (1 mL). The solution is stirred at -78 °C for 1 h and then quenched by the additon of aqueous saturated NH₄Cl solution. The aqueous mixture is extracted with ethyl acetate, and the combined extract is concentrated to give the crude intermediate (0.52 g) that is taken up in ethanol (5 mL). The solution is cooled in an ice bath and NaBH₄ (58 mg) is added. The cooling bath is removed, and the resulting mixture is stirred at room temperature overnight. Saturated aqueous NaHCO₃ solution is added, and the resulting mixture is stirred for 1 h. The mixture is extracted with tert-butyl methyl ether, and the combined extract is dried (MgSO₄) and concentrated. The residue is chromatographed on silica gel (cyclohexane/ethyl acetate 4:1→1:1) to give the title compound as a colorless oil. Yield: 0.20 g (40% of theory); Mass spectrum (ESI⁻): m/z = 327 [M-H]⁻.

### Step 3: tert-butyl-[4-(2,2-difluoro-1-methoxy-ethyl)-indan-1-yloxy]-dimethyl-silane

Sodium hydride (60% in mineral oil; 22 mg) is added to a solution of 1-[1-(tert-butyldimethyl-silanyloxy)-indan-4-yl]-2,2-difluoro-ethanol (0.18 g) in tetrahydrofuran (1 mL) at room temperature. The mixture is stirred for 30 min prior to the addition of methyl iodide (2 mol/L in tert-butyl methyl ether; 0.41 mL). The mixture is stirred at room temperature overnight. Water is added and the resulting mixture is extracted with ethyl acetate. The combined extract is dried (MgSO₄) and concentrated to give the title compound as an oil. Yield: 0.19 g (crude); LC (method 1): t_{R} = 1.52 min.

### Step 4: 4-(2,2-difluoro-1-methoxy-ethyl)-indan-1-ol

Tetrabutylammonium fluoride (1 mol/L in tetrahydrofuran; 1.6 mL) is added to a solution of tert-butyl-[4-(2,2-difluoro-1-methoxy-ethyl)-indan-1-yloxy]-dimethyl-silane (0.18 g) in tetrahydrofuran (1 mL) chilled in an ice bath. The solution is stirred with cooling for 3 h and then quenched by the addition of water. The resulting mixture is extracted with ethyl acetate and the combined extract is dried (MgSO₄). The solvent is evaporated and the residue is chromatographed on silica gel (cyclohexane/ethyl acetate 9:1→1:9) to give the title compound as a mixture of diastereomers. Yield: 80 mg (67% of theory); LC (method 2): t_{R} = 1.14/1.15 min; Mass spectrum (ESI⁺): m/z = 211 [M+H-H₂O]⁺.

### Step 5: {6-[4-(2,2-difluoro-1-methoxy-ethyl)-indan-1-yloxy]-2,3-dihydro-benzofuran-3-yl}-acetic acid methyl ester

The title compound is prepared from 4-(2,2-difluoro-1-methoxy-ethyl)-indan-1-ol and (6-hydroxy-2,3-dihydro-benzofuran-3-yl)-acetic acid methyl ester following a procedure analogous to that described in Step 3 of Intermediate 1. Yield: 68% of theory; LC (method 1): t_{R} = 1.31 min; Mass spectrum (ESI⁺): m/z = 419 [M+H]⁺.

### Intermediate 8

### {6-[(R)-4-(Tetrahydro-pyran-4-yl)-indan-1-yloxy]-2,3-dihydro-benzofuran-3-yl}-acetic acid methyl ester

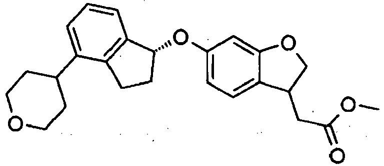

A mixture of {6-[(*R*)-4-(3,6-dihydro-2H-pyran-4-yl)-inden-1-yloxy]-2,3-dihydro-benzofuran-3-yl}-acetic acid methyl ester (70 mg), PtO₂ (20 mg), and ethyl acetate (5 mL) is shaken under hydrogen atmosphere (3 bar) at room temperature for 8 h. The catalyst is separated by filtration and the filtrate is concentrated to give the crude title compound that is used without further purification. Yield: 70 mg (quantitative); Mass spectrum (ESI⁺): m/z = 409 [M+H]⁺.

### Intermediate 9

### {6-[4-(1-Methoxy-2,2-dimethyl-propyl)-indan-1-yloxy]-2,3-dihydro-benzofuran-3-yl}-acetic acid methyl ester

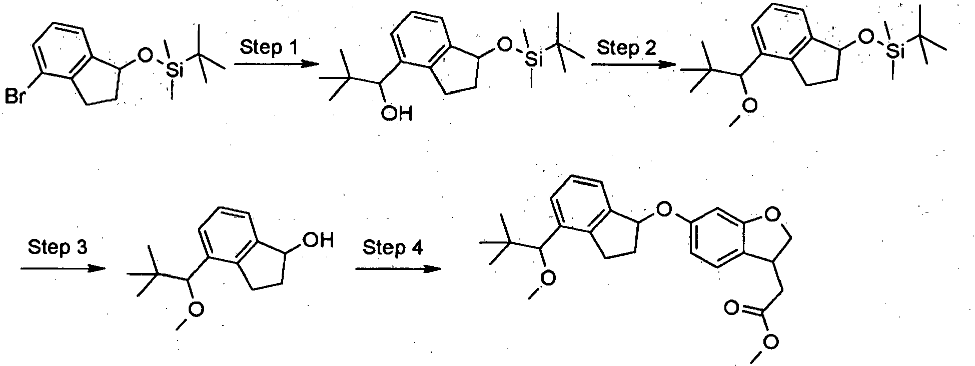

### Step 1: 1-[1-(tert-butyl-dimethyl-silanyloxy)-indan-4-yl]-2,2-dimethyl-propan-1-ol

The title compound is prepared from (4-bromo-indan-1-yloxy)-tert-butyl-dimethylsilane and pivaldehyde following a procedure analogous to that described in Step 2 of Intermediate 7 and obtained as a mixture of two diastereomers; since pivaldehyde is used as the electrophile subsequent reduction as described for Intermediate 7, step 2 is omitted. Yield: 35% of theory; LC (method 1): t_{R} = 1.54/1.56 min; Mass spectrum (ESI⁺): m/z = 352 [M+NH₄]⁺.

### Step 2: tert-butyl-[4-(1-methoxy-2,2-dimethyl-propyl)-indan-1-yloxy]-dimethyl-silane

The title compound is prepared from 1-[1-(tert-butyl-dimethyl-silanyloxy)-indan-4-yl]-2,2-dimethyl-propan-1-ol following a procedure analogous to that described in Step 3 of Intermediate 7 and obtained as a mixture of two diastereomers. Yield: quantitative (crude product).

### Step 3: 4-(1-methoxy-2,2-dimethyl-propyl)-indan-1-ol

The title compound is prepared from tert-butyl-[4-(1-methoxy-2,2-dimethyl-propyl)-indan-1-yloxy]-dimethyl-silane following a procedure analogous to that described in Step 4 of Intermediate 7 and obtained as a mixture of two diastereomers. Yield: 50% of theory; Mass spectrum (ESI⁺): m/z = 217 [M+H-H₂O]⁺.

### Step 4: {6-[4-(1-methoxy-2,2-dimethyl-propyl)-indan-1-yloxy]-2,3-dihydro-benzofuran-3-yl}-acetic acid methyl ester

The title compound is prepared from 4-(1-methoxy-2,2-dimethyl-propyl)-indan-1-ol and (6-hydroxy-2,3-dihydro-benzofuran-3-yl)-acetic acid methyl ester following a procedure analogous to that described in Step 3 of Intermediate 1; the product is obtained as a mixture of diastereomers. Yield: 46% of theory; Mass spectrum (ESI⁺): m/z = 425 [M+H]⁺.

### Intermediate 10

### {(S)-6-[4-(Cyclobutyl-methoxy-methyl)-indan-1-yloxy]-2,3-dihydro-benzofuran-3-yl}-acetic acid methyl ester

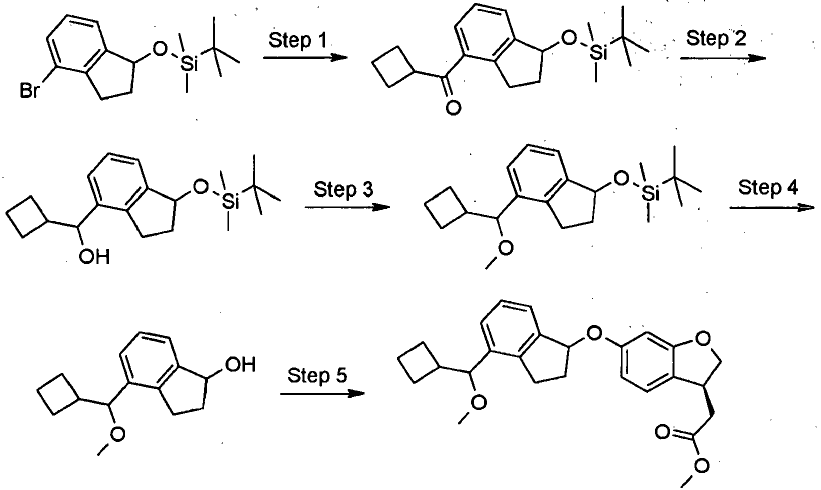

### Step 1: [1-(tert-butyl-dimethyl-silanyloxy)-indan-4-yl]-cyclobutyl-methanone

n-Butyl lithium (1.6 mol/L in hexane, 0.96 mL) is added to a solution of (4-bromo-indan-1-yloxy)-tert-butyl-dimethyl-silane (0.50 g) in tetrahydrofuran (5 mL) at -78 °C. The resulting solution is stirred at -78 °C for 20 min prior to the addition of cyclobutanecarboxylic acid methoxy-methyl-amide (0.22 g) dissolved in tetrahydrofuran (1 mL). The solution is stirred at -78 °C for 1 h and then warmed in the cooling bath to room temperature overnight. Aqueous saturated NH₄Cl solution is added and resulting mixture is extracted with ethyl acetate. The combined extract is concentrated and the residue is chromatographed on silica gel (cyclohexane/ethyl acetate 9:1→7:3) to give the title compound. Yield: 0.38 g (75% of theory); LC (method 1): t_{R} = 1.58 min; Mass spectrum (ESI⁺): m/z = 331 [M+H]⁺.

### Step 2: [1-(tert-butyl-dimethyl-silanyloxy)-indan-4-yl]-cyclobutyl-methanol

A mixture of [1-(tert-butyl-dimethyl-silanyloxy)-indan-4-yl]-cyclobutyl-methanone (0.38 g), NaBH₄ (40 mg), and ethanol (2 mL) is stirred at room temperature overnight. Saturated aqueous NaHCO₃ solution is added, and the resulting mixture is stirred for 1 h. The mixture is extracted with tert-butyl methyl ether, and the combined extract is dried (MgSO₄) and concentrated. The residue is chromatographed on silica gel (cyclohexane/ethyl acetate 9:1→1:1) to give the title compound. Yield: 0.11 g (29% of theory); Mass spectrum (ESI⁺): m/z = 350 [M+NH₄]⁺.

### Step 3: tert-butyl-[4-(cyclobutyl-methoxy-methyl)-indan-1-yloxy]-dimethyl-silane

The title compound is prepared from [1-(tert-butyl-dimethyl-silanyloxy)-indan-4-yl]-cyclobutyl-methanol following a procedure analogous to that described in Step 3 of Intermediate 7. Yield: 96% of theory (crude product).

### Step 4: 4-(cyclobutyl-methoxy-methyl)-indan-1-ol

The title compound is prepared from tert-butyl-[4-(cyclobutyl-methoxy-methyl)-indan-1-yloxy]-dimethyl-silane following a procedure analogous to that described in Step 4 of Intermediate 7. Yield: 75% of theory; LC (method 1): t_{R} = 1.56 min; Mass spectrum (ESI⁺): m/z = 215 [M+H-H₂O]⁺.

### Step 5: {(S)-6-[4-(cyclobutyl-methoxy-methyl)-indan-1-yloxy]-2,3-dihydro-benzofuran-3-yl}-acetic acid methyl ester

The title compound is prepared from 4-(cyclobutyl-methoxy-methyl)-indan-1-ol and (*S*)-(6-hydroxy-2,3-dihydro-benzofuran-3-yl)-acetic acid methyl ester following a procedure analogous to that described in Step 3 of Intermediate 1. Yield: 63% of theory; LC (method 3): t_{R} = 1.89 min; Mass spectrum (ESI⁺): m/z = 423 [M+H]⁺.

### Intermediate 11

### 2-(5,5-Dimethyl-cyclopent-1-enyl)-4,4,5,5-tetramethyl-[1,3,2]dioxaborolane

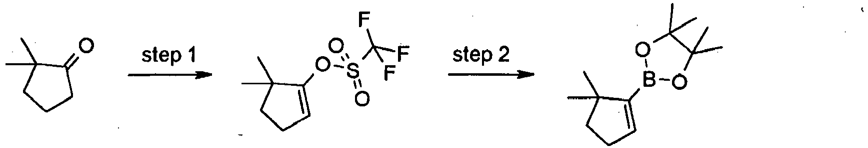

### Step 1: trifluoro-methanesulfonic acid 5,5-dimethyl-cyclopent-1-enyl ester

n-Butyl lithium (1.6 mol/l, 18.4 mL) is added to a solution of diisopropylamine (4.2 mL) in tetrahydrofuran (50 mL) cooled to -78 °C. The solution is stirred at -78 °C for 20 min and then at 0 °C for another 20 min. The solution is cooled to -78 °C, and 2,2-dimethyl-cyclopentanone (3.00 g) is added. The solution is stirred at -78 °C for 3 h prior to the dropwise addition of N-phenyltriflimide (10.00 g) dissolved in tetrahydrofuran (50 mL). The solution is warmed in the cooling bath to room temperature overnight. The solution is diluted with heptane (100 mL) and saturated aqueous Na₂CO₃ solution is added. The organic phase is separated and washed with saturated aqueous Na₂CO₃ solution, water, and brine. The organic phase is dried (MgSO₄) and the solvent is evaporated. The residue is chromatographed on silica gel (cyclohexane/ethyl acetate 9:1→1:1) to give the title compound as an oil. Yield: 1.80 g (28% of theory).

### Step 2: 2-(5,5-dimethyl-cyclopent-1-enyl)-4,4,5,5-tetramethyl-[1,3,2]dioxaborolane

A flask charged with trifluoromethanesulfonic acid 5,5-dimethyl-cyclopent-1-enyl ester (1.70 g), bis(pinacolato)diboron (1.80 g), Pd(PPh₃)₂Cl₂ (0.15 g), PPh₃ (0.17 g), and sodium phenoxide (1.21 g) is purged with argon for 10 min. Toluene (20 mL) is then added, and the resulting mixture is stirred at 50 °C for 2 h. After cooling to room temperature, water is added and the resulting mixture is extracted with ethyl acetate. The combined extract is dried (MgSO₄) and concentrated. The residue is chromatographed on silica gel (cyclohexane/ethyl acetate 4:1→1:2) to give the title compound. Yield: 1.25 g (81 % of theory).

### Intermediate 12

### {6-[(R)-4-(5,5-Dimethyl-cyclopent-1-enyl)-indan-1-yloxy]-2,3-dihydro-benzofuran-3-yl}-acetic acid methyl ester

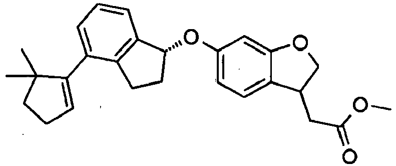

A flask charged with a stir bar, {6-[(*R*)-4-bromo-indan-1-yloxy]-2,3-dihydrobenzofuran-3-yl}-acetic acid methyl ester (0.23 g), 3-(5,5-dimethyl-cyclopent-1-enyl)-1,5-dimethyl-2,4-dioxa-3-bora-bicyclo[3.1.0]hexane (0.18 g), 2-dicyclohexylphosphino-2',6'-dimethoxy-1,1'-biphenyl (45 mg), K₃PO₄ (0.30 g), water (0.15 mL), and N,N-dimethylformamide (3 mL) at room temperature is sparged with argon for 5 min. Palladium(II) acetate (12 mg) is added and the mixture is heated to 75 °C. After stirring at 75 °C overnight, the mixture is cooled to ambient temperature and diluted with water. The resulting mixture is extracted with ethyl acetate, and the combined extract is dried (MgSO₄). The solvent is evaporated and the residue is chromatographed on silica gel (cyclohexane/ethyl acetate 4:1→1:2) to give the title compound as an oil. Yield: 0.12 g (50% of theory); LC (method 1): t_{R} = 1.56 min; Mass spectrum (ESI⁺): m/z = 419 [M+H]⁺.

### Intermediate 13

### {(S)-6-[(R)-4-Difluoromethoxy-indan-1-yloxy]-2,3-dihydro-benzofuran-3-yl}-acetic acid methyl ester

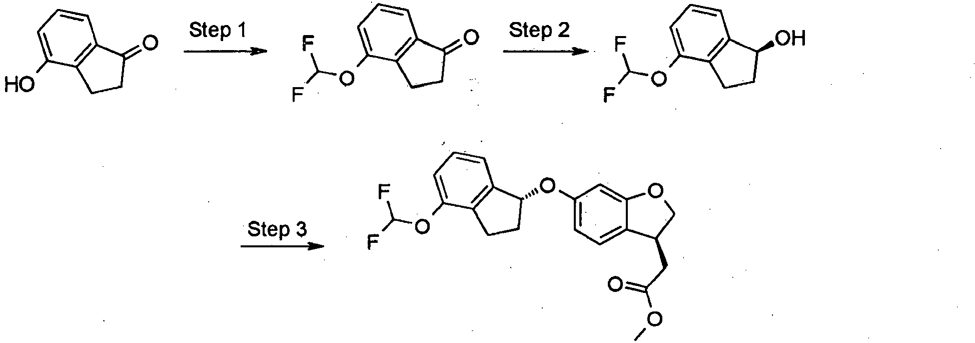

### Step 1: 4-difluoromethoxy-indan-1-one

A mixture of 4-hydroxy-indan-1-one (0.50 g), ethyl 1-bromo-1,1-difluoroacetate (0.65 mL), K₂CO₃ (1.00 g), and N,N-dimethylformamide (5 mL) is stirred at room temperature for 16 h. The mixture is diluted with ethyl acetate and the organic phase is washed with 1 M aqueous HCl solution and brine. The organic phase is dried (MgSO₄) and concentrated. The residue is chromatographed on silica gel (cyclohexane/ethyl acetate 20:1→7:3) to give difluoro-(1-oxo-indan-4-yloxy)-acetic acid ethyl ester (0.21 g) which is taken up in methanol (3 mL). 4 M aqueous NaOH solution (0.25 mL) is added and the mixture is stirred at room temperature for 1 h. 4 M aqueous HCl solution (0.3 mL) is added and the solution is stirred at room temperature for 1 h. The solution is extracted with ethyl acetate and the combined extract is dried (MgSO₄) and concentrated to give the title compound as an oil. Yield: 0.18 g (26% of theory).

### Step 2: (S)-4-difluoromethoxy-indan-1-ol

Formic acid (0.12 mL) is added to a solution of triethylamine (0.39 mL) in dichloromethane (5 mL) chilled in an ice bath. 4-Difluoromethoxy-indan-1-one (0.18 g) is added and the flask is purged with argon. Chloro{[(1S,2S)-(-)-2-amino-1,2-diphenylethyl](4-toluenesulfonyl)amido}-(mesitylene)ruthenium(II) (30 mg; alternatively, the catalyst is formed in situ from N-[(1S,2S)-2-amino-1,2-diphenylethyl]-4-methylbenzenesulfonamide and dichloro(p-cymene)-ruthenium(II) dimer) is added and the mixture is stirred at room temperature for 16 h. Water is added and the resulting mixture is extracted with dichloromethane. The combined extract is washed with saturated aqueous NaHCO₃ solution and dried (MgSO₄). The solvent is evaporated and the residue is chromatographed on silica gel (cyclohexane/ethyl acetate 9:1→1:1) to give the title compound. Yield: 70 mg (39% of theory); LC (method 1): t_{R} = 0.93 min; Mass spectrum (ESI⁺): m/z = 183 [M+H-H₂O]⁺.

### Step 3: {(S)-6-[(R)-4-difluoromethoxy-indan-1-yloxy]-2,3-dihydro-benzofuran-3-yl}-acetic acid methyl ester

The title compound is prepared from (*S*)-4-difluoromethoxy-indan-1-ol and (*S*)-(6-hydroxy-2,3-dihydro-benzofuran-3-yl)-acetic acid methyl ester following a procedure analogous to that described in Step 3 of Intermediate 1. Yield: 52% of theory; LC (method 3): t_{R} = 1.36 min; Mass spectrum (ESI⁺): m/z = 391 [M+H]⁺.

### Intermediate 14

### {(S)-6-[(R)-4-(1-Methoxy-cyclobutyl)-indan-1-yloxy]-2,3-dihydro-benzofuran-3-yl}-acetic acid methyl ester

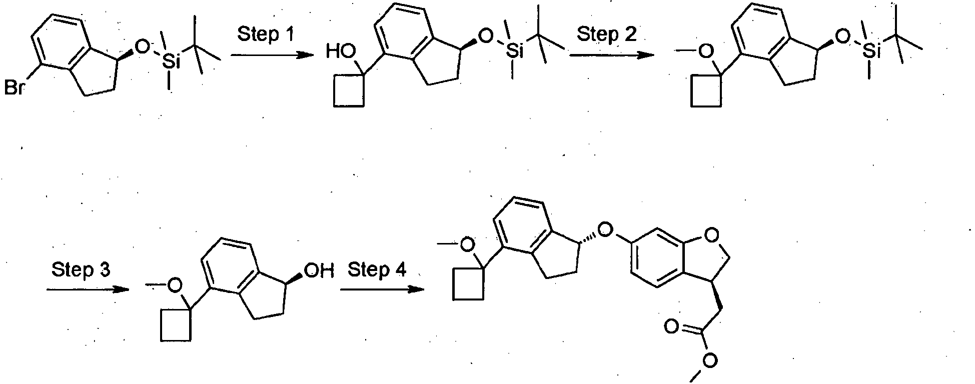

### Step 1: 1-[(S)-1-(tert-butyl-dimethyl-silanyloxy)-indan-4-yl]-cyclobutanol

The title compound is prepared from [(*S*)-4-bromo-indan-1-yloxy]-tert-butyl-dimethylsilane (prepared in analogy to the compound in Intermediate 7/step 1 from (S)-4-bromo-indan-1-ol which, in turn, is obtained as described in WO 2009157418 or WO 8908096) and cyclobutanone following a procedure analogous to that described in Step 2 of Intermediate 7; since a ketone is used as the electrophile subsequent reduction as described for Intermediate 7, step 2 is omitted. TLC: r_{f} = 0.30 (silicagel, cyclohexane/ethyl acetate 4:1); Mass spectrum (ESI⁺): m/z = 336 [M+NH₄]⁺.

### Step 2: (S)-tert-butyl-[4-(1-methoxy-cyclobutyl)-indan-1-yloxy]-dimethyl-silane

The title compound is prepared from 1-[(*S*)-1-(tert-butyl-dimethyl-silanyloxy)-indan-4-yl]-cyclobutanol following a procedure analogous to that described in Step 3 of Intermediate 7. TLC: r_{f} = 0.85 (silicagel, cyclohexane/ethyl acetate 4:1).

### Step 3: (S)-4-(1-methoxy-cyclobutyl)-indan-1-ol

The title compound is prepared from (*S*)-tert-butyl-[4-(1-methoxy-cyclobutyl)-indan-1-yloxy]-dimethyl-silane following a procedure analogous to that described in Step 4 of Intermediate 7. Yield: 75% of theory; LC (method 3): t_{R} = 1.07 min; Mass spectrum (ESI⁺): m/z = 201 [M+H-H₂O]⁺.

### Step 4: {(S)-6-[(R)-4-(1-methoxy-cyclobutyl)-indan-1-yloxy]-2,3-dihydro-benzofuran-3-yl}-acetic acid methyl ester

The title compound is prepared from (*S*)-4-(1-methoxy-cyclobutyl)-indan-1-ol and (S)-(6-hydroxy-2,3-dihydro-benzofuran-3-yl)-acetic acid methyl ester following a procedure analogous to that described in Step 3 of Intermediate 1. LC (method 1): t_{R} = 1.42 min; Mass spectrum (ESI⁺): m/z = 431 [M+Na]⁺.

### Intermediate 15

### {(S)-6-[(R)-6-Trifluoromethyl-indan-1-yloxy]-2,3-dihydro-benzofuran-3-yl}-acetic acid methyl ester

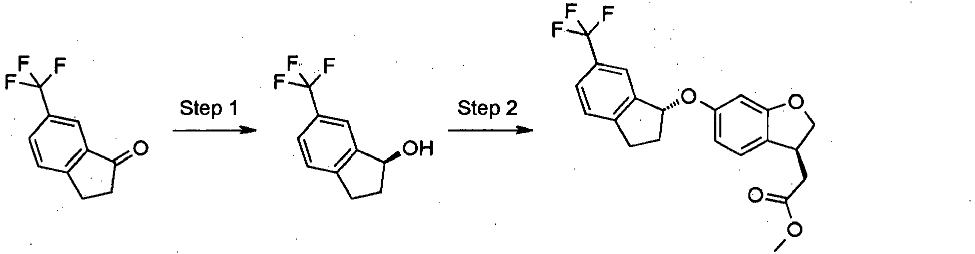

### Step 1: (S)-6-trifluoromethyl-indan-1-ol

The title compound is prepared from 6-trifluoromethyl-indan-1-one following a procedure analogous to that described in Step 2 of Intermediate 13. Yield: 92% of theory; LC (method 4): t_{R} = 1.72 min; Mass spectrum (ESI⁺): m/z = 185 [M-OH]⁺. Step 2: {(*S*)-6-[(*R*)-6-trifluoromethyl-indan-1-yloxy]-2,3-dihydro-benzofuran-3-yl}-acetic acid methyl ester

The title compound is prepared from (*S*)-6-trifluoromethyl-indan-1-ol and (*S*)-(6-hydroxy-2,3-dihydro-benzofuran-3-yl)-acetic acid methyl ester following a procedure analogous to that described in Step 3 of Intermediate 1. Yield: 52% of theory; LC (method 4): t_{R} = 2.12 min; Mass spectrum (ESI⁻): m/z = 391 [M-H]⁻.

### Intermediate 16

### {(S)-6-[(R)-5-Trifluoromethyl-indan-1-yloxy]-2,3-dihydro-benzofuran-3-yl}-acetic acid methyl ester

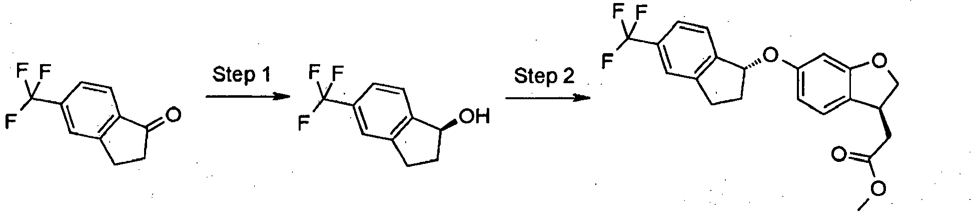

### Step 1: (S)-5-trifluoromethyl-indan-1-ol

The title compound is prepared from 5-trifluoromethyl-indan-1-one following a procedure analogous to that described in Step 2 of Intermediate 13. Yield: 84% of theory; LC (method 4): t_{R} = 1.76 min; Mass spectrum (ESI⁺): m/z = 185 [M-OH]⁺.

### Step 2: {(S)-6-[(R)-5-trifluoromethyl-indan-1-yloxy]-2,3-dihydro-benzofuran-3-yl}-acetic acid methyl ester

The title compound is prepared from (*S*)-5-trifluoromethyl-indan-1-ol and (*S*)-(6-hydroxy-2,3-dihydro-benzofuran-3-yl)-acetic acid methyl ester following a procedure analogous to that described in Step 3 of Intermediate 1. Yield: 52% of theory; LC (method 4): t_{R} = 2.14 min; Mass spectrum (ESI⁻): m/z = 391 [M-H]⁻.

### Intermediate 17

### {(S)-6-[(R)-5-Chloro-indan-1-yloxy]-2,3-dihydro-benzofuran-3-yl}-acetic acid methyl ester

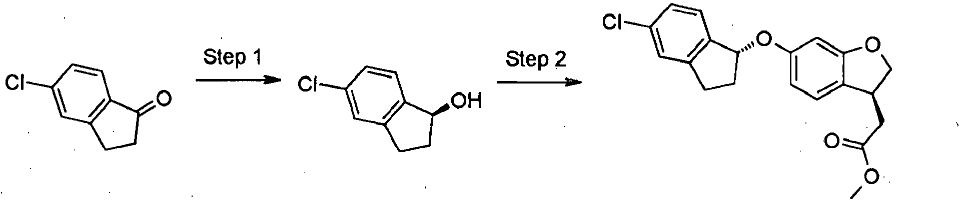

### Step 1: (S)-5-chloro-indan-1-ol

The title compound is prepared from 5-chloro-indan-1-one following a procedure analogous to that described in Step 2 of Intermediate 13. Yield: 87% of theory; LC (method 4): t_{R} = 1.64 min; Mass spectrum (ESI⁺): m/z = 151/153 (CI) [M-OH]⁺.

### Step 2: {(S)-6-[(R)-5-chloro-indan-1-yloxy]-2,3-dihydro-benzofuran-3-yl}-acetic acid methyl ester

The title compound is prepared from (*S*)-5-chloro-indan-1-ol and (*S*)-(6-hydroxy-2,3-dihydro-benzofuran-3-yl)-acetic acid methyl ester following a procedure analogous to that described in Step 3 of Intermediate 1. Yield: 45% of theory; LC (method 4): t_{R} = 2.11 min; Mass spectrum (ESI⁻): m/z = 357/359 (CI) [M-H]⁻.

### Intermediate 18

### {(S)-6-[(R)-4-Chloro-indan-1-yloxy]-2,3-dihydro-benzofuran-3-yl}-acetic acid methyl ester

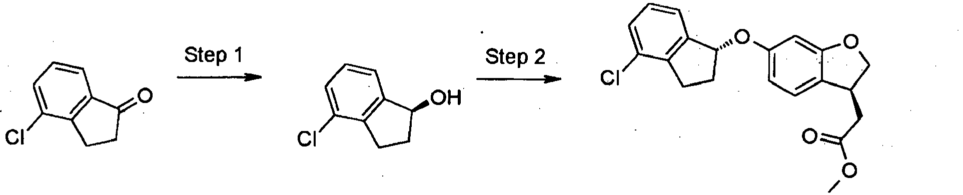

### Step 1: (S)-4-chloro-indan-1-ol

The title compound is prepared from 4-chloro-indan-1-one following a procedure analogous to that described in Step 2 of :Intermediate 13. Yield: 82% of theory; LC (method 4): t_{R} = 1.64 min; Mass spectrum (ESI⁺): m/z = 151/153 (CI) [M-OH]⁺.

### Step 2: {(S)-6-[(R)-4-chloro-indan-1-yloxy]-2,3-dihydro-benzofuran-3-yl}-acetic acid methyl ester

The title compound is prepared from (*S*)-4-chloro-indan-1-ol and (*S*)-(6-hydroxy-2,3-dihydro-benzofuran-3-yl)-acetic acid methyl ester following a procedure analogous to that described in Step 3 of Intermediate 1. Yield: 48% of theory; LC (method 4): t_{R} = 2.13 min; Mass spectrum (ESI⁻): m/z = 357/359 (CI) [M-H]⁻.

### Intermediate 19

### {(S)-6-[(R)-7-Trifluoromethyl-indan-1-yloxy]-2,3-dihydro-benzofuran-3-yl}-acetic acid methyl ester

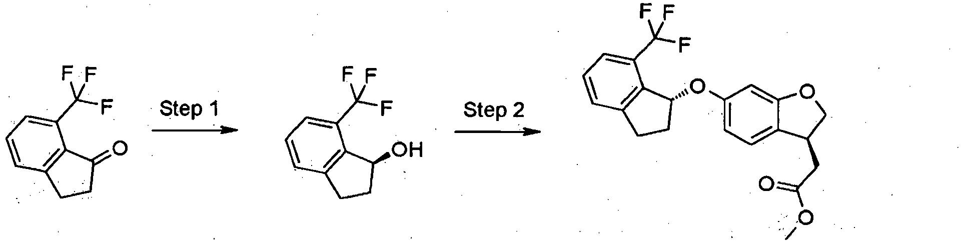

### Step 1: (S)-7-trifluoromethyl-indan-1-ol

The title compound is prepared from 7-trifluoromethyl-indan-1-one following a procedure analogous to that described in Step 2 of Intermediate 13. Yield: 72% of theory; LC (method 4): t_{R} = 1.69 min; Mass spectrum (ESI⁺): m/z = 185 [M-OH]⁺.

### Step 2: {(S)-6-[(R)-7-trifluoromethyl-indan-1-yloxy]-2,3-dihydro-benzofuran-3-yl}-acetic acid methyl ester

The title compound is prepared from (*S*)-7-trifluoromethyl-indan-1-ol and (*S*)-(6-hydroxy-2,3-dihydro-benzofuran-3-yl)-acetic acid methyl ester following a procedure analogous to that described in Step 3 of Intermediate 1. Yield: 48% of theory; LC (method 4): t_{R} = 2.08 min; Mass spectrum (ESI⁺): m/z = 393 [M+H]⁺.

### Intermediate 20

### {(S)-6-[(R)-7-Fluoro-4-trifluoromethyl-indan-1-yloxy]-2,3-dihydro-benzofuran-3-yl}-acetic acid methyl ester

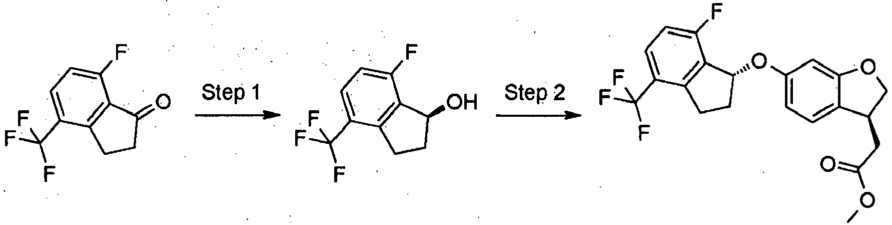

### Step 1: (S)-7-fluoro-4-trifluoromethyl-indan-1-ol

The title compound is prepared from 7-fluoro-4-trifluoromethyl-indan-1-one following a procedure analogous to that described in Step 2 of Intermediate 13. Yield: 70% of theory; LC (method 4): t_{R} = 1.73 min; Mass spectrum (ESI⁺): m/z = 203 [M-OH]⁺.

### Step 2: {(S)-6-[(R)-7-fluoro-4-trifluoromethyl-indan-1-yloxy]-2,3-dihydro-benzofuran-3-yl}-acetic acid methyl ester

The title compound is prepared from (*S*)-7-fluoro-4-trifluoromethyl-indan-1-ol and (S)-(6-hydroxy-2,3-dihydro-benzofuran-3-yl)-acetic acid methyl ester following a procedure analogous to that described in Step 3 of Intermediate 1. Yield: 69% of theory; LC (method 4): t_{R} = 2.10 min; Mass spectrum (ESI⁺): m/z = 411 [M+H]⁺.

### Intermediate 21

### {(S)-6-[(R)-4-Trifluoromethoxy-indan-1-yloxy]-2,3-dihydro-benzofuran-3-yl}-acetic acid methyl ester

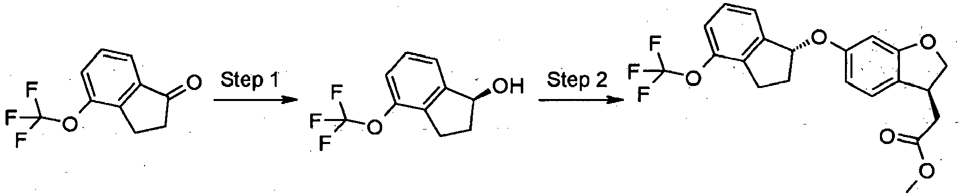

### Step 1: (S)-4-trifluoromethoxy-indan-1-ol

The title compound is prepared from 4-trifluoromethoxy-indan-1-one following a procedure analogous to that described in Step 2 of Intermediate 13. Yield: 82% of theory; LC (method 4): t_{R} = 1.76 min; Mass spectrum (ESI⁺): m/z = 201 [M-OH]⁺.

### Step 2: {(S)-6-[(R)-trifluoromethoxy-indan-1-yloxy]-2,3-dihydro-benzofuran-3-yl}-acetic acid methyl ester

The title compound is prepared from (*S*)-4-trifluoromethoxy-indan-1-ol and (*S*)-(6-hydroxy-2,3-dihydro-benzofuran-3-yl)-acetic acid methyl ester following a procedure analogous to that described in Step 3 of Intermediate 1. Yield: 49% of theory; LC (method 4): t_{R} = 2.14 min; Mass spectrum (ESI⁺): m/z = 409 [M+H]⁺.

### Intermediate 22

### {(S)-6-[(R)-6-Methyl-4-trifluoromethyl-indan-1-yloxy]-2,3-dihydro-benzofuran-3-yl}-acetic acid methyl ester

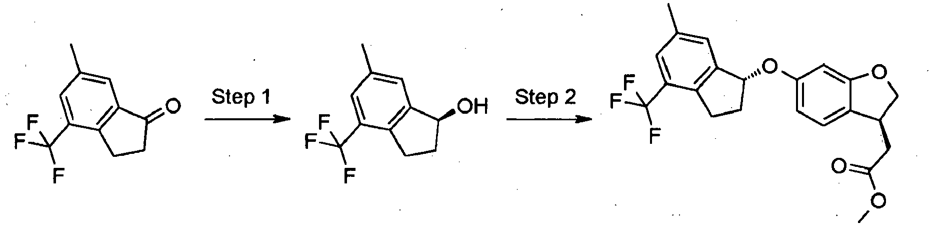

### Step 1: (S)-6-methyl-4-trifluoromethyl-indan-1-ol

The title compound is prepared from 6-methyl-4-trifluoromethyl-indan-1-one following a procedure analogous to that described in Step 2 of Intermediate 13.

### Step 2: {(S)-6-[(R)-6-methyl-4-trifluoromethyl-indan-1-yloxy]-2,3-dihydro-benzofuran-3-yl}-acetic acid methyl ester

The title compound is prepared from (*S*)-6-methyl-4-trifluoromethyl-indan-1-ol and (*S*)-(6-hydroxy-2,3-dihydro-benzofuran-3-yl)-acetic acid methyl ester following a procedure analogous to that described in Step 3 of Intermediate 1. Yield: 39% of theory; LC (method 4): t_{R} = 2.17 min; Mass spectrum (ESI⁺): m/z = 429 [M+Na]⁺.

### Intermediate 23

### {(S)-6-[(R)-4-(1-methoxy-2,2-dimethyl-propyl)-indan-1-yloxy]-2,3-dihydro-benzofuran-3-yl}-acetic acid methyl ester (mixture of 2 diastereomers)

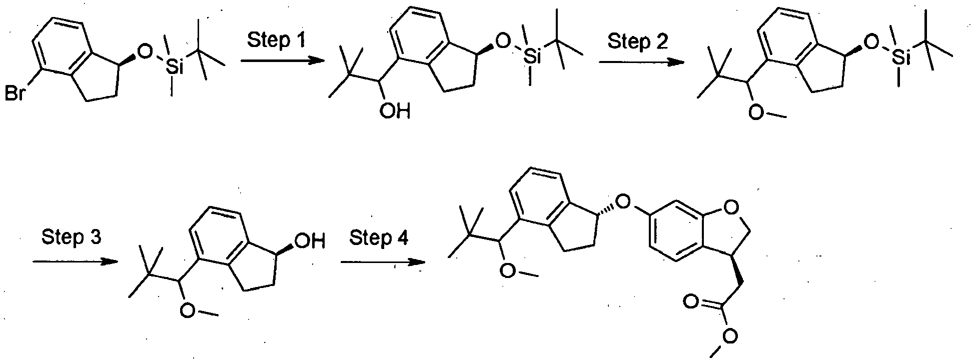

### Step 1: (S)-1-[1-(tert-butyl-dimethyl-silanyloxy)-indan-4-yl]-2,2-dimethyl-propan-1-ol (mixture of 2 diastereomers)

The title compound is prepared from [(*S*)-4-bromo-indan-1-yloxy]-tert-butyl-dimethylsilane (prepared in analogy to the compound in Intermediate 7/step 1 from (S)-4-bromo-indan-1-ol which, in turn, is obtained as described in WO 2009157418 or WO 8908096) and pivaldehyde following a procedure analogous to that described in Step 2 of Intermediate 7; since an aldehyde is used as the electrophile subsequent reduction as described for Intermediate 7, step 2 is omitted. Yield: 89% of theory; LC (method 1): t_{R} = 1.54/1.56 min; Mass spectrum (ESI⁺): m/z = 357 [M+Na]⁺.

### Step 2: tert-butyl-[4-(1-methoxy-2,2-dimethyl-propyl)-indan-1-yloxy]-dimethyl-silane (mixture of 2 diastereomers)

The title compound is prepared from (*S*)-1-[1-(tert-butyl-dimethyl-silanyloxy)-indan-4-yl]-2,2-dimethyl-propan-1-ol (mixture of 2 diastereomers) following a procedure analogous to that described in Step 3 of Intermediate 7. Yield: quantitative (crude product). LC (method 1): t_{R} = 1.65 min.

### Step 3: (S)-4-(1-methoxy-2,2-dimethyl-propyl)-indan-1-ol (mixture of 2 diastereomers)

The title compound is prepared from tert-butyl-[(*S*)-4-(1-methoxy-2,2-dimethyl-propyl)-indan-1-yloxy]-dimethyl-silane (mixture of 2 diastereomers) following a procedure analogous to that described in Step 4 of Intermediate 7. Yield: 61% of theory; LC (method 1): t_{R} = 1.24/1.26 min; Mass spectrum (ESI⁺): m/z = 217 [M+H-H₂O]⁺.

### Step 4: {(S)-6-[(R)-4-(1-methoxy-2,2-dimethyl-propyl)-indan-1-yloxy]-2,3-dihydrobenzofuran-3-yl}-acetic acid methyl ester (mixture of 2 diastereomers)

The title compound is prepared from (*S*)-4-(1-methoxy-2,2-dimethyl-propyl)-indan-1-ol and (*S*)-(6-hydroxy-2,3-dihydro-benzofuran-3-yl)-acetic acid methyl ester following a procedure analogous to that described in Step 3 of Intermediate 1. Yield: 39% of theory; LC (method 1): t_{R} = 1.50 min; Mass spectrum (ESI⁻): m/z = 425 [M+H]⁺.

### Intermediate 24

### {(S)-6-[(R)-7-Methyl-4-trifluoromethyl-indan-1-yloxy]-2,3-dihydro-benzofuran-3-yl}-acetic acid methyl ester

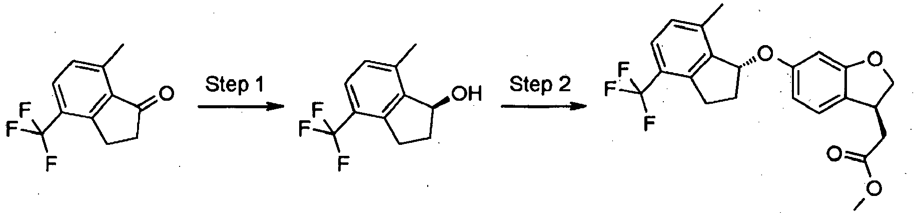

### Step 1: (S)-7-methyl-4-trifluoromethyl-indan-1-ol

The title compound is prepared from 7-methyl-4-trifluoromethyl-indan-1-one following a procedure analogous to that described in Step 2 of Intermediate 13. Yield: 73% of theory; LC (method 4): t_{R} = 1.79 min; Mass spectrum (ESI⁺): m/z = 199 [M-OH]⁺.

### Step 2: {(S)-6-[(R)-7-methyl-4-trifluoromethyl-indan-1-yloxy]-2,3-dihydro-benzofuran-3-yl}-acetic acid methyl ester

The title compound is prepared from (*S*)-7-methyl-4-trifluoromethyl-indan-1-ol and (*S*)-(6-hydroxy-2,3-dihydro-benzofuran-3-yl)-acetic acid methyl ester following a procedure analogous to that described in Step 3 of Intermediate 1. Yield: 39% of theory; LC (method 4): t_{R} = 2.13 min; Mass spectrum (ESI⁻): m/z = 405 [M-H]⁻.

### Intermediate 25

### {(S)-6-[(R)-4-Cyclopropyl-indan-1-yloxy]-2,3-dihydro-benzofuran-3-yl}-acetic acid methyl ester

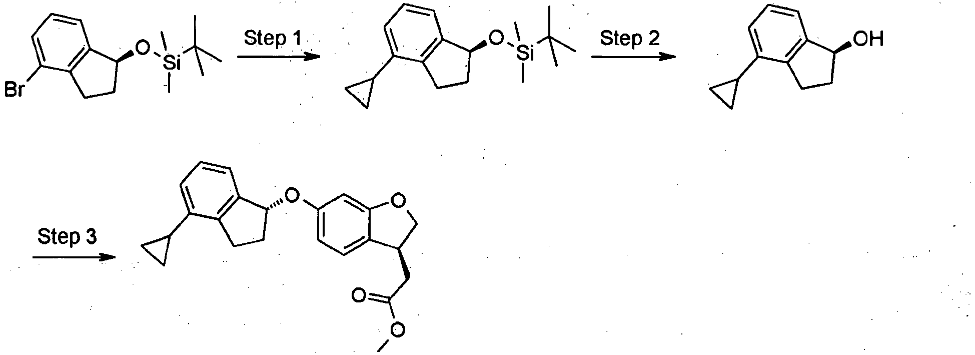

### Step 1: (S)-tert-butyl-(4-cyclopropyl-indan-1-yloxy)-dimethyl-silane

Cyclopropylzinc bromide (0.5 mol/L in tetrahydrofuran; 12 mL) is added slowly to a mixture of [(*S*)-4-bromo-indan-1-yloxy]-tert-butyl-dimethyl-silane (1.00 g), palladium(II) acetate (69 mg), 2-dicyclohexylphosphino-2',6'-dimethoxy-1,1'-biphenyl (0.25 g), and tetrahydrofuran (50 mL) chilled in an ice bath under argon atmosphere. The cooling bath is removed and the resulting mixture is stirred at room temperature overnight. More cyclopropylzinc bromide (0.5 mol/L in tetrahydrofuran; 2 mL) is then added and the same amount is added after an additional day of stirring. After stirring for another 1 h, water is added and the resulting mixture is extracted with ethyl acetate. The combined organic layers are washed with water and brine, dried (Na₂SO₄), and concentrated. The residue is chromatographed on silica gel to afford the title compound as an oil. Yield: 0.72 g (73% of theory); LC (method 4): t_{R} = 2.46 min.

### Step 2: (S)-4-cyclopropyl-indan-1-ol

The title compound is prepared from (*S*)-tert-butyl-(4-cyclopropyl-indan-1-yloxy)-dimethyl-silane following a procedure analogous to that described in Step 4 of Intermediate 7. Yield: 70% of theory; LC (method 4): t_{R} = 1.71 min; Mass spectrum (ESI⁺): m/z = 157 [M+H-H₂O]⁺.

### Step 3: {(S)-6-[(R)-4-cyclopropyl-indan-1-yloxy]-2,3-dihydro-benzofuran-3-yl}-acetic acid methyl ester

The title compound is prepared from (*S*)-4-cyclopropyl-indan-1-ol and (*S*)-(6-hydroxy-2,3-dihydro-benzofuran-3-yl)-acetic acid methyl ester following a procedure analogous to that described in Step 3 of Intermediate 1. Yield: 37% of theory; LC (method 4): t_{R} = 2.12 min; Mass spectrum (ESI⁺): m/z = 387 [M+Na]⁺.

### Intermediate 26

### {(S)-6-[(R)-4-Isopropyl-indan-1-yloxy]-2,3-dihydro-benzofuran-3-yl}-acetic acid methyl ester

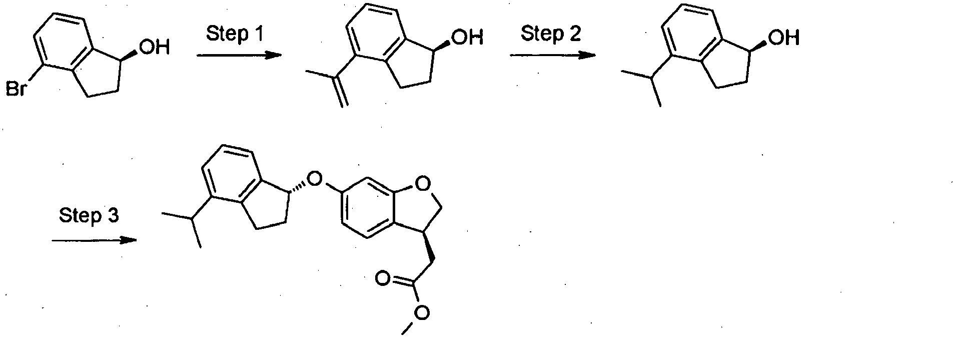

### Step 1: (S)-4-isopropenyl-indan-1-ol

Tetrakis(triphenylphosphine)palladium(0) (0.27 g) is added to a mixture of (*S*)-4-bromo-indan-1-ol (1.00 g; for preparation see WO 2009157418 and WO 8908096), 4,4,5,5-tetramethyl-2-(prop-1-en-2-yl)-1,3,2-dioxaborolane (1.58 g), K₃PO₄ (2.99 g), 1,2-dimethoxyethane (10 mL), and water (5 mL) under argon atmosphere. The mixture is stirred in a microwave oven at 90 °C for 4 h. Water is added and the resulting mixture is extracted with ethyl acetate. The combined organic layer is washed with water and brine, dried (Na₂SO₄) and concentrated. The residue is chromatographed on silica gel (heptane/ethyl acetate). Yield: 0.96 g (quantitative); LC (method 4): t_{R} = 1.73 min; Mass spectrum (ESI⁺): m/z = 157 [M+H-H₂O]⁺.

### Step 2: (S)-4-isopropyl-indan-1-ol

Platinum oxide (0.11 g) is added to (*S*)-4-isopropenyl-indan-1-ol (0.81 g) dissolved in ethyl acetate (40 mL) under Ar atmosphere. Hydrogen gas (balloon pressure) is applied and the resulting mixture is stirred at room temperature for 1 h. The mixture is filtered over celite and the filtrate is concentrated. The residue is chromatographed on silica gel (heptane/ethyl acetate) to afford the title compound as colorless oil. Yield: 0.82 g (87% of theory); LC (method 4): t_{R} = 1.77 min; Mass spectrum (ESI⁺): m/z = 159 [M+H-H₂O]⁺.

### Step 3: {(S)-6-[(R)-4-isopropyl-indan-1-yloxy]-2,3-dihydro-benzofuran-3-yl}-acetic acid methyl ester

The title compound is prepared from (*S*)-4-isopropyl-indan-1-ol and (*S*)-(6-hydroxy-2,3-dihydro-benzofuran-3-yl)-acetic acid methyl ester following a procedure analogous to that described in Step 3 of Intermediate 1. Yield: 33% of theory; LC (method 4): t_{R} = 2.17 min; Mass spectrum (ESI⁻): m/z = 365 [M-H]⁻.

### Intermediate 27

### {(S)-6-[(R)-4-(2,6-Dimethyl-phenyl)-indan-1-yloxy]-2,3-dihydro-benzofuran-3-yl}-acetic acid methyl ester

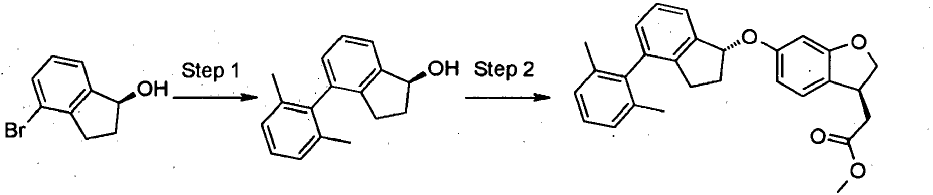

### Step 1: (S)-4-(2,6-dimethyl-phenyl)-indan-1-ol

Tetrakis(triphenylphosphine)palladium(0) (0.14 g) is added to a microwave vial charged with (S)-4-bromo-2,3-dihydro-1H-inden-1-ol (0.50 g), K₂CO₃ (0.97 g), 2,6-dimethylphenylboronic acid (0.70 g), 1,2-dimethoxyethane (10 mL), and water (2.5 mL) under Ar atmosphere. The vial is sealed and the mixture is stirred at 100 °C for 3 h in a microwave oven. The mixture is diluted with water and extracted with ethyl acetate. The combined organic extract is washed with brine, dried (Na₂SO₄), and concentrated. The residue is chromatographed on silica gel (heptane/ethyl acetate) to afford the title compound as an oil which crystallizes upon standing. Yield: 0.15 g (27% of theory); LC (method 4): t_{R} = 1.93 min; Mass spectrum (ESI⁺): m/z = 221 [M+H-H₂O]⁺.

### Step 2: {(S)-6-[(R)-4-(2,6-dimethyl-phenyl)-indan-1-yloxy]-2,3-dihydro-benzofuran-3-yl}-acetic acid methyl ester

The title compound is prepared from (*S*)-4-(2,6-dimethyl-phenyl)-indan-1-ol and (*S*)-(6-hydroxy-2,3-dihydro-benzofuran-3-yl)-acetic acid methyl ester following a procedure analogous to that described in Step 3 of Intermediate 1. Yield: 32% of theory; LC (method 4): t_{R} = 2.23 min; Mass spectrum (ESI⁺): m/z = 451 [M+Na]⁺.

### Intermediate 28

### {(S)-6-[(R)-4-(2,2-Dimethyl-propyl)-indan-1-yloxy]-2,3-dihydro-benzofuran-3-yl}-acetic acid methyl ester

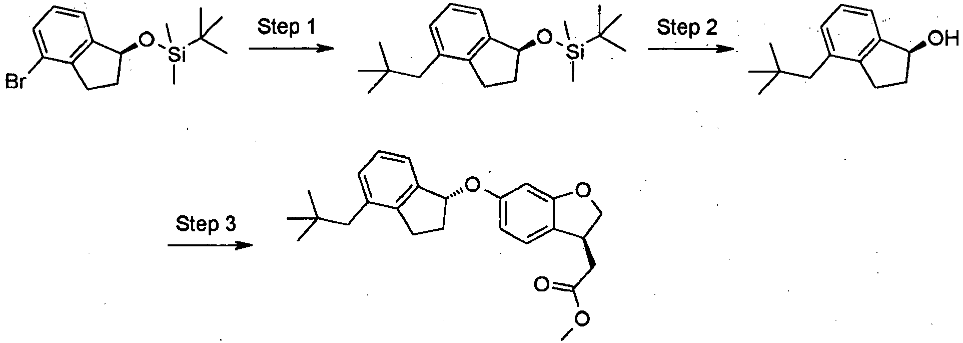

### Step 1: (S)-tert-butyl-[4-(2,2-dimethyl-propyl)-indan-1-yloxy]-dimethyl-silane

Neopentylzinc bromide (0.5 mol/L, 12 mL) is added slowly to a mixture of [(*S*)-4-bromo-indan-1-yloxy]-tert-butyl-dimethyl-silane (1.00 g), palladium(II) acetate (0.07 g), 2-dicyclohexylphosphino-2',6'-dimethoxy-1,1'-biphenyl (0.25 g), and tetrahydrofuran (50 mL) chilled in an ice bath under argon atmosphere. The cooling bath is removed and the resulting mixture is stirred at room temperature for 2 h. Water is added and the resulting mixture is extracted with ethyl acetate. The combined organic layer is washed with water and brine, dried (Na₂SO₄), and concentrated. The residue is chromatographed on silica gel (heptane/ethyl acetate) to afford the title compound as colorless oil. Yield: 0.88 g (86% of theory); LC (method 4): t_{R} = 2.70 min; Mass spectrum (ESI⁺): m/z = 187 [M-OSiMe₂tBu]⁺.

### Step 2: (S)-4-(2,2-dimethyl-propyl)-indan-1-ol

The title compound is prepared from (*S*)-tert-butyl-[4-(2,2-dimethyl-propyl)-indan-1-yloxy]-dimethyl-silane following a procedure analogous to that described in Step 4 of Intermediate 7. Yield: 87% of theory; LC (method 4): t_{R} = 1.94 min; Mass spectrum (ESI⁺): m/z = 187 [M+H-H₂O]⁺.

### Step 3: {(S)-6-[(R)-4-(2,6-dimethyl-phenyl)-indan-1-yloxy]-2,3-dihydro-benzofuran-3-yl}-acetic acid methyl ester

The title compound is prepared from (*S*)-4-(2,2-dimethyl-propyl)-indan-1-ol and (S)-(6-hydroxy-2,3-dihydro-benzofuran-3-yl)-acetic acid methyl ester following a procedure analogous to that described in Step 3 of Intermediate 1. Yield: 32% of theory; LC (method 4): t_{R} = 2.26 min; Mass spectrum (ESI⁺): m/z = 417 [M+Na]⁺.

### Intermediate 29

### {(S)-6-[(R)-4-(2-Isopropyl-phenyl)-indan-1-yloxy]-2,3-dihydro-benzofuran-3-yl}-acetic acid methyl ester

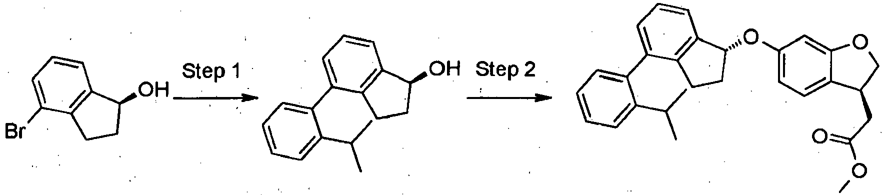

### Step 1: (S)-4-(2-isopropyl-phenyl)-indan-1-ol

The title compound is prepared from (*S*)-4-bromo-indan-1-ol and (2-isopropylphenyl)boronic acid following a procedure analogous to that described in Step 1 of Intermediate 27. Yield: 97% of theory; LC (method 4): t_{R} = 1.96 min; Mass spectrum (ESI⁺): m/z = 235 [M-OH]⁺.

### Step 2: {(S)-6-[(R)-4-(2-isopropyl-phenyl)-indan-1-yloxy]-2,3-dihydro-benzofuran-3-yl}-acetic acid methyl ester

The title compound is prepared from (*S*)-4-(2-isopropyl-phenyl)-indan-1-ol and (*S*)-(6-hydroxy-2,3-dihydro-benzofuran-3-yl)-acetic acid methyl ester following a procedure analogous to that described in Step 3 of Intermediate 1. Yield: 54% of theory; LC (method 4): t_{R} = 2.24 min; Mass spectrum (ESI⁺): m/z = 465 [M+Na]⁺.

### Intermediate 30

### {(S)-6-[(R)-5-Methyl-4-trifluoromethyl-indan-1-yloxy]-2,3-dihydro-benzofuran-3-yl}-acetic acid methyl ester

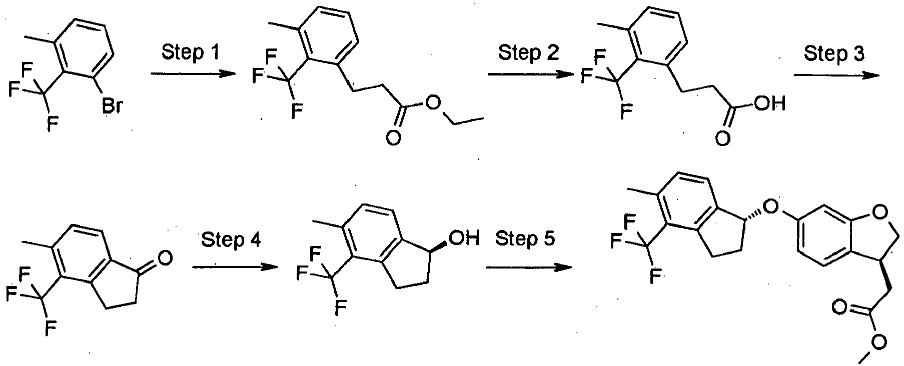

### Step 1: 3-(3-methyl-2-trifluoromethyl-phenyl)-propionic acid ethyl ester

1-Bromo-3-methyl-2-(trifluoromethyl)benzene (0.17 mL), N,N-diisopropylethylamine (0.36 mL), and acrolein diethyl acetal (0.48mL) are added to a flask charged with a stir bar, tetrabutylammonium chloride (0.29 g), and dry N,N-dimethylformamide (5 mL) under Ar atmosphere. The mixture is purged with Ar for 5 min prior to the addition of palladium(II) acetate (2.4 mg). The flask is placed in a preheated (90 °C) oil bath and the mixture is stirred for 10 min. After cooling to room temperature, the mixture is poured into 2 N aqueous HCl (30 mL) and the resulting mixture is extracted with Et₂O. The combined organic extract is washed with water and brine, dried (Na₂SO₄), and concentrated. The residue is chromatographed (heptane/ethyl acetate) to give the title compound. Yield: 0.11 g (39% of theory).

### Step 2: 3-(3-methyl-2-trifluoromethyl-phenyl)-propionic acid

The title compound is prepared from 3-(3-methyl-2-trifluoromethyl-phenyl)-propionic acid ethyl ester following a procedure analogous to that described in Example 1. Yield: 94% of theory.

### Step 3: 5-methyl-4-trifluoromethyl-indan-1-one

3-[3-Methyl-2-(trifluoromethyl)phenyl]propanoic acid (0.61 g) dissolved in trifluoromethanesulfonic acid (5.8 ml) is stirred at 60 °C for 2 h. After cooling to room temperature, the mixture is poured into water (500 mL) and the resulting mixture is extracted with diethyl ether. The combined organic extract is washed with brine and concentrated. The residue is chromatographed on silica gel (heptane/ethyl acetate) to afford the title compound as a colorless solid. Yield: 0.31 g (56% of theory).

### Step 4: (S)-5-methyl-4-trifluoromethyl-indan-1-ol

The title compound is prepared from 5-methyl-4-trifluoromethyl-indan-1-one following a procedure analogous to that described in Step 2 of Intermediate 13. Yield: 70% of theory; LC (method 4): t_{R} = 1.80 min; Mass spectrum (ESI⁺): m/z = 199 [M-OH]⁺.

### Step 5: {(S)-6-[(R)-5-methyl-4-trifluoromethyl-indan-1-yloxy]-2,3-dihydro-benzofuran-3-yl}-acetic acid methyl ester

The title compound is prepared from (*S*)-5-methyl-4-trifluoromethyl-indan-1-ol and (*S*)-(6-hydroxy-2,3-dihydro-benzofuran-3-yl)-acetic acid methyl ester following a procedure analogous to that described in Step 3 of Intermediate 1. Yield: 54% of theory; LC (method 4): t_{R} = 2.14 min; Mass spectrum (ESI⁻): m/z = 405 [M-H]⁻.

### Intermediate 31

### {(S)-6-[(R)-4,5-difluoro-indan-1-yloxy]-2,3-dihydro-benzofuran-3-yl}-acetic acid methyl ester

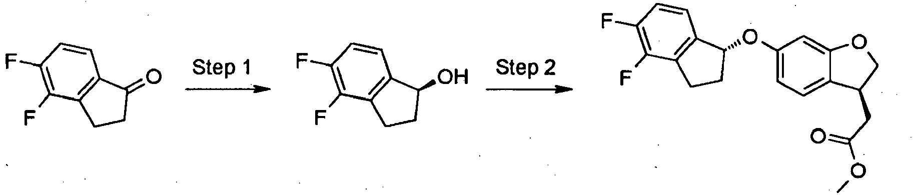

### Step 1: (S)-4,5-difluoro-indan-1-ol

The title compound is prepared from 4,5-difluoro-indan-1-one following a procedure analogous to that described in Step 2 of Intermediate 13. Yield: 87% of theory; LC (method 4): t_{R} = 1.62 min; Mass spectrum (ESI⁺): m/z = 153 [M-OH]⁺.

### Step 2: {(S)-6-[(R)-4,5-difluoro-indan-1-yloxy]-2-,3-dihydro-benzofuran-3-yl}-acetic acid methyl ester

The title compound is prepared from (*S*)-4,5-difluoro-indan-1-ol and (*S*)-(6-hydroxy-2,3-dihydro-benzofuran-3-yl)-acetic acid methyl ester following a procedure analogous to that described in Step 3 of Intermediate 1. Yield: 60% of theory; LC (method 4): t_{R} = 2.01 min; Mass spectrum (ESI⁻): m/z = 359 [M-H]⁻.

### Intermediates 32 and 33

### (S)-4-[(R)-2,2,2-Trifluoro-1-methoxy-ethyl]-indan-1-ol and (S)-4-[(S)-2,2,2-Trifluoro-1-methoxy-ethyl]-indan-1-ol

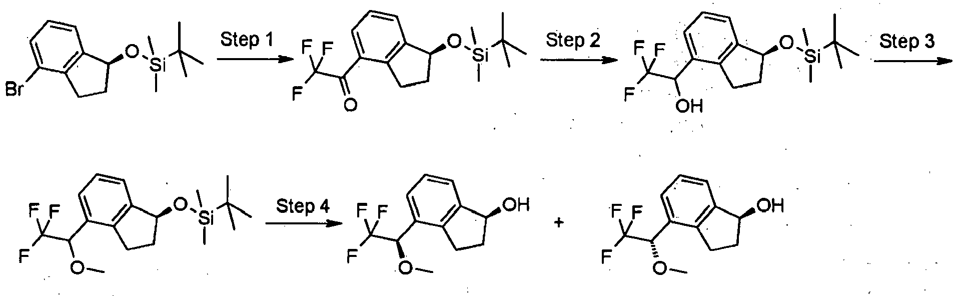

### Step 1: 1-[(S)-1-(tert-butyl-dimethyl-silanyloxy)-indan-4-yl]-2,2,2-trifluoro-ethanone

n=Butyl lithium (2.5 mol/L in hexane, 0.92 mL) is added slowly to a solution of [(*S*)-4-bromo-indan-1-yloxy]-tert-butyl-dimethyl-silane (0.50 g) in tetrahydrofuran (25 mL) cooled to -78 °C. The solution is stirred for 30 min prior to the dropwise addition of ethyl trifluoroacetate (0.55 mL) dissolved in tetrahydrofuran (5 ml). The mixture is stirred at -78 °C for 30 min and then warmed to room temperature by removing the cooling bath. Water is added and the resulting mixture is extracted with ethyl acetate. The organic layer is washed with brine, dried (Na₂SO₄), and concentrated. The residue is used without further purification in the next step. Yield: 0.52 g (99% of theory); LC (method 4): t_{R} = 2.64 min; Mass spectrum (ESI⁻): m/z = 343 [M-H]⁻.

### Step 2: 1-[(S)-1-(tert-butyl-dimethyl-silanyloxy)-indan-4-yl]-2,2,2-trifluoro-ethanol (mixture of 2 diastereomers)

NaBH₄ (57 mg) is added to a solution of 1-[(*S*)-1-(tert-butyl-dimethyl-silanyloxy)-indan-4-yl]-2,2,2-trifluoro-ethanone (0.52 g) in ethanol (8 mL) cooled to 0 °C. The mixture is stirred at 0 °C for 5 min and then the cooling bath is removed. After stirring the mixture at room temperature for 1 h, water is added and the resulting mixture is extracted with ethyl acetate. The combined organic layer is washed with brine, dried (Na₂SO₄), and concentrated. The residue is chromatographed on silica gel (heptane/ethyl acetate) to afford the title compound. Yield: 0.39 g (75% of theory). LC (method 4): t_{R} = 2.45 min.

### Step 3: tert-butyl-dimethyl-[(S)-4-(2,2,2-trifluoro-1-methoxy-ethyl)-indan-1-yloxy]-silane (mixture of 2 diastereomers)

The title compound is prepared from 1-[(*S*)-1-(tert-butyl-dimethyl-silanyloxy)-indan-4-yl]-2,2,2-trifluoro-ethanol (mixture of 2 diastereomers) following a procedure analogous to that described in Step 3 of Intermediate 7. Yield: 94% of theory. LC (method 4): t_{R} = 2.73 min; Mass spectrum (ESI⁻): m/z = 229 [M-OSiMe₂tBu]⁺.

### Step 4: (S)-4-[(R)-2,2,2-trifluoro-1-methoxy-ethyl]-indan-1-ol and (S)-4-[(S)-2,2,2-trifluoro-1-methoxy-ethyl]-indan-1-ol

The title compounds are prepared from tert-butyl-dimethyl-[(*S*)-4-(2,2,2-trifluoro-1-methoxy-ethyl)-indan-1-yloxy]-silane (mixture of 2 diastereomers) following a procedure analogous to that described in Step 4 of Intermediate 7. The two diastereomers obtained are separated by chromatography on silica gel (heptane/ethyl acetate).
(*S*)-4-[(*R*)*-2,2,2-trifluoro-1-methoxy-ethyl]-indan-1-ol (configuration at stereocenter indicated (*) arbitrarily assigned): LC (method 4): t_{R} = 1.73 min; Mass spectrum (ESI⁺): m/z = 229 [M-OH]⁺;
(*S*)-4-[(*S*)*-2,2,2-trifluoro-1-methoxy-ethyl]-indan-1-ol (configuration at stereocenter indicated (*) arbitrarily assigned): LC (method 4): t_{R} = 1.73 min; Mass spectrum (ESI⁺): m/z = 229 [M-OH]⁺.

### Intermediate 34

### {(S)-6-[(R)-4-((R)*-2,2,2-Trifluoro-1-methoxy-ethyl)-indan-1-yloxy]-2,3-dihydrobenzofuran-3-yl}-acetic acid methyl ester (configuration at the stereocenter indicated (*) arbitrarily assigned)

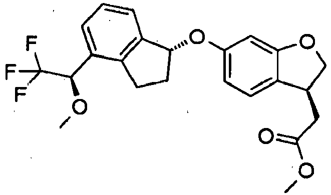

The title compound is prepared from (*S*)-4-[(*R*)*-2,2,2-trifluoro-1-methoxy-ethyl]-indan-1-ol (configuration at the stereocenter indicated (*) arbitrarily assigned) and (*S*)-(6-hydroxy-2,3-dihydro-benzofuran-3-yl)-acetic acid methyl ester following a procedure analogous to that described in Step 3 of Intermediate 1. Yield: 31% of theory; LC (method 4): t_{R} = 2.05 min; Mass spectrum (ESI⁻): m/z = 435 [M-H]⁻.

### Intermediate 35

### {(S)-6-[(R)-4-((S)*-2,2,2-Trifluoro-1-methoxy-ethyl)-indan-1-yloxy]-2,3-dihydrobenzofuran-3-yl}-acetic acid methyl ester (configuration at the stereocenter indicated (*) arbitrarily assigned)

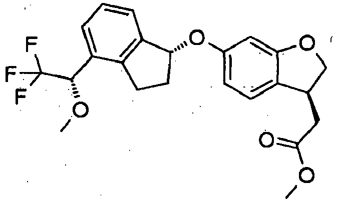

The title compound is prepared from (*S*)-4-[(*S*)*-2,2,2-trifluoro-1-methoxy-ethyl]-indan-1-ol (configuration at the stereocenter indicated (*) arbitrarily assigned) and (*S*)-(6-hydroxy-2,3-dihydro-benzofuran-3-yl)-acetic acid methyl ester following a procedure analogous to that described in Step 3 of Intermediate 1. Yield: 63% of theory; LC (method 4): t_{R} = 2.04 min; Mass spectrum (ESI⁻): m/z = 435 [M-H]⁻.

### Intermediate 36

### (S)-4-(3,3,4-Trimethyl-oxetan-2-yl)-indan-1-ol (2 fractions of different diastereomers)

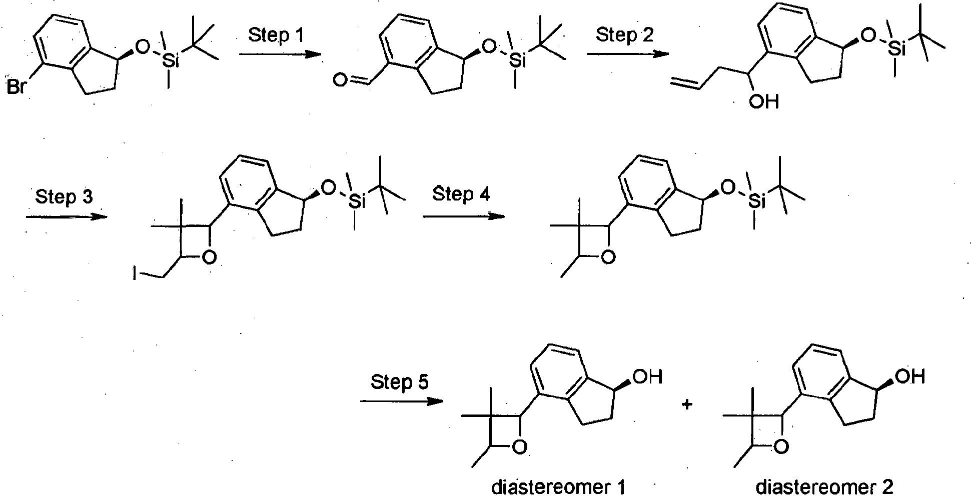

### Step 1: (S)-1-(tert-butyl-dimethyl-silanyloxy)-indan-4-carbaldehyde

n-Butyl lithium (15% in hexane, 1.15 mL) is added to a solution of (*S*)-4-bromo-indan-1-yloxy)-tert-butyl-dimethyl-silane (0.40 g) in tetrahydrofuran (10 mL) cooled to -78 °C. The resulting solution is stirred at -50 °C for 1 h prior to the addition of N,N-dimethylformamide (1 mL). The solution is stirred at -78 °C for 1 h and then quenched by the additon of aqueous saturated NH₄Cl solution. The aqueous mixture is extracted with diethylether and the combined extract is washed with water and dried (Na₂SO₄). The solvent is evaporated to give the crude title compound. Yield: 0.33 g (96% of theory); LC (method 6): t_{R} = 1.64 min; Mass spectrum (ESI⁺): m/z = 277 [M+H]⁺.

### Step 2: (S)-1-[1-(tert-butyl-dimethyl-silanyloxy)-indan-4-yl]-but-3-en-1-ol

1-Bromo-3-methyl-but-2-ene (0.40 mL), NaI (0.52 g), and In powder (0.27 g) are added in the given order to a solution of (*S*)-1-(tert-butyl-dimethyl-silanyloxy)-indan-4-carbaldehyde (0.32 g) in N,N-dimethylformamide (5 mL) at room temperature. The resulting solution is stirred at room temperature for 4 h. Diethylether is added and the resulting mixture is washed with water and aqueous Na₂S₂O₃ solution and dried (Na₂SO₄). The solvent is evaporated to give the crude title compound. Yield: 0.39 g (97% of theory); LC (method 6): t_{R} = 1.78/1.82 min (mixture of diastereomers); Mass spectrum (ESI⁺): m/z = 369 [M+Na]⁺.

### Step 3: (S)-tert-butyl-[4-(4-iodomethyl-3,3-dimethyl-oxetan-2-yl)-indan-1-yloxy]-dimethyl-silane

Li₂CO₃ (0.56 g) and iodine (0.56 g) are added to a solution of (*S*)-1-[1-(tert-butyl-dimethyl-silanyloxy)-indan-4-yl]-but-3-en-1-ol (0.38 g) in acetonitrile (3 mL) at room temperature; The resulting mixture is stirred at room temperature for 72 h. Diethylether is added and the resulting mixture is washed with aqueous Na₂S₂O₃ solution and brine and dried (Na₂SO₄). The solvent is evaporated and the residue is chromatographed on silica gel (petrol ether/ethyl acetate) to give the title compound. Yield: 0.20 g (39% of theory).

### Step 4: (S)-tert-butyl-dimethyl-[4-(3,3,4-trimethyl-oxetan-2-yl)-indan-1-yloxy]-silane

A mixture of (*S*)-tert-butyl-[4-(4-iodomethyl-3,3-dimethyl-oxetan-2-yl)-indan-1-yloxy]-dimethyl-silane (0.19 g), 10% palladium on carbon (30 mg), triethylamine (0.15 mL), and methanol (10 mL) is shaken under hydrogen atmosphere (3 bar) at room temperature for 6 h. The catalyst is separated by filtration and the filtrate is concentrated. The residue is chromatographed on silica gel (petrol ether/ethyl acetate) to give the title compound. Yield: 0.08 g (57% of theory); LC (method 6): t_{R} = 1.84 min; Mass spectrum (ESI⁺): m/z = 369 [M+Na]⁺.

### Step 5: (S)-4-(3,3,4-trimethyl-oxetan-2-yl)-indan-1-ol (2 fractions of different diastereomers)

The title compounds are prepared from (*S*)-tert-butyl-dimethyl-[4-(3,3,4-trimethyl-oxetan-2-yl)-indan-1-yloxy]-silane following a procedure analogous to that described in Step 4 of Intermediate 7. Two fractions with different diastereomers are obtained after chromatography on silica gel (petrol ether/ethyl acetate).
(*S*)-4-(3,3,4-trimethyl-oxetan-2-yl)-indan-1-ol (fraction 1): LC (method 6): t_{R} = 0.48 min.
(*S*)-4-(3,3,4-trimethyl-oxetan-2-yl)-indan-1-ol (fraction 2): LC (method 6): t_{R} = 0.62 min.

### Intermediates 37 and 38

### {(S)-6-[(R)-4-(3,3,4-Trimethyl-oxetan-2-yl)-indan-1-yloxy]-2,3-dihydro-benzofuran-3-yl}-acetic acid methyl ester (2 fractions of different diastereomers)

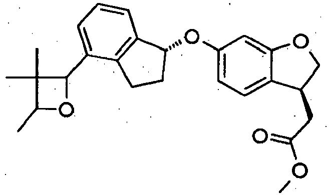

The title compounds are prepared from (*S*)-4-(3,3,4-trimethyl-oxetan-2-yl)-indan-1-ol (Intermediate 36, Step 5, fraction 1; or Intermediate 36, Step 5, fraction 2) and (S)-(6-hydroxy-2,3-dihydro-benzofuran-3-yl)-acetic acid methyl ester following a procedure analogous to that described in Step 3 of Intermediate 1.
Intermediate 37: {(*S*)-6-[(*R*)-4-(3,3,4-trimethyl-oxetan-2-yl)-indan-1-yloxy]-2,3-dihydro-benzofuran-3-yl}-acetic acid methyl ester from fraction 1: LC (method 6): t_{R} = 1.45 min; Mass spectrum (ESI⁺): m/z = 445 [M+Na]⁺.
Intermediate 38: {(*S*)-6-[(*R*)-4-(3,3,4-trimethyl-oxetan-2-yl)-indan-1-yloxy]-2,3-dihydrobenzofuran-3-yl}-acetic acid methyl ester from fraction 2: LC (method 6): t_{R} = 1.43 min; Mass spectrum (ESI⁺): m/z = 445 [M+Na]⁺.

### Intermediate 39

### [(S)-(Indan-1-yloxy)-2,3-dihydro-benzofuran-3-yl]-acetic acid methyl ester

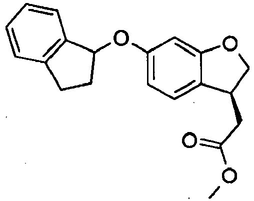

The title compound is prepared from indan-1-ol and (*S*)-(6-hydroxy-2,3-dihydrobenzofuran-3-yl)-acetic acid methyl ester following a procedure analogous to that described in Step 3 of Intermediate 1.

### Intermediate 40

### {(S)-6-[(R)-4-Chloro-5-fluoro-indan-1-yloxy]-2,3-dihydro-benzofuran-3-yl}-acetic acid methyl ester

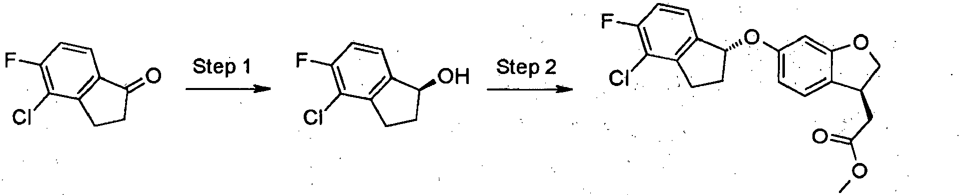

### Step 1: (S)-4-chloro-5-fluoro-indan-1-ol

The title compound is prepared from 4-chloro-5-fluoro-indan-1-one following a procedure analogous to that described in Step 2 of Intermediate 13.

### Step 2: {(S)-6-[(R)-4-chloro-5-fluoro-indan-1-yloxy]-2,3-dihydro-benzofuran-3-yl}-acetic acid methyl ester

The title compound is prepared from (*S*)-4-chloro-5-fluoro-indan-1-ol and (S)-(6-hydroxy-2,3-dihydro-benzofuran-3-yl)-acetic acid methyl ester following a procedure analogous to that described in Step 3 of Intermediate 1. Yield: 53% of theory; LC (method 4): t_{R} = 2.28 min; Mass spectrum (ESI⁻): m/z = 375/377 (CI) [M-H]⁻.

### Intermediate 41

### {(S)-6-[(R)-4-Cyano-indan-1-yloxy]-2,3-dihydro-benzofuran-3-yl}-acetic acid methyl ester

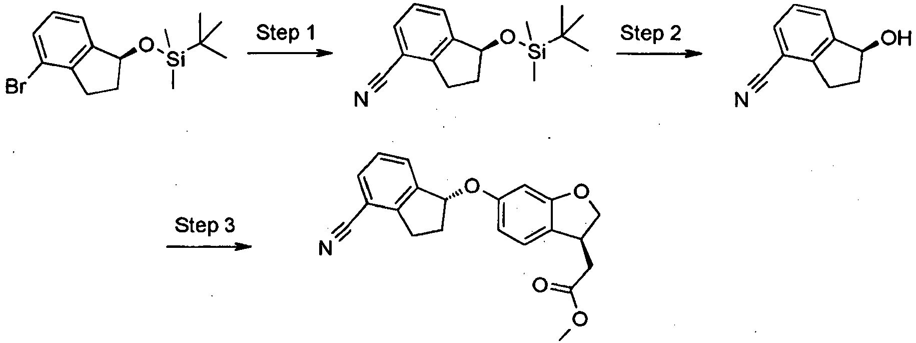

### Step 1: (S)-1-(tert-butyl-dimethyl-silanyloxy)-indan-4-carbonitrile

A mixture of [(*S*)-4-bromo-indan-1-yloxy]-tert-butyl-dimethyl-silane (0.20 g), copper(I) cyanide (0.08 g), and N-methyl-pyrrolidone (1 mL) is stirred under microwave irradiation at 170 °C for 1 h. The mixture is diluted with diethyl ether and the resulting mixture is washed with 5% aqueous NaHCO₃ solution and dried (Na₂SO₄). The solvent is evaporated to give the crude title compound. Yield: 0:16 g (93% of theory).

### Step 2: (S)-4-cyano-indan-1-ol

The title compound is prepared from (*S*)-1-(tert-butyl-dimethyl-silanyloxy)-indan-4-carbonitrile following a procedure analogous to that described in Step 4 of Intermediate 7.

### Step 3: {(S)-6-[(R)-4-cyano-indan-1-yloxy]-2,3-dihydro-benzofuran-3-yl}-acetic acid methyl ester

The title compound is prepared from (*S*)-4-cyano-indan-1-ol and (*S*)-(6-hydroxy-2,3-dihydro-benzofuran-3-yl)-acetic acid methyl ester following a procedure analogous to that described in Step 3 of Intermediate 1. Yield: 85% of theory; LC (method 9): t_{R} = 8.43 min; Mass spectrum (ESI⁺): m/z = 350 [M+Na]⁺.

### Intermediate 42

### {(S)-6-[(R)-4-(3-Cyano-pyridin-2-yl)-indan-1-yloxy]-2,3-dihydro-benzofuran-3-yl}-acetic acid methyl ester

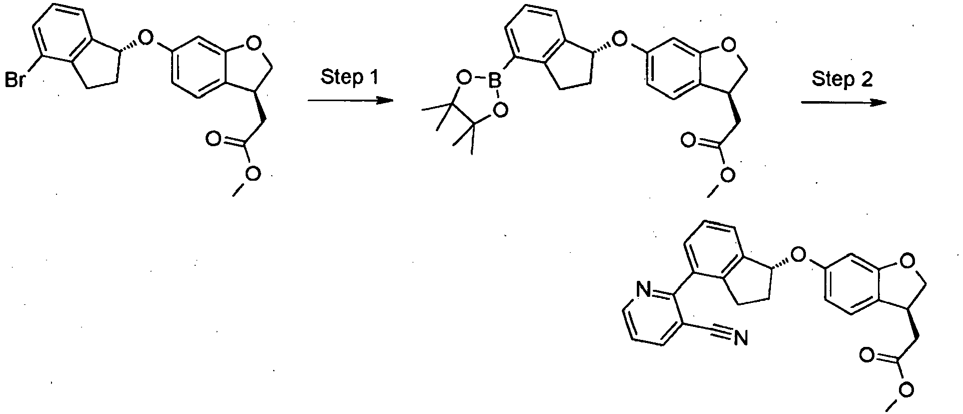

### Step 1: {(S)-6-[(R)-4-(4,4,5,5-tetramethyl-[1,3,2]dioxaborolan-2-yl)-indan-1-yloxy]-2,3-dihydro-benzofuran-3-yl}-acetic acid methyl ester

A flask charged with a stir bar, {(S)-6-[(R)-4-bromo-indan-1-yloxy]-2,3-dihydro-benzofuran-3-yl}-acetic acid methyl ester (0.85 g), bis(pinacolato)diboron (0.54 g), potassium acetate (0.56 g), and 1,4-dioxane (10 mL) is purged with argon for 10 min. 1,1'-Bis(diphenylphosphino)ferrocene-dichloropalladium dichloromethane adduct (0.08 g) is added and the mixture is stirred at 100 °C for 1.5 h. After cooling to room temperature, the mixture is concentrated and the residue is taken up in methanol. The resulting mixture is filtered and the filtrate is concentrated. The residue is chromatographed on reversed phase (methanol/water) to give the title compound. Yield: 0.15 g (27% of theory); LC (method 1): t_{R} = 1.50 min; Mass spectrum (ESI⁺): m/z = 473 [M+Na]⁺.

### Step 2: {(S)-6-[(R)-4-(3-cyano-pyridin-2-yl)-indan-1-yloxy]-2,3-dihydro-benzofuran-3-yl}-acetic acid methyl ester

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and 2-chloro-3-cyano-pyridine following a procedure analogous to that described in Step 1 of Intermediate 27. Yield: 45% of theory; LC (method 1): t_{R} = 1.24 min; Mass spectrum (ESI⁺): m/z = 427 [M+H]⁺.

### Intermediate 43

### Trifluoro-methanesulfonic acid 8-oxa-spiro[4.5]dec-1-en-1-yl ester

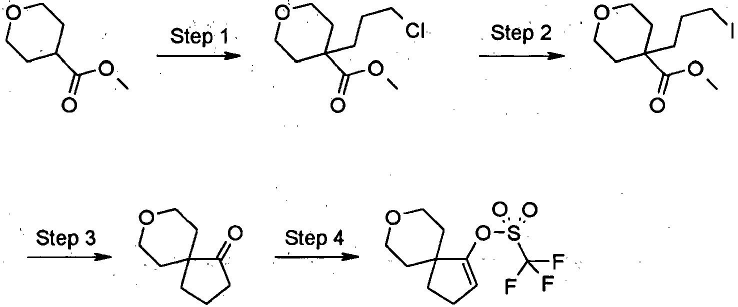

### Step 1: 4-(3-chloro-propyl)-tetrahydro-pyran-4-carboxylic acid methyl ester

Lithium hexamethyldisilazide (1 mol/l in toluene, 7.5 mL) is added dropwise to a solution of tetrahydropyran-4-carboxylic acid methyl ester (1.0 mL) in tetrahydrofuran (10 mL) cooled to -78 °C. The solution is stirred at -78 °C for 1 h prior to the addition of 1-chloro-3-iodo-propane (0.8 mL). The solution is warmed to room temperature overnight and then diluted with diethyl ether. The resulting mixture is washed with aqueous Na₂S₂O₃ solution and aqueous NaHCO₃ solution and dried (Na₂SO₄). The solvent is evaporated to give the crude title compound. Yield: 1.49 g (91 % of theory). Step 2: 4-(3-iodo-propyl)-tetrahydro-pyran-4-carboxylic acid methyl ester
A mixture of 4-(3-chloro-propyl)-tetrahydropyran-4-carboxylic acid methyl ester (1.49 g), sodium iodide (1.43 g), and acetone (5 mL) is stirred at reflux temperature overnight. After cooling to room temperature, diethyl ether is added and the resulting mixture is washed with aqueous Na₂S₂O₃ solution. The organic phase is dried and concentrated to give the title compound. Yield: 1.79 g (85% of theory); Mass spectrum (ESI⁺): m/z = 313 [M+H]⁺.

### Step 3: 8-oxa-spiro[4.5]decan-1-one

tert-Butyl lithium (1.7 mol/l in pentane, 4.9 mL) is added dropwise to a solution of 4-(3-iodo-propyl)-tetrahydropyran-4-carboxylic acid methyl ester (1.29 g) in tetrahydrofuran (13 mL) cooled to -78 °C. The solution is stirred at -78 °C for 1 h and then quenched by the addition of aqueous NH₄Cl solution. The resulting mixture is extracted with diethyl ether and the combined extract is dried (Na₂SO₄). The solvent is evaporated and the residue is chromatographed on silica gel (cyclohexane/ethyl acetate 9:1→7:3) to give the title compound. Yield: 0.37 g (58% of theory); Mass spectrum (ESI⁺): m/z = 155 [M+H]⁺.

### Step 4: trifluoro-methanesulfonic acid 8-oxa-spiro[4.5]dec-1-en-1-yl ester

Potassium hexamethyldisilazide (0.5 mol/l in toluene, 2.7 mL) is added to a solution of 8-oxa-spiro[4.5]decan-1-one (0.17 g) in tetrahydrofuran (5 mL) cooled to -78 °C. The solution is stirred at -78 °C for 30 min prior to the slow addition of N,N-bis(trifluoromethanesulfonyl)aniline (0.41 g) in tetrahydrofuran (2 mL). The solution is warmed to room temperature over a period of 1 h. Diethyl ether is added and the resulting mixture is washed with 1 M aqueous HCl solution and dried (Na₂SO₄). The solvent is evaporated and the residue is chromatographed on silica gel (cyclohexane/ethyl acetate 9:1→7:3) to give the title compound. Yield: 0.23 g (73% of theory); Mass spectrum (ESI⁺): m/z = 287 [M+H]⁺.

### Intermediate 44

### {(S)-6-[(R)-4-(8-Oxa-spiro[4.5]dec-1-en-1-yl)-indan-1-yloxy]-2,3-dihydro-benzofuran-3-yl}-acetic acid methyl ester

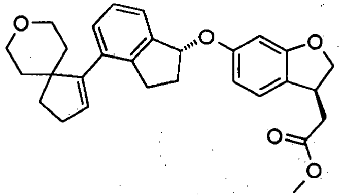

A flask charged with a stir bar, {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester (0.15 g), trifluoro-methanesulfonic acid 8-oxa-spiro[4.5]dec-1-en-1-yl ester (0.08 g), K₃PO₄ (0.12 g), and tetrahydrofuran (4 mL) is purged with argon for 10 min. 1,1'-Bis(diphenylphosphino)ferrocene-dichloropalladium dichloromethane adduct (8 mg) is added and the mixture is stirred at 100 °C for 5 h. After cooling to room temperature, the mixture is diluted with diethyl ether, washed with aqueous NH₄Cl solution, and dried (Na₂SO₄). The solvent is evaporated and the residue is chromatographed on silica gel (cyclohexane/ethyl acetate) to give the title compound. Yield: 0.09 g (75% of theory); LC (method 1): t_{R} = 1.46 min; Mass spectrum (ESI⁺): m/z = 483 [M+Na]⁺.

### Intermediate 45

### {(S)-6-[(R)-4-(2,6,6-Trimethyl-cyclohex-1-enyl)-indan-1-yloxy]-2,3-dihydrobenzofuran-3-yl}-acetic acid methyl ester

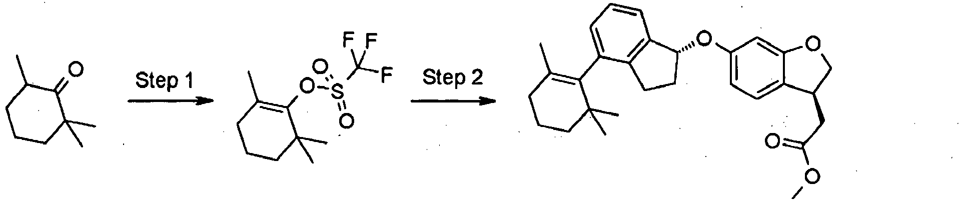

### Step 1: trifluoro-methanesulfonic acid 2,6,6-trimethyl-cyclohex-1-enyl ester

The title compound is prepared from 2,2,6-trimethyl-cyclohexanone following a procedure analogous to that described in Step 4 of Intermediate 43. Yield: 27% of theory.

### Step 2: {(S)-6-[(R)-4-(2,6,6-trimethyl-cyclohex-1-enyl)-indan-1-yloxy]-2,3-dihydrobenzofuran-3-yl}-acetic acid methyl ester

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and trifluoro-methanesulfonic acid 2,6,6-trimethyl-cyclohex-1-enyl ester following a procedure analogous to that described in Intermediate 44. Yield: 73% of theory; LC (method 6): t_{R} = 1.89 min; Mass spectrum (ESI⁺): m/z = 469 [M+Na]⁺.

### Intermediate 46

### {(S)-6-[(R)-4-(1,3,5-Trimethyl-1H-pyrazol-4-yl)-indan-1-yloxy]-2,3-dihydro-benzofuran-3-yl}-acetic acid methyl ester

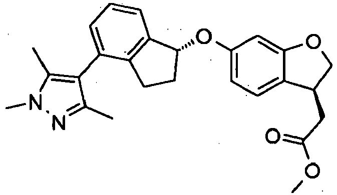

The title compound is prepared from [(*S*)-6-((*R*)-4-bromo-indan-1-yloxy)-2,3-dihydrobenzofuran-3-yl]-acetic acid methyl ester and 1,3,5-trimethyl-4-(4,4,5,5-tetramethyl-[1,3,2]dioxaborolan-2-yl)-1 H-pyrazole following a procedure analogous to that described in Step 1 of Intermediate 27.

### Intermediate 47

### {(S)-6-[(S)-6-Trifluoromethyl-indan-1-yloxy]-2,3-dihydro-benzofuran-3-yl}-acetic acid methyl ester

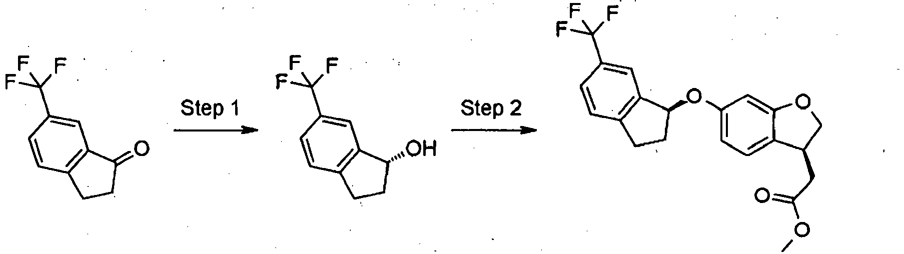

### Step 1: (R)-6-trifluoromethyl-indan-1-ol

The title compound is prepared from 6-trifluoromethyl-indan-1-one following a procedure analogous to that described in Step 2 of Intermediate 13; chloro{[(1R,2R)-(-)-2-amino-1,2-diphenlyethyl](4-toluenesulfonyl)amido}(mesitylene)ruthenium(II) is used as catalyst. Yield: 92% of theory; LC (method 1): t_{R} = 1.08 min; Mass spectrum (ESI⁻): m/z = 201 [M-H]⁻.

### Step 2: {(S)-6-[(S)-6-trifluoromethyl-indan-1-yloxy]-2,3-dihydro-benzofuran-3-yl}-acetic acid methyl ester

The title compound is prepared from (*R*)-6-trifluoromethyl-indan-1-ol and (*S*)-(6-hydroxy-2,3-dihydro-benzofuran-3-yl)-acetic acid methyl ester following a procedure analogous to that described in Step 3 of Intermediate 1. Yield: 45% of theory; LC (method 1): t_{R} = 1.41 min; Mass spectrum (ESI⁺): m/z = 393 [M+H]⁺.

### Intermediate 48

### {(S)-6-[(R)-4-(2-oxo-2H-pyridin-1-yl)-indan-1-yloxy]-2,3-dihydro-benzofuran-3-yl}-acetic acid methyl ester

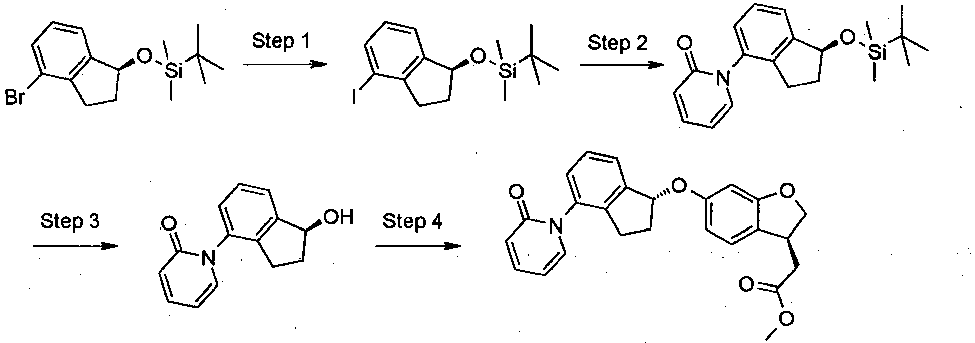

### Step 1: (S)-tert-butyl-(4-iodo-indan-1-yloxy)-dimethyl-silane

Nal (2.06 g), Cul (0.16 g), and N,N'-dimethylethylene-1,2-diamine (0.18 mL) are added to a degassed solution of (*S*)-tert-butyl-(4-bromo-indan-1-yloxy)-dimethyl-silane (0.90 g) in 1,4 dioxane (15 ml). The resulting mixture is stirred at 110 °C until the starting material is completely converted. Water is added and the mixture is extracted with ethyl acetate. The combined extracts are washed with brine and dried (Na₂SO₄). The solvent is evaporated and the residue is chromatographed on silica gel (heptane/ethyl acetate) to give the title compound. Yield: 0.78 g (92% of theory); LC (method 4): t_{R} = 2.73 min.

### Step 2: 1-[(S)-1-(tert-butyl-dimethyl-silanyloxy)-indan-4-yl]-1H-pyridin-2-one

A hot solution of (*S*)-tert-butyl-(4-iodo-indan-1-yloxy)-dimethyl-silane (1.81 g) in dimethylsulfoxide (50 mL) is added to an autoclave charged with K₂CO₃ (1.34 g), copper iodide (0.09 g), 2-hydroxy-pyridine (0.55 g), and 4,7-dimethoxy-1,10-phenanthroline (0.17 g). The autoclave is purged with argon and then heated at 130 °C overnight. After cooling to room temperature, water is added and the aqueous mixture is extracted with ethyl acetate. The combined organic extracts are washed with brine, dried (Na₂SO₄), and concentrated. The residue is chromatographed on silica gel (heptane/ethyl acetate) to give the title compound. Yield: 0.54 g (33% of theory); LC (method 4): t_{R} = 2.31 min; Mass spectrum (ESI⁺): m/z = 342 [M+H]⁺.

### Step 3: (S)-1-(1-hydroxy-indan-4-yl)-1H-pyridin-2-one

The title compound is prepared from 1-[(*S*)-1-(tert-butyl-dimethyl-silanyloxy)-indan-4-yl]-1H-pyridin-2-one following a procedure analogous to that described in Step 4 of Intermediate 7. 54% of theory; LC (method 4): t_{R} = 1.46 min; Mass spectrum (ESI⁺): m/z = 288 [M+H]⁺.

### Step 4: {(S)-6-[(R)-4-(2-oxo-2H-pyridin-1-yl)-indan-1-yloxy]-2,3-dihydro-benzofuran-3-yl}-acetic acid methyl ester

The title compound is prepared from (*S*)-1-(1-hydroxy-indan-4-yl)-1H-pyridin-2-one and (*S*)-(6-hydroxy-2,3-dihydro-benzofuran-3-yl)-acetic acid methyl ester following a procedure analogous to that described in Step 3 of Intermediate 1. Yield: 47% of theory.

### Intermediate 49

### {(S)-6-[(R)-5-(2,6-Dimethyl-phenyl)-indan-1-yloxy]-2,3-dihydro-benzofuran-3-yl}-acetic acid methyl ester

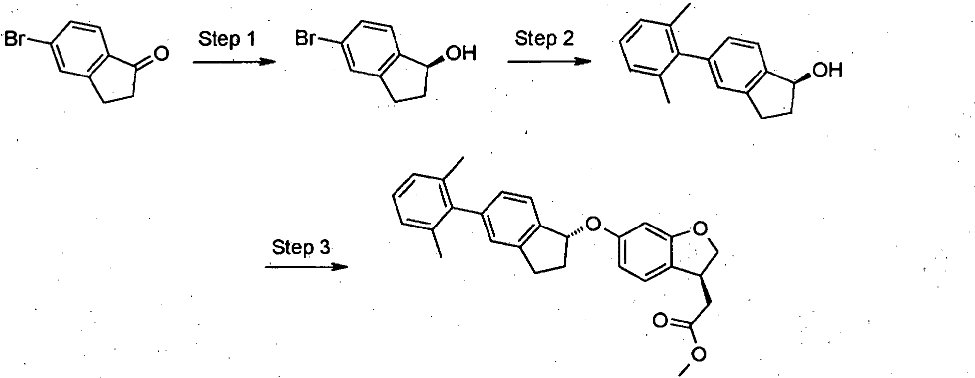

### Step 1: (S)-5-bromo-indan-1-ol

The title compound is prepared from 5-bromo-indan-1-one following a procedure analogous to that described in Step 2 of Intermediate 13. Yield: 79% of theory; LC (method 1): t_{R} = 1.04 min; Mass spectrum (ESI⁺): m/z = 195/197 (Br) [M-OH]⁺.

### Step 2: (S)-5-(2,6-dimethyl-phenyl)-indan-1-ol

The title compound is prepared from (*S*)-5-bromo-indan-1-ol and 2,6-dimethylphenylboronic acid following a procedure analogous to that described in Step 1 of Intermediate 27. Yield: 36% of theory; LC (method 1): t_{R} = 1.31 min; Mass spectrum (ESI⁺): m/z = 221 [M-OH]⁺.

### Step 3: {(S)-6-[(R)-5-(2,6-dimethyl-phenyl)-indan-1-yloxy]-2,3-dihydro-benzofuran-3-yl}-acetic acid methyl ester

The title compound is prepared from (*S*)-5-(2,6-dimethyl-phenyl)-indan-1-ol and (S)-(6-hydroxy-2,3-dihydro-benzofuran-3-yl)-acetic acid methyl ester following a procedure analogous to that described in Step 3 of Intermediate 1. Yield: 42% of theory; LC (method 1): t_{R} = 1.52 min; Mass spectrum (ESI⁺): m/z = 451 [M+Na]⁺.

### Intermediate 50

### {(S)-6-[(S)-6-Bromo-indan-1-yloxy]-2,3-dihydro-benzofuran-3-yl}-acetic acid methyl ester

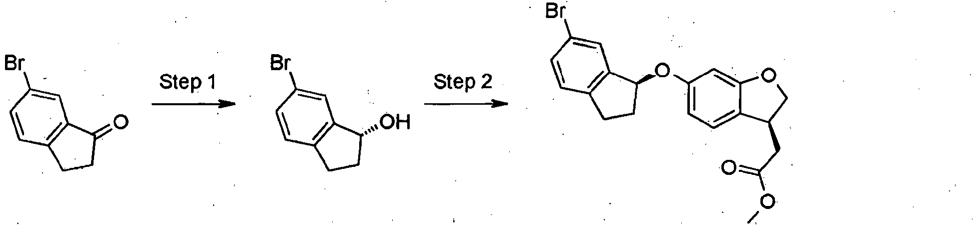

### Step 1: (R)-6-bromo-indan-1-ol

The title compound is prepared from 6-bromo-indan-1-one following a procedure analogous to that described in Step 2. of Intermediate 13; chloro{[(1R,2R)-(-)-2-amino-1,2-diphenylethyl](4-toluenesulfonyl)amido}(mesitylene)ruthenium(II) is used as catalyst. Yield: 99% of theory; LC (method 1): t_{R} = 1.03 min; Mass spectrum (ESI⁺): m/z = 195 [M-OH]⁺.

### Step 2: {(S)-6-[(S)-6-bromo-indan-1-yloxy]-2,3-dihydro-benzofuran-3-yl}-acetic acid methyl ester

The title compound is prepared from (*R*)-6-bromo-indan-1-ol and (*S*)-(6-hydroxy-2,3-dihydro-benzofuran-3-yl)-acetic acid methyl ester following a procedure analogous to that described in Step 3 of Intermediate 1. Yield: 53% of theory; LC (method 1): tR = 1.43 min; Mass spectrum (ESI⁺): m/z = 403/405 (Br) [M+H]⁺.

### Intermediate 51.

### {(S)-6-[(R)-5-(2-Isopropyl-phenyl)-indan-1-yloxy]-2,3-dihydro-benzofuran-3-yl}-acetic acid methyl ester

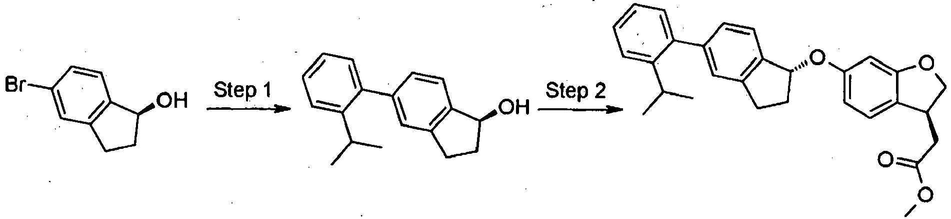

### Step 1: (S)-5-(2-isopropyl-phenyl)-indan-1-ol

The title compound is prepared from (*S*)-5-bromo-indan-1-ol and 2-isopropylphenylboronic acid following a procedure analogous to that described in Step 1 of Intermediate 27. Yield: 93% of theory; LC (method 1): tR = 1.34 min; Mass spectrum (ESI⁺): m/z = 235 [M-OH]⁺.

### Step 2: {(S)-6-[(R)-5-(2-isopropyl-phenyl)-indan-1-yloxy]-2,3-dihydro-benzofuran-3-yl}-acetic acid methyl ester

The title compound is prepared from (*S*)-5-(2-isopropyl-phenyl)-indan-1-ol and (*S*)-(6-hydroxy-2,3-dihydro-benzofuran-3-yl)-acetic acid methyl ester following a procedure analogous to that described in Step 3 of Intermediate 1. Yield: 41% of theory; LC (method 1): t_{R} = 1.55 min; Mass spectrum (ESI⁺): m/z = 465 [M+Na]⁺.

### Intermediate 52

### {(S)-6-[(R)-4-(3-Oxa-spiro[5.5]undec-7-en-7-yl)-indan-1-yloxy]-2,3-dihydro-benzofuran-3-yl}-acetic acid methyl ester

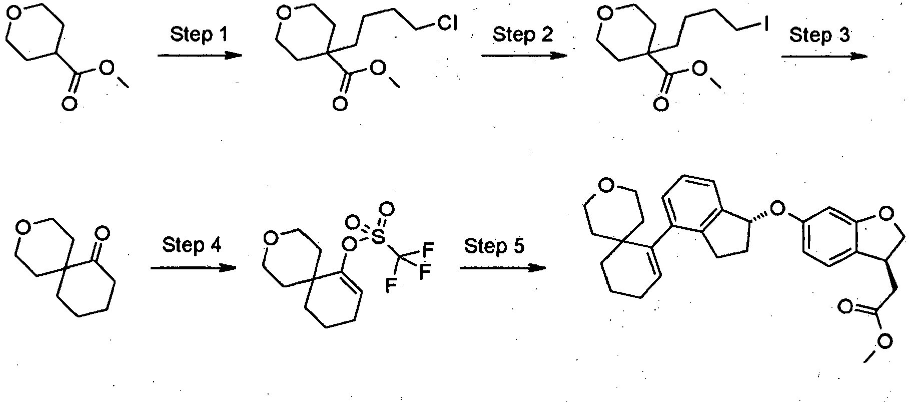

The title compound is obtained following the synthetic scheme depicted above and the corresponding procedures described for Intermediate 43 and Intermediate 44. LC (method 1): t_{R} = 1.49 min; Mass spectrum (ESI⁺): m/z = 497 [M+Na]⁺.

### Intermediate 53

### {(S)-6-[(R)-4-(1-Methyl-1H-imidazol-2-yl)-indan-1-yloxy]-2,3-dihydro-benzofuran-3-yl}-acetic acid methyl ester

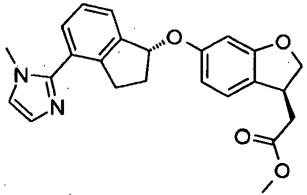

The title compound is prepared from {(S)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and 2-iodo-1-methyl-imidazole following a procedure analogous to that described in Example 53; instead of toluene 1,4-dioxane is employed and the reaction is carried out in a microwave oven at 120 °C for 0.5 h.

### Intermediate 54

### {(S)-6-[(R)-4-(3-Methyl-pyridin-2-yl)-indan-1-yloxy]-2,3-dihydro-benzofuran-3-yl}-acetic acid methyl ester

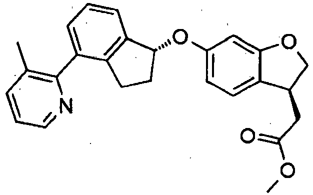

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and 2-bromo-3-methyl-pyridine following a procedure analogous to that described in Example 53. LC (method 1): t_{R} = 1.06 min; Mass spectrum (ESI⁺): m/z = 416 [M+H]⁺.

### Intermediate 55

### {(S)-6-[(R)-4-(4-Methoxy-2,6-dimethyl-phenyl)-indan-1-yloxy]-2,3-dihydro-benzofuran-3-yl}-acetic acid methyl ester

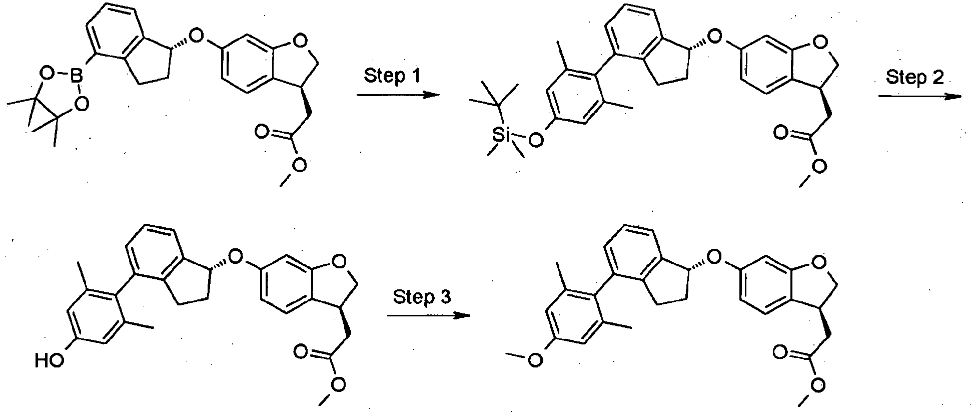

### Step 1: ((S)-6-{(R)-4-[4-(tert-butyl-dimethyl-silanyloxy)-2,6-dimethyl-phenyl]-indan-1-yloxy}-2,3-dihydro-benzofuran-3-yl)-acetic acid methyl ester

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and (4-bromo-3,5-dimethyl-phenoxy)-tert-butyl-dimethyl-silane following a procedure analogous to that described in Example 53; tetrabutylammonium bromide is added to the reaction mixture and the reaction is carried out in a microwave oven at 120 °C. Yield: 69% of theory; LC (method 1): t_{R} = 1.65 min; Mass spectrum (ESI⁺): m/z = 559 [M+H]⁺.

### Step 2: {(S)-6-[(R)-4-(4-hydroxy-2,6-dimethyl-phenyl)-indan-1-yloxy]-2,3-dihydro-benzofuran-3-yl}-acetic acid methyl ester

The title compound is prepared from ((*S*)-6-{(*R*)-4-[4-(tert-butyl-dimethyl-silanyloxy)-2,6-dimethyl-phenyl]-indan-1-yloxy}-2,3-dihydro-benzofuran-3-yl)-acetic acid methyl ester following a procedure analogous to that described in Step 4 of Intermediate 7. 81% of theory; LC (method 1): t_{R} = 1.36 min; Mass spectrum (ESI⁺): m/z = 445 [M+H]⁺.

### Step 3: {(S)-6-[(R)-4-(4-methoxy-2,6-dimethy)-phenyl)-indan-1-yloxy]-2,3-dihydrobenzofuran-3-yl}-acetic acid methyl ester

lodomethane (9 µL) is added to a mixture of {(*S*)-6-[(*R*)-4-(4-hydroxy-2,6-dimethyl-phenyl)-indan-1-yloxy]-2,3-dihydro-benzofuran-3-yl}-acetic acid methyl ester (40 mg); K₂CO₃ (25 mg), and N,N-dimethylformamide (2 mL) at room temperature. The mixture is stirred at room temperature overnight and then diluted with ethyl acetate. The mixture is washed with water and brine and dried (MgSO₄). The solvent is evaporated to give the title compound. Yield: 31 mg (75% of theory); LC (method 1): t_{R} = 1.50 min; Mass spectrum (ESI⁺): m/z = 459 [M+H]⁺.

### Intermediate 56

### 4-Bromo-2-methoxy-3,5-dimethyl-pyridine

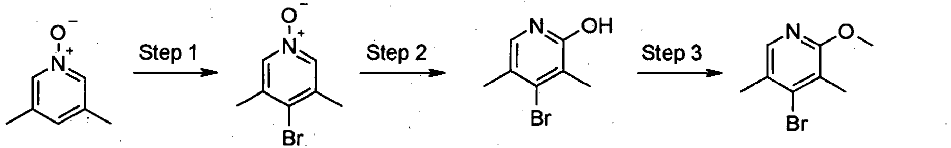

### Step 1: 4-bromo-3,5-dimethyl-pyridine 1-oxide

Bromine (1.1 mL) is added dropwise to a mixture of lead tetraacetate (13.01 g), 3,5-dimethyl-pyridine-N-oxide (2.46 g), and acetic acid (20 mL) stirred at 70 °C. After stirring the mixture at 70 °C overnight, another portion of bromine (0.56 mL) is added and stirring is continued for another 24 h. After cooling to room temperature, the mixture is neutralized using NaOH. The precipitate is separated by filtration and the filtrate is concentrated. The residue is taken up in water/methanol and the resulting mixture is filtered again. The filtrate is concentrated and the residue is chromatographed on reversed phase (acetonitrile/water) to give the title compound. Yield: 2.60 g (64% of theory); LC (method 1): t_{R} = 0.69 min; Mass spectrum (ESI⁺): m/z = 202/204 (Br) [M+H]⁺.

### Step 2: 4-bromo-3,5-dimethyl-pyridin-2-ol

Trifluoroacetic anhydride (0.87 mL) is added to a solution of 4-bromo-3,5-dimethyl-pyridine 1-oxide (1.01 g) and triethylamine (3.5 mL) in tetrahydrofuran (50 mL) chilled in an ice bath. The cooling bath is removed and the solution is stirred at room temperature for 2.5 h. Water is added and the solution is concentrated. The residue is chromatographed on reversed phase (acetonitrile/water) to give the title compound. Yield: 0.29 g (29% of theory).

### Step 3: 4-bromo-2-methoxy-3,5-dimethyl-pyridine

Iodomethane (0.15 mL) is added to a mixture of 4-bromo-3,5-dimethyl-pyridin-2-ol (0.10 g), Ag₂CO₃ (0.21 g), and chloroform (6 mL) at room temperature. The mixture is stirred at reflux temperature overnight. After cooling to room temperature, water and dichlormethane are added and the resulting mixture is filtered. The filtrate is extracted with dichloromethane. The combined extracts are dried (MgSO₄) and concentrated to give the title compound. Yield: 0.08 g (71% of theory); LC (method 1): t_{R} = 1.29 min.

### Intermediate 57

### {(S)-6-[(R)-4-(2-Methoxy-3,5-dimethyl-pyridin-4-yl)-indan-1-yloxy]-2,3-dihydrobenzofuran-3-yl}-acetic acid methyl ester

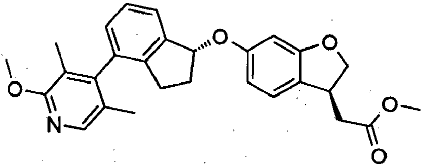

A vial charged with a stir bar, {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester (0.11 g), 4-bromo-3,5-dimethyl-2-methoxy-pyridine (0.06 g), and 1,4-dioxane (4 mL) is purged with Ar for 10 min. Bis(dibenzylideneacetone)palladium(0) (14 mg), 2-dicyclohexylphosphino-2',6'-dimethoxybiphenyl (21 mg), and Ba(OH)₂ (0.13 g) are added in the given order and the resulting mixture is stirred at 80 °C overnight. After cooling to room temperature, the mixture is filtered and the filtrate is slightly acidified using trifluoroacetic acid and chromatographed on reversed phase (acetonitrile/water) to give the title compound. Yield: 0.07 g (57% of theory).

### Intermediate 58

### {(S)-6-[(R)-4-(1,3,5-Trimethyl-2-oxo-1,2-dihydro-pyridin-4-yl)-indan-1-yloxy]-2,3-dihydro-benzofuran-3-yl}-acetic acid methyl ester

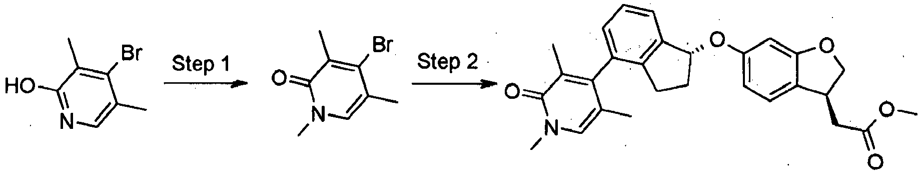

### Step 1: 4-bromo-1,3,5-trimethyl-1H-pyridin-2-one

A mixture of 4-bromo-3,5-dimethyl-pyridin-2-ol (0.10 g), dimethyl carbonate (0.42 mL), and K₂CO₃ (0.10 g) is stirred at 120 °C overnight. After cooling to room temperature, water is added and the mixture is extracted with dichloromethane. The combined extracts are dried (Na₂SO₄) and concentrated to give the crude title compound. Yield: 0.11 g (quantitative); LC (method 1): t_{R} = 0.95 min.

### Step 2: {(S)-6-[(R)-4-(1,3,5-trimethyl-2-oxo-1,2-dihydro-pyridin-4-yl)-indan-1-yloxy]-2,3-dihydro-benzofuran-3-yl}-acetic acid methyl ester

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and 4-bromo-1,3,5-trimethyl-1H-pyridin-2-one following a procedure analogous to that described in Intermediate 57. Yield: 36% of theory.

### Intermediate 59

### {(S)-6-[(R)-4-o-Tolyl-indan-1-yloxy]-2,3-dihydro-benzofuran-3-yl}-acetic acid methyl ester

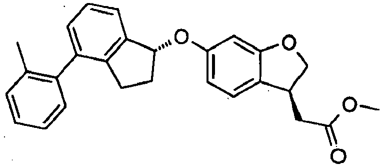

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and 2-bromo-toluene following a procedure analogous to that described in Example 53; tetrabutylammonium bromide is added to the reaction mixture and the reaction is carried out in a microwave oven at 120 °C. Yield: 67% of theory; LC (method 1): t_{R} = 1.50 min; Mass spectrum (ESI⁺): m/z = 437 [M+Na]⁺.

### Intermediate 60

### {(S)-6-[(R)-4-(1,2,4-Trimethyl-6-oxo-1,6-dihydro-pyridin-3-yl)-indan-1-yloxy]-2,3-dihydro-benzofuran-3-yl}-acetic acid methyl ester

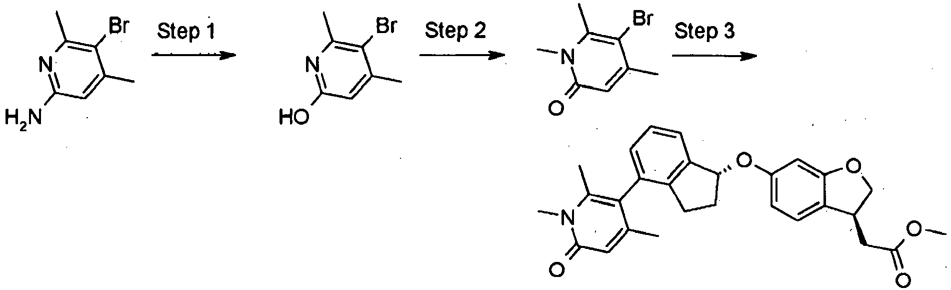

### Step 1: 5-bromo-4,6-dimethyl-pyridin-2-ol

5-Bromo-4,6-dimethyl-pyridin-2-ylamine (2.01 g) in water (120 mL) and 32% aqueous HCl (40 mL) is heated to reflux temperature and the solution is filtered hot. NaNO₂ (2.00 g) dissolved in water (40 mL) is added to the hot filtrate with vigorous stirring. The resulting mixture is stirred at room temperature overnight. The mixture is cooled in an ice bath and the precipitate is separated by filtration. The precipitate is washed with water and dried to give the title compound. Yield: 1.17 g (58% of theory); LC (method 1): t_{R} = 0.82 min; Mass spectrum (ESI⁺): m/z = 202/204 (Br) [M+H)⁺.

### Step 2: 5-bromo-1,4,6-trimethyl-1H-pyridin-2-one

The title compound is prepared from 5-bromo-4,6-dimethyl-pyridin-2-ol following a procedure analogous to that described in Step 1 of Intermediate 58. Yield: 65% of theory; LC (method 1): t_{R} = 0.87 min; Mass spectrum (ESI⁺): m/z = 216/218 (Br) [M+H]⁺.

### Step 3: {(S)-6-[(R)-4-(1,2,4-trimethyl-6oxo-1,6-dihydro-pyridin-3-yl)-indan-1-yloxy]-2,3-dihydro-benzofuran-3-yl}-acetic acid methyl ester

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and 5-bromo-1,4,6-trimethyl-1H-pyridin-2-one following a procedure analogous to that described in Intermediate 57. Yield: 33% of theory.

### Intermediate 61

### {(S)-6[(R)-4-(6-Methoxy-2,4-dimethyl-pyridin-3-yl)-indan-1-yloxy]-2,3-dihydrobenzofuran-3-yl}-acetic acid methyl ester

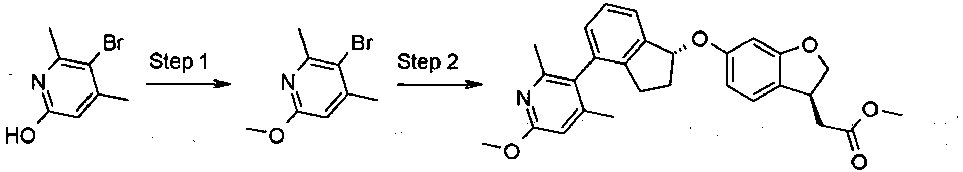

### Step 1: 3-bromo-6-methoxy-2,4-dimethyl-pyridine

The title compound is prepared from 5-bromo-4,6-dimethyl-pyridin-2-ol and iodomethane following a procedure analogous to that described in Step 3 of Intermediate 56. Yield: 60% of theory; LC (method 1): t_{R} = 1.21 min; Mass spectrum (ESI⁺): m/z = 216/218 (Br) [M+H]⁺.

### Step 2: {(S)-6-[(R)-4-(6-methoxy-2,4-dimethyl-pyridin-3-yl)-indan-1-yloxy]-2,3-dihydrobenzofuran-3-yl}-acetic acid methyl ester

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and 3-bromo-6-methoxy-2,4-dimethyl-pyridine following a procedure analogous to that described in Intermediate 57. Yield: 36% of theory.

### Intermediate 62

### {(S)-6-[(R)-4-Morpholin-4-yl-indan-1-yloxy]-2,3-dihydro-benzofuran-3-yl}-acetic acid methyl ester

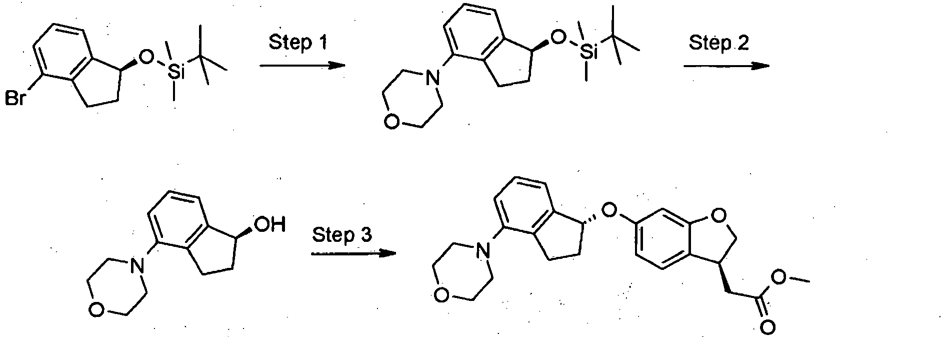

### Step 1: 4-[(S)-1-(tert-butyl-dimethyl-silanyloxy)-indan-4-yl]-morpholine

A microwave vial charged with a stir bar, ((*S*)-4-bromo-indan-1-yloxy)-tert-butyldimethyl-silane (0.10 g), morpholine (37 µL), sodium tert-butoxide (42 mg), and 1,4-dioxane (1 mL) is purged with Ar for 10 min. Chloro(2-dicyclohexylphosphino-2',6'-di-i-propoxy-1,1'-biphenyl)[2-(2-aminoethylphenyl)]palladium (II) methyl-tert-butyl-ether adduct (2.5 mg) is added and the mixture is stirred at 100 °C in a microwave oven for 10 min. After cooling to room temperature, ethyl acetate is added and the mixture is washed with water and brine and dried (Na₂SO₄). The solvent is evaporated and the residue is chromatographed on silica gel (cyclohexane/ethyl acetate) to give the title compound.

### Step 2: (S)-4-morpholin-4-yl-indan-1-ol

The title compound is prepared from (*S*)-4-[1-(tert-butyl-dimethyl-silanyloxy)-indan-4-yl]-morpholine following a procedure analogous to that described in Step 4 of Intermediate 7. Yield: 94% of theory; LC (method 1): t_{R} = 0.59 min; Mass spectrum (ESI⁺): m/z = 220 [M+H]⁺.

### Step 3: {(S)-6-[(R)-4-morpholin-4-yl-indan-1-yloxy]-2,3-dihydro-benzofuran-3-yl}-acetic acid methyl ester

The title compound is prepared from (*S*)-4-morpholin-4-yl-indan-1-ol and (*S*)-(6-hydroxy-2,3-dihydro-benzofuran-3-yl)-acetic acid methyl ester following a procedure analogous to that described in Step 3 of Intermediate 1. Yield: 23% of theory; LC (method 1): t_{R} = 1.28 min; Mass spectrum (ESI⁺): m/z = 410 [M+H]⁺.

### Intermediates 63 and 64

### (S)-4-[(R)-3,3-Dimethyl-tetrahydro-pyran-2-yl]-indan-1-ol and (S)-4-[(S)-3,3-Dimethyl-tetrahydro-pyran-2-yl]-indan-1-ol

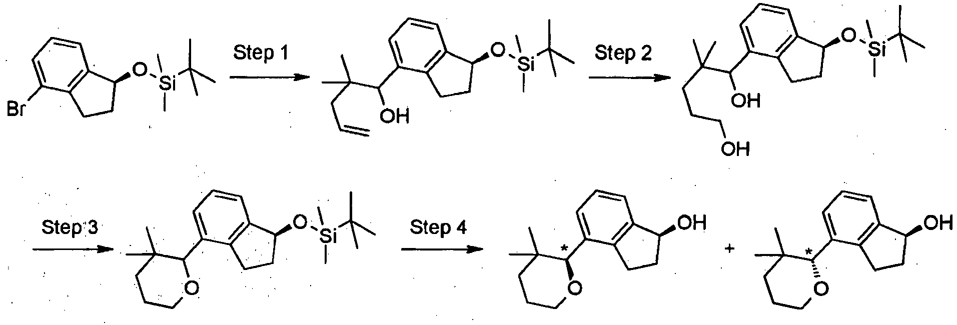

### Step 1: 1-[(S)-1-(tert-butyl-dimethyl-silanyloxy)-indan-4-yl]-2,2-dimethyl-pent-4-en-1-ol (mixture of 2 diastereomers)

The title compound is prepared from [(*S*)-4-bromo-indan-1-yloxy]-tert-butyl-dimethyl-silane and 2,2-dimethyl-pent-4-enal following a procedure analogous to that described in Step 1 of Intermediate 36. Yield: 58% of theory; LC (method 6): t_{R} = 1.83/1.87 min (2 diastereomers).

### Step 2: 1-[(S)-1-(tert-butyl-dimethyl-silanyloxy)-indan-4-yl]-2,2-dimethyl-pentane-1,5-diol (mixture of 2 diastereomers)

Borane tetrahydrofuran complex (1 mol/L, 3.6 mL) is added to a solution of 1-[(S)-1-(tert-butyl-dimethyl-silanyloxy)-indan-4-yl]-2,2-dimethyl-pent-4-en-1-ol (0.43 g) in tetrahydrofuran (6 mL) chilled in an ice bath. The solution is stirred for 1 h prior to the addition of aqueous NaOH solution (4 mol/L, 1.8 mL) and aqueous hydrogen peroxide solution (35%, 1.8 mL). The mixture is stirred for 1 h and then half-saturated NaCl solution is added. The mixture is extracted with diethyl ether and the combined extracts are dried (Na₂SO₄) and concentrated to give the crude product. Yield: 0.45 g (quantitative). LC (method 6): t_{R} = 1.62/1.66 min; Mass spectrum (ESI⁺): m/z = 401 [M+Na]⁺.

### Step 3: tert-butyl-[(S)-4-(3,3-dimethyl-tetrahydropyran-2-yl)-indan-1-yloxy]-dimethyl-silane (mixture of 2 diastereomers)

Triethylamine (0.5 mL), 4-methyl-benzenesulfonyl chloride (0.25 g), and 4-dimethylaminopyridine (catalytic amount) are added in the given order to a solution of 1-[(*S*)-1-(tert-butyl-dimethyl-silanyloxy)-indan-4-yl]-2,2-dimethyl-pentane-1,5-diol (0.45 g) in dichloromethane (6 mL) at room temperature. The solution is stirred at room temperature for 72 h and then heated in a microwave oven to 60 °C. After stirring at 60 °C for 2 h, the solution is cooled to room temperature and diluted with diethyl ether. The resulting mixture is washed with water, 1 M aqueous NaOH solution, and aqueous NH₄Cl solution and dried (Na₂SO₄). The solvent is evaporated and the residue is chromatographed on silica gel (cyclohexane/ethyl acetate) to give the title compound. Yield: 0.20 g (46% of theory); LC (method 1): t_{R} = 1.94 min; Mass spectrum (ESI⁺): m/z = 378 [M+NH₄]⁺.

### Step 4: (S)-4-[(S)-3,3-dimethyl-tetrahydro-pyran-2-yl]-indan-1-ol and (S)-4-[(R)-3,3-dimethyl-tetrahydro-pyran-2-yl]-indan-1-ol

The title compounds are prepared from tert-butyl-[(S)-4-(3,3-dimethyl-tetrahydropyran-2-yl)-indan-1-yloxy]-dimethyl-silane following a procedure analogous to that described in Step 4 of Intermediate 7. The two diastereomers are separated by chromatography on silica gel (petrol ether/ethyl acetate). The configurations at the stereocenters indicated (*) are arbitrarily assigned.
Intermediate 63: LC (method 6): t_{R} = 0.85 min; Mass spectrum (ESI⁺): m/z = 229 [M-OH]⁺.
Intermediate 64: LC (method 6): t_{R} = 0.98 min; Mass spectrum (ESI⁺): m/z = 229 [M-OH]⁺.

### Intermediate 65

### {(S)-6-[(R)-4-((S)-3,3-Dimethyl-tetrahydro-pyran-2-yl)-indan-1-yloxy]-2,3-dihydrobenzofuran-3-yl}-acetic acid methyl ester, one isomer, configuration at the stereocenter indicated (*) arbitrarily assigned

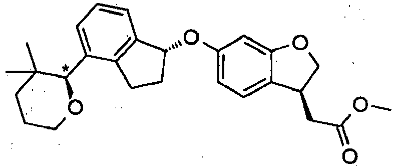

The title compound is prepared from (*S*)-4-[(*S*)-3,3-dimethyl-tetrahydro-pyran-2-yl]-indan-1-ol (pure isomer, Intermediate 63) and (*S*)-(6-hydroxy-2,3-dihydro-benzofuran-3-yl)-acetic acid methyl ester following a procedure analogous to that described in Step 3 of Intermediate 1. Yield: 64% of theory; LC (method 6): t_{R} = 1.61 min; Mass spectrum (ESI⁺): m/z = 437 [M+H]⁺.

### Intermediate 66

### {(S)-6-[(R)-4-((R)-3,3-Dimethyl-tetrahydro-pyran-2-yl)-indan-1-yloxy]-2,3-dihydrobenzofuran-3-yl}-acetic acid methyl ester, one isomer, configuration at the stereocenter indicated (*) arbitrarily assigned

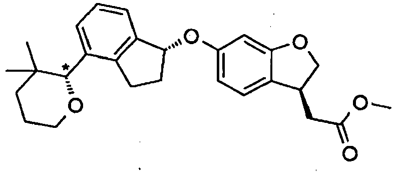

The title compound is prepared from (*S*)-4-[(*R*)-3,3-dimethyl-tetrahydro-pyran-2-yl]-indan-1-ol (pure isomer, Intermediate 64) and (*S*)-(6-hydroxy-2,3-dihydro-benzofuran-3-yl)-acetic acid methyl ester following a procedure analogous to that described in Step 3 of Intermediate 1. Yield: 54% of theory; LC (method 6): t_{R} = 1.59 min; Mass spectrum (ESI⁺): m/z = 437 [M+H]⁺.

### Intermediate 67

### {(S)6-[(R)-4-(5-Fluoro-2-methoxy-pyridin-4-yl)-indan-1-yloxy]-2,3-dihydrobenzofuran-3-yl}-acetic acid methyl ester

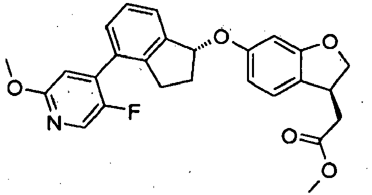

The title compound is prepared from {(*S*)-6-[(*R*)-4-bromo-indan-1-yloxy]-2,3-dihydro-benzofuran-3-yl}-acetic acid methyl ester and 5-fluoro-2-methoxy-4-(4,4,5,5-tetramethyl-[1,3,2]dioxaborolan-2-yl)-pyridine following a procedure analogous to that described in Intermediate 44. Yield: 73% of theory; LC (method 6): t_{R} = 1.53 min; Mass spectrum (ESI⁺): m/z = 450 [M+H]⁺.

### Intermediate 68

### {(S)-6-[(R)-4-(2-Trifluoromethyl-phenyl)-indan-1-yloxy]-2,3-dihydro-benzofuran-3-yl}-acetic acidmethyl ester

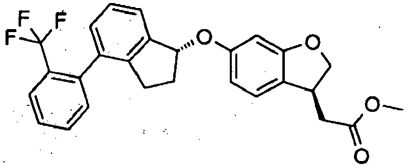

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and 1-iodo-2-trifluoromethyl-benzene following a procedure analogous to that described in Example 53; tetrabutylammonium bromide is added to the reaction mixture and the reaction is carried out in a microwave oven at 120 °C. Yield: 76% of theory; LC (method 1): t_{R} = 1.47 min; Mass spectrum (ESI⁺): m/z = 491 [M+Na]⁺.

### Intermediate 69

### {(S)-6-[(R)-4-(6,6-Dimethyl-cyclohex-1-enyl)-indan-1-yloxy]-2,3-dihydro-benzofuran-3-yl}-acetic acid methyl ester

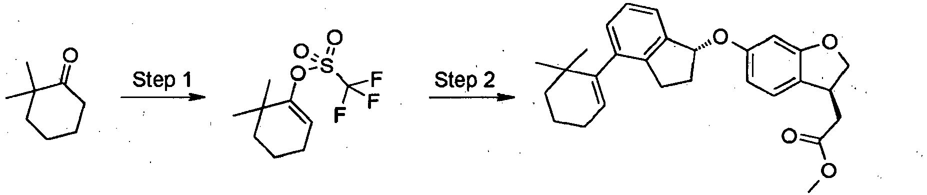

### Step 1: trifluoro-methanesulfonic acid 6,6-dimethyl-cyclohex-1-enyl ester

The title compound is prepared from 2,2-dimethyl-cyclohexanone following a procedure analogous to that described in Step 4 of Intermediate 43. Yield: 34% of theory.

### Step 2: {(S)-6-[(R)-4-(6,6-dimethyl-cyclohex-1-enyl)-indan-1-yloxy]-2,3-dihydro-benzofuran-3-yl}-acetic acid methyl ester

The title compound is prepared from trifluoro-methanesulfonic acid 6,6-dimethyl-cyclohex-1-enyl ester and {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester following a procedure analogous to that described in Intermediate 44. Yield: 74% of theory; LC (method 6): t_{R} = 1.85 min; Mass spectrum (ESI⁺): m/z = 455 [M+Na]⁺.

### Intermediate 70

### {(S)-6-[(R)-4-(5-Methoxy-2-methyl-phenyl)-indan-1-yloxy]-2,3-dihydro-benzofuran-3-yl}-acetic acid methyl ester

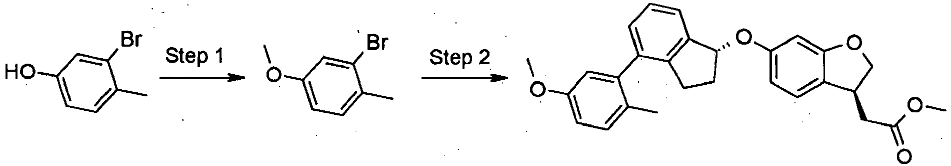

### Step 1: 2-bromo-4-methoxy-1-methyl-benzene

The title compound is prepared from 3-bromo-4-methyl-phenol following a procedure analogous to that described in Step 3 of Intermediate 56. Yield: 76% of theory; LC (method 1): t_{R} = 1.26 min.

### Step 2: {(S)-6-[(R)-4-(5-methoxy-2-methyl-phenyl)-indan-1-yloxy]-2,3-dihydro-benzofuran-3-yl}-acetic acid methyl ester

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and 2-bromo-4-methoxy-1-methyl-benzene following a procedure analogous to that described in Example 53; tetrabutylammonium bromide is added to the reaction mixture in toluene and water and the reaction is carried out in a microwave oven at 120 °C. Yield: 73% of theory; LC (method 1): t_{R} = 1.48 min; Mass spectrum (ESI⁺): m/z = 467 [M+Na]⁺.

### Intermediate 71

### {(S)-6-[(R)-4-(5-Hydroxy-2-methyl-phenyl)-indan-1-yloxy]-2,3-dihydro-benzofuran-3-yl}-acetic acid methyl ester

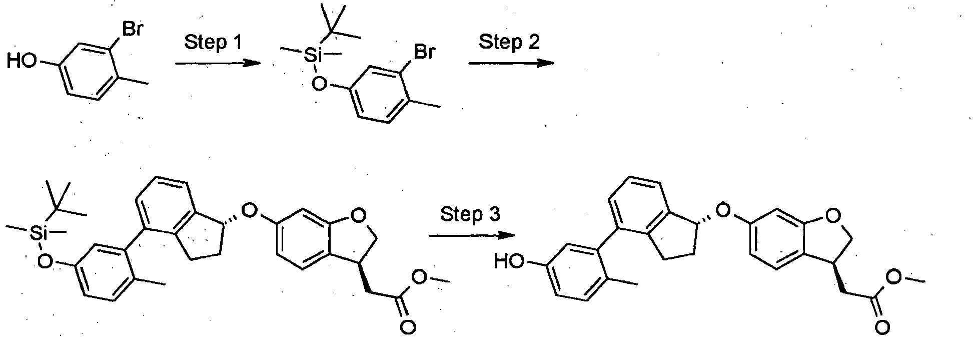

### Step 1: (3-bromo-4-methyl-phenoxy)-tert-butyl-dimethyl-silane

tert-Butyldimethylsilyl chloride (0.18 g) is added to a solution of 3-bromo-4-methyl-phenol (0.20 g) and triethylamine (0.23 mL) in dichloromethane (3 mL) chilled in an ice bath. 4-Dimethylaminopyridine (13 mg) is added and the solution is stirred at room temperature overnight. Dichloromethane is added and the solution is washed with 1 M hydrochloric acid, aqueous NaHCO₃ solution, and brine, and dried (MgSO₄). The solvent is evaporated and the residue is chromatographed on silica gel (cyclohexane/ethyl acetate) to give the title compound. Yield: 0.25 g (78% of theory); LC (method 1): t_{R} = 1.59 min.

### Step 2: ((S)-6-{(R)-4-[5-(tert-butyl-dimethyl-silanyloxy)-2-methyl-phenyl]-indan-1-yloxy}-2,3-dihydro-benzofuran-3-yl)-acetic acid methyl ester

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and (3-bromo-4-methyl-phenoxy)-tert-butyl-dimethyl-silane following a procedure analogous to that described in Example 53; tetrabutylammonium bromide is added to the reaction mixture in toluene and water and the reaction is carried out in a microwave oven at 120 °C. Yield: 33% of theory; LC (method 1): t_{R} = 1.64 min.

### Step 2: {(S)-6-[(R)-4-(5-hydroxy-2-methyl-phenyl)-indan-1-yloxy]-2,3-dihydrobenzofuran-3-yl}-acetic acid methyl ester

The title compound is prepared from ((*S*)-6-{(*R*)-4-[5-(tert-butyl-dimethyl-silanyloxy)-2-methyl-phenyl]-indan-1-yloxy}-2,3-dihydro-benzofuran-3-yl)-acetic acid methyl ester following a procedure analogous to that described in Step 4 of Intermediate 7. LC (method 1): t_{R} = 1.36 min; Mass spectrum (ESI⁺): m/z = 453 [M+Na]⁺.

### Intermediate 72

### {(S)-6-[(R)-4-Isoquinolin-1-yl-indan-1-yloxy]-2,3-dihydro-benzofuran-3-yl}-acetic acid methyl ester

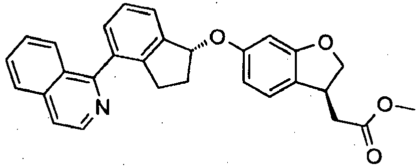

The title compound is prepared from 1-bromo-isoquinoline and {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester following a procedure analogous to that described in Intermediate 44. Yield: 46% of theory; LC (method 6): t_{R} = 1.15 min; Mass spectrum (ESI⁺): m/z = 452 [M+H]⁺.

### Intermediate 73

### {(S)-6-[(R)-4-(1-Methoxy-1,2,2-trimethyl-propyl)-indan-1-yloxy]-2,3-dihydrobenzofuran-3-yl}-acetic acid methyl ester

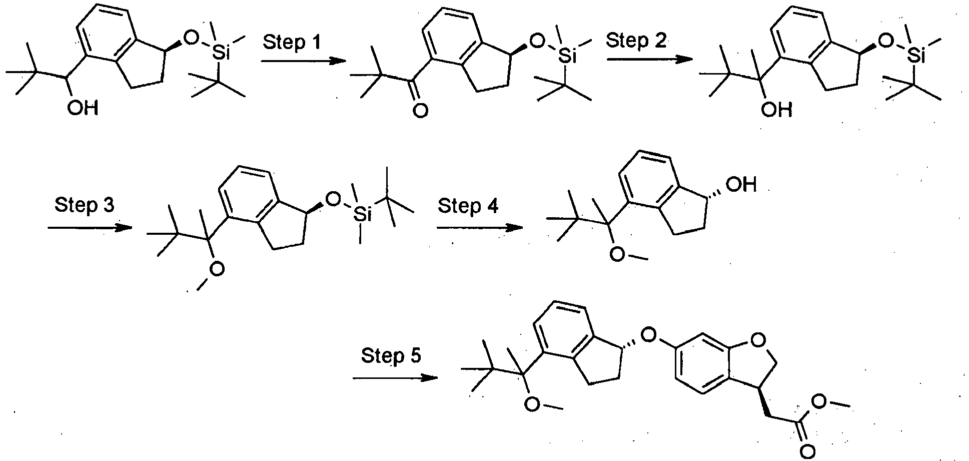

### Step 1: 1-[(S)-1-(tert-butyl-dimethyl-silanyloxy)-indan-4-yl]-2,2-dimethyl-propan-1-one

1,1-Dihydro-1,1,1-triacetoxy-1,2-benziodoxol-3(1H)-on (15% in dichloromethane, 1.4 mL) is added to a solution of 1-[(*S*)-1-(tert-butyl-dimethyl-silanyloxy)-indan-4-yl]-2,2-dimethyl-propan-1-ol (0.19 g) in dichloromethane (5 mL) chilled in an ice bath. The solution is stirred at room temperature for 1 h and then concentrated. The residue is chromatographed on silica gel (cyclohexane/ethyl acetate) to give the title compound. Yield: 0.16 g (85% of theory); LC (method 1): t_{R} = 1.56 min; Mass spectrum (ESI⁺): m/z = 355 [M+Na]⁺.

### Step 2: 2-[(S)-1-(tert-butyl-dimethyl-silanyloxy)-indan-4-yl]-3,3-dimethyl-butan-2-ol

Methylmagnesium bromide (1.4 mol/l in tetrahydrofuran/toluene; 3 mL) is added to a solution of 1-[(*S*)-1-(tert-butyl-dimethyl-silanyloxy)-indan-4-yl]-2,2-dimethyl-propan-1-one (0.14 g) in toluene (15 mL) at room temperature. The solution is stirred at 45 °C for 1 h. The solution is poured into ice water and dichloromethane is added to the mixture. The mixture is acidified with 1 M hydrochloric acid and stirred for 15 min. The organic layer is separated, dried (Na₂SO₄), and concentrated to give the crude title compound. Yield: 0.16 g (quantitative); LC (method 1): t_{R} = 1.58 min.

### Step 3: tert-butyl-[(S)-4-(1-methoxy-1,2,2-trimethyl-propyl)-indan-1-yloxy]-dimethyl-silane

2-[(*S*)-1-(tert-Butyl-dimethyl-silanyloxy)-indan-4-yl]-3,3-dimethyl-butan-2-ol (0.17 g) dissolved in tetrahydrofuran (2 mL) is added to a flask charged with a stir bar and potassium hydride (30% in oil, 0.19 g) at room temperature. The mixture is stirred at room temperature for 20 min prior to the addition of methyl iodide (2 mol/L in tert-butyl methyl ether, 0.36 mL). The mixture is stirred for 1 h and then chilled in an ice bath. Aqueous NH₄Cl solution is slowly added and the resulting mixture is extracted with diethyl ether. The combined extracts are dried (Na₂SO₄) and concentrated. The residue is chromatographed on silica gel (cyclohexane/ethyl acetate) to give the title compound. Yield: 0.11 g (64% of theory); LC (method 1): t_{R} = 1.67 min.

### Step 4: (S)-4-(1-methoxy-1,2,2-trimethyl-propyl)-indan-1-ol

The title compound is prepared from tert-butyl-[(*S*)-4-(1-methoxy-1,2,2-trimethylpropyl)-indan-1-yloxy]-dimethyl-silane following a procedure analogous to that described in Step 4 of Intermediate 7. Yield: 80% of theory; LC (method 1): t_{R} = 1.31 min; Mass spectrum (ESI⁺): m/z = 471 [M+Na]⁺.

### Step 5: {(S)-6-[(R)-4-(1-methoxy-1,2,2-trimethyl-propyl)-indan-1-yloxy]-2,3-dihydro-benzofuran-3-yl}-acetic acid methyl ester

The title compound is prepared from (*S*)-4-(1-methoxy-1,2,2-trimethyl-propyl)-indan-1-ol and (*S*)-(6-hydroxy-2,3-dihydro-benzofuran-3-yl)-acetic acid methyl ester following a procedure analogous to that described in Step 3 of Intermediate 1. Yield: 47% of theory; LC (method 1): t_{R} = 1.54 min; Mass spectrum (ESI⁺): m/z = 461 [M+Na]⁺.

### Intermediate 74

### {(S)-6-[(R)-4-(1,1-difluoro-2-methoxy-ethyl)-indan-1-yloxy]-2,3-dihydro-benzofuran-3-yl}-acetic acid methyl ester

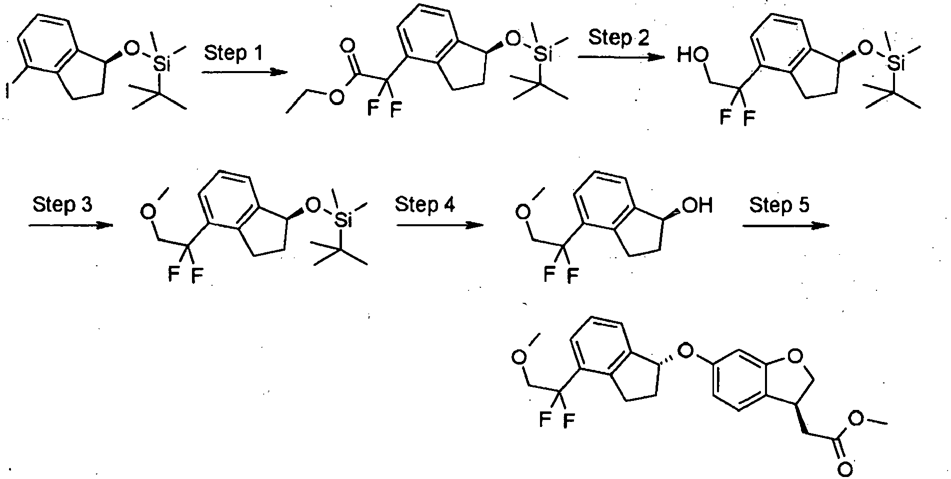

### Step 1: [(S)-1-(tert-butyl-dimethyl-silanyloxy)-indan-4-yl]-difluoro-acetic acid ethyl ester

A mixture of tert-butyl-[(*S*)-4-iodo-indan-1-yloxy]-dimethyl-silane (2.43 g), bromo-difluoro-acetic acid ethyl ester (1.1 mL), Cu powder (1.65 g), and dimethylsulfoxide (25 mL) is stirred under Ar atmosphere at 60 °C for 6 h. Ethyl acetate and aqueous NH₄Cl solution are added and the resulting mixture is extracted with ethyl acetate. The combined extracts are washed with water and dried (Na₂SO₄). The solvent is evaporated and the residue is chromatographed on silica gel (cyclohexane/ethyl acetate) to give the title compound. Yield: 2.13 g (89% of theory); Mass spectrum (ESI⁺): m/z = 388 [M+NH₄]⁺.

### Step 2: 2-[(S)-1-(tert-butyl-dimethyl-silanyloxy)-indan-4-yl]-2,2-difluoro-ethanol

Sodium borohydride (0.20 g) is added to a solution of [(*S*)-1-(tert-butyl-dimethyl-silanyloxy)-indan-4-yl]-difluoro-acetic acid ethyl ester (0.37 g) in methanol (25 mL) chilled in an ice bath. The mixture is stirred in the cooling bath while warming to room temperature overnight. Brine is added and the methanol is evaporated. The residue is extracted with ethyl acetate and the combined extracts are dried (Na₂SO₄) and concentrated to give the crude title compound.

### Step 3: tert-butyl-[(S)-4-(1,1-difluoro-2-methoxy-ethyl)-indan-1-yloxy]-dimethyl-silane

NaH (ca. 60% in mineral oil; 22 mg) is added to a solution of 2-[(*S*)-1-(tert-butyl-dimethyl-silanyloxy)-indan-4-yl]-2,2-difluoro-ethanol (0.17 g) in tetrahydrofuran (4 mL) at room temperature. The mixture is stirred at room temperature for 20 min prior to the addition of methyl iodide (17 µL). The mixture is stirred overnight and then water is added. The resulting mixture is extracted with ethyl acetate and the combined extracts are dried (Na₂SO₄) and concentrated to give the crude title compound.

### Step 4: (S)-4-(1,1-difluoro-2-methoxy-ethyl)-indan-1-ol

The title compound is prepared from tert-butyl-[(*S*)-4-(1,1-difluoro-2-methoxy-ethyl)-indan-1-yloxy]-dimethyl-silane following a procedure analogous to that described in Step 4 of Intermediate 7.

### Step 5: {(S)-6-[(R)-4-(1,1-difluoro-2-methoxy-ethyl)-indan-1-yloxy]-2,3-dihydro-benzofuran-3-yl}-acetic acid methyl ester

The title compound is prepared from (*S*)-4-(1,1-difluoro-2-methoxy-ethyl)-indan-1-ol and (*S*)-(6-hydroxy-2,3-dihydro-benzofuran-3-yl)-acetic acid methyl ester following a procedure analogous to that described in Step 3 of Intermediate 1.

### Intermediate 75

### {(S)-6-[(R)-4-(2-Methyl-naphthalen-1-yl)-indan-1-yloxy]-2,3-dihydro-benzofuran-3-yl}-acetic acid methyl ester

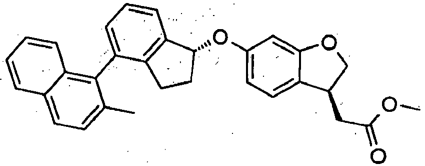

The title compound is prepared from 1-bromo-2-methyl-naphthalene and {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester following a procedure analogous to that described in Intermediate 44. Yield: 77% of theory; LC (method 6): t_{R} = 1.77 min; Mass spectrum (ESI⁺): m/z = 487 [M+Na]⁺.

### Intermediate 76

### {(S)-6-[(R)-4-(Tetrahydro-pyran-4-carbonyl)-indan-1-yloxy]-2,3-dihydro-benzofuran-3-yl}-acetic acid

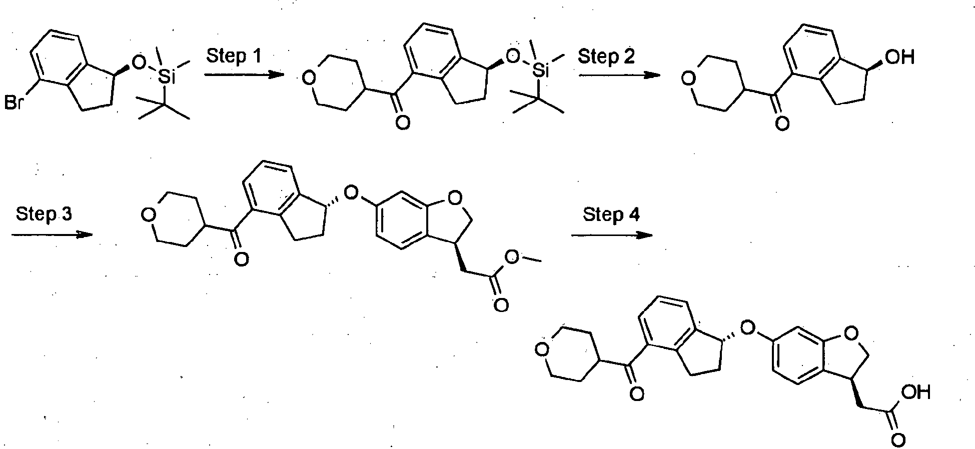

### Step 1: [(S)-1-(tert-butyl-dimethyl-silanyloxy)-indan-4-yl]-(tetrahydro-pyran-4-yl)-methanone

n-Butyl lithium (1.6 mol/L in hexane, 1.0 mL) is added to a solution of [(*S*)-4-bromo-indan-1-yloxy]-tert-butyl-dimethyl-silane (0.49 g) in tetrahydrofuran (10 mL) cooled to -78 °C. The resulting solution is stirred at -78 °C for 1 h prior to the addition of tetrahydro-pyran-4-carboxylic acid methoxy-methyl-amide (0.30 g). The solution is stirred at -78 °C for 1 h and then warmed to room temperature in the cooling bath overnight. Aqueous NaHCO₃ solution is added and resulting mixture is extracted with ethyl acetate. The combined extract is dried (Na₂SO₄) and concentrated to give the crude title compound. Yield: 0.52 g (97% of theory).

### Step 2: [(S)-1-(tert-butyl-dimethyl-silanyloxy)-indan-4-yl]-(tetrahydro-pyran-4-yl)-methanone

The title compound is prepared from tert-butyl-[(*S*)-4-(1,1-difluoro-2-methoxy-ethyl)-indan-1-yloxy]-dimethyl-silane following a procedure analogous to that described in Step 4 of Intermediate 7. Yield: 36% of theory; Mass spectrum (ESI⁺): m/z = 247 [M+H]⁺.

### Step 3: {(S)-6-[(R)-4-(tetrahydro-pyran-4-carbonyl)-indan-1-yloxy]-2,3-dihydrobenzofuran-3-yl}-acetic acid methyl ester

The title compound is prepared from [(*S*)-1-hydroxy-indan-4-yl]-(tetrahydro-pyran-4-yl)-methanone and (*S*)-(6-hydroxy-2,3-dihydro-benzofuran-3-yl)-acetic acid methyl ester following a procedure analogous to that described in Step 3 of Intermediate 1. Yield: 49% of theory; Mass spectrum (ESI⁺): m/z = 437 [M+H]⁺.

### Step 4: {(S)-6-[(R)-4-(tetrahydro-pyran-4-carbonyl)-indan-1-yloxy]-2,3-dihydrobenzofuran-3-yl}-acetic acid

The title compound is prepared from {(*S*)-6-[(R)-4-(tetrahydro-pyran-4-carbonyl)-indan-1-yloxy]-2,3-dihydro-benzofuran-3-yl}-acetic acid methyl ester following a procedure analogous to that described in Example 1. Yield: 74% of theory; Mass spectrum (ESI⁻): m/z = 421 [M-H]⁻.

### Intermediate 77

### {(S)-6-[(R)-4-pent-4-enoyl-indan-1-yloxy]-2,3-dihydro-benzofuran-3-yl}-acetic acid

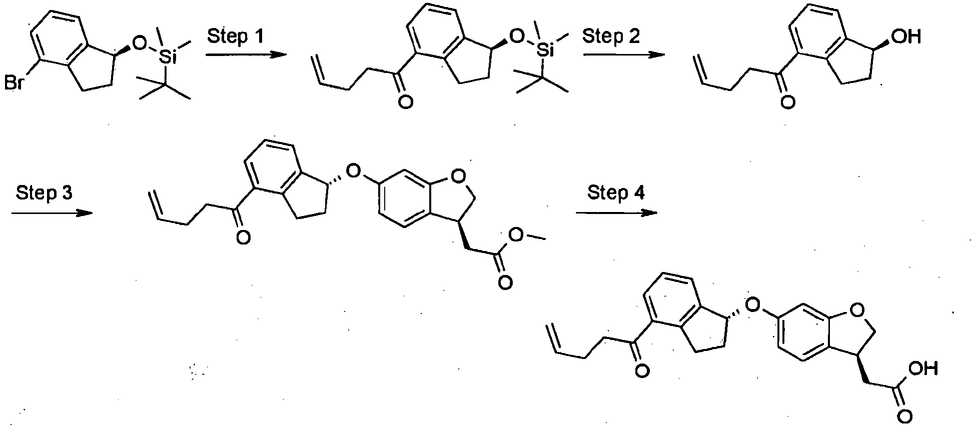

The title compound is obtained following the synthetic scheme depicted above and the corresponding procedures described for Intermediate 76. Pent-4-enoic acid methoxy-methyl-amide is used as electrophile in Step 1. Mass spectrum (ESI⁺): m/z = 393 [M+H]⁺.

### Intermediate 78

### {(S)-6-[(R)-4-(2-tetrahydro-pyran-4-yl-acetyl)-indan-1-yloxy]-2,3-dihydro-benzofuran-3-yl}-acetic acid

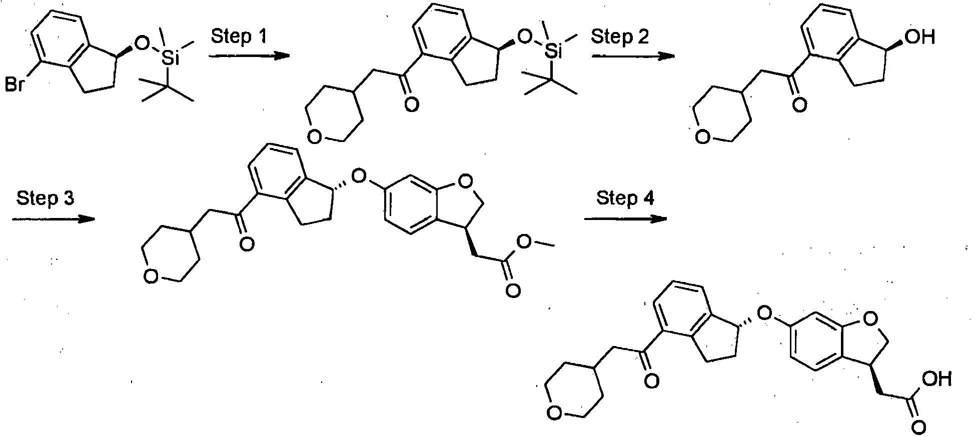

The title compound is obtained following the synthetic scheme depicted above and the corresponding procedures described for Intermediate 76. N-Methoxy-N-methyl-2-(tetrahydro-pycan-4-yl)-acetamide is used as electrophile in Step 1. Mass spectrum (ESI⁻): m/z = 435 [M-H]⁻.

### Intermediate 79

### {(S)-6-[(R)-4-(1,1-Difluoro-2-methoxy-2-methyl-propyl)-indan-1-yloxy]-2,3-dihydrobenzofuran-3-yl}-acetic acid methyl ester

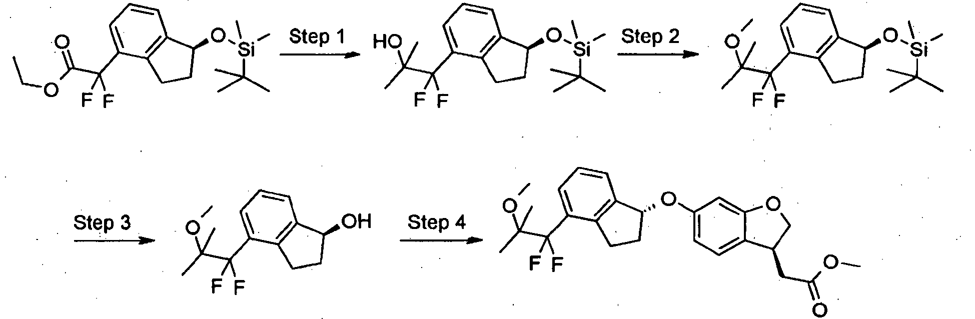

### Step 1: 1-[(S)-1-(tert-butyl-dimethyl-silanyloxy)-indan-4-yl]-1,1-difluoro-2-methyl-propan-2-ol

Methylmagnesium bromide (1.4 mol/l in tetrahydrofuran/toluene; 3.6 mL) is added to a solution of [(*S*)-1-(tert-butyl-dimethyl-silanyloxy)-indan-4-yl]-difluoro-acetic acid ethyl ester (0.37 g) in tetrahydrofuran (10 mL) chilled in an ice bath. The solution is warmed to room temperature in the cooling bath overnight. Aqueous NH₄Cl solution is slowly added and the resulting mixture is extracted with ethyl acetate. The combined extracts are dried (Na₂SO₄) and concentrated to give the crude title compound.

### Step 2: tert-butyl-[(S)-4-(1,1-difluoro-2-methoxy-2-methyl-propyl)-indan-1-yloxy]-dimethyl-silane

The title compound is prepared from 1-[(*S*)-1-(tert-butyl-dimethyl-silanyloxy)-indan-4-yl]-1,1-difluoro-2-methyl-propan-2-ol following a procedure analogous to that described in Step 3 of Intermediate 74.

### Step 3: (S)-4-(1,1-difluoro-2-methoxy-2-methyl-propyl)-indan-1-ol

The title compound is prepared from tert-butyl-[(*S*)-4-(1,1-difluoro-2-methoxy-2-methyl-propyl)-indan-1-yloxy]-dimethyl-silane following a procedure analogous to that described in Step 4 of Intermediate 7.

### Step 4: {(S)-6-[(R)-4-(1,1-difluoro-2-methoxy-2-methyl-propyl)-indan-1-yloxy]-2,3-dihydro-benzofuran-3-yl}-acetic acid methyl ester

The title compound is prepared from (*S*)-4-(1,1-difluoro-2-methoxy-2-methyl-propyl)-indan-1-ol and (*S*)-(6-hydroxy-2,3-dihydro-benzofuran-3-yl)-acetic acid methyl ester following a procedure analogous to that described in Step 3 of Intermediate 1.

### Intermediate 80

### {(S)-6-[(R)-4-(1,1-Difluoro-2-hydroxy-2-methyl-propyl)-indan-1-yloxy]-2,3-dihydro-benzofuran-3-yl}-acetic acid methyl ester

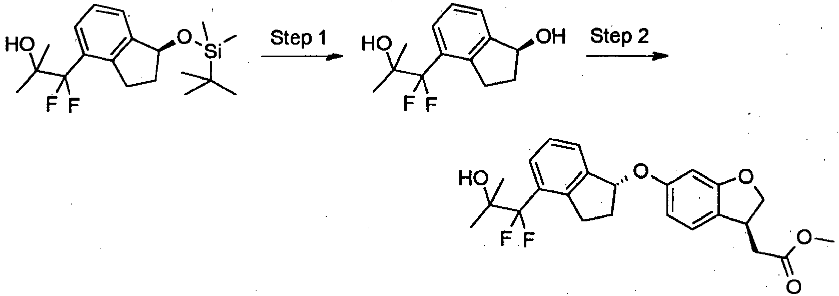

### Step 1: (S)-4-(1,1-difluoro-2-hydroxy-2-methyl-propyl)-indan-1-ol

The title compound is prepared from tert-butyl-[(*S*)-4-(1,1-difluoro-2-hydroxy-2-methyl-propyl)-indan-1-yloxy]-dimethyl-silane following a procedure analogous to that described in Step 4 of Intermediate 7.

### Step 2: {(S)-6-[(R)-4-(1,1-difluoro-2-hydroxy-2-methyl-propyl)-indan-1-yloxy]-2,3-dihydro-benzofuran-3-yl}-acetic acid methyl ester

The title compound is prepared from (*S*)-4-(1,1-difluoro-2-hydroxy-2-methyl-propyl)-indan-1-ol and (*S*)-(6-hydroxy-2,3-dihydro-benzofuran-3-yl)-acetic acid methyl ester following a procedure analogous to that described in Step 3 of Intermediate 1.

### Intermediate 81

### {(S)-6-[(R)-4-(4,6-dimethyl-pyrimidin-5-yl)-indan-1-yloxy]-2,3-dihydro-benzofuran-3-yl}-acetic acid methyl ester

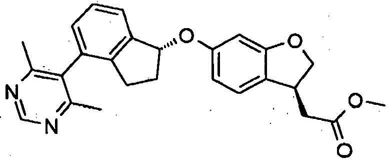

The title compound is prepared from 5-bromo-4,6-dimethyl-pyrimidine and {(*S*)-6-[(*R*)-4-(4,4,5,5-tetramethyl-[1,3,2]dioxaborolan-2-yl)-indan-1-yloxy]-2,3-dihydrobenzofuran-3-yl}-acetic acid methyl ester following a procedure analogous to that described in Intermediate 44. Yield: 47% of theory; LC (method 6): t_{R} = 1.14 min; Mass spectrum (ESI⁺): m/z = 431 [M+H]⁺.

### Intermediate 82

### {(S)-6-[4-(1-Methyl-cyclopropyl)-indan-1-yloxy]-2,3-dihydro-benzofuran-3-yl}-acetic acid methyl ester

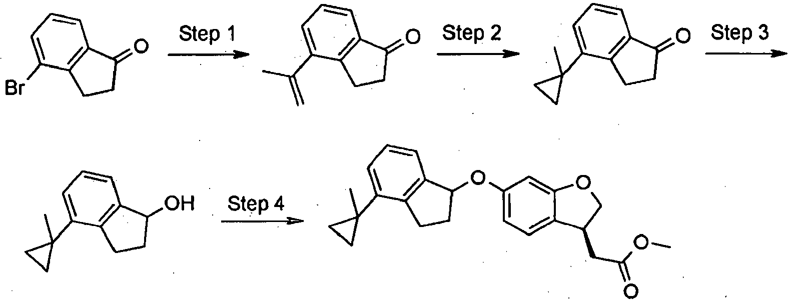

### Step 1: 4-isopropenyl-indan-1-one

The title compound is prepared from 4-bromo-indan-1-one and potassium isopropenyltrifluoroborate following a procedure analogous to that described in Intermediate 44. Yield: 67% of theory; LC (method 6): t_{R} = 0.67 min; Mass spectrum (ESI⁺): m/z = 173 [M+H]⁺.

### Step 2: 4-(1-methyl-cyclopropyl)-indan-1-one

Trifluoroacetic acid (0.20 mL) in dichloromethane (4 mL) is added to an ice-cold solution of diethylzinc (1 mol/l in hexane; 3 mL) in dichloromethane (4 mL). The solution is stirred for 20 min prior to the addition of diiodomethane (0.29 mL) in dichloromethane (4 mL). The solution is stirred for another 20 min and then 4-isopropenyl-indan-1-one (0.21 g) in dichloromethane (4 mL) is added. The solution is stirred at room temperature overnight. The solution is diluted with dichloromethane and washed with aqueous NH₄Cl solution. The solution is dried (MgSO₄) and concentrated. The residue is chromatographed on reversed phase (methanol/water/trifluoroacetic acid) to give the title compound. Yield: 0.07 g (32% of theory); LC (method 6): tR = 0.84 min; Mass spectrum (ESI⁺): m/z = 187 [M+N]⁺.

### Step 3: 4-(1-methyl-cyclopropyl)-indan-1-ol

The title compound is prepared from 4-(1-methyl-cyclopropyl)-indan-1-one following a procedure analogous to that described in Step 2 of Intermediate 74. Yield: 84% of theory; LC (method 6): t_{R} = 0.93 min; Mass spectrum (ESI⁺): m/z = 171 [M+H]⁺.

### Step 4: {(S)-6-[4-(1-methyl-cyclopropyl)-indan-1-yloxy]-2,3-dihydro-benzofuran-3-yl}-acetic acid methyl ester

The title compound is prepared from 4-(1-methyl-cyclopropyl)-indan-1-ol and (*S*)-(6-hydroxy-2,3-dihydro-benzofuran-3-yl)-acetic acid methyl ester following a procedure analogous to that described in Step 3 of Intermediate 1. Yield: 74% of theory; LC (method 6): t_{R} = 1.63 min; Mass spectrum (ESI⁺): m/z = 401 [M+Na]⁺.

### Intermediate 83

### {(S)-6-[(R)-4-(2,6-Dichloro-phenyl)-indan-1-yloxy]-2,3-dihydro-benzofuran-3-yl}-acetic acid methyl ester

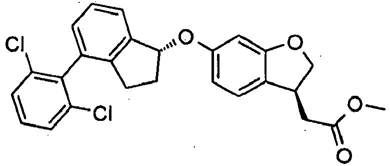

The title compound is prepared from 1,3-dichloro-2-iodo-benzene and {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester following a procedure analogous to that described in Intermediate 53; 1,4-dioxane is used as solvent at 85 °C.

### Intermediate 84

### {(S)-6-[(R)-4-(2.2,2-Trifluoro-acetyl)-indan-1-yloxy]-2,3-dihydro-benzofuran-3-yl}-acetic acid

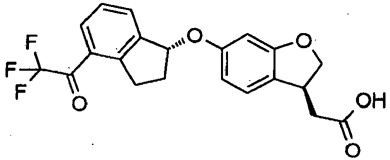

n-Butyl lithium (1.6 mol/L in hexane, 0.25 mL) is added to a solution of {(*S*)-6-[(*R*)-4-bromo-indan-1-yloxy]-2,3-dihydro-benzofuran-3-yl}-acetic acid (50 mg) in tetrahydrofuran (5 mL) cooled to -78 °C. The solution is stirred at -78 °C for 0.5 h prior to the addition of ethyl trifluoroacetate (0.10 mL) in tetrahydrofuran (1 mL). The solution is stirred at -78 °C for 1 h and then warmed to room temperature in the cooling bath overnight. Diethyl ether is added and the solution is washed with aqueous NH₄Cl solution. The solution is dried (Na₂SO₄) and concentrated to give the crude title compound. Yield: 51 mg (98% of theory); LC (method 6): t_{R} = 1.06 min; Mass spectrum (ESI⁻): m/z = 405 [M-H]⁻.

### Intermediate 85

### {(S)-6-[(R)-4-(2-Chloro-6-methyl-phenyl)-indan-1-yloxy]-2,3-dihydro-benzofuran-3-yl}-acetic acid methyl ester

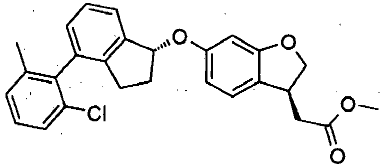

The title compound is prepared from 1-chloro-2-iodo-3-methyl-benzene and {(*S*)-6-[(*R*)-4-(4,4,5,5-tetramethyl-[1,3,2]dioxaborolan-2-yl)-indan-1-yloxy]-2,3-dihydrobenzofuran-3-yl}-acetic acid methyl ester following a procedure analogous to that described in Intermediate 53; 1,4-dioxane is used as solvent at 85 °C.

### Intermediate 86

### {(S)-6-[(R)-4-(6-Methoxy-3-trifluoromethyl-pyridin-2-yl)-indan-1-yloxy]-2,3-dihydrobenzofuran-3-yl}-acetic acid methyl ester

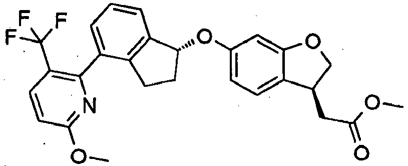

The title compound is prepared from 2-iodo-6-methoxy-3-trifluoromethyl-pyridine and {(*S*)-6-[(*R*)-4-(4,4,5,5-tetramethyl-[1,3,2]dioxaborolan-2-yl)-indan-1-yloxy]-2,3-dihydrobenzofuran-3-yl}-acetic acid methyl ester following a procedure analogous to that described in Intermediate 44. Yield: 67% of theory; LC (method 1): t_{R} = 1.44 min; Mass spectrum (ESI⁺): m/z = 500 [M+H]⁺.

### Intermediate 87

### {(S)-6-[(R)-4-(8-Methyl-naphthalen-1-yl)-indan-1-yloxy]-2,3-dihydro-benzofuran-3-yl}-acetic acid methyl ester

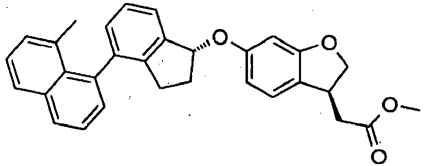

The title compound is prepared from 1-bromo-8-methyl-naphthalene and {(*S*)-6-[(*R*)-4-(4,4,5,5-tetramethyl-[1,3,2]dioxaborolan-2-yl)-indan-1-yloxy]-2,3-dihydrobenzofuran-3-yl}-acetic acid methyl ester following a procedure analogous to that described in Intermediate 44. Yield: 90% of theory; LC (method 1): t_{R} = 1.55 min; Mass spectrum (ESI⁺): m/z = 487 [M+Na]⁺.

### Intermediate 88

### {(S)-6-[(R)-4-(2,4-Dimethyl-pyridin-3-yl)-indan-1-yloxy]-2,3-dihydro-benzofuran-3-yl}-acetic acid methyl ester

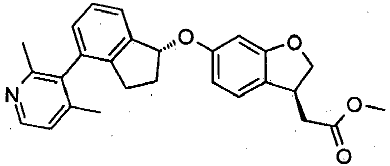

The title compound is prepared from 3-bromo-2,4-dimethyl-pyridine and {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester following a procedure analogous to that described in Intermediate 44. Yield: 47% of theory; LC (method 1): t_{R} = 1.06 min; Mass spectrum (ESI⁺): m/z = 430 [M+H]⁺.

### Intermediate 89

### {(S)-6-[(R)-4-(2-Pentafluoroethyl-phenyl)-indan-1-yloxy]-2,3-dihydro-benzofuran-3-yl}-acetic acid methyl ester

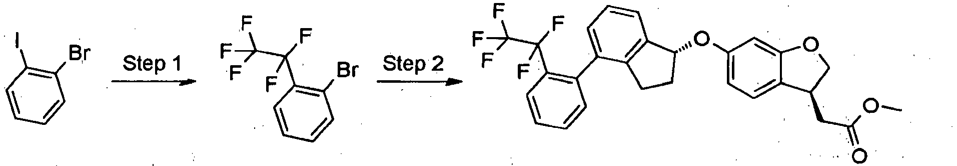

### Step 1: 1-bromo-2-pentafluoroethyl-benzene

Trimethyl-pentafluoroethyl-silane (1.0 mL) is added to a mixture of 1-bromo-2-iodobenzene (0.50 mL), KF (0.27 g), CuI (0.89 mg), and N,N-dimethylformamide (1 mL) under Ar atmosphere at room temperature. The reaction vessel is sealed and the mixture is stirred at 80 °C overnight. After cooling to room temperature, diethyl ether is added and the mixture is washed with aqueous NH₄Cl solution. The organic phase is dried (MgSO₄) and concentrated to give the crude title compound. Yield: 1.05 g (98% of theory); LC (method 7): t_{R} = 1.77 min.

### Step 2: {(S)-6-[(R)-4-(2-pentafluoroethyl-phenyl)-indan-1-yloxy]-2,3-dihydro-benzofuran-3-yl}-acetic acid methyl ester

The title compound is prepared from 1-bromo-2-pentafluoroethyl-benzene and {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester following a procedure analogous to that described in Intermediate 44. Yield: 68% of theory; LC (method 1): t_{R} = 1.69 min; Mass spectrum (ESI⁺): m/z = 541 [M+Na]⁺.

### Intermediate 90

### {(S)-6-[(R)-5-tert-Butyl-indan-1-yloxy]-2,3-dihydro-benzofuran-3-yl}-acetic acid methyl ester

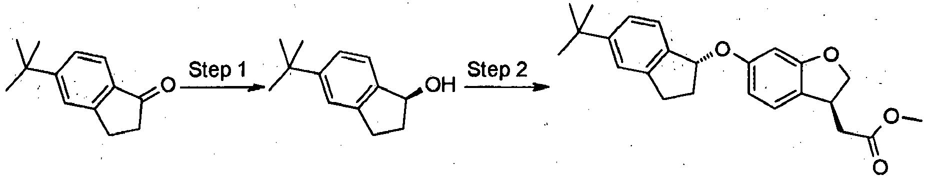

### Step 1: (S)-5-tert-butyl-indanol

The title compound is prepared from 5-tert-butyl-indan-1-one following a procedure analogous to that described in Step 2 of Intermediate 13. Yield: 84% of theory; LC (method 4): t_{R} = 2.07 min; Mass spectrum (ES⁺): m/z = 173 [M-OH]⁺.

### Step 2: {(S)-6-[(R)-5-tert-butyl-indan-1-yloxy]-2,3-dihydro-benzofuran-3-yl}-acetic acid methyl ester

The title compound is prepared from (*S*)-5-tert-butyl-indanol and (*S*)-(6-hydroxy-2,3-dihydro-benzofuran-3-yl)-acetic acid methyl ester following a procedure analogous to that described in Step 3 of Intermediate 1. Yield: 46% of theory; LC (method 1): t_{R} = 1.51 min; Mass spectrum (ESI⁺): m/z = 403 [M+Na]⁺.

### Intermediate 91

### {(S)-6-[(R)-4-(Cyano-dimethyl-methyl)-indan-1-yloxy]-2,3-dihydro-benzofuran-3-yl}-acetic acid methyl ester

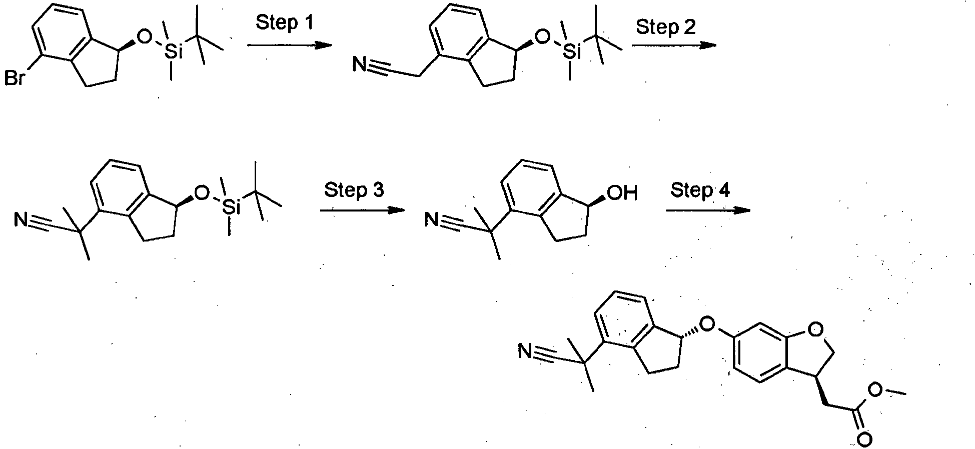

### Step 1: (S)-[1-(tert-butyl-dimethyl-silanyloxy)-indan-4-yl]-acetonitrile

Bis(diphenylphosphino)ferrocene-dichloropalladium dichloromethane adduct (0.47 g) is added to a flask charged with a stir bar, (*S*)-(4-bromo-2,3-dihydro-1H-inden-1-yloxy)-tert-butyl-dimethylsilane (2.10 g), 4-(4,4,5,5-tetramethyl-1,3,2-dioxaborolan-2-yl)isoxazole (1.50 g), Cs₂CO₃ (6.27 g), N,N-dimethylformamide (50 mL), and water (10 mL) under Ar atmosphere at room temperature. The mixture is stirred at 90 °C overnight. After cooling to room temperature, water is added and the mixture is extracted with ethyl acetate. The combined organic extracts are washed with water and brine and dried (Na₂SO₄). The solvent is evaporated and the residue is chromatographed on silica gel (heptane/ethyl acetate) to give the title compound. Yield: 1.84 g (37% of theory); LC (method 4): t_{R} = 2.37 min.

### Step 2: (S)-2-[1-(tert-butyl-dimethyl-silanyloxy)-indan-4-yl]-2-methyl-propionitrile

Potassium hexamethyldisilazide (0.5 mol/L in toluene; 2.1 mL) is added to a solution of (*S*)-[1-(tert-butyl-dimethyl-silanyloxy)-indan-4-yl]-acetonitrile (0.30 g) in tetrahydrofuran (10 mL) cooled to -60 °C. The solution is stirred at -60 °C for 20 min prior to the addition of methyl iodide (65 µL). The solution is stirred at -60 °C for another 30 min prior to the addition of another portion of potassium hexamethyldisilazide (0.5 mol/L in toluene; 2.1 mL) and after another 20 min of stirring of another portion of methyl iodide (65 µL). The solution is stirred for another 2 h before potassium hexamethyldisilazide (0.5 mol/L in toluene; 2.1 mL) is added followed after another 5 min by methyl iodide (65 µL). The solution is stirred at -50 °C for 1 h and then diluted with diethyl ether. The solution is washed with 1 M aqueous HCl solution and aqueous Na₂S₂O₃ solution and dried (Na₂SO₄). The solvent is evaporated and the residue is chromatographed on silica gel (cyclohexane/ethyl acetate) to give the title compound. Yield: 0.24 g (72% of theory); LC (method 1): t_{R} = 1.51 min; Mass spectrum (ESI⁺): m/z = 338 [M+Na]⁺.

### Step 3: (S)-2-(1-hydroxy-indan-4-yl)-2-methyl-propionitrile

The title compound is prepared from (*S*)-2-[1-(tert-butyl-dimethyl-silanyloxy)-indan-4-yl]-2-methyl-propionitrile following a procedure analogous to that described in Step 4 of Intermediate 7. Yield: 76% of theory; LC (method 1): t_{R} = 0.91 min; Mass spectrum (ESI⁺): m/z = 184 [M-OH]⁺.

### Step 4: {(S)-6-[4-(cyano-dimethyl-methyl)-indan-1-yloxy]-2,3-dihydro-benzofuran-3-yl}-acetic acid methyl ester

The title compound is prepared from (*S*)-2-(1-hydroxy-indan-4-yl)-2-methyl-propionitrile and (*S*)-(6-hydroxy-2,3-dihydro-benzofuran-3-yl)-acetic acid methyl ester following a procedure analogous to that described in Step 3 of Intermediate 1. Yield: 65% of theory; LC (method 1): t_{R} = 1.30 min; Mass spectrum (ESI⁺): m/z = 414 [M+Na]⁺.

### Intermediate 92

### {(S)-6-[(R)-4-Chloro-5-trifluoromethyl-indan-1-yloxy]-2,3-dihydro-benzofuran-3-yl}-acetic acid methyl ester

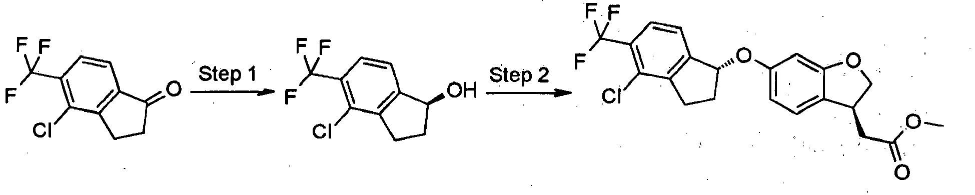

### Step 1: (S)-4-chloro-5-trifluoromethyl-indanol

The title compound is prepared from 4-chloro-5-trifluoromethyl-indan-1-one following a procedure analogous to that described in Step 2 of Intermediate 13. Yield: 55% of theory; Mass spectrum (ESI⁻): m/z = 235/237 (CI) [M-H]⁻.

### Step 2: {(S)-6-[(R)-4-chloro-5-trifluoromethyl-indan-1-yloxy]-2,3-dihydro-benzofuran-3-yl}-acetic acid methyl ester

The title compound is prepared from (*S*)-4-chloro-5-trifluoromethyl-indanol and (S)-(6-hydroxy-2,3-dihydro-benzofuran-3-yl)-acetic acid methyl ester following a procedure analogous to that described in Step 3 of Intermediate 1. Yield: 70% of theory; LC (method 6): t_{R} = 1.60 min; Mass spectrum (ESI⁺): m/z = 427/429 (CI) [M+H]⁺.

### Intermediate 93

### {(S)-6-[(R)-4-(3-Methyl-pyrazin-2-yl)-indan-1-yloxy]-2,3-dihydro-benzofuran-3-yl}-acetic acid methyl ester

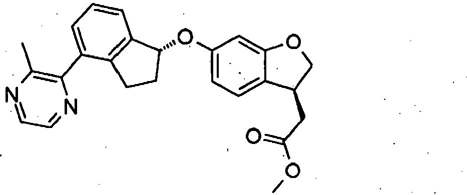

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and 2-chloro-3-methyl-pyrazine following a procedure analogous to that described in Example 53; the reaction is carried out in tetrahydrofuran at 100 °C. LC (method 7): t_{R} = 1.68 min; Mass spectrum (ESI⁺): m/z = 417 [M+H]⁺.

### Intermediate 94

### {(S)-6-[(R)-4-(3-Methoxy-2,6-dimethyl-phenyl)-indan-1-yloxy]-2,3-dihydro-benzofuran-3-yl}-acetic acid methyl ester

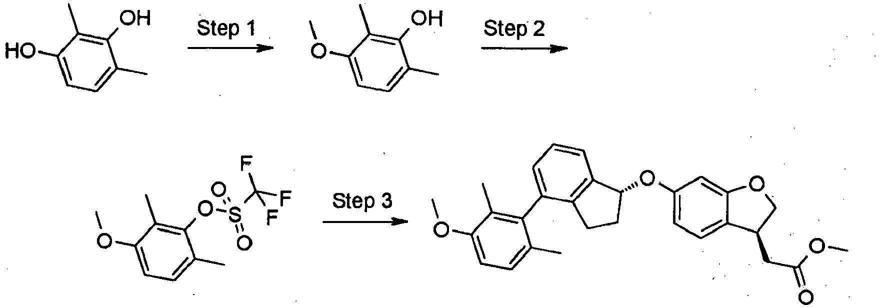

### Step 1: 3-methoxy-2,6-dimethyl-phenol

The title compound is prepared from 2,4-dimethyl-benzene-1,3-diol and iodomethane following a procedure analogous to that described in Step 3 of Intermediate 55. Yield: 55% of theory; LC (method 7): t_{R} = 1.28 min; Mass spectrum (ESI⁺): m/z = 153 [M+H]⁺.

### Step 2: trifluoro-methanesulfonic acid 3-methoxy-2,6-dimethyl-phenyl ester

Trifluoromethanesulfonic anhydride (0.10 mL) is added to a solution of 3-methoxy-2,6-dimethyl-phenol (65 mg) and lutidine (0.10 mL) in dichloromethane (2 mL) chilled in an ice bath. The cooling bath is removed and the solution is stirred at room temperature overnight. Dichloromethane is added and the solution is washed with 1 M aqueous HCl solution. The organic phase is dried (Na₂SO₄) and concentrated. The residue is chromatographed on silica gel (petrol ether/ethyl acetate) to give the title compound. Yield: 80 mg (75% of theory); LC (method 7): t_{R} = 1.79 min.

### Step 3: {(S)-6-[(R)-4-(3-methoxy-2,6-dimethyl-phenyl)-indan-1-yloxy]-2,3-dihydro-benzofuran-3-yl}-acetic acid methyl ester

The title compound is prepared from trifluoro-methanesulfonic acid 3-methoxy-2,6-dimethyl-phenyl ester and {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester following a procedure analogous to that described in Intermediate 44; the reaction is conducted in a mixture of tetrahydrofuran and toluene at 100 °C. Yield: 40% of theory; LC (method 6): t_{R} = 1.64 min; Mass spectrum (ESI⁺): m/z = 481 [M+Na]⁺.

### Intermediate 95

### {(S)-6-[(R)-4-Pentafluoroethyl-indan-1-yloxy]-2,3-dihydro-benzofuran-3-yl}-acetic acid methyl ester

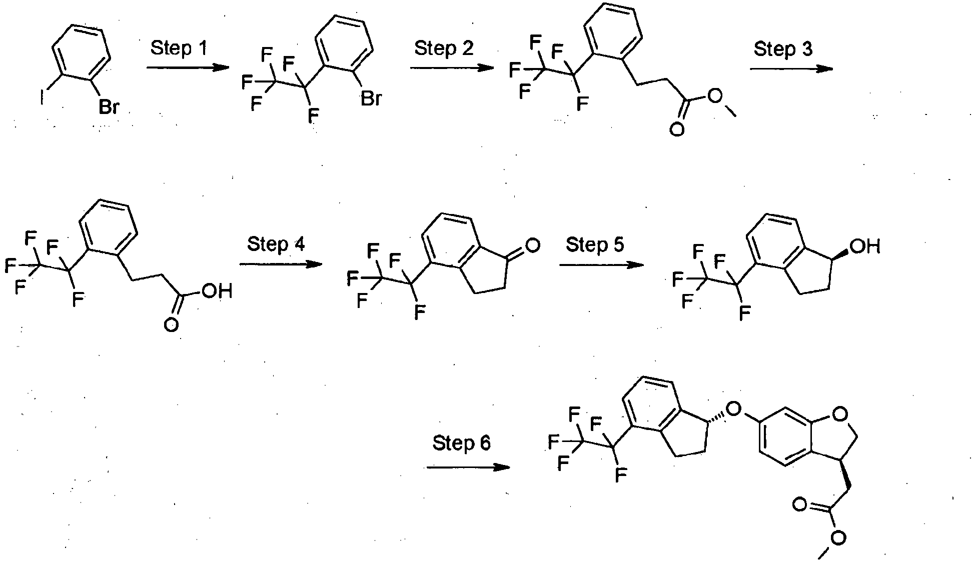

### Step 1: 1-bromo-2-pentafluoroethyl-benzene

The title compound is prepared as described in Step 1 of Intermediate 89.

### Step 2: 3-(2-pentafluoroethyl-phenyl)-propionic acid methyl ester

The title compound is prepared from 1-bromo-2-pentafluoroethyl-benzene and 3,3-dimethoxy-propene following a procedure analogous to that described in Step 1 of intermediate 30. Yield: 26% of theory; LC (method 6): t_{R} = 1.16 min.

### Step 3: 3-(2-pentafluoroethyl-phenyl)-propionic acid

The title compound is prepared from 3-(2-pentafluoroethyl-phenyl)-propionic acid methyl ester following a procedure analogous to that described in Example 1. Yield: 87% of theory; LC (method 6): t_{R} = 0.93 min; Mass spectrum (ESI⁻): m/z = 267 [M-H]⁻.

### Step 4: 4-pentafluoroethyl-indan-1-one

The title compound is prepared from 3-(2-pentafluoroethyl-phenyl)-propionic acid following a procedure analogous to that described in Step 3 of Intermediate 30; the reaction is carried out with and in chlorosulfonic acid at room temperature. Yield: 69% of theory; LC (method 6): t_{R} = 0.88 min; Mass spectrum (ESI⁺): m/z = 251 [M+H]⁺.

### Step 5: (S)-4-pentafluoroethyl-indan-1-ol

The title compound is prepared from 4-pentafluoroethyl-indan-1-one following a procedure analogous to that described in Step 2 of Intermediate 13. Yield: 80% of theory; LC (method 6): t_{R} = 0.97 min; Mass spectrum (ESI⁺): m/z = 235 [M-OH]⁺. Step 6: {(*S*)-6-[(*R*)-4-pentafluoroethyl-indan-1-yloxy]-2,3-dihydro-benzofuran-3-yl}-acetic acid methyl ester

The title compound is prepared from (*S*)-4-pentafluoroethyl-indan-1-ol and (S)-(6-hydroxy-2,3-dihydro-benzofuran-3-yl)-acetic acid methyl ester following a procedure analogous to that described in Step 3 of Intermediate 1. Yield: 68% of theory; LC (method 6): t_{R} = 1.60 min; Mass spectrum (ESI⁺): m/z = 443 [M+H]⁺.

### Intermediate 96

### {(S)-6-[(R)-6-Fluoro-4-trifluoromethyl-indan-1-yloxy]-2,3-dihydro-benzofuran-3-yl}-acetic acid methyl ester

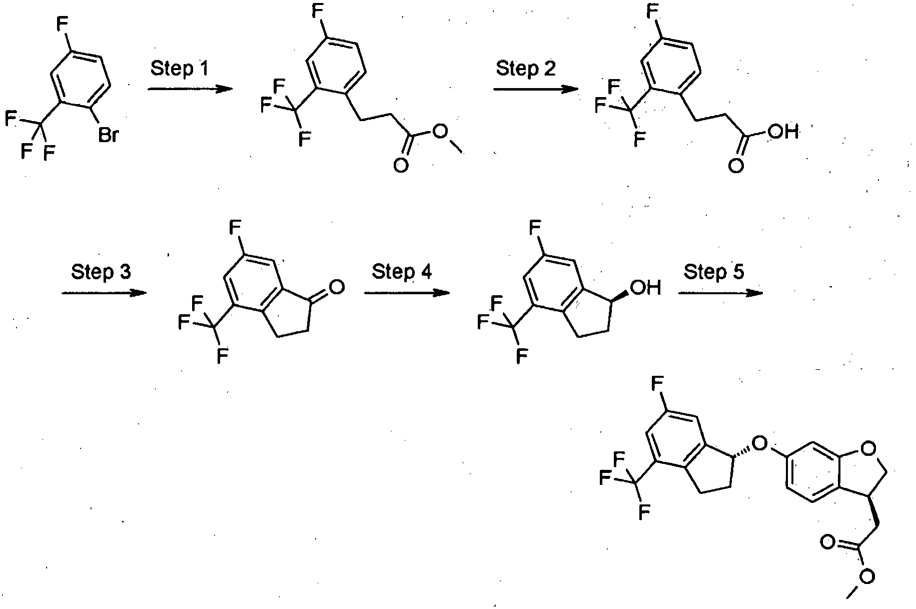

The title compound is obtained following the synthetic scheme depicted above and the corresponding procedures described for Intermediates 30 and 95. LC (method 7): t_{R} = 1.92 min; Mass spectrum (ESI⁺): m/z = 411 [M+H]⁺.

### Intermediate 97

### {(S)-6-[(R)-4-(2-Methoxy-4-trifluoromethyl-pyridin-3-yl)-indan-1-yloxy]-2,3-dihydro-benzofuran-3-yl}-acetic acid methyl ester

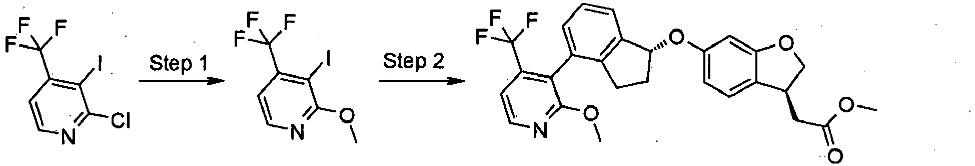

### Step 1: 3-iodo-2-methoxy-4-trifluoromethyl-pyridine

Sodium (18 mg) dissolved in methanol (0.5 mL) is added to a solution of 2-chloro-3-iodo-4-trifluoromethyl-pyridine (0.20 g) in methanol (1.5 mL) at room temperature. The solution is stirred at 60 °C overnight. After cooling to room temperature, diethyl ether is added and the solution is washed with water and brine. The organic phase is dried (Na₂SO₄) and concentrated. The residue is chromatographed on silica gel (dichloromethane) to give the title compound. Yield: 0.06 g (30% of theory); LC (method 1): t_{R} = 1.25 min; Mass spectrum (ESI⁺): m/z = 304 [M+H]⁺.

### Step 3: {(S)-6-[(R)-4-(2-methoxy-4-trifluoromethyl-pyridin-3-yl)-indan-1-yloxy]-2,3-dihydro-benzofuran-3-yl}-acetic acid methyl ester

The title compound is prepared from 3-iodo-2-methoxy-4-trifluoromethyl-pyridine and {(*S*)-6-[(*R*)-4-(4,4,5,5-tetramethyl-[1,3,2]dioxaborolan-2-yl)-indan-1-yloxy]-2,3-dihydrobenzofuran-3-yl}-acetic acid methyl ester following a procedure analogous to that described in Intermediate 44. Yield: 61% of theory; LC (method 1): t_{R} = 1.44 min; Mass spectrum (ESI⁺): m/z = 500 [M+H]⁺.

### Intermediate 98

### {(S)-6-[(R)-4-(3-Methoxy-2-methyl-phenyl)-indan-1-yloxy]-2,3-dihydro-benzofuran-3-yl}-acetic acid methyl ester

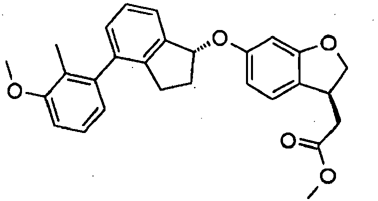

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and 1-bromo-3-methoxy-2-methyl-benzene following a procedure analogous to that described in Example 44. LC (method 7): t_{R} = 1.66 min; Mass spectrum (ESI⁺): m/z = 445 [M+H]⁺.

### Intermediate 98

### {7-Methyl-6-[(R)-4-trifluoromethyl-indan-1-yloxy]-2,3-dihydro-benzofuran-3-yl}-acetic acid methyl ester

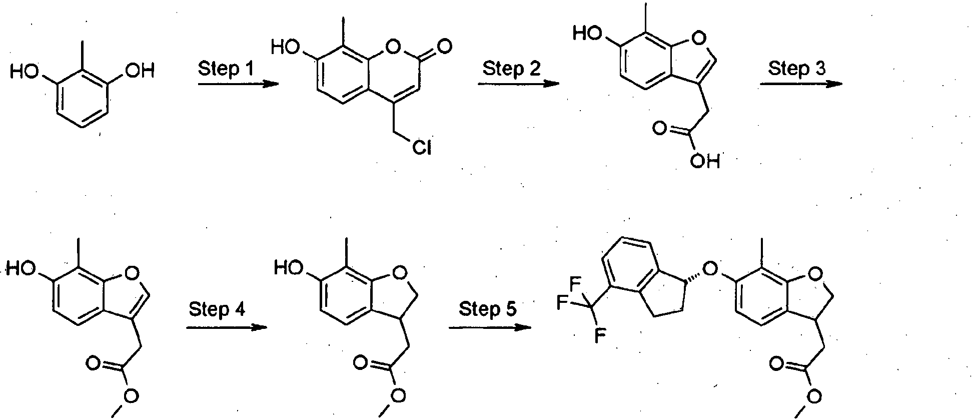

### Step 1: 4-chloromethyl-7-hydroxy-8-methyl-chromen-2-one

Ethyl 4-chloroacetoacetate (6.63 g) is added slowly to concentrated sulphuric acid (17 mL) at 0 °C with stirring. 2-Methylresorcinol (5.0 g) is added portionwise after which the mixture is stirred at room temperature for 3 hours. The mixture is poured slowly into a mixture of ice and water and the precipitated product collected by filtration, washed with water and dried under vacuum to give the title compound. Yield 8.29 g (91% of theory); Mass spectrum (ESI⁺): m/z = 225/227 (CI) [M+H]⁺.

### Step 2: (6-hydroxy-7-methyl-benzofuran-3-yl)-acetic acid

4-Chloromethyl-7-hydroxy-8-methyl-chromen-2-one (8.29 g) is suspended in 250 mL of 1 M NaOH in water and heated at reflux for 3 h. The mixture is cooled to 0 °C, acidified with concentrated sulphuric acid then extracted with ethyl acetate. The organic extract is dried over magnesium sulphate, filtered and the solvent removed under vacuum to give the title compound. Yield 7.35 g (96% of theory); Mass spectrum (ESI⁻): m/z 205 [M-H]⁻.

### Step 3: (6-hydroxy-7-methyl-benzofuran-3-yl)-acetic acid methyl ester

(6-Hydroxy-7-methyl-benzofuran-3-yl)-acetic acid (7.35 g) is dissolved in methanol (80 mL), concentrated sulphuric acid (2 mL) is added and the mixture heated at reflux for 3 h. The mixture is cooled to room temperature, concentrated under vacuum then partitioned between ethyl acetate and saturated aqueous sodium bicarbonate solution. The organic phase is washed with brine, dried over magnesium sulphate, filtered and the solvent removed. The residue is purified by flash chromatography on silica gel (cyclohexane/ethyl acetate 9:2) to give the title compound. Yield 2.93 g (38% of theory); Mass spectrum (ESI⁻): m/z 219 [M-H]⁻.

### Step 4: (6-hydroxy-7-methyl-2,3-dihydro-benzofuran-3-yl)-acetic acid methyl ester

(6-Hydroxy-7-methyl-benzofuran-3-yl)-acetic acid methyl ester (1.0 g) is suspended in methanol (100 mL) and hydrogenated at a pressure of 5 bar for 4 days using 10% palladium on activated carbon (100 mg) as the catalyst. The mixture is filtered through dicalite to remove the catalyst then the solvent is removed under vacuum. The residue is purified by flash chromatography on silica gel (cyclohexane/ethyl acetate 9:1) to give the title compound. Yield 467 mg (46% of theory); Mass spectrum (ESI⁻): m/z 223 [M+H]⁺.

### Step 5: {7-methyl-6-[(R)-4-trifluoromethyl-indan-1-yloxy]-2,3-dihydro-benzofuran-3-yl}-acetic acid methyl ester

(6-Hydroxy-7-methyl-2,3-dihydro-benzofuran-3-yl)-acetic acid methyl ester (200 mg) is dissolved in dry tetrahydrofuran (5 mL) and (*S*)-4-trifluoromethyl-indan-1-ol (181 mg) is added followed by triphenylphosphine (236 mg). The mixture is cooled to 0 °C and di-tert-butyl-azodicarboxylate (207 mg) is added. The mixture is stirred at 0 °C for 4 h then the solvent is removed under vacuum. The residue is purified by flash chromatography on silica gel (cyclohexane/ethyl acetate 9:1) to give the title compound. Yield 190 mg (52% of theory); Mass spectrum (ESI⁺): m/z 407 [M+H]⁺.

### Intermediate 99

### {4-Methyl-6-[(R)-4-trifluoromethyl-indan-1-yloxy]-2,3-dihydro-benzofuran-3-yl}-acetic acid methyl ester

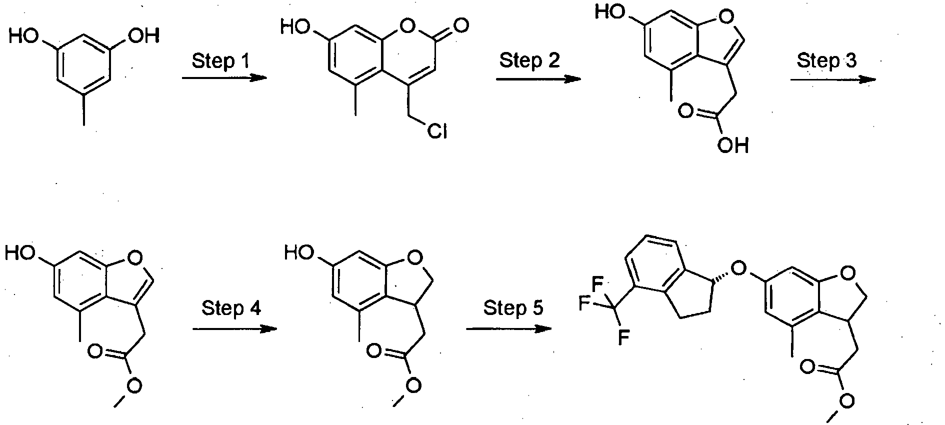

### Step 1: 4-chloromethyl-7-hydroxy-5-methyl-chromen-2-one

Ethyl 4-chloroacetoacetate (3.98 g) is added slowly to concentrated sulphuric acid (10 mL) at 0 °C with stirring. Orcinol (3.0 g) is added portionwise after which the mixture is stirred at room temperature for 3 h. The mixture is poured slowly into a mixture of ice and water and the precipitated product collected by filtration, washed with water and dried under vacuum to give the title compound. Yield 5.13 g (94% of theory); Mass spectrum (ESI⁺): m/z = 225/227 (CI) [M+H]⁺.

### Step 2: (6-hydroxy-4-methyl-benzofuran-3-yl)-acetic acid

4-Chloromethyl-7-hydroxy-5-methyl-chromen-2-one (5.13 g) is suspended in 125 mL of 1 M NaOH in water and heated at reflux for 3 hours. The mixture is cooled to 0 °C, acidified with concentrated sulphuric acid then extracted with ethyl acetate. The organic extract is dried over magnesium sulphate, filtered and the solvent removed under vacuum to give the title compound. Yield 4.27 g (90% of theory); Mass spectrum (ESI⁻): m/z 205 [M-H]⁻.

### Step 3: (6-hydroxy-4-methyl-benzofuran-3-yl)-acetic acid methyl ester

(6-Hydroxy-4-methyl-benzofuran-3-yl)-acetic acid (4.27 g) is dissolved in methanol (50 mL), concentrated sulphuric acid (1.5 mL) is added and the mixture heated at reflux for 3 h. The mixture is cooled to room temperature, concentrated under vacuum then partitioned between ethyl acetate and saturated aqueous sodium bicarbonate solution. The organic phase is washed with brine, dried over magnesium sulphate, filtered and the solvent removed. The residue is purified by flash chromatography on silica gel (cyclohexane/ethyl acetate 9:1) to give the title compound. Yield 3.70 g (81% of theory); Mass spectrum (ESI⁻): m/z 219 [M-H]⁻.

### Step 4: (6-hydroxy-4-methyl-2,3-dihydro-benzofuran-3-yl)-acetic acid methyl ester

(6-Hydroxy-4-methyl-benzofuran-3-yl)-acetic acid methyl ester (1.0 g) is suspended in methanol (150 mL) and hydrogenated at a pressure of 5 bar for 3 d using 10% palladium on activated carbon (100 mg) as the catalyst. The mixture is filtered through dicalite to remove the catalyst then the solvent is removed under vacuum. The residue is purified by flash chromatography on silica gel (cyclohexane/ethyl acetate 9:1) to give the title compound. Yield 470 mg (47% of theory); Mass spectrum (ESI⁺): m/z 223 [M+H]⁺.

### Step 5: {4-methyl-6-[(R)-4-trifluoromethyl-indan-1-yloxy]-2,3-dihydro-benzofuran-3-yl}-acetic acid methyl ester

(6-Hydroxy-4-methyl-2,3-dihydro-benzofuran-3-yl)-acetic acid methyl ester (200 mg) is dissolved in dry tetrahydrofuran (5 mL) and (*S*)-4-trifluoromethyl-indan-1-ol (181 mg) is added followed by triphenylphosphine (236 mg). The mixture is cooled to 0 °C and di-tert-butyl-azodicarboxylate (207 mg) is added. The mixture is stirred at 0 °C for 4 h then the solvent is removed under vacuum. The residue is purified by flash chromatography on silica gel (cyclohexane/ethyl acetate 9:1) to give the title compound. Yield 114 mg (31% of theory); Mass spectrum (ESI⁺): m/z 407 [M+H]⁺.

### Intermediate 100

### {5-Methyl-6-[(R)-4-trifluoromethyl-indan-1-yloxy]-2,3-dihydro-benzofuran-3-yl}-acetic acid methyl ester

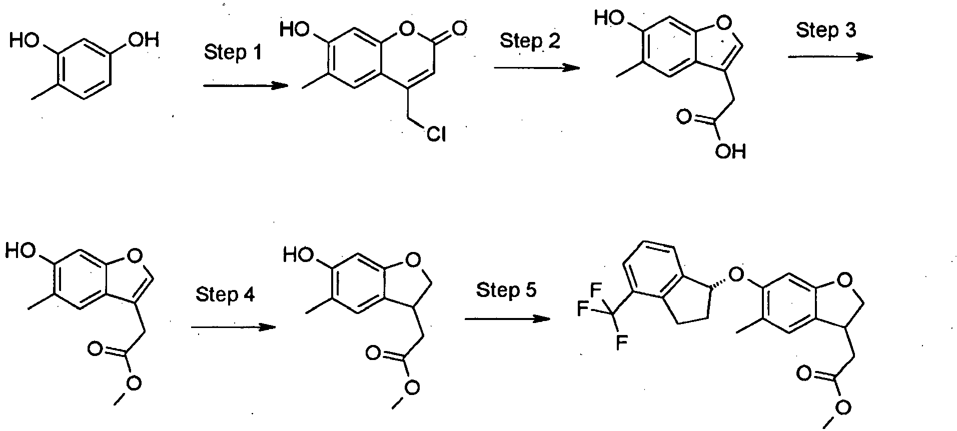

### Step 1: 4-chloromethyl-7-hydroxy-6-methyl-chromen-2-one

Ethyl 4-chloroacetoacetate (1.33 g) is added slowly to concentrated sulphuric acid (3.5 mL) at 0 °C with stirring. 4-Methylresorcinol (1.0 g) is added portionwise after which the mixture is stirred at room temperature overnight. The mixture is poured slowly into a mixture of ice and water and the precipitated product collected by filtration, washed with water and dried under vacuum to give the title compound. Yield 1.80g (99% of theory); Mass spectrum (ESI⁺): m/z = 225/227 (CI) [M+H]⁺.

### Step 2: (6-hydroxy-5-methyl-benzofuran-3-yl)-acetic acid

4-Chloromethyl-7-hydroxy-6-methyl-chromen-2-one (1.80 g) is suspended in 58 mL of 1 M NaOH in water and heated at reflux for 3 hours. The mixture is cooled to 0 °C, acidified with concentrated sulphuric acid then extracted with ethyl acetate. The organic extract is dried over magnesium sulphate, filtered and the solvent removed under vacuum to give the title compound. Yield 1.53 g (90% of theory); Mass spectrum (ESI⁻): m/z 205 [M-H]⁻.

### Step 3: (6-hydroxy-5-methyl-benzofuran-3-yl)-acetic acid methyl ester

(6-Hydroxy-5-methyl-benzofuran-3-yl)-acetic acid (1.80 g) is dissolved in methanol (21 mL), concentrated sulphuric acid (0.63 mL) is added and the mixture heated at reflux for 3 h. The mixture is cooled to room temperature, concentrated under vacuum then partitioned between ethyl acetate and saturated aqueous sodium bicarbonate solution. The organic phase is washed with brine, dried over magnesium sulphate, filtered and the solvent removed. The residue is purified by flash chromatography on silica gel (cyclohexane/ethyl acetate 9:1→9:2) to give the title compound. Yield 1.18 g (61 % of theory); Mass spectrum (ESI⁺): m/z 221 [M+H]⁺.

### Step 4: (6-hydroxy-5-methyl-2,3-dihydro-benzofuran-3-yl)-acetic acid methyl ester

(6-Hydroxy-5-methyl-benzofuran-3-yl)-acetic acid methyl ester (1.16 g) is suspended in methanol (50 mL) and hydrogenated at a pressure of 5 bar for 3 h using 10% palladium on activated carbon (100 mg) as the catalyst. The mixture is filtered through dicalite to remove the catalyst then the solvent is removed under vacuum. The residue is purified by flash chromatography on silica gel (cyclohexane/ethyl acetate 9:1) to give the title compound. Yield 1.02 mg (87% of theory); Mass spectrum (ESI⁺): m/z 223 [M+H]⁺.

### Step 5: [5-methyl-6-((R)-4-trifluoromethyl-indan-1-yloxy)-2,3-dihydro-benzofuran-3-yl]-acetic acid methyl ester

(6-Hydroxy-5-methyl-2,3-dihydro-benzofuran-3-yl)-acetic acid methyl ester (200 mg) is dissolved in dry tetrahydrofuran (5 mL) and (*S*)-4-trifluoromethyl-indan-1-ol (181 mg) is added followed by triphenylphosphine (236 mg). The mixture is cooled to 0 °C and di-tert-butyl-azodicarboxylate (207 mg) is added. The mixture is stirred at 0 °C for 4 h then the solvent is removed under vacuum. The residue is purified by flash chromatography on silica gel (cyclohexane/ethyl acetate 9:0.2) to give the title compound. Yield 175 mg (48% of theory); Mass spectrum (ESI⁺): m/z 407 [M+H]⁺.

### Example 1

### [6-(4-Trifluoromethyl-indan-1-yloxy)-2,3-dihydro-benzofuran-3-yl]-acetic acid

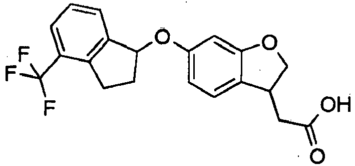

4 M aqueous NaOH solution (0.48 mL) is added to a solution of [6-(4-trifluoromethyl-indan-1-yloxy)-2,3-dihydro-benzofuran-3-yl]-acetic acid methyl ester (0.25 g) in tetrahydrofuran (0.5 mL) and methanol (0.5 mL) at room temperature. The mixture is stirred at room temperature for 2 h. The mixture is diluted with water and acidified using 1 M aqueous HCl solution. The resulting mixture is extracted with ethyl acatete, and the combined extract is washed with brine and dried (MgSO₄). The solvent is evaporated to give the title compound as a mixture of four stereoisomers. Yield: 0.24 g (quantitative); Mass spectrum (ESI⁺): m/z = 379 [M+H]⁺.

### Examples 2, 3, 4, and 5

### Stereoisomers of [6-(4-Trifluoromethyl-indan-1-yloxy)-2,3-dihydro-benzofuran-3-yl]-acetic acid

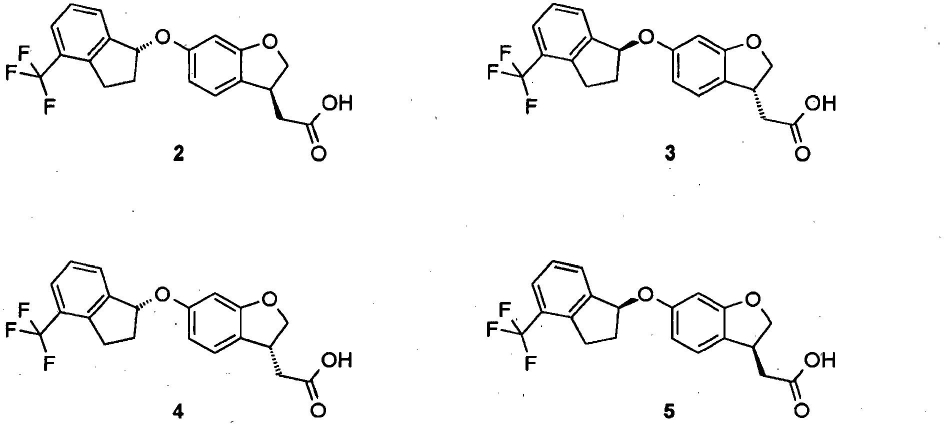

The mixture of stereoisomers obtained in Example 1 is separated by SFC on chiral phase (column: Daicel IC, 250 mm x 4.6 mm, 5 µm, 40 °C; eluent: CO₂/10% ethanol containing 0.2% diethylamine, 4 mL/min). Two stereoisomers are obtained in pure form and the two others in a 1:1 mixture with each other; the configurations of the stereocenters are arbitrarily assigned (retention times of the Examples on the SFC on chiral phase (conditions as described above): Example 2: t_{R} = 4.18 min; Examples 3 and 4: t_{R} = 4.73 min; Example 5: t_{R} = 5.74 min).

### Example 6

### {6-[(R)-4-Bromo-indan-1-yloxy]-2,3-dihydro-benzofuran-3-yl}-acetic acid

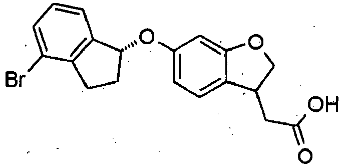

The title compound is prepared from {6-[(*R*)-4-bromo-indan-1-yloxy]-2,3-dihydrobenzofuran-3-yl}-acetic acid methyl ester following a procedure analogous to that described in Example 1. LC (method 1): t_{R} = 1.36 min; Mass spectrum (ESI⁺): m/z = 389/391 (Br) [M+H]⁺.
The enantiomeric pure compound, {(*S*)-6-[(*R*)-4-bromo-indan-1-yloxy]-2,3-dihydrobenzofuran-3-yl}-acetic acid, is obtained by chromatography of the diastereomeric mixture, Example 6, or by employing the enantiomerically pure starting material, {(*S*)-6-[(*R*)-4-bromo-indan-1-yloxy]-2,3-dihydro-benzofuran-3-yl}-acetic acid methyl ester, for saponification.

### Example 7

### {6-[(R)-4-(1-Methyl-1H-pyrazol-4-yl)-indan-1-yloxy]-2,3-dihydro-benzofuran-3-yl}-acetic acid

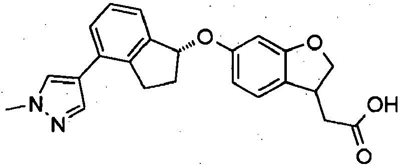

The title compound is prepared from {6-[(*R*)-4-(1-methyl-1H-pyrazol-4-yl)-indan-1-yloxy]-2,3-dihydro-benzofuran-3-yl}-acetic acid methyl ester following a procedure analogous to that described in Example 1. LC (method 1): t_{R} = 1.16 min; Mass spectrum (ESI⁺): m/z = 391 [M+H]⁺.

### Example 8

### {6-[(R)-4-(3,6-Dihydro-2H-pyran-4-yl)-indan-1-yloxy]-2,3-dihydro-benzofuran-3-yl}-acetic acid

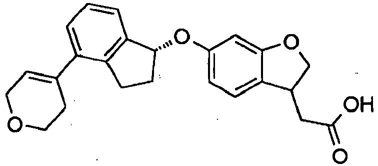

The title compound is prepared from {6-[(*R*)-4-(3,6-dihydro-2H-pyran-4-yl)-indan-1-yloxy]-2,3-dihydro-benzofuran-3-yl}-acetic acid methyl ester following a procedure analogous to that described in Example 1. LC (method 1): t_{R} = 1.26 min; Mass spectrum (ESI⁺): m/z = 393 [M+H]⁺.

### Example 9

### {6-[(R)-4-(1-Methyl-2-oxo-1,2-dihydro-pyridin-4-yl)-indan-1-yloxy]-2,3-dihydrobenzofuran-3-yl}-acetic acid

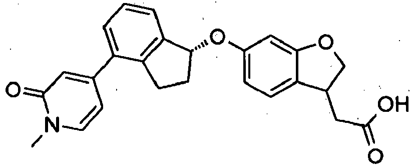

The title compound is prepared from {6-[(*R*)-4-(1-methyl-2-oxo-1,2-dihydro-pyridin-4-yl)-indan-1-yloxy]-2,3-dihydro-benzofuran-3-yl}-acetic acid methyl ester following a procedure analogous to that described in Example 1: LC (method 2): t_{R} = 1.34 min; Mass spectrum (ESI⁺): m/z = 418 [M+H]⁺.

### Example 10

### {6-[(R)-4-(4-Methoxy-phenyl)-indan-1-yloxy]-2,3-dihydro-benzofuran-3-yl}-acetic acid

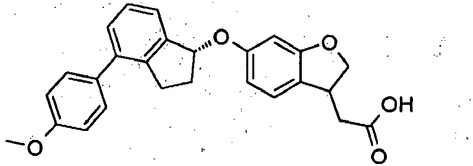

The title compound is prepared from {6-[(*R*)-4-(4-methoxy-phenyl)-indan-1-yloxy]-2,3-dihydro-benzofuran-3-yl}-acetic acid methyl ester following a procedure analogous to that described in Example 1. LC (method 2): t_{R} = 1.61 min; Mass spectrum (ESI⁺): m/z = 417 [M+H]⁺.

### Example 11

### {6-[4-(2,2-Difluoro-1-methoxy-ethyl)-indan-1-yloxy]-2,3-dihydro-benzofuran-3-yl}-acetic acid

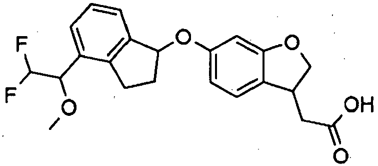

The title compound is prepared from {6-[4-(2,2-difluoro-1-methoxy-ethyl)-indan-1-yloxy]-2,3-dihydro-benzofuran-3-yl}-acetic acid methyl ester following a procedure analogous to that described in Example 1. LC (method 1): t_{R} = 1.23 min; Mass spectrum (ESI⁺): m/z = 405 [M+H]⁺.

### Example 12

### {6-[(R)-4-(Tetrahydro-pyran-4-yl)-indan-1-yloxy]-2,3-dihydro-benzofuran-3-yl}-acetic acid

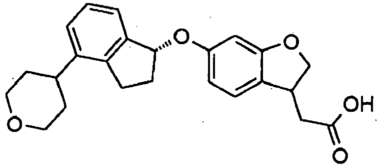

The title compound is prepared from {6-[(*R*)-4-(tetrahydro-pyran-4-yl)-indan-1-yloxy]-2,3-dihydro-benzofuran-3-yl}-acetic acid methyl ester following a procedure analogous to that described in Example 1. LC (method 1): t_{R} = 1.27 min; Mass spectrum (ESI⁺): m/z = 395 [M+H]⁺.

### Example 13

### {6-[4-(1-Methoxy-2,2-dimethyl-propyl)-indan-1-yloxy]-2,3-dihydro-benzofuran-3-yl}-acetic acid

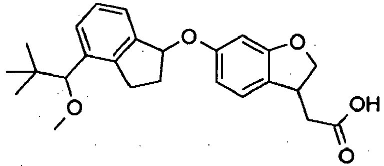

The title compound is prepared from {6-[4-(1-methoxy-2,2-dimethyl-propyl)-indan-1-yloxy]-2,3-dihydro-benzofuran-3-yl}-acetic acid methyl ester following a procedure analogous to that described in Example 1. LC (method 3): t_{R} = 1.45 min; Mass spectrum (ESI⁺): m/z = 411 [M+H]⁺.

### Example 14

### {(S)-6-[4-(Cyclobutyl-methoxy-methyl)-indan-1-yloxy]-2,3-dihydro-benzofuran-3-yl}-acetic acid

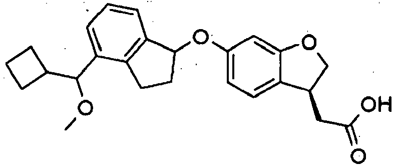

The title compound is prepared from {(*S*)-6-[4-(cyclobutyl-methoxy-methyl)-indan-1-yloxy]-2,3-dihydro-benzofuran-3-yl}-acetic acid methyl ester following a procedure analogous to that described in Example 1. LC (method 1): t_{R} = 1.39 min; Mass spectrum (ESI⁺): m/z = 409 [M+H]⁺.

### Example 15

### {6-[(R)-4-(5,5-Dimethyl-cyclopent-1-enyl)-indan-1-yloxy]-2,3-dihydro-benzofuran-3-yl}-acetic acid

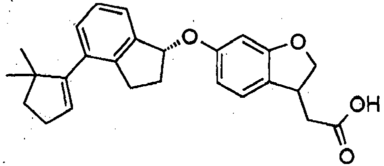

The title compound is prepared from {6-[(*R*)-4-(5,5-dimethyl-cyclopent-1-enyl)-indan-1-yloxy]-2,3-dihydro-benzofuran-3-yl}-acetic acid methyl ester following a procedure analogous to that described in Example 1. LC (method 1): t_{R} = 1.51 min; Mass spectrum (ESI⁺): m/z = 405 [M+H]⁺.

### Example 16

### {6-[(R)-4-(2,2-Dimethyl-cyclopentyl)-indan-1-yloxy]-2,3-dihydro-benzofuran-3-yl}-acetic acid

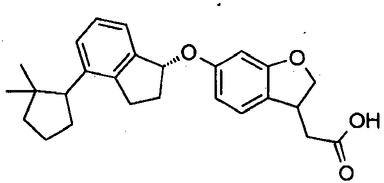

A mixture of {6-[(*R*)-4-(5,5-dimethyl-cyclopent-1-enyl)-indan-1-yloxy]-2,3-dihydro-benzofuran-3-yl}-acetic acid (40 mg), PtO₂ (20 mg), and ethyl acetate (3 mL) is shaken under hydrogen atmosphere (3 bar) at room temperature for 4 h. The catalyst is separated by filtration and the filtrate is concentrated to give the title compound. Yield: 40 mg (quantitative); LC (method 1): t_{R} = 1.52 min; Mass spectrum (ESI⁺): m/z = 407 [M+H]⁺.

### Example 17

### {(S)-6-[(R)-4-Difluoromethoxy-indan-1-yloxy]-2,3-dihydro-benzofuran-3-yl}-acetic acid

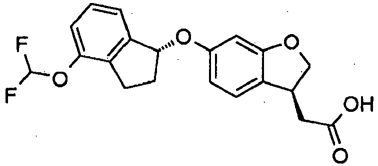

The title compound is prepared from {(*S*)-6-[(*R*)-4-difluoromethoxy-indan-1-yloxy]-2,3-dihydro-benzofuran-3-yl}-acetic acid methyl ester following a procedure analogous to that described in Example 1. LC (method 2): t_{R} = 1.31 min; Mass spectrum (ESI⁺): m/z = 377 [M+H]⁺.

### Example 18

### {(S)-6-[(R)-4-(1-Methoxy-cyclobutyl)-indan-1-yloxy]-2,3-dihydro-benzofuran-3-yl}-acetic acid

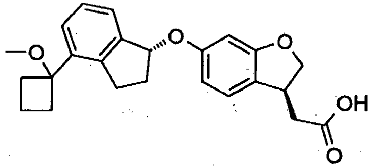

The title compound is prepared from {(*S*)-6-[(*R*)-4-(1-methoxy-cyclobutyl)-indan-1-yloxy]-2,3-dihydro-benzofuran-3-yl}-acetic acid methyl ester following a procedure analogous to that described in Example 1. LC (method 1): t_{R} = 1.35 min; Mass spectrum (ESI⁺): m/z = 395 [M+H]⁺.

### Example 19

### {(S)-6-[(R)-6-Trifluoromethyl-indan-1-yloxy]-2,3-dihydro-benzofuran-3-yl}-acetic acid

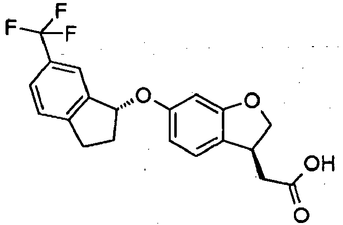

The title compound is prepared from {(*S*)-6-[(*R*)-6-trifluoromethyl-indan-1-yloxy]-2,3-dihydro-benzofuran-3-yl}-acetic acid methyl ester following a procedure analogous to that described in Example 1. LC (method 4): t_{R} = 1.98 min; Mass spectrum (ESI⁻): m/z = 377 [M-H]⁻.

### Example 20

### {(S)-6-[(R)-5-Trifluoromethyl-indan-1-yloxy]-2,3-dihydro-benzofuran-3-yl}-acetic acid

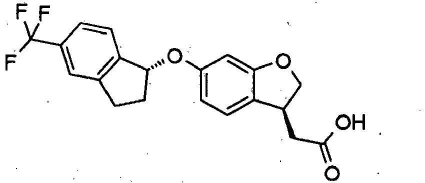

The title compound is prepared from {(*S*)-6-[(*R*)-5-trifluoromethyl-indan-1-yloxy]-2,3-dihydro-benzofuran-3-yl}-acetic acid methyl ester following a procedure analogous to that described in Example 1. LC (method 4): t_{R} = 2.00 min; Mass spectrum (ESI⁻): m/z = 377 [M-H]⁻.

### Example 21

### {(S)-6-[(R)-5-Chloro-indan-1-yloxy]-2,3-dihydro-benzofuran-3-yl}-acetic acid

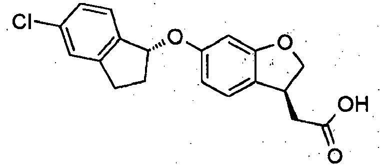

The title compound is prepared from {(*S*)-6-[(*R*)-5-chloro-indan-1-yloxy]-2,3-dihydro-benzofuran-3-yl}-acetic acid methyl ester following a procedure analogous to that described in Example 1. LC (method 5): t_{R} = 3.41 min; Mass spectrum (ESI⁻): m/z = 343/345 (Cl) [M-H]⁻.

### Example 22

### {(S)-6-[(R)-4-Chloro-indan-1-yloxy]-2,3-dihydro-benzofuran-3-yl}-acetic acid

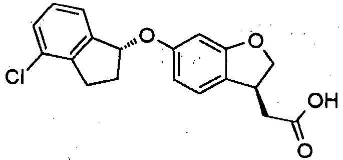

The title compound is prepared from {(*S*)-6-[(*R*)-4-chloro-indan-1-yloxy]-2,3-dihydro-benzofuran-3-yl}-acetic acid methyl ester following a procedure analogous to that described in Example 1. LC (method 4): t_{R} = 1.99 min; Mass spectrum (ESI⁻): m/z = 343/345 (CI) [M-H]⁻.

### Example 23

### {(S)-6-[(R)-7-Trifluoromethyl-indan-1-yloxy]-2,3-dihydro-benzofuran-3-yl}-acetic acid

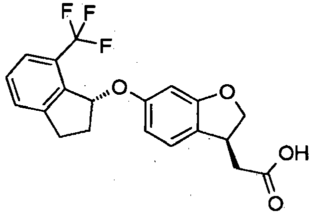

The title compound is prepared from {(*S*)-6-[(*R*)-7-trifluoromethyl-indan-1-yloxy]-2,3-dihydro-benzofuran-3-yl}-acetic acid methyl ester following a procedure analogous to that described in Example 1. LC (method 5): t_{R} = 3.33 min; Mass spectrum (ESI⁻): m/z = 377 [M-H]⁻.

### Example 24

### {(S)-6-[(R)-7-Fluoro-4-trifluoromethyl-indan-1-yloxy]-2,3-dihydro-benzofuran-3-yl}-acetic acid

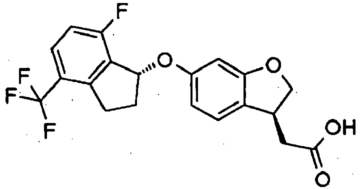

The title compound is prepared from {(*S*)-6-[(*R*)-7-fluoro-4-trifluoromethyl-indan-1-yloxy]-2,3-dihydro-benzofuran-3-yl}-acetic acid methyl ester following a procedure analogous to that described in Example 1. LC (method 4): t_{R} = 1.98 min; Mass spectrum (ESI⁻): m/z = 395 [M-H]⁻.

### Example 25

### {(S)-6-[(R)-4-Trifluoromethoxy-indan-1-yloxy]-2,3-dihydro-benzofuran-3-yl}-acetic acid

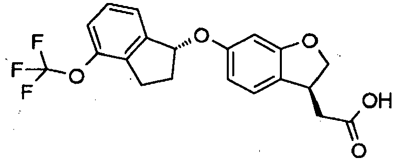

The title compound is prepared from {(*S*)-6-[(*R*)-4-trifluoromethoxy-indan-1-yloxy]-2,3-dihydro-benzofuran-3-yl}-acetic acid methyl ester following a procedure analogous to that described in Example 1. LC (method 4): t_{R} = 2.02 min; Mass spectrum (ESI⁻): m/z = 393 [M-H]⁻.

### Example 26

### {(S)-6-[(R)-6-Methyl-4-trifluoromethyl-indan-1-yloxy]-2,3-dihydro-benzofuran-3-yl}-acetic acid

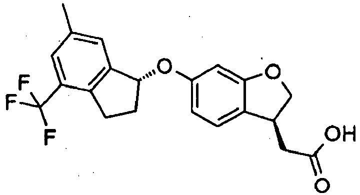

The title compound is prepared from {(*S*)-6-[(*R*)-6-methyl-4-trifluoromethyl-indan-1-yloxy]-2,3-dihydro-benzofuran-3-yl}-acetic acid methyl ester following a procedure analogous to that described in Example 1. LC (method 4): t_{R} = 2.05 min; Mass spectrum (ESI⁻): m/z = 391 [M-H]⁻.

### Example 27

### {(S)-6-[(R)-4-(1-Methoxy-2,2-dimethyl-propyl)-indan-1-yloxy]-2,3-dihydro-benzofuran-3-yl}-acetic acid (mixture of 2 diastereomers)

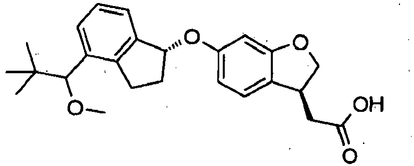

The title compound is prepared from {(*S*)-6-[(*R*)-4-(1-methoxy-2,2-dimethyl-propyl)-indan-1-yloxy]-2,3-dihydro-benzofuran-3-yl}-acetic acid methyl ester (mixture of 2 diastereomers) following a procedure analogous to that described in Example 1. LC (method 1): t_{R} = 1.43/1.45 min (diastereomeric mixture); Mass spectrum (ESI⁻): m/z = 409 [M-H]⁻.

### Example 28 and Example 29

### {(S)-6-[(R)-4-((R)*-1-Methoxy-2,2-dimethyl-propyl)-indan-1-yloxy]-2,3-dihydro-benzofuran-3-yl}-acetic acid (Example 28) and {(S)-6-[(R)-4-((S)*-1-Methoxy-2,2-dimethyl-propyl)-indan-1-yloxy]-2,3-dihydro-benzofuran-3-yl}-acetic acid (Example 29)

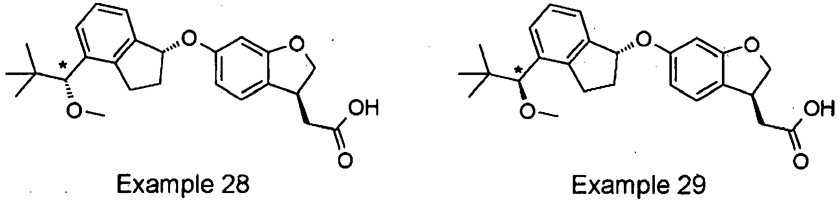

The title compounds are obtained in separate fractions upon SFC on chiral phase of the diastereomeric mixture of Example 27 (column: Daicel ADH, 250x4.6 mm; mobile phase: methanol containing 0.2% diethylamine/sc carbon dioxide 25:75; flow rate: 4 mL/min). The configuration of the stereocenter indicated (*) is arbitrarily assigned. Example 28: LC (method 1): t_{R} = 1.43 min; Mass spectrum (ESI⁻): m/z = 409 [M-H]⁻. Example 29: LC (method 1): t_{R} = 1.45 min; Mass spectrum (ESI⁻): m/z = 409 [M-H]⁻.

### Example 30

### {(S)-6-[(R)-7-Methyl-4-trifluoromethyl-indan-1-yloxy]-2,3-dihydro-benzofuran-3-yl}-acetic acid

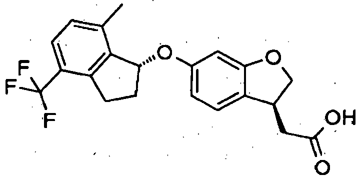

The title compound is prepared from {(*S*)-6-[(*R*)-7-methyl-4-trifluoromethyl-indan-1-yloxy]-2,3-dihydro-benzofuran-3-yl}-acetic acid methyl ester following a procedure analogous to that described in Example 1. LC (method 4): t_{R} = 2.01 min; Mass spectrum (ESI⁻): m/z = 391 [M-H]⁻.

### Example 31

### {(S)-6-[(R)-4-Cyclopropyl-indan-1-yloxy]-2,3-dihydro-benzofuran-3-yl}-acetic acid

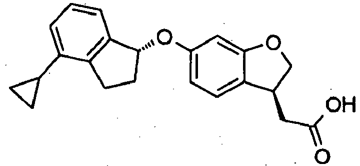

The title compound is prepared from {(*S*)-6-[(*R*)-4-cyclopropyl-indan-1-yloxy]-2,3-dihydro-benzofuran-3-yl}-acetic acid methyl ester following a procedure analogous to that described in Example 1. LC (method 4): t_{R} = 2.00 min; Mass spectrum (ESI⁻): m/z = 349 [M-H]⁻.

### Example 32

### {(S)-6-[(R)-4-Isopropyl-indan-1-yloxy]-2,3-dihydro-benzofuran-3-yl}-acetic acid

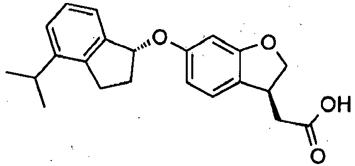

The title compound is prepared from {(*S*)-6-[(*R*)-4-isopropyl-indan-1-yloxy]-2,3-dihydro-benzofuran-3-yl}-acetic acid methyl ester following a procedure analogous to that described in Example 1. LC (method 4): t_{R} = 2.04 min; Mass spectrum (ESI⁻): m/z = 351 [M-H]⁻.

### Example 33

### {(S)-6-[(R)-4-(2,6-Dimethyl-phenyl)-indan-1-yloxy]-2,3-dihydro-benzofuran-3-yl}-acetic acid

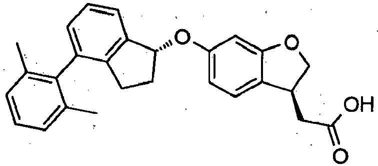

The title compound is prepared from {(*S*)-6-[(*R*)-4-(2,6-dimethyl-phenyl)-indan-1-yloxy]-2,3-dihydro-benzofuran-3-yl}-acetic acid methyl ester following a procedure analogous to that described in Example 1. LC (method 5): t_{R} = 3.74 min; Mass spectrum (ESI⁻): m/z = 413 [M-H]⁻.

### Example 34

### {(S)-6-[(R)-4-(2,2-Dimethyl-propyl)-indan-1-yloxy]-2,3-dihydro-benzofuran-3-yl}-aceti acid

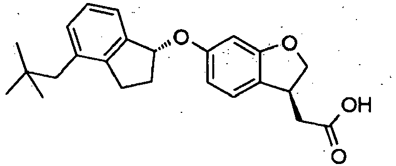

The title compound is prepared from {(*S*)-6-[(*R*)-4-(2,2-dimethyl-propyl)-indan-1-yloxy]-2,3-dihydro-benzofuran-3-yl}-acetic acid methyl ester following a procedure analogous to that described in Example 1. LC (method 5): t_{R} = 3.79 min; Mass spectrum (ESI⁻): m/z = 379 [M-H]⁻.

### Example 35

### {(S)-6-[(R)-4-(2-Isopropyl-phenyl)-indan-1-yloxy]-2,3-dihydro-benzofuran-3-yl}-acetic acid

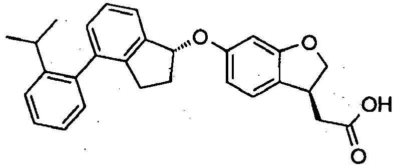

The title compound is prepared from {(*S*)-6-[(*R*)-4-(2-isopropyl-phenyl)-indan-1-yloxy]-2,3-dihydro-benzofuran-3-yl}-acetic acid methyl ester following a procedure analogous to that described in Example 1. LC (method 5): t_{R} = 3.76 min; Mass spectrum (ESI⁻): m/z = 427 [M-H]⁻.

### Example 36

### {(S)-6-[(R)-5-Methyl-4-trifluoromethyl-indan-1-yloxy]-2,3-dihydro-benzofuran-3-yl}-acetic acid

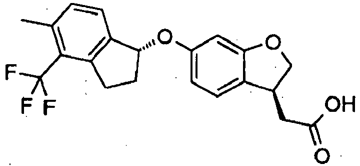

The title compound is prepared from {(*S*)-6-[(*R*)-5-methyl-4-trifluoromethyl-indan-1-yloxy]-2,3-dihydro-benzofuran-3-yl}-acetic acid methyl ester following a procedure analogous to that described in Example 1. LC (method 5): t_{R} = 3.52 min; Mass spectrum (ESI⁻): m/z = 391 [M-H]⁻.

### Example 37

### {(S)-6-[(R)-4,5-Difluoro-indan-1-yloxy]-2,3-dihydro-benzofuran-3-yl}-acetic acid

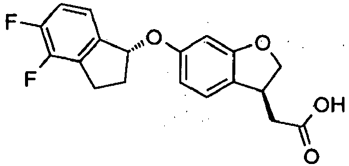

The title compound is prepared from {(*S*)-6-[(*R*)-4,5-difluoro-indan-1-yloxy]-2,3-dihydro-benzofuran-3-yl}-acetic acid methyl ester following a procedure analogous to that described in Example 1. LC (method 5): t_{R} = 3.25 min; Mass spectrum (ESI⁻): m/z = 345 [M-H]⁻.

### Example 38

### {(S)-6-[(R)-4-((R)*-2,2,2-Trifluoro-1-methoxy-ethyl)-indan-1-yloxy]-2,3-dihydro-benzofuran-3-yl}-acetic acid (configuration at the stereocenter indicated (*) is arbitrarily assigned)

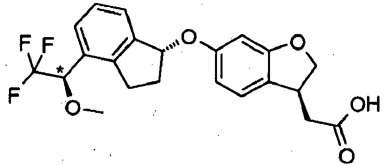

The title compound is prepared from {(*S*)-6-[(*R*)-4-((*R*)*-2,2,2-trifluoro-1-methoxy-ethyl)-indan-1-yloxy]-2,3-dihydro-benzofuran-3-yl}-acetic acid methyl ester following a procedure analogous to that described in Example 1; configuration at the stereocenter indicated (*) is arbitrarily assigned, diastereomer of Example 39. LC (method 5): t_{R} = 3.30 min; Mass spectrum (ESI⁻): m/z = 421 [M-H]⁻.

### Example 39

### {(S)-6-[(R)-4-((S)*-2,2,2-Trifluoro-1-methoxy-ethyl)-indan-1-yloxy]2,3-dihydrobenzofuran-3-yl}-acetic acid (configuration at the stereocenter indicated (*) is arbitrarily assigned)

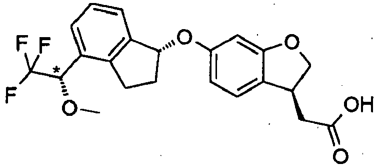

The title compound is prepared from {(*S*)-6-[(*R*)-4-((*S*)*-2,2,2-trifluoro-1-methoxy-ethyl)-indan-1-yloxy]-2,3-dihydro-benzofuran-3-yl}-acetic acid methyl ester following a procedure analogous to that described in Example 1; configuration at the stereocenter indicated (*) is arbitrarily assigned, diastereomer of Example 38. LC (method 5): t_{R} = 3.30 min; Mass spectrum (ESI⁻): m/z = 421 [M-H]⁻.

### Example 40

### {(S)-6-[(R)-4-(5,5-Dimethyl-cyclopent-1-enyl)-indan-1-yloxy]-2,3-dihydro-benzofuran-3-yl}-acetic acid

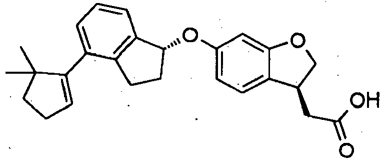

The title compound is prepared from {(*S*)-6-[(*R*)-4-(5,5-dimethyl-cyclopent-1-enyl)-indan-1-yloxy]-2,3-dihydro-benzofuran-3-yl}-acetic acid methyl ester following a procedure analogous to that described in Example 1. {(*S*)-6-[(*R*)-4-(5,5-dimethyl-cyclopent-1-enyl)-indan-1-yloxy]-2,3-dihydro-benzofuran-3-yl}-acetic acid methyl ester in turn is obtained from {(*S*)-6-[(*R*)-4-bromo-indan-1-yloxy]-2,3-dihydro-benzofuran-3-yl}-acetic acid methyl ester and 3-(5,5-dimethyl-cyclopent-1-enyl)-1,5-dimethyl-2,4-dioxa-3-bora-bicyclo[3.1.0]hexane as described for Intermediate 12. LC (method 4): t_{R} = 2.35 min; Mass spectrum (ESI⁺): m/z = 427 [M+Na]⁺.

Alternatively, the title compound is obtained by chromatography of the diastereomeric mixture Example 15.

### Example 41

### {(S)-6-[(R)-4-(3,3,4-Trimethyl-oxetan-2-yl)-indan-1-yloxy]-2,3-dihydro-benzofuran-3-yl}-acetic acid, configurations at the stereocenters indicated (*) not determined, diastereomer of Example 42

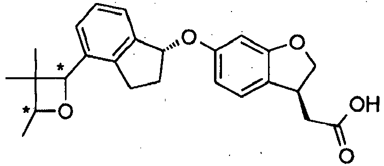

The title compound is prepared from {(*S*)-6-[(*R*)-4-(3,3,4-trimethyl-oxetan-2-yl)-indan-1-yloxy]-2,3-dihydro-benzofuran-3-yl}-acetic acid methyl ester, Intermediate 37, following a procedure analogous to that described in Example 1; configuration at the stereocenters indicated (*) not determined, diastereomer of Example 42. LC (method 6): t_{R} = 1.31 min; Mass spectrum (ESI⁻): m/z = 407 [M-H]⁻.

### Example 42

### {(S)-6-[(R)-4-(3,3,4-Trimethyl-oxetan-2-yl)-indan-1-yloxy]-2,3-dihydro-benzofuran-3-yl}-acetic acid, configurations at the stereocenters indicated (*) not determined, diastereomer of Example 41

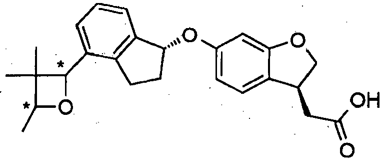

The title compound is prepared from {(*S*)-6-[(*R*)-4-(3,3,4-trimethyl-oxetan-2-yl)-indan-1-yloxy]-2,3-dihydro-benzofuran-3-yl}-acetic acid methyl ester, Intermediate 38, following a procedure analogous to that described in Example 1; configuration at the stereocenters indicated (*) not determined, diastereomer of Example 41. LC (method 6): t_{R} = 1.26 min; Mass spectrum (ESI⁻): m/z = 407 [M-H]⁻.

### Example 43

### {(S)-[6-(Indan-1-yloxy)-2,3-dihydro-benzofuran-3-yl]-acetic acid

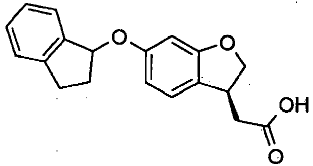

The title compound is prepared from [(*S*)-6-(indan-1-yloxy)-2,3-dihydro-benzofuran-3-yl]-acetic acid methyl ester following a procedure analogous to that described in Example 1. LC (method 8): t_{R} = 5.87 min; Mass spectrum (ESI⁻): m/z = 309 [M-H]⁻.

### Example 44

### {(S)-6-[(R)-4-Chloro-5-fluoro-indan-1-yloxy]-2,3-dihydro-benzofuran-3-yl}-acetic acid

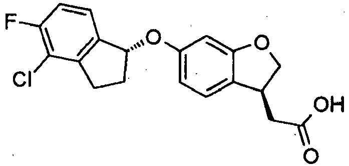

The title compound is prepared from {(*S*)-6-[(*R*)-4-chloro-5-fluoro-indan-1-yloxy]-2,3-dihydro-benzofuran-3-yl}-acetic acid methyl ester following a procedure analogous to that described in Example 1. LC (method 5): t_{R} = 3.65 min; Mass spectrum (ESI⁻): m/z = 361/363 (Cl) [M-H]⁻.

### Example 45

### {(S)-[(R)-6-(4-Cyano-indan-1-yloxy)-2,3-dihydro-benzofuran-3-yl]-acetic acid

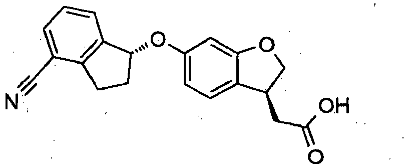

The title compound is prepared from {(*S*)-[(*R*)-6-(4-cyano-indan-1-yloxy)-2,3-dihydro-benzofuran-3-yl]-acetic acid methyl ester following a procedure analogous to that described in Example 1. LC (method 9): t_{R} = 7.24 min; Mass spectrum (ESI⁺): m/z = 336 [M+H]⁺.

### Example 46

### {(S)-6-[(R)-4-(3-Cyano-pyridin-2-yl)-indan-1-yloxy]-2,3-dihydro-benzofuran-3-yl}-acetic acid

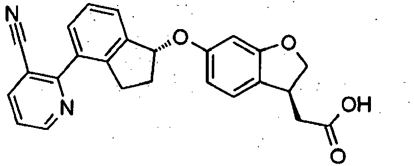

The title compound is prepared from {(*S*)-6-[(*R*)-4-(3-cyano-pyridin-2-yl)-indan-1-yloxy]-2,3-dihydro-benzofuran-3-yl}-acetic acid methyl ester following a procedure analogous to that described in Example 1. LC (method 1): t_{R} = 1.13 min; Mass spectrum (ESI⁻): m/z = 411 [M-H]⁻.

### Example 47

### {(S)-6-[(R)-4-(8-Oxa-spiro[4.5]dec-1-en-1-yl)-indan-1-yloxy]-2,3-dihydro-benzofuran-3-yl}-acetic acid

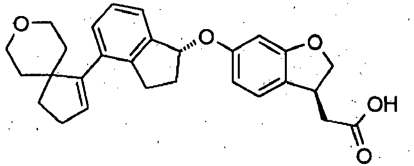

The title compound is prepared from {(*S*)-6-[(*R*)-4-(8-oxa-spiro[4.5]dec-1-en-1-yl)-indan-1-yloxy]-2,3-dihydro-benzofuran-3-yl}-acetic acid methyl ester following a procedure analogous to that described in Example 1. LC (method 1): t_{R} = 1.40 min; Mass spectrum (ESI⁺): m/z = 447 [M+H]⁺.

### Example 48

### {(S)-6-[(R)-4-(2,6,6-Trimethyl-cyclohex-1-enyl)-indan-1-yloxy]-2,3-dihydrobenzofuran-3-yl}-acetic acid

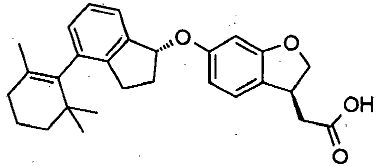

The title compound is prepared from {(*S*)-6-[(*R*)-4-(2,6,6-trimethyl-cyclohex-1-enyl)-indan-1-yloxy]-2,3-dihydro-benzofuran-3-yl}-acetic acid methyl ester following a procedure analogous to that described in Example 1. LC (method 6): t_{R} = 1.81 min; Mass spectrum (ESI⁻): m/z = 431 [M-H]⁻.

### Example 49

### {(S)-6-[(R)-4-(1,3,5-Trimethyl-1H-pyrazol-4-yl)-indan-1-yloxy]-2,3-dihydro-benzofuran-3-yl}-acetic acid

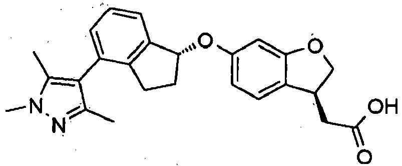

The title compound is prepared from {(*S*)-6-[(*R*)-4-(1,3,5-trimethyl-1H-pyrazol-4-yl)-indan-1-yloxy]-2,3-dihydro-benzofuran-3-yl}-acetic acid methyl ester following a procedure analogous to that described in Example 1. LC (method 1): t_{R} = 1.19 min; Mass spectrum (ESI⁻): m/z = 417 [M-H]⁻.

### Example 50

### {(S)-6-[(S)-6-Trifluoromethyl-indan-1-yloxy]-2,3-dihydro-benzofuran-3-yl]-acetic acid

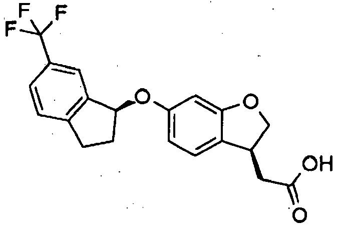

The title compound is prepared from {(*S*)-6-[(*S*)-6-trifluoromethyl-indan-1-yloxy]-2,3-dihydro-benzofuran-3-yl]-acetic acid methyl ester following a procedure analogous to that described in Example 1. LC (method 1): t_{R} = 1.34 min; Mass spectrum (ESI⁻): m/z = 377 [M-H]⁻.

### Example 51

### {(S)-6-[(R)-4-(2-Oxo-2H-pyridin-1-yl)-indan-1-yloxy]-2,3-dihydro-benzofuran-3-yl}-acetic acid

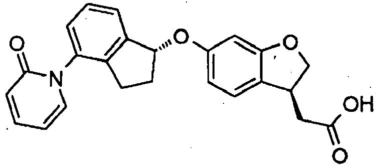

The title compound is prepared from {(*S*)-6-[(*R*)-4-(2-oxo-2H-pyridin-1-yl)-indan-1-yloxy]-2,3-dihydro-benzofuran-3-yl}-acetic acid methyl ester following a procedure analogous to that described in Example 1. LC (method 5): t_{R} = 2.96 min; Mass spectrum (ESI⁻): m/z = 402 [M-H]⁻.

### Example 52

### {(S)-6-[(R)-5-(2,6-Dimethyl-phenyl)-indan-1-yloxy]-2,3-dihydro-benzofuran-3-yl}-acetic acid

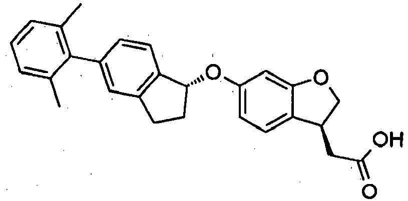

The title compound is prepared from {(3*S*)-6-[(*R*)-5-(2,6-dimethyl-phenyl)-indan-1-yloxy]-2,3-dihydro-benzofuran-3-yl}-acetic acid methyl ester following a procedure analogous to that described in Example 1. LC (method 1): t_{R} = 1.47 min; Mass spectrum (ESI⁻): m/z = 413 [M-H]⁻.

### Example 53

### {(S)-6-[(S)-6-(2,6-Dimethyl-phenyl)-indan-1-yloxy]-2,3-dihydro-benzofuran-3-yl}-acetic acid

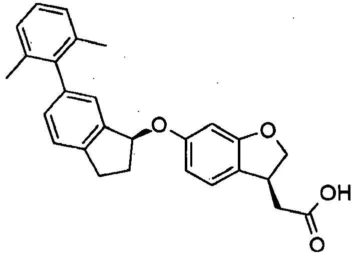

A vial charged with a stir bar, {(S)-6-[(S)-6-bromo-indan-1-yloxy]-2,3-dihydro-benzofuran-3-yl}-acetic acid methyl ester (0.20 g), 1 M aqueous Na₂CO₃ solution (1.5 mL), 2,6-dimethylphenylboronic acid (0.08 g), ethanol (1 mL), and toluene (3 mL) is purged with Ar. Tetrakis(triphenylphosphine)palladium(0) (26 mg) is added and the mixture is stirred at 100 °C overnight. After cooling to room temperature, diethyl ether is added and the mixture is extracted with ethyl acetate. The combined organic extracts are washed with aqueous NH₄Cl solution and brine and dried (Na₂SO₄). The solvent is evaporated and the residue is chromatographed on silica gel (heptane/ethyl acetate/acetic acid) to afford the title compound (under the reaction conditions the ester is saponified to give the acid). Yield: 0.10 g (49% of theory); LC (method 1): t_{R} = 1.47 min; Mass spectrum (ESI⁻): m/z = 413 [M-H]⁻.

### Example 54

### {(S)-6-[(R)-5-(2-Isopropyl-phenyl)-indan-1-yloxy]-2,3-dihydro-benzofuran-3-yl}-acetic acid

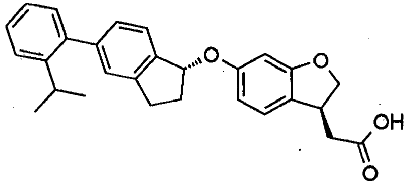

The title compound is prepared from {(*S*)-6-[(*R*)-5-(2-isopropy)-phenyl)-indan-1-yloxy]-2,3-dihydro-benzofuran-3-yl}-acetic acid methyl ester following a procedure analogous to that described in Example 1. LC (method 1): t_{R} = 1.50 min; Mass spectrum (ESI⁻): m/z = 427 [M-H]⁻.

### Example 55

### {(S)-6-[(S)-6-Bromo-indan-1-yloxy]-2,3-dihydro-benzofuran-3-yl}-acetic acid

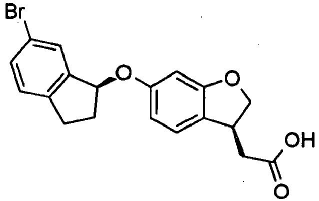

The title compound is prepared from {(*S*)-6-[(*S*)-6-bromo-indan-1-yloxy]-2,3-dihydrobenzofuran-3-yl}-acetic acid methyl ester following a procedure analogous to that described in Example 1. LC (method 1): t_{R} = 1.34 min; Mass spectrum (ESI⁻): m/z = 387/389 (Br) [M-H]⁻.

### Example 56

### {(S)-6-[(R)-4-(3-Oxa-spiro[5.5]undec-7-en-7-yl)-indan-1-yloxy]-2,3-dihydro-benzofuran-3-yl}-acetic acid

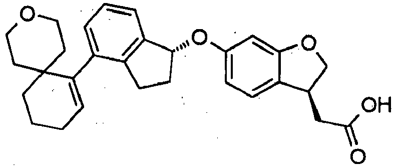

The title compound is prepared from {(*S*)-6-[(*R*)-4-(3-oxa-spiro[5.5]undec-7-en-7-yl)-indan-1-yloxy]-2,3-dihydro-benzofuran-3-yl}-acetic acid methyl ester following a procedure analogous to that described in Example 1. LC (method 1): t_{R} = 1.43 min; Mass spectrum (ESI⁻): m/z = 459 [M-H]⁻.

### Example 57

### {(S)-6-[(R)-4-(1-methyl-1H-imidazol-2-yl)-indan-1-yloxy]-2,3-dihydro-benzofuran-3-yl}-acetic acid

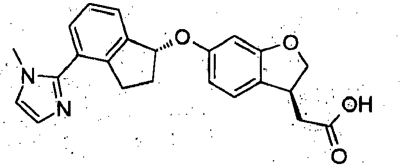

The title compound is prepared from {(*S*)-6-[(*R*)-4-(1-methy)-1H-imidazol-2-yl)-indan-1-yloxy]-2,3-dihydro-benzofuran-3-yl}-acetic acid methyl ester following a procedure analogous to that described in Example 1. LC (method 1): t_{R} = 0.85 min; Mass spectrum (ESI⁻): m/z = 389 [M-H]⁻.

### Example 58

### {(S)-6-[(R)-4-(3-Methyl-pyridin-2-yl)-indan-1-yloxy]-2,3-dihydro-benzofuran-3-yl}-acetic acid

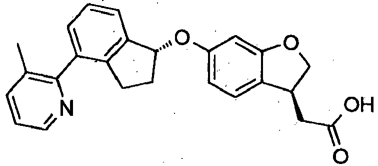

The title compound is prepared from {(*S*)-6-[(*R*)-4-(3-methyl-pyridin-2-yl)-indan-1-yloxy]-2,3-dihydro-benzofuran-3-yl}-acetic acid methyl ester following a procedure analogous to that described in Example 1. LC (method 1): t_{R} = 0.95 min; Mass spectrum (ESI⁻): m/z = 400 [M-H]⁻.

### Example 59

### {(S)-6-[(R)-4-(4-Methoxy-2,6-dimethyl-phenyl)-indan-1-yloxy]-2,3-dihydro-benzofuran-3-yl}-acetic acid

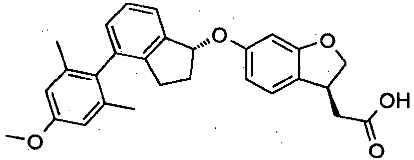

The title compound is prepared from {(*S*)-6-[(*R*)-4-(4-methoxy-2,6-dimethyl-phenyl)-indan-1-yloxy]-2,3-dihydro-benzofuran-3-yl}-acetic acid methyl ester following a procedure analogous to that described in Example 1. LC (method 1): t_{R} = 1.44 min; Mass spectrum (ESI⁻): m/z = 443 [M-H]⁻.

### Example 60

### {(S)-6-[(R)-4-(2-Methoxy-3,5-dimethyl-pyridin-4-yl)-indan-1-yloxy]-2,3-dihydrobenzofuran-3-yl}-acetic acid

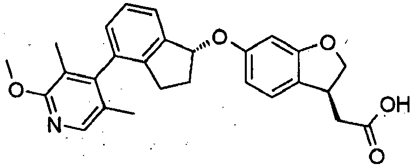

The title compound is prepared from {(*S*)-6-[(*R*)-4-(2-methoxy-3,5-dimethyl-pyridin-4-yl)-indan-1-yloxy]-2,3-dihydro-benzofuran-3-yl}-acetic acid methyl ester following a procedure analogous to that described in Example 1. LC (method 1): t_{R} = 1.39 min; Mass spectrum (ESI⁻): m/z = 444 [M-H]⁻.

### Example 61

### {(S)-6-[(R)-4-(1,3,5-Trimethyl-2-oxo-1,2-dihydro-pyridin-4-yl)-indan-1-yloxy]-2,3-dihydro-benzofuran-3-yl}-acetic acid

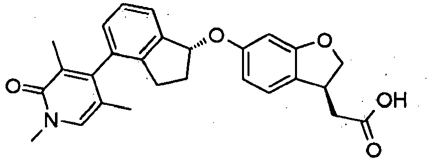

The title compound is prepared from {(*S*)-6-[(*R*)-4-(1,3,5-trimethyl-2-oxo-1,2-dihydro-pyridin-4-yl)-indan-1-yloxy]-2,3-dihydro-benzofuran-3-yl}-acetic acid methyl ester following a procedure analogous to that described in Example 1. LC (method 1): t_{R} = 1.20 min; Mass spectrum (ESI⁻): m/z = 444 [M-H]⁻.

### Example 62

### {(S)-6-[(R)-4-o-Tolyl-indan-1-yloxy]-2,3-dihydro-benzofuran-3-yl}-acetic acid

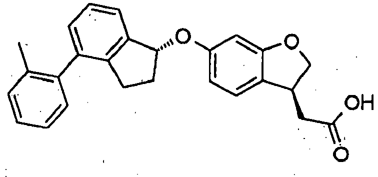

The title compound is prepared from {(*S*)-6-[(*R*)-4-o-tolyl-indan-1-yloxy]-2,3-dihydro-benzofuran-3-yl}-acetic acid methyl ester following a procedure analogous to that described in Example 1. LC (method 1): t_{R} = 1.44 min; Mass spectrum (ESI⁻): m/z = 399 [M-H]⁻.

### Example 63

### {(S)-6-[(R)-4-(1,2,4-Trimethyl-6-oxo-1,6-dihydro-pyridin-3-yl)-indan-1-yloxy]-2,3-dihydro-benzofuran-3-yl}-acetic acid

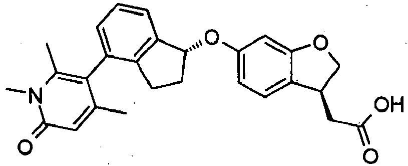

The title compound is prepared from {(*S*)-6-[(*R*)-4-(1,2,4-trimethyl-6-oxo-1,6-dihydro-pyridin-3-yl)-indan-1-yloxy]-2,3-dihydro-benzofuran-3-yl}-acetic acid methyl ester following a procedure analogous to that described in Example 1. LC (method 1): t_{R} = 1.18 min; Mass spectrum (ESI⁻): m/z = 444 [M-H]⁻.

### Example 64

### {(S)-6-[(R)-4-(6-Methoxy-2,4-dimethyl-pyridin-3-yl)-indan-1-yloxy]-2,3-dihydro-benzofuran-3-yl}-acetic acid

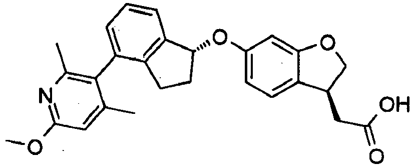

The title compound is prepared from {(*S*)-6-[(*R*)-4-(6-methoxy-2,4-dimethyl-pyridin-3-yl)-indan-1-yloxy]-2,3-dihydro-benzofuran-3-yl}-acetic acid methyl ester following a procedure analogous to that described in Example 1. LC (method 1): t_{R} = 1.16 min; Mass spectrum (ESI⁻): m/z = 444 [M-H]⁻.

### Example 65

### {(S)-6-[(R)-4-Morpholin-4-yl-indan-1-yloxy]-2,3-dihydro-benzofuran-3-yl}-acetic acid

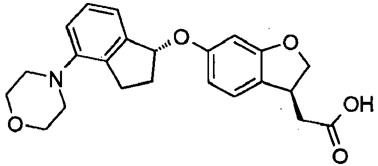

The title compound is prepared from {(*S*)-6-[(*R*)-4-morpholin-4-yl-indan-1-yloxy]-2,3-dihydro-benzofuran-3-yl}-acetic acid methyl ester following a procedure analogous to that described in Example 1. LC (method 1): t_{R} = 1.18 min; Mass spectrum (ESI⁻): m/z = 394 [M-H]⁻.

### Example 66

### {(S)-6-[(R)-4-((S)-3,3-Dimethyl-tetrahydro-pyran-2-yl)-indan-1-yloxy]-2,3-dihydrobenzofuran-3-yl}-acetic acid, pure isomer, configuration at stereocenter indicated (*) arbitrarily assigned

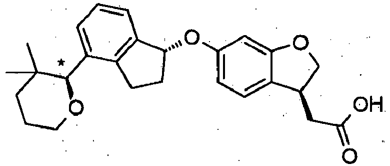

The title compound is prepared from {(*S*)-6-[(*R*)-4-((*S*)-3,3-dimethyl-tetrahydropyran-2-yl)-indan-1-yloxy]-2,3-dihydro-benzofuran-3-yl}-acetic acid methyl ester (pure isomer, Intermediate 65) following a procedure analogous to that described in Example 1. LC (method 6): t_{R} = 1.48 min; Mass spectrum (ESI⁻): m/z = 421 [M-H]⁻.

### Example 67

### {(S)-6-[(R)-4-((R)-3,3-Dimethyl-tetrahydro-pyran-2-yl)-indan-1-yloxy]-2,3-dihydrobenzofuran-3-yl}-acetic acid, pure isomer, configuration at stereocenter indicated (*) arbitrarily assigned

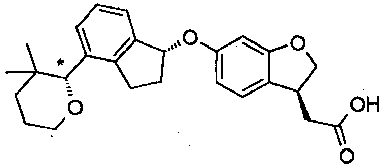

The title compound is prepared from {(*S*)-6-[(*R*)-4-((*R*)-3,3-dimethyl-tetrahydropyran-2-yl)-indan-1-yloxy]-2,3-dihydro-benzofuran-3-yl}-acetic acid methyl ester (pure isomer, Intermediate 66) following a procedure analogous to that described in Example 1. LC (method 6): t_{R} = 1.44 min; Mass spectrum (ESI⁻): m/z = 421 [M-H]⁻.

### Example 68

### {(S)-6-[(R)-4-(5-Fluoro-2-methoxy-pyridin-4-yl)-indan-1-yloxy]-2,3-dihydrobenzofuran-3-yl}-acetic acid

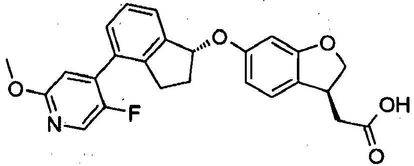

The title compound is prepared from {(*S*)-6-[(*R*)-4-(5-fluoro-2-methoxy-pyridin-4-yl)-indan-1-yloxy]-2,3-dihydro-benzofuran-3-yl}-acetic acid methyl ester following a procedure analogous to that described in Example 1. LC (method 6): t_{R} = 1.36 min; Mass spectrum (ESI⁻): m/z = 434 [M-H]⁻.

### Example 69

### {(S)-6-[(R)-4-(2-Trifluoromethyl-phenyl)-indan-1-yloxy]-2,3-dihydro-benzofuran-3-yl}-acetic acid

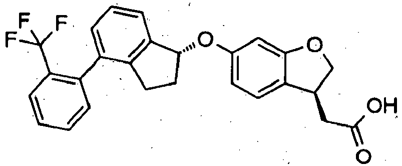

The title compound is prepared from {(*S*)-6-[(*R*)-4-(2-trifluoromethyl-phenyl)-indan-1-yloxy]-2,3-dihydro-benzofuran-3-yl}-acetic acid methyl ester following a procedure analogous to that described in Example 1. LC (method 6): t_{R} = 1.41 min; Mass spectrum (ESI⁻): m/z = 453 [M-H]⁻.

### Example 70

### {(S)-6-[(R)-4-(6,6-Dimethyl-cyclohex-1-enyl)-indan-1-yloxy]-2,3-dihydro-benzofuran-3-yl}-acetic acid

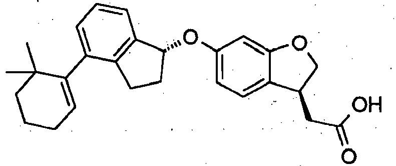

The title compound is prepared from {(*S*)-6-[(*R*)-4-(6,6-dimethyl-cyclohex-1-enyl)-indan-1-yloxy]-2,3-dihydro-benzofuran-3-yl}-acetic acid methyl ester following a procedure analogous to that described in Example 1. LC (method 6): t_{R} = 1.75 min; Mass spectrum (ESI⁻): m/z = 417 [M-H]⁻.

### Example 71

### {(S)-6-[(R)-4-(5-Methoxy-2-methyl-phenyl)-indan-1-yloxy]-2,3-dihydro-benzofuran-3-yl}-acetic acid

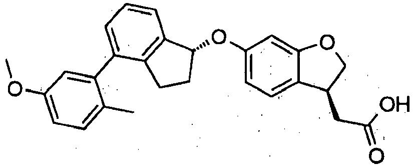

The title compound is prepared from {(*S*)-6-[(*R*)-4-(5-methoxy-2-methyl-phenyl)-indan-1-yloxy]-2,3-dihydro-benzofuran-3-yl}-acetic acid methyl ester following a procedure analogous to that described in Example 1. LC (method 1): t_{R} = 1.41 min; Mass spectrum (ESI⁻): m/z = 429 [M-H]⁻.

### Example 72

### {(S)-6-[(R)-4-(5-Hydroxy-2-methyl-phenyl)-indan-1-yloxy]-2,3-dihydro-benzofuran-3-yl}-acetic acid

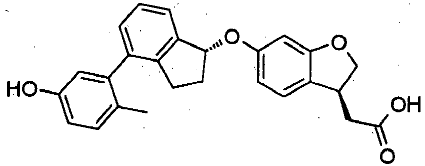

The title compound is prepared from {(*S*)-6-[(*R*)-4-(5-hydroxy-2-methyl-phenyl)-indan-1-yloxy]-2,3-dihydro-benzofuran-3-yl}-acetic acid methyl ester following a procedure analogous to that described in Example 1. LC (method 1): t_{R} = 1.28 min; Mass spectrum (ESI⁻): m/z = 415 [M-H]⁻.

### Example 73

### {(S)-6-[(R)-4-(2-Trifluoromethyl-pyridin-3-yl)-indan-1-yloxy]-2,3-dihydro-benzofuran-3-yl}-acetic acid

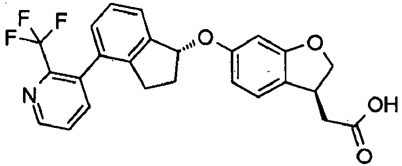

The title compound is prepared from {(*S*)-6-[(*R*)-4-(4,4,5,5-tetramethyl-[1,3,2]dioxaborolan-2-yl)-indan-1-yloxy]-2,3-dihydro-benzofuran-3-yl}-acetic acid methyl ester and 3-bromo-2-trifluoromethyl-pyridine following a procedure analogous to that described in Example 53; the ester is saponified under the reaction conditions. LC (method 7): t_{R} = 1.71 min; Mass spectrum (ESI⁻): m/z = 454 [M-H]⁻.

### Example 74

### {(S)-6-[(R)-4-(3,5-Dimethyl-isoxazol-4-yl)-indan-1-yloxy]-2,3-dihydro-benzofuran-3-yl}-acetic acid

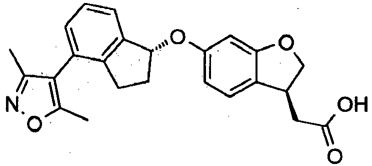

The title compound is prepared from {(S)-6-[(R)-4-(4,4,5,5-tetramethyl-[1,3,2]dioxaborolan-2-yl)-indan-1-yloxy]-2,3-dihydro-benzofuran-3-yl}-acetic acid methyl ester and 4-iodo-3,5-dimethyl-isoxazole following a procedure analogous to that described in Example 53; the ester is saponified under the reaction conditions. LC (method 1): t_{R} = 1.70 min; Mass spectrum (ESI⁻): m/z = 404 [M-H]⁻.

### Example 75 and Example 76

### {(S)-6-[(R)-4-((S)-2,2-Dimethyl-cyclopentyl)-indan-1-yloxy]-2,3-dihydro-benzofuran-3-yl}-acetic acid and {(S)-6-[(R)-4-((R)-2,2-Dimethyl-cyclopentyl)-indan-1-yloxy]-2,3-dihydro-benzofuran-3-yl}-acetic acid, pure isomers, configuration at the stereocenter indicated (*) arbitrarily assigned

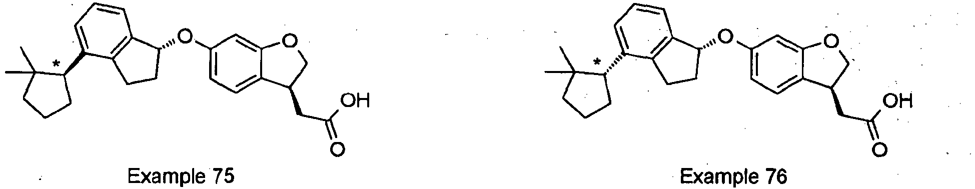

A mixture of {(*S*)-6-[(*R*)-(5,5-dimethyl-cyclopent-1-enyl)-indan-1-yloxy]-2,3-dihydrobenzofuran-3-yl}-acetic acid (0.32 g), platinum(IV) oxide (36 mg), and ethyl acetate (18 mL) is stirred under hydrogen atmosphere (1 bar) at room temperature for 1 h. The mixture is filtered and the filtrate is concentrated. The mixture of diastereomers is separated by HPLC on reversed phase (heptane/isopropanol/trifluoroacetic acid) to give the two isomerically pure title compounds. The configuration at the stereocenter indicated is arbitrarily assigned.
Example 75: LC (method 5): t_{R} = 4.20 min; Mass spectrum (ESI⁻): m/z = 405 [M-H].
Example 76: LC (method 5): t_{R} = 4.23 min; Mass spectrum (ESI⁻): m/z = 405 [M-H]⁻.

### Example 77

### {(S)-6-[(R)-4-Isoquinolin-1-yl-indan-1-yloxy]-2,3-dihydro-benzofuran-3-yl}-acetic acid

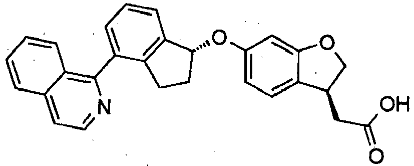

The title compound is prepared from {(*S*)-6-[(*R*)-4-isoquinolin-1-yl-indan-1-yloxy]-2,3-dihydro-benzofuran-3-yl}-acetic acid methyl ester following a procedure analogous to that described in Example 1. LC (method 6): t_{R} = 0.86 min; Mass spectrum (ESI⁻): m/z = 436 [M-H]⁻.

### Example 78

### {(S)-6-[(R)-4-(1-Methoxy-1,2,2-trimethyl-propyl)-indan-1-yloxy]-2,3-dihydrobenzofuran-3-yl}-acetic acid

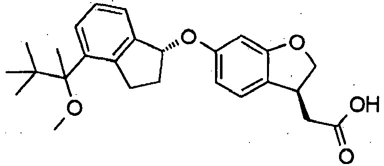

The title compound is prepared from {(*S*)-6-[(*R*)-4-(1-methoxy-1,2,2-trimethyl-propyl)-indan-1-yloxy]-2,3-dihydro-benzofuran-3-yl}-acetic acid methyl ester following a procedure analogous to that described in Example 1. LC (method 1): t_{R} = 1.48 min; Mass spectrum (ESI⁻): m/z = 423 [M-H]⁻.

### Example 79

### {(S)-6-[(R)-4-(1,1-Difluoro-2-methoxy-ethyl)-indan-1-yloxy]-2,3-dihydro-benzofuran-3-yl}-acetic acid

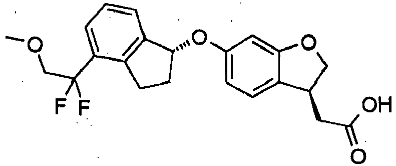

The title compound is prepared from {(*S*)-6-[(*R*)-4-(1,1-difluoro-2-methoxy-ethyl)-indan-1-yloxy]-2,3-dihydro-benzofuran-3-yl}-acetic acid methyl ester following a procedure analogous to that described in Example 1. LC (method 7): t_{R} = 1.69 min; Mass spectrum (ESI⁻): m/z = 403 [M-H]⁻.

### Example 80

### {(S)-6-[(R)-4-(2-Methyl-naphthalen-1-yl)-indan-1-yloxy]-2,3-dihydro-benzofuran-3-yl}-acetic acid

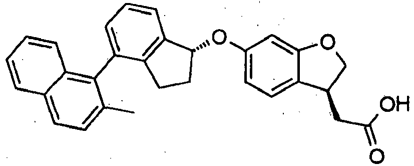

The title compound is prepared from {(*S*)-6-[(*R*)-4-(2-methyl-naphthaten-1-yl)-indan-1-yloxy]-2,3-dihydro-benzofuran-3-yl}-acetic acid methyl ester following a procedure analogous to that described in Example 1. LC (method 6): t_{R} = 1.68 min; Mass spectrum (ESI⁻): m/z = 449 [M-H]⁻.

### Example 81

### ((S)-6-{(R)-4-[1-Hydroxy-1-(tetrahydro-pyran-4-yl)-ethyl]-indan-1-yloxy}-2,3-dihydrobenzofuran-3-yl)-acetic acid

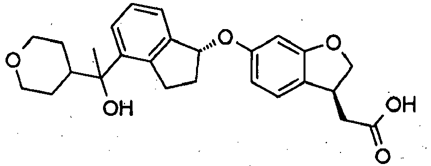

The title compound is prepared from {(*S*)-6-[(*R*)-4-(tetrahydro-pyran-4-carbonyl)-indan-1-yloxy]-2,3-dihydro-benzofuran-3-yl}-acetic acid following a procedure analogous to that described in Step 2 of Intermediate 73; excess of methylmagnesium bromide is used. LC (method 7): t_{R} = 1.59 min; Mass spectrum (ESI⁻): m/z = 437 [M-H]⁻.

### Example 82

### {(S)-6-[(R)-4-(1-Hydroxy-1-methyl-pent-4-enyl)-indan-1-yloxy]-2,3-dihydrobonzofuran-3-yl}-acetic acid

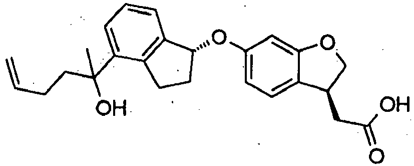

The title compound is prepared from {(*S*)-6-[(*R*)-4-pent-4-enoyl-indan-1-yloxy]-2,3-dihydro-benzofuran-3-yl}-acetic acid following a procedure analogous to that described in Step 2 of Intermediate 73; excess of methylmagnesium bromide is used. LC (method 7): t_{R} = 1.72 min; Mass spectrum (ESI⁻): m/z = 407 [M-H]⁻.

### Example 83

### ((S)-6-{(R)-4-[1-Hydroxy-1-methyl-2-(tetrahydro-pyran-4-yl)-ethyl]-indan-1-yloxy}-2,3-dihydro-benzofuran-3-yl)-acetic acid

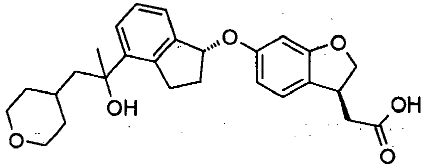

The title compound is prepared from {(*S*)-6-[(*R*)-4-(2-tetrahydro-pyran-4-yl-acetyl)-indan-1-yloxy]-2,3-dihydro-benzofuran-3-yl}-acetic acid following a procedure analogous to that described in Step 2 of Intermediate 73; excess of methylmagnesium bromide is used. LC (method 7): t_{R} = 1.61 min; Mass spectrum (ESI⁻): m/z = 451 [M-H]⁻.

### Example 84

### {(S)-6-[(R)-4-(1,1-Difluoro-2-methoxy-2-methyl-propyl)-indan-1-yloxy]-2,3-dihydro-benzofuran-3-yl}-acetic acid

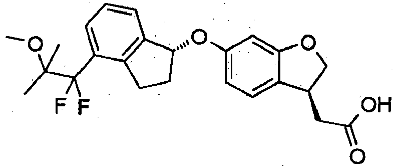

The title compound is prepared from {(*S*)-6-[(*R*)-4-(1,1-difluoro-2-methoxy-2-methylpropyl)-indan-1-yloxy]-2,3-dihydro-benzofuran-3-yl}-acetic acid methyl ester following a procedure analogous to that described in Example 1. LC (method 7): t_{R} = 1.80 min; Mass spectrum (ESI⁻): m/z = 431 [M-H]⁻.

### Example 85

### {(S)-6-[(R)-4-(1,1-Difluoro-2-hydroxy-2-methyl-propyl)-indan-1-yloxy]-2,3-dihydrobenzofuran-3-yl}-acetic acid

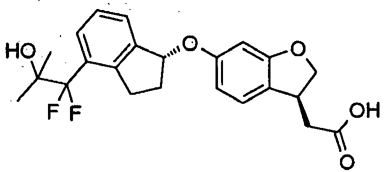

The title compound is prepared from {(*S*)-6-[(*R*)-4-(1,1-difluoro-2-hydroxy-2-methylpropyl)-indan-1-yloxy]-2,3-dihydro-benzofuran-3-yl}-acetic acid methyl ester following a procedure analogous to that described in Example 1. LC (method 7): t_{R} = 1.66 min; Mass spectrum (ESI⁻): m/z = 417 [M-H]⁻.

### Example 86

### {(S)-6-[(R)-4-(4,6-Dimethyl-pyrimidin-5-yl)-indan-1-yloxy]-2,3-dihydro-benzofuran-3-yl}-acetic acid

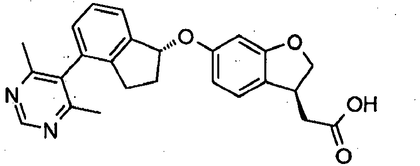

The title compound is prepared from {(*S*)-6-[(*R*)-4-(4,6-dimethyl-pyrimidin-5-yl)-indan-1-yloxy]-2,3-dihydro-benzofuran-3-yl}-acetic acid methyl ester following a procedure analogous to that described in Example 1. LC (method 6): t_{R} = 0.95 min; Mass spectrum (ESI⁻): m/z = 415 [M-H]⁻.

### Example 87

### {(S)-6-[(R)-4-(1-Methyl-cyclopropyl)-indan-1-yloxy]-2,3-dihydro-benzofuran-3-yl}-acetic acid

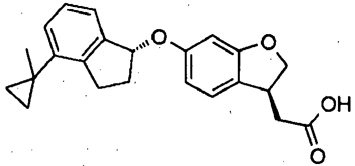

The title compound is prepared from {(*S*)-6-[(*R*)-4-(1-methyl-cyclopropyl)-indan-1-yloxy]-2,3-dihydro-benzofuran-3-yl}-acetic acid methyl ester following a procedure analogous to that described in Example 1. LC (method 6): tR = 1.51 min; Mass spectrum (ESI⁻): m/z = 363 [M-H]⁻.

### Example 88

### {(S)-6-[(R)-4-(2,6-Dichloro-phenyl)-indan-1-yloxy]-2,3-dihydro-benzofuran-3-yl}-acetic acid

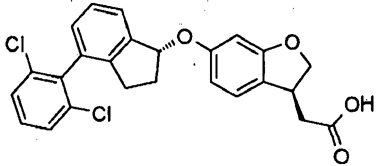

The title compound is prepared from {(*S*)-6-[(*R*)-4-(2,6-dichloro-phenyl)-indan-1-yloxy]-2,3-dihydro-benzofuran-3-yl}-acetic acid methyl ester following a procedure analogous to that described in Example 1. LC (method 7): t_{R} = 1.89 min; Mass spectrum (ESI⁻): m/z = 453/455/457 (2 Cl) [M-H]⁻.

### Example 89

### {(S)-6-[(R)-4-(2,2,2-Trifluoro-1-hydroxy-1-methyl-ethyl)-indan-1-yloxy]-2,3-dihydrobenzofuran-3-yl}-acetic acid

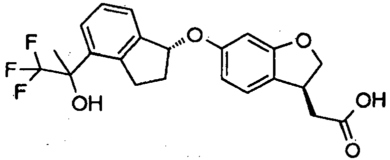

The title compound is prepared from {(*S*)-6-[(*R*)-4-(2,2,2-trifluoro-acetyl)-indan-1-yloxy]-2,3-dihydro-benzofuran-3-yl}-acetic acid following a procedure analogous to that described in Step 2 of Intermediate 73; excess of methylmagnesium bromide is used. LC (method 6): t_{R} = 1.03 min; Mass spectrum (ESI⁻): m/z = 421 [M-H]⁻.

### Example 90

### {(S)-6-[(R)-4-(2-Chloro-6-methyl-phenyl)-indan-1-yloxy]-2,3-dihydro-benzofuran-3-yl}-acetic acid

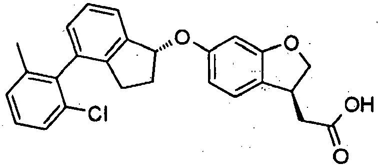

The title compound is prepared from {(*S*)-6-[(*R*)-4-(2-chloro-6-methyl-phenyl)-indan-1-yloxy]-2,3-dihydro-benzofuran-3-yl}-acetic acid methyl ester following a procedure analogous to that described in Example 1. LC (method 7): t_{R} = 1.92 min; Mass spectrum (ESI⁻): m/z = 433/435 (Cl) [M-H]⁻.

### Example 91

### {(S)-6-[(R)-4-(6-Methoxy-3-trifluoromethyl-pyridin-2-yl)-indan-1-yloxy]-2,3-dihydro-benzofuran-3-yl}-acetic acid

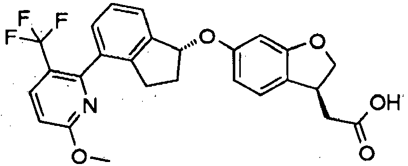

The title compound is prepared from {(*S*)-6-[(*R*)-4-(6-methoxy-3-trifluoromethyl-pyridin-2-yl)-indan-1-yloxy]-2,3-dihydro-benzofuran-3-yl}-acetic acid methyl ester following a procedure analogous to that described in Example 1. LC (method 1): t_{R} = 1.39 min; Mass spectrum (ESI⁻): m/z = 484 [M-H]⁻.

### Example 92

### {(S)-6-[(R)-4-(8-Methyl-naphthalen-1-yl)-indan-1-yloxy]-2,3-dihydro-benzofuran-3-yl}-acetic acid

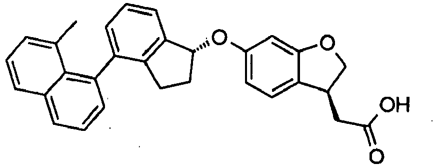

The title compound is prepared from {(*S*)-6-[(*R*)-4-(8-methyl-naphthalen-1-yl)-indan-1-yloxy]-2,3-dihydro-benzofuran-3-yl}-acetic acid methyl ester following a procedure analogous to that described in Example 1. LC (method 7): t_{R} = 1.51 min; Mass spectrum (ESI⁻): m/z = 449 [M-H]⁻.

### Example 93

### {(S)-6-[(R)-4-(2,4-Dimethyl-pyridin-3-yl)-indan-1-yloxy]-2,3-dihydro-benzofuran-3-yl}-acetic acid

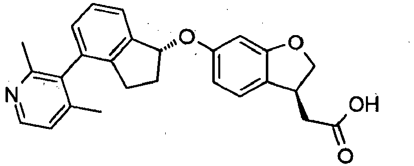

The title compound is prepared from {(*S*)-6-[(*R*)-4-(2,4-dimethyl-pyridin-3-yl)-indan-1-yloxy]-2,3-dihydro-benzofuran-3-yl}-acetic acid methyl ester following a procedure analogous to that described in Example 1. LC (method 1): t_{R} = 0.98 min; Mass spectrum (ESI⁻): m/z = 414 [M-H]⁻.

### Example 94

### {(S)-6-[(R)-4-(2-Pentafluoroethyl-phenyl)-indan-1-yloxy]-2,3-dihydro-benzofuran-3-yl}-acetic acid

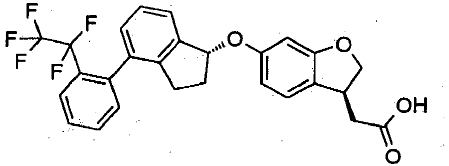

The title compound is prepared from {(*S*)-6-[(*R*)-4-(2-pentafluoroethyl-phenyl)-indan-1-yloxy]-2,3-dihydro-benzofuran-3-yl}-acetic acid methyl ester following a procedure analogous to that described in Example 1. LC (method 6): t_{R} = 1.57 min; Mass spectrum (ESI⁻): m/z = 503 [M-H]⁻.

### Example 95

### {(S)-6-[(R)-5-tert-Butyl-indan-1-yloxy]-2,3-dihydro-benzofuran-3-yl}-acetic acid

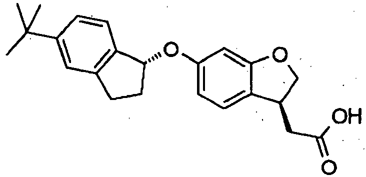

The title compound is prepared from {(*S*)-6-[(*R*)-5-tert-butyl-indan-1-yloxy]-2,3-dihydro-benzofuran-3-yl}-acetic acid methyl ester following a procedure analogous to that described in Example 1. LC (method 1): t_{R} = 1.45 min; Mass spectrum (ESI⁻): m/z = 365 [M-H]⁻.

### Example 96

### {(S)-6-[(R)-4-tert-Butyl-indan-1-yloxy]-2,3-dihydro-benzofuran-3-yl}-acetic acid

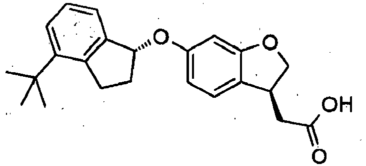

A solution of NiCl₂*1.5H₂O (for preparation see J. Am. Chem. Soc. 2011, 133, 8478-81; 6 mg), 1,3-dicyclohexyl-imidazolium tetrafluoroborate (12 mg), and {(*S*)-6-[(*R*)-4-bromo-indan-1-yloxy]-2,3-dihydro-benzofuran-3-yl}-acetic acid (0.15 g) in tetrahydrofuran (3 mL) is purged with Ar. The solution is cooled in an ice/sodium chloride bath and tert-butylmagnesium chloride (1 mol/L in tetrahydrofuran; 1.2 mL) is added over a period of 5 min. The solution is stirred in the cooling bath over 1.5 h while warming to 0 °C. The solution is poured into a stirred mixture of ethyl acetate and aqueous NH₄Cl solution and the resulting mixture is stirred for 5 min. The organic phase is separated and washed with brine and dried (Na₂SO₄). The solvent is evaporated and the residue is chromatographed on reversed phase (HPLC; acetonitrile/water) and then again on silica gel (cyclohexane/ethyl acetate) to give the title compound. LC (method 6): t_{R} = 1.54 min; Mass spectrum (ESI⁻): m/z = 365 [M-H]⁻

### Example 97

### {(S)-6-[(R)-4-(Cyano-dimethyl-methyl)-indan-1-yloxy]-2,3-dihydro-benzofuran-3-yl}-acetic acid

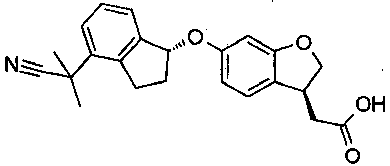

The title compound is prepared from {(*S*)-6-[(*R*)-4-(cyano-dimethyl-methyl)-indan-1-yloxy]-2,3-dihydro-benzofuran-3-yl}-acetic acid methyl ester following a procedure analogous to that described in Example 1. LC (method 1): t_{R} = 1.22 min; Mass spectrum (ESI⁻): m/z = 376 [M-H]⁻.

### Example 98

### {(S)-6-[(R)-4-Chloro-5-trifluoromethyl-indan-1-yloxy]-2,3-dihydro-benzofuran-3-yl}-acetic acid

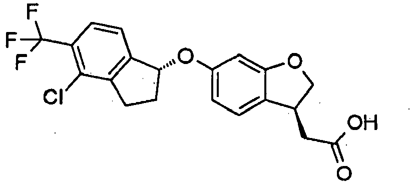

The title compound is prepared from {(*S*)-6-[(*R*)-4-chloro-5-trifluoromethyl-indan-1-yloxy]-2,3-dihydro-benzofuran-3-yl}-acetic acid methyl ester following a procedure analogous to that described in Example 1. LC (method 6): t_{R} = 1.48 min; Mass spectrum (ESI⁻): m/z = 411/413 (Cl) [M-H]⁻.

### Example 99

### {(S)-6-[(R)-4-(3-Methyl-pyrazin-2-yl)-indan-1-yloxy]-2,3-dihydro-benzofuran-3-yl}-acetic acid

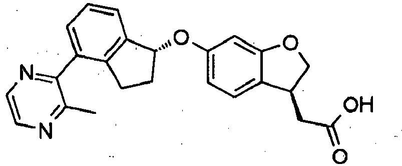

The title compound is prepared from {(*S*)-6-[(*R*)-4-(3-methyl-pyrazin-2-yl)-indan-1-yloxy]-2,3-dihydro-benzofuran-3-yl}-acetic acid methyl ester following a procedure analogous to that described in Example 1. LC (method 7): t_{R} = 1.57 min; Mass spectrum (ESI⁻): m/z = 401 [M-H]⁻.

### Example 100

### {(S)-6-[(R)-4-(3-Methoxy-2,6-dimethyl-phenyl)-indan-1-yloxy]-2,3-dihydro-benzofuran-3-yl}-acetic acid

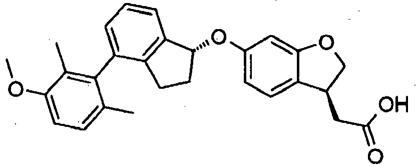

The title compound is prepared from {(*S*)-6-[(*R*)-4-(3-methoxy-2,6-dimethyl-phenyl)-indan-1-yloxy]-2,3-dihydro-benzofuran-3-yl}-acetic acid methyl ester following a procedure analogous to that described in Example 1. LC (method 6): t_{R} = 1.55 min; Mass spectrum (ESI⁻): m/z = 443 [M-H]⁻.

### Example 101

### {(S)-6-[(R)-4-Pentafluoroethyl-indan-1-yloxy]-2,3-dihydro-benzofuran-3-yl}-acetic acid

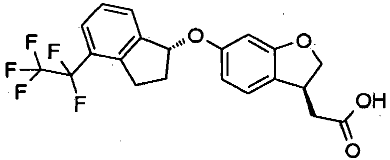

The title compound is prepared from {(*S*)-6-[(*R*)-4-pentafluoroethyl-indan-1-yloxy]-2,3-dihydro-benzofuran-3-yl}-acetic acid methyl ester following a procedure analogous to that described in Example 1. LC (method 6): t_{R} = 1.51 min; Mass spectrum (ESI⁻): m/z = 427 [M-H]⁻.

### Example 102

### {(S)-6-[(R)-6-Fluoro-4-trifluoromethyl-indan-1-yloxy]-2,3-dihydro-benzofuran-3-yl}-acetic acid

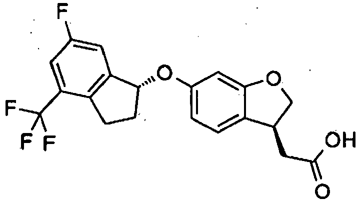

The title compound is prepared from {(*S*)-6-[(*R*)-6-fluoro-4-trifluoromethyl-indan-1-yloxy]-2,3-dihydro-benzofuran-3-yl}-acetic acid methyl ester following a procedure analogous to that described in Example 1. LC (method 7): t_{R} = 1.84 min; Mass spectrum (ESI⁻): m/z = 395 [M-H]⁻.

### Example 103

### {(S)-6-[(R)-4-(2-Methoxy-4-trifluoromethyl-pyridin-3-yl)-indan-1-yloxy]-2,3-dihydrobenzofuran-3-yl}-acetic acid

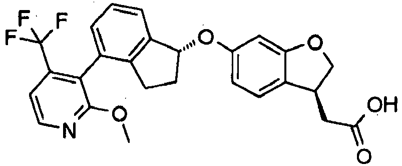

The title compound is prepared from {(*S*)-6-[(*R*)-4-(2-methoxy-4-trifluoromethyl-pyridin-3-yl)-indan-1-yloxy]-2,3-dihydro-benzofuran-3-yl}-acetic acid methyl ester following a procedure analogous to that described in Example 1. LC (method 1): t_{R} = 1.37/1.38 min (mixture of 2 atropic isomers); Mass spectrum (ESI⁻): m/z = 484 [M-H]⁻.

### Example 104

### {(S)-6-[(R)-4-(3-Methoxy-2-methyl-phenyl)-indan-1-yloxy]-2,3-dihydro-benzofuran-3-yl}-acetic acid

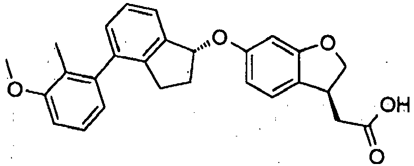

The title compound is prepared from {(*S*)-6-[(*R*)-4-(3-methoxy-2-methyl-phenyl)-indan-1-yloxy]-2,3-dihydro-benzofuran-3-yl}-acetic acid methyl ester following a procedure analogous to that described in Example 1. LC (method 7): t_{R} = 1.55 min; Mass spectrum (ESI⁻): m/z = 429 [M-H]⁻.

### Example 105

### {7-Methyl-6-[(R)-4-trifluoromethyl-indan-1-yloxy]-2,3-dihydro-benzofuran-3-yl}-acetic acid

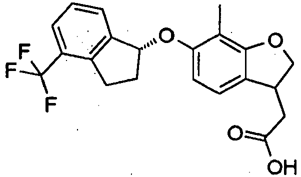

The title compound is prepared from {7-methyl-6-[(R)-4-trifluoromethyl-indan-1-yloxy]-2,3-dihydro-benzofuran-3-yl}-acetic acid methyl ester following a procedure analogous to that described in Example 1. The compound is purified by semipreparative HPLC to give the title compound as a mixture of two diastereoisomers. LC (method 8): t_{R} = 7.32 min; Mass spectrum (ESI⁻): m/z 391 [M-H]⁻.

### Example 106

### {4-Methyl-6-[(R)-4-trifluoromethyl-indan-1-yloxy]-2,3-dihydro-benzofuran-3-yl}-acetic acid

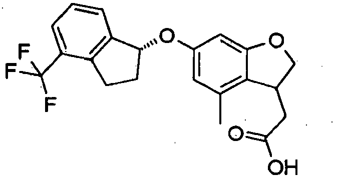

The title compound is prepared from {4-methyl-6-[(R)-4-trifluoromethyl-indan-1-yloxy]-2,3-dihydro-benzofuran-3-yl}-acetic acid methyl ester following a procedure analogous to that described in Example 1. The compound is purified by semipreparative HPLC to give the title compound as a mixture of two diastereoisomers. LC (method 8): t_{R} = 7.30 min; Mass spectrum (ESI⁻): m/z 391 [M-H]⁻.

### Example 107

### {5-Methyl-6-[(R)-4-trifluoromethyl-indan-1-yloxy]-2,3-dihydro-benzofuran-3-yl}-acetic acid

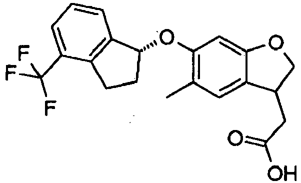

The title compound is prepared from {5-methyl-6-[(R)-4-trifluoromethyl-indan-1-yloxy]-2,3-dihydro-benzofuran-3-yl}-acetic acid methyl ester following a procedure analogous to that described in Example 1. The compound is purified by semipreparative HPLC to give the title compound as a mixture of two diastereoisomers. HPLC-MS (method 9): t_{R} = 9.14 min; Mass spectrum (ESI⁺): m/z 393 [M+H]⁺.

The following compounds are prepared from (*S*)-(6-hydroxy-2,3-dihydro-benzofuran-3-yl)-acetic acid methyl ester and the respective indan-1-ol following a procedure analogous to that described in Step 3 of Intermediate 1; the resulting carboxylic ester may be cleaved as described in Example 1 to obtain the carboxylic acid. The individual indan-1-ol compounds are prepared analogously to the above-mentioned Examples and other methods known from the literature.

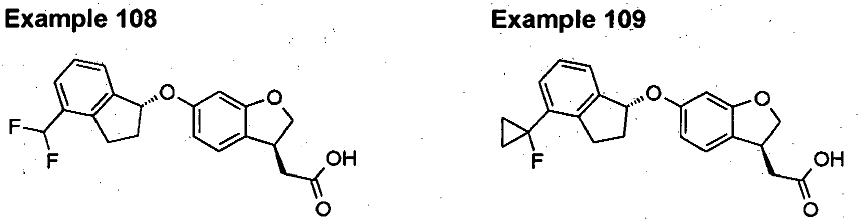

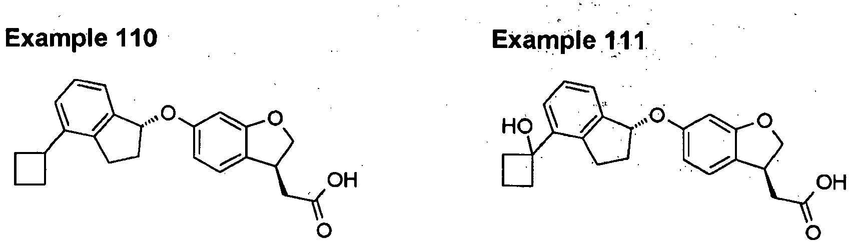

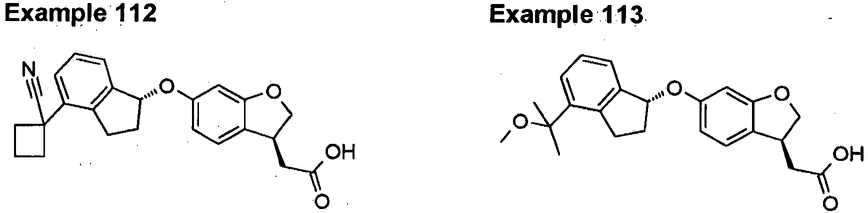

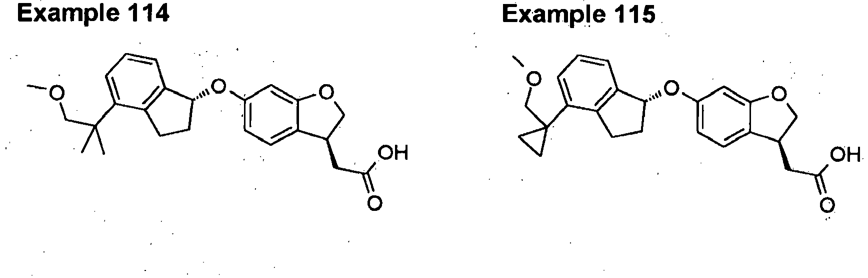

## Claims

1. A compound of formula (I) 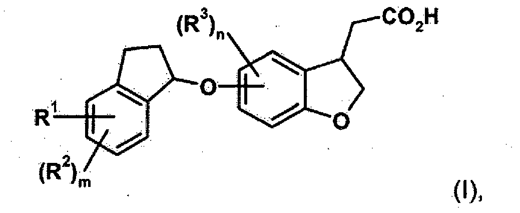 wherein:
R¹ is selected from the group consisting of H, F, Cl, Br, I,
C₁₋₈-alkyl, C₂₋₆-alkenyl, C₂₋₆-alkynyl, C₃₋₁₂-cycloalkyl, C₃₋₁₂-cycloalkyl-C₁₋₄-alkyl, C₅-₁₂-bicycloalkyl-, C₅₋₁₂-bicycloalkyl-C₁₋₆-alkyl-, C₅₋₆-cycloalkenyl, C₅₋₆-cycloalkenyl-C₁₋₄-alkyl, C₁₋₈-alkyloxy, C₃₋₆-cycloalkyl-oxy, and C₃₋₆-cycloalkyl-C₁₋₄-alkyloxy, wherein any of these groups optionally and independently is substituted with 1 to 3 fluorine atoms and/or 1 to 3 R⁴ groups;
C₃₋₁₂-heterocyclyl, C₃₋₁₂-heterocyclyl-C₁₋₄-alkyl, bicycloheterocyclyl-, bicycloheterocyclyl-C₁₋₆-alkyl-, heterocyclyl-C₁₋₄-alkyl-oxy, and C₃₋₁₂-heterocyclyloxy, wherein any of these groups optionally and independently is substituted with 1 to 3 R⁴ groups,
aryl, aryl-C₁₋₄-alkyl, aryl-C₁₋₄-alkyl-oxy, aryloxy, heteroaryl, heteroaryl-C₁₋₄-alkyl, heteroaryl-C₁₋₄-alkyl-oxy, and heteroaryloxy, wherein any of these groups optionally and independently is substituted with 1 to 5 R⁵ groups,
C₁₋₄-alkyl-sulfanyl, C₁₋₄-alkyl-sulfinyl, and C₁₋₄-alkyl-sulfonyl, wherein any of these groups optionally is substituted with 1 to 3 fluorine atoms, and
cyano, nitro, amino, C₁₋₄-alkylamino, and di-(C₁₋₄-alkyl)-amino;
wherein the aforementioned heterocyclyl groups and submoieties may be partially unsaturated and comprise 1 to 3 heteroatoms or groups selected from N, NR^{N}, O and S, with the proviso that only up to two of the heteroatoms are O and S and no O-O, S-S, and S-O bond is formed, While a methylene group bound to a heteroatom may be replaced by a carbonyl group, whilst the heterocyclyl groups are bound to the respective residue via a carbon or nitrogen atom; RN denotes H, C₁₋₄-alkyl-, C₁₋₄-alkyl-C(O)- or C₁₋₄-alkyl-O-C(O)-,
wherein the aforementioned bicycloheterocyclyl groups and submoieties may be partially unsaturated and comprise 6 to 12 ring members and 1 to 3 heteroatoms or groups selected from N, NR^{N}, O and S, with the proviso that only up to two of the heteroatoms are O and S and no O-O, S-S, and S-O bond is formed, while a methylene group bound to a heteroatom may be replaced by a carbonyl group, whilst the heterocyclyl groups are bound to the respective residue via a carbon or nitrogen atom; R^{N} denotes H, C₁₋₄-alkyl-, C₁₋₄-alkyl-C(O)- or C₁₋₄-alkyl-O-C(O)-,
wherein the aforementioned bicycloalkyl and bicycloheterocyclyl groups and submoieties comprise fused, bridged and spiro ring systems,
wherein the aforementioned aryl groups and submoieties comprise 6 to 10 carbon atoms, wherein in bicyclic aromatic groups the ring not attached to the respective residue may be partially saturated, and
wherein the aforementioned heteroaryl groups and submoieties consist of 5 to 14 atoms containing 1 to 3 heteroatoms selected from N, O or S(O)ᵣ, wherein r = 0, 1 or 2, wherein in polycyclic heteroaromatic groups the rings not attached to the respective residue may be partially or fully saturated, while at least one aromatic ring includes one or more hetereoatoms,
R² is selected from the group consisting of F, Cl, Br, I, cyano, C₁₋₄-alkyl, C₃₋₆-cycloalkyl and C₁₋₄-alkyloxy, wherein any alkyl and cycloalkyl group or submoiety is optionally substituted with 1 to 3 fluorine atoms,
R³ is selected from the group consisting of F, Cl, Br, I, C₁₋₄-alkyl and C₁₋₄-alkyloxy, wherein any alkyl group or submoiety is optionally substituted with 1 to 3 fluorine atoms,
R⁴ is selected from the group consisting of F, Cl, Br, I, cyano, C₁₋₄-alkyl, hydroxy, hydroxy-C₁₋₄-alkyl, C₁₋₄-alkyl-oxy, C₁₋₄-alkyloxy-C₁₋₄-alkyl, C₁₋₄-alkyl-sulfanyl, C₁₋₄-alkyl-sulfinyl, C₁₋₄-alkyl-sulfonyl, C₃₋₆-cycloalkyl-, C₃₋₆-cycloalkyl-oxy-, wherein any alkyl and cycloalkyl group or submoiety is optionally substituted with 1 to 3 fluorine atoms, and
R⁵ is selected from the group consisting of F, Cl, Br, I, cyano, nitro, amino, C₁₋₄-alkyl-amino, di-(C₁₋₄-alkyl)-amino, C₁₋₄-alkyl, C₂₋₄-alkenyl, C₂₋₄-alkynyl, hydroxy, hydroxy-C₁₋₄-alkyl, C₁₋₄-alkyl-oxy, C₁₋₄-alkyloxy-C₁₋₄-alkyl, C₁₋₄-alkyl-sulfanyl, C₁₋₄-alkyl-sulfinyl, C₁₋₄-alkyl-sulfonyl, C₃₋₆-cycloalkyl-; C₃₋₆-cycloalkyl-oxy-, wherein any alkyl and cycloalkyl group or submoiety is optionally substituted with 1 to 5 fluorine atoms,
m is selected from the numbers 0, 1, 2 and 3, preferably from the numbers 0, 1 and 2, or, more preferably, from the numbers 0 and 1, whereas the number 0 is most preferred and
n is selected from the numbers 0, 1, 2 and 3, preferably from the numbers 0 and 1, whereas the number 0 is most preferred,
wherein in any definition mentioned hereinbefore and if not specified otherwise, any alkyl moiety mentioned hereinbefore may be straight-chained or branched,
or a salt thereof.

2. A compound according to claim 1 wherein
R¹ is selected from the group consisting of H, F, Cl, Br,
C₁₋₆-alkyl, C₅₋₆-alkenyl, C₃₋₁₀-cycloalkyl, C₃₋₁₀-cycloalkyl-C₁₋₄-alkyl, C₅₋₆-cycloalkenyl, C₁₋₆-alkyloxy, C₃₋₆-cyclolkyl-oxy, and C₃₋₆-cycloalkyl-C₁₋₄-alkyloxy, wherein any of these groups optionally and independently is substituted with 1 to 3 fluorine atoms and/or 1 to 3 R⁴ groups,
C₅₋₁₀-heterocyclyl, C₅₋₁₀-heterocyclyl-C₁₋₄-alkyl, bicycloheterocyclyl-, bicycloheterocyclyl-C₁₋₄-alkyl-, C₅₋₁₀-heterocyclyl-C₁₋₄-alkyl-oxy, and C₅₋₁₀-heterocyclyloxy, wherein any of these groups optionally and independently is substituted with 1 to 3 R⁴ groups,
phenyl, phenyl-C₁₋₄-alkyl, phenyl-C₁₋₄-alkyl-oxy, phenyloxy, heteroaryl, heteroaryl-C₁₋₄-alkyl, heteroaryl-C₁₋₄-alkyl-oxy, and heteroaryloxy, wherein any of these groups optionally and independently is substituted with 1 to 3 R⁵ groups,
C₁₋₂-alkyl-sulfanyl, optionally substituted with 1 to 3 fluorine-atoms, and
cyano,
wherein the aforementioned heterocyclyl groups and submoieties may be partially unsaturated and comprise 1 to 3 heteroatoms or groups selected from N, NR^{N}, O and S, with the proviso that only up to two of the heteroatoms are O and S and no O-O, S-S, and S-O bond is formed, while a methylene group bound to a heteroatom may be replaced by a carbonyl group, whilst the heterocyclyl groups are bound to the respective residue via a carbon or nitrogen atom; R^{N} denotes H, C₁₋₄-alkyl-, C₁₋₄-alkyl-C(O)- or C₁₋₄-alkyl-O-C(O)-,
wherein the aforementioned bicycloheterocyclyl groups and submoieties may be partially unsaturated and comprise 6 to 10 ring members and 1 to 3 heteroatoms or groups selected from N, NR^{N}, O and S, with the proviso that only up to two of the heteroatoms are O and S and no O-O, S-S, and S-O bond is formed, while a methylene group bound to a heteroatom may be replaced by a carbonyl group, whilst the heterocyclyl groups are bound to the respective residue via a carbon or nitrogen atom; R^{N} denotes H, C₁₋₄-alkyl-, C₁₋₄-alkyl-C(O)- or C₁₋₄-alkyl-O-C(O)-,
wherein the aforementioned bicycloalkyl and bicycloheterocyclyl groups and submoieties comprise fused and bridged ring systems,
wherein the aforementioned heteroaryl groups and submoieties consist of 5 to 10 atoms containing 1 to 3 heteroatoms selected from N, O or S(O)ᵣ, wherein r = 0, 1 or 2, wherein in polycyclic heteroaromatic groups the ring or rings not attached to the respective residue may be partially or fully saturated, while at least one aromatic ring includes one or more hetereoatoms,
or a salt thereof.

3. A compound according to claim 1 wherein
R¹ is selected from the group consisting of H, F, Cl, Br,
C₁₋₆-alkyl, C₅₋₆-alkenyl, C₃₋₆-cycloalkyl, C₃₋₆-cycloalkyl-C₁₋₄-alkyl, C₅₋₆-cycloalkenyl, tetrahydropyranyl-C₁₋₂-alkyl, and C₁₋₆-alkyloxy, wherein any of these groups optionally and independently is substituted with 1 to 3 fluorine atoms and/or 1 to 3 R⁴ groups,
oxetanyl, tetrahydrofuranyl, dihydropyranyl, tetrahydropyranyl, morpholinyl, tetrahydrofuran-3-yl-oxy, tetrahydropyran-3-yloxy, tetrahydropyran-4-yloxy, 8-oxa-spiro[4.5]decenyl, 3-oxa-spiro[5.5]undecenyl, 2-oxa-bicyclo[3.1.1]heptyl, 2-oxa-bicyclo[2.2.1]heptyl, 2-oxa-bicyclo[3.1.1]hept-4-yloxy, 2-oxa-bicyclo[3.1.1]hept-5-yloxy, 2-oxa-bicyclo[3.1.1]hept-6-yloxy, 2-oxa-bicyclo[2.2.1]hept-4-yloxy, 2-oxa-bicyclo[2.2.1]hept-5-yloxy, and 2-oxa-bicyclo[2.2.1]hept-6-yloxy, wherein any of these groups optionally and independently is substituted with 1 to 3 R⁴ groups,
2-oxo-1,2-dihydro-pyridyl, optionally substituted with 1 to 3 R⁴ groups,
phenyl, and heteroaryl, wherein any of these groups optionally and independently is substituted with 1 to 3 R⁵ groups,
trifluoromethylsulfanyl and cyano,
wherein the aforementioned heteroaryl groups and submoieties comprise 5-and 6-membered monocyclic ring systems containing 1 or 2 heteroatoms selected from N, NR^{N}, and O, wherein R^{N} denotes H and C₁₋₄-alkyl, and
m and n both denote the number 1,
or a salt thereof.

4. A compound according to claim 1 wherein
R¹ is selected from the group consisting of H, F, Cl, Br,
C₁₋₆-alkyl, C₅₋₆-alkenyl, C₃₋₁₀-cycloalkyl, C₃₋₁₀-cycloalkyl-C₁₋₄-alkyl, C₅₋₆-cycloalkenyl, C₁₋₆-alkyloxy, C₃₋₆-cycloalkyl-oxy, and C₃₋₆-cycloalkyl-C₁₋₄-alkyloxy, wherein any of these groups optionally and independently is substituted with 1 to 3 fluorine atoms and/or 1 to 3 R⁴ groups,
C₅₋₁₀-heterocyclyl, C₅₋₁₀-heterocyclyl-C₁₋₄-alkyl, bicycloheterocyclyl-, bicycloheterocyclyl-C₁₋₄-alkyl-, C₅₋₁₀-heterocyclyl-C₁₋₄-alkyl-oxy, and C₅₋₁₀-heterocyclyloxy, wherein any of these groups optionally and independently is substituted with 1 to 3 R⁴ groups,
phenyl, phenyl-C₁₋₄-alkyl, phenyl-C₁₋₄-alkyl-oxy, phenyloxy, heteroaryl, heteroaryl-C₁₋₄-alkyl, heteroaryl-C₁₋₄-alkyl-oxy, and heteroaryloxy, wherein any of these groups optionally and independently is substituted with 1 to 3 R⁵ groups,
C_{1-**2**}-alkyl-sulfanyl, optionally substituted with 1 to 3 fluorine atoms, and
cyano,
wherein the aforementioned heterocyclyl groups and submoieties may be partially unsaturated and comprise 1 to 3 heteroatoms or groups selected from N, NR^{N}, O and S, with the proviso that only up to two of the heteroatoms are O and S and no O-O, S-S, and S-O bond is formed, while a methylene group bound to a heteroatom may be replaced by a carbonyl group, whilst the heterocyclyl groups are bound to the respective residue via a carbon or nitrogen atom; R^{N} denotes H, C₁₋₄-alkyl-, C₁₋₄-alkyl-C(O)- or C₁₋₄-alkyl-O-C(O)-,
wherein the aforementioned bicycloheterocyclyl groups and submoieties may be partially unsaturated and comprise 6 to 10 ring members and 1 to 3 heteroatoms or groups selected from N, NR^{N}, O and S, with the proviso that only up to two of the heteroatoms are O and S and no O-O, S-S, and S-O bond is formed, while a methylene group bound to a heteroatom may be replaced by a carbonyl group, whilst the heterocyclyl groups are bound to the respective residue via a carbon or nitrogen atom; R^{N} denotes H, C₁₋₄-alkyl-, C₁₋₄-alkyl-C(O)- or C₁₋₄-alkyl-O-C(O)-,
wherein the aforementioned bicycloalkyl and bicycloheterocyclyl groups and submoieties comprise fused and bridged ring systems,
wherein the aforementioned heteroaryl groups and submoieties consist of 5 to 10 atoms containing 1 to 3 heteroatoms selected from N, O or S(O)ᵣ, wherein r = 0, 1 or 2, wherein in polycyclic heteroaromatic groups the ring or rings not attached to the respective residue may be partially or fully saturated, while at least one aromatic ring includes one or more hetereoatoms,
R² is selected from the group consisting of F, Cl, Br, methyl, difluoromethyl, trifluoromethyl, cyclopropyl, methoxy, difluoromethoxy and trifluoromethoxy,
R³ is selected from the group consisting of F, Cl, Br, methyl, difluoromethyl, trifluoromethyl, methoxy, difluoromethoxy and trifluoromethoxy,
R⁴ is selected from the group consisting of F, cyano, hydroxy, methyl, difluoromethyl, trifluoromethyl, methoxy, difluoromethoxy and trifluoromethoxy,
R⁵ is selected from the group consisting of F, Cl, cyano, C₁₋₃-alkyl (preferably methyl), difluoromethyl, trifluoromethyl, pentafluoroethyl, hydroxy, methoxy, difluoromethoxy and trifluoromethoxy, and
m and n both independently denote the number 0 or 1,
or a salt thereof.

5. A compound according to claim 1 wherein
R¹ is selected from the group consisting of H, F, Cl, Br,
C₁₋₆-alkyl, C₅₋₆-alkenyl, C₃₋₁₀-cycloalkyl, C₃₋₁₀-cycloalkyl-C₁₋₄-alkyl, C₅₋₆-cycloalkenyl, C₁₋₆-alkyloxy, C₃₋₆-cycloalkyl-oxy, and C₃₋₆-cycloalkyl-C₁₋₄-alkyloxy,
wherein any of these groups optionally and independently is substituted with 1 to 3 fluorine atoms and/or 1 to 3 R⁴ groups,
C₅₋₁₀-heterocyclyl, C₅₋₁₀-heterocyclyl-C₁₋₄-alkyl, bicycloheterocyclyl-, bicycloheterocyclyl-C₁₋₄-alkyl-, C₅₋₁₀-heterocyclyl-C₁₋₄-alkyl-oxy, and C₅₋₁₀-heterocyclyloxy, wherein any of these groups optionally and independently is substituted with 1 to 3 R⁴ groups,
phenyl, phenyl-C₁₋₄-alkyl, phenyl-C₁₋₄-alkyl-oxy, phenyloxy, heteroaryl, heteroaryl-C₁₋₄-alkyl, heteroaryl-C₁₋₄-alkyl-oxy, and heteroaryloxy, wherein any of these groups optionally and independently is substituted with 1 to 3 R⁵ groups,
C_{1-**2**}-alkyl-sulfanyl, optionally substituted with 1 to 3 fluorine atoms, and
cyano,
wherein the aforementioned heterocyclyl groups and submoieties may be partially unsaturated and comprise 1 to 3 heteroatoms or groups selected from N, NR^{N}, O and S, with the proviso that only up to two of the heteroatoms are O and S and no O-O, S-S, and S-O bond is formed, while a methylene group bound to a heteroatom may be replaced by a carbonyl group, whilst the heterocyclyl groups are bound to the respective residue via a carbon or nitrogen atom; R^{N} denotes H, C₁₋₄-alkyl-, C₁₋₄-alkyl-C(O)- or C₁₋₄-alkyl-O-C(O)-,
wherein the aforementioned bicycloheterocyclyl groups and submoieties may be partially unsaturated and comprise 6 to 10 ring members and 1 to 3 heteroatoms or groups selected from N, NR^{N}, O and S, with the proviso that only up to two of the heteroatoms are O and S and no O-O, S-S, and S-O bond is formed, while a methylene group bound to a heteroatom may be replaced by a carbonyl group, whilst the heterocyclyl groups are bound to the respective residue via a carbon or nitrogen atom; R^{N} denotes H, C₁₋₄-alkyl-, C₁₋₄-alkyl-C(O)- or C₁₋₄-alkyl-O-C(O)-,
wherein the aforementioned bicycloalkyl and bicycloheterocyclyl groups and submoieties comprise fused and bridged ring systems,
wherein the aforementioned heteroaryl groups and submoieties consist of 5 to 10 atoms containing 1 to 3 heteroatoms selected from N, O or S(O)ᵣ, wherein r = 0, 1 or 2, wherein in polycyclic heteroaromatic groups the ring or rings not attached to the respective residue may be partially or fully saturated, while at least one aromatic ring includes one or more heteroatoms,
R² is selected from the group consisting of F, Cl, Br, methyl, difluoromethyl, trifluoromethyl, cyclopropyl, methoxy, difluoromethoxy and trifluoromethoxy,
R³ is selected from the group consisting of F, Cl, Br, methyl, difluoromethyl, trifluoromethyl, methoxy, difluoromethoxy and trifluoromethoxy,
R⁴ is selected from the group consisting of F, cyano, hydroxy, methyl, difluoromethyl, trifluoromethyl, methoxy, difluoromethoxy and trifluoromethoxy,
R⁵ is selected from the group consisting of F, Cl, cyano, C₁₋₃-alkyl (preferably methyl), difluoromethyl, trifluoromethyl, pentafluoroethyl, hydroxy, methoxy, difluoromethoxy and trifluoromethoxy,
m denotes 0 and
n denotes 0,
or a salt thereof.

6. A compound according to claim 1 wherein
R¹ is selected from the group consisting of H, F, Cl, Br,
C₁₋₆-alkyl, C₅₋₆-alkenyl, C₃₋₆-cycloalkyl, C₃₋₆-cycloalkyl-C₁₋₄-alkyl, C₅₋₆-cycloalkenyl, tetrahydropyranyl-C₁₋₂-alkyl, and C₁₋₆-alkyloxy, wherein any of these groups optionally and independently is substituted with 1 to 3 fluorine atoms and/or 1 to 3 R⁴ groups,
oxetanyl, tetrahydrofuranyl, dihydropyranyl, tetrahydropyranyl, morpholinyl, tetrahydrofuran-3-yl-oxy, tetrahydropyran-3-yloxy, tetrahydropyran-4-yloxy, 8-oxa-spiro[4.5]decenyl, 3-oxa-spiro[5.5]undecenyl, 2-oxa-bicyclo[3.1.1]heptyl, 2-oxa-bicyclo[2.2.1]heptyl, 2-oxa-bicyclo[3.1.1]hept-4-yloxy, 2-oxa-bicyclo[3.1.1]hept-5-yloxy, 2-oxa-bicyclo[3.1.1]hept-6-yloxy, 2-oxa-bicyclo[2.2.1]hept-4-yloxy, 2-oxa-bicyclo[2.2.1]hept-5-yloxy, and 2-oxa-bicyclo[2.2.1]hept-6-yloxy, wherein any of these groups optionally and independently is substituted with 1 to 3 R⁴ groups,
2-oxo-1,2-dihydro-pyridyl, optionally substituted with 1 to 3 R⁴ groups,
phenyl and heteroaryl, wherein any of these groups optionally and independently is substituted with 1 to 3 R⁵ groups,
trifluoromethylsulfanyl and cyano,
wherein the aforementioned heteroaryl groups and submoieties comprise 5-and 6-membered monocyclic ring systems containing 1 or 2 heteroatoms selected from N, NR^{N}, and O, wherein R^{N} denotes H and C₁₋₄-alkyl,
and
R², R³, R⁴, R⁵, m and n are defined as in claim 5,
or a salt thereof.

7. A pharmaceutically acceptable salt of a compound according to one or more of the claims 1 to 6.

8. A pharmaceutical composition comprising one or more compounds according to one or more of the claims 1 to 6 or one or more pharmaceutically acceptable salts thereof, optionally together with one or more inert carriers and/or diluents.

9. A compound according to one or more of the claims 1 to 6 or a pharmaceutically acceptable salt thereof for use as a medicament.

10. A compound according to one or more of the claims 1 to 6 or a pharmaceutically acceptable salt thereof for the treatment of diseases or conditions which can be influenced by the modulation of the function of GPR40, particularly, for the prophylaxis and/or therapy of metabolic diseases, such as diabetes, more specifically type 2 diabetes mellitus, and conditions associated with the disease, including insulin resistance, obesity, cardiovascular disease and dyslipidemia.

11. A pharmaceutical composition comprising one or more compounds according to one or more of the claims 1 to 6 or one or more pharmaceutically acceptable salts thereof and one or more additional therapeutic agents, optionally together with one or more inert carriers and/or diluents.

## Patentansprüche

1. Verbindung der Formel (I) 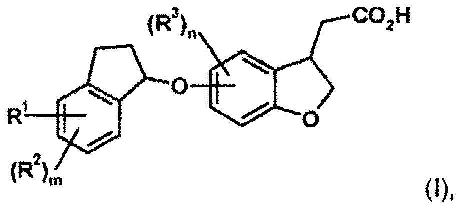 wobei:
R¹ ausgewählt ist aus der Gruppe, bestehend aus H, F, Cl, Br, I,
C₁₋₈-Alkyl, C₂₋₆-Alkenyl, C₂₋₆-Alkinyl, C₃₋₁₂-Cycloalkyl, C₃₋₁₂-Cycloalkyl-C₁₋₄-alkyl, C₅₋₁₂-Bicycloalkyl-, C₅₋₁₂-Bicycloalkyl-C₁₋₆-alkyl-, C₅₋₆-Cycloalkenyl, C₅₋₆-Cycloalkenyl-C₁₋₄-alkyl, C₁₋₈-Alkyloxy, C₃₋₆-Cycloalkyl-oxy und C₃₋₆-Cycloalkyl-C₁₋₄-alkyloxy, wobei jede dieser Gruppen gegebenenfalls und unabhängig mit 1 bis 3 Fluoratomen und/oder 1 bis 3 R⁴-Gruppen substituiert ist,
C₃₋₁₂-Heterocyclyl, C₃₋₁₂-Heterocyclyl-C₁₋₄-alkyl, Bicycloheterocyclyl-, Bicycloheterocyclyl-C₁₋₆-alkyl-, Heterocyclyl-C₁₋₄-alkyl-oxy und C₃₋₁₂-Heterocyclyloxy, wobei jede dieser Gruppen gegebenenfalls und unabhängig mit 1 bis 3 R⁴-Gruppen substituiert ist,
Aryl, Aryl-C₁₋₄-alkyl, Aryl-C₁₋₄-alkyl-oxy, Aryloxy, Heteroaryl, Heteroaryl-C₁₋₄-alkyl, Heteroaryl-C₁₋₄-alkyl-oxy und Heteroaryloxy, wobei jede dieser Gruppen gegebenenfalls und unabhängig mit 1 bis 5 R⁵-Gruppen substituiert ist,
C₁₋₄-Alkyl-sulfanyl, C₁₋₄-Alkyl-sulfinyl und C₁₋₄-Alkyl-sulfonyl, wobei jede dieser Gruppen gegebenenfalls mit 1 bis 3 Fluoratomen substituiert ist, und
Cyano, Nitro, Amino, C₁₋₄-Alkylamino und Di-(C₁₋₄-alkyl)-amino;
wobei die vorgenannten Heterocyclyl-Gruppen und -Untergruppen teilweise ungesättigt sein können und 1 bis 3 Heteroatome oder Gruppen umfassen, ausgewählt aus N, NR^{N}, O und S, mit der Maßgabe, dass nur bis zu zwei der Heteroatome O und S darstellen und keine O-O-, S-S- und S-O-Bindung gebildet wird, wobei eine MethylenGruppe, die an ein Heteroatom gebunden ist, durch eine Carbonyl-Gruppe ersetzt sein kann, wobei die Heterocyclyl-Gruppen über ein Kohlenstoff- oder ein Stickstoffatom an den jeweiligen Rest gebunden sind; R^{N} bezeichnet H, C₁₋₄-Alkyl-, C₁₋₄-Alkyl-C(O)- oder C₁₋₄-Alkyl-O-C(O)-,
wobei die vorgenannten Bicycloheterocyclyl-Gruppen und -Untergruppen teilweise ungesättigt sein können und 6 bis 12 Ringglieder und 1 bis 3 Heteroatome oder Gruppen umfassen, ausgewählt aus N, NR^{N}, O und S, mit der Maßgabe, dass nur bis zu zwei der Heteroatome O und S darstellen und keine O-O-, S-S- und S-O-Bindung gebildet wird, wobei eine Methylen-Gruppe, die an ein Heteroatom gebunden ist, durch eine Carbonyl-Gruppe ersetzt sein kann, wobei die Heterocyclyl-Gruppen über ein Kohlenstoff- oder Stickstoffatom an den jeweiligen Rest gebunden sind; R^{N} bezeichnet H, C₁₋₄-Alkyl-, C₁₋₄-Alkyl-C(O)- oder C₁₋₄-Alkyl-O-C(O)-,
wobei die vorgenannten Bicycloalkyl- und Bicycloheterocyclyl-Gruppen und -Untergruppen kondensierte, verbrückte und Spiro-Ringsysteme umfassen,
wobei die vorgenannten Aryl-Gruppen und -Untergruppen 6 bis 10 Kohlenstoffatome umfassen, wobei in bicyclischen aromatischen Gruppen der Ring, der nicht mit dem jeweiligen Rest verknüpft ist, teilweise gesättigt sein kann, und
wobei die vorgenannten Heteroaryl-Gruppen und -Untergruppen aus 5 bis 14 Atomen bestehen, enthaltend 1 bis 3 Heteroatome, ausgewählt aus N, O oder S(O)ᵣ, wobei r = 0, 1 oder 2, wobei in polycyclischen heteroaromatischen Gruppen die Ringe, die nicht mit dem jeweiligen Rest verknüpft sind, teilweise oder vollständig gesättigt sein können, wobei mindestens ein aromatischer Ring ein oder mehrere Hetereoatome umfasst,
R² ist ausgewählt aus der Gruppe, bestehend aus F, Cl, Br, I, Cyano, C₁₋₄-Alkyl, C₃₋₆-Cycloalkyl und C₁₋₄-Alkyloxy, wobei jede Alkyl- und Cycloalkyl-Gruppe oder -Untergruppe gegebenenfalls mit 1 bis 3 Fluoratomen substituiert ist,
R³ ist ausgewählt aus der Gruppe, bestehend aus F, Cl, Br, I, C₁₋₄-Alkyl und C₁₋₄-Alkyloxy, wobei jede Alkyl-Gruppe oder -Untergruppe gegebenenfalls mit 1 bis 3 Fluoratomen substituiert ist,
R⁴ ist ausgewählt aus der Gruppe, bestehend aus F, Cl, Br, I, Cyano, C₁₋₄-Alkyl, Hydroxy, Hydroxy-C₁₋₄-alkyl, C₁₋₄-Alkyl-oxy, C₁₋₄-Alkyloxy-C₁₋₄-alkyl, C₁₋₄-Alkyl-sulfanyl, C₁₋₄-Alkyl-sulfinyl, C₁₋₄-Alkyl-sulfonyl, C₃₋₆-Cycloalkyl- C₃₋₆-Cycloalkyl-oxy-, wobei jede Alkyl- und Cycloalkyl-Gruppe oder -Untergruppe gegebenenfalls mit 1 bis 3 Fluoratomen substituiert ist, und
R⁵ ist ausgewählt aus der Gruppe, bestehend aus F, Cl, Br, I, Cyano, Nitro, Amino, C₁₋₄-Alkyl-amino, Di-(C₁₋₄-alkyl)-amino, C₁₋₄-Alkyl, C₂₋₄-Alkenyl, C₂₋₄-Alkinyl, Hydroxy, Hydroxy-C₁₋₄-alkyl, C₁₋₄-Alkyl-oxy, C₁₋₄-Alkyloxy-C₁₋₄-alkyl, C₁₋₄-Alkyl-sulfanyl, C₁₋₄-Alkyl-sulfinyl, C₁₋₄-Alkyl-sulfonyl, C₃₋₆-Cycloalkyl-, C₃₋₆-Cycloalkyl-oxy-, wobei jede Alkyl- und Cycloalkyl-Gruppe oder -Untergruppe gegebenenfalls mit 1 bis 5 Fluoratomen substituiert ist,
m ist ausgewählt aus den Zahlen 0, 1, 2 und 3, bevorzugt aus den Zahlen 0, 1 und 2, oder bevorzugter aus den Zahlen 0 und 1, wobei die Zahl 0 am meisten bevorzugt ist, und
n ist ausgewählt aus den Zahlen 0, 1, 2 und 3, bevorzugt aus den Zahlen 0 und 1, wobei die Zahl 0 am meisten bevorzugt ist,
wobei in jeder hier zuvor genannten Definition und, wenn nicht anders angegeben, jeder hier zuvor genannte Alkylrest geradkettig oder verzweigt sein kann,
oder ein Salz hiervon.

2. Verbindung nach Anspruch 1, wobei
R¹ ausgewählt ist aus der Gruppe, bestehend aus H, F, Cl, Br,
C₁₋₆-Alkyl, C₅₋₆-Alkenyl, C₃₋₁₀-Cycloalkyl, C₃₋₁₀-Cycloalkyl-C₁₋₄-alkyl, C₅₋₆-Cycloalkenyl, C₁₋₆-Alkyloxy, C₃₋₆-Cycloalkyl-oxy und C₃₋₆-Cycloalkyl-C₁₋₄-alkyloxy, wobei jede dieser Gruppen gegebenenfalls und unabhängig mit 1 bis 3 Fluoratomen und/oder 1 bis 3 R⁴-Gruppen substituiert ist,
C₅₋₁₀-Heterocyclyl, C₅₋₁₀-Heterocyclyl-C₁₋₄-alkyl, Bicycloheterocyclyl-, Bicycloheterocyclyl-C₁₋₄-alkyl-, C₅₋₁₀-Heterocyclyl-C₁₋₄-alkyl-oxy und C₅₋₁₀-Heterocyclyloxy, wobei jede dieser Gruppen gegebenenfalls und unabhängig mit 1 bis 3 R⁴-Gruppen substituiert ist,
Phenyl, Phenyl-C₁₋₄-alkyl, Phenyl-C₁₋₄-alkyl-oxy, Phenyloxy, Heteroaryl, Heteroaryl-C₁₋₄-alkyl, Heteroaryl-C₁₋₄-alkyl-oxy und Heteroaryloxy, wobei jede dieser Gruppen gegebenenfalls und unabhängig mit 1 bis 3 R⁵-Gruppen substituiert ist,
C₁₋₂-Alkyl-sulfanyl, gegebenenfalls substituiert mit 1 bis 3 Fluoratomen, und
Cyano,
wobei die vorgenannten Heterocyclyl-Gruppen und -Untergruppen teilweise ungesättigt sein können und 1 bis 3 Heteroatome oder Gruppen umfassen, ausgewählt aus N, NR^{N}, O und S, mit der Maßgabe, dass nur bis zu zwei der Heteroatome O und S darstellen und keine O-O-, S-S- und S-O-Bindung gebildet wird, wobei eine Methylen-Gruppe, die an ein Heteroatom gebunden ist, durch eine Carbonyl-Gruppe ersetzt sein kann, wobei die Heterocyclyl-Gruppen über ein Kohlenstoff- oder ein Stickstoffatom an den jeweiligen Rest gebunden sind; R^{N} bezeichnet H, C₁₋₄-Alkyl-, C₁₋₄-Alkyl-C(O)- oder C₁₋₄-Alkyl-O-C(O)-,
wobei die vorgenannten Bicycloheterocyclyl-Gruppen und -Untergruppen teilweise ungesättigt sein können und 6 bis 10 Ringglieder und 1 bis 3 Heteroatome oder Gruppen umfassen, ausgewählt aus N, NR^{N}, O und S, mit der Maßgabe, dass nur bis zu zwei der Heteroatome O und S darstellen und keine O-O-, S-S- und S-O-Bindung gebildet wird, wobei eine Methylen-Gruppe, die an ein Heteroatom gebunden ist, durch eine Carbonyl-Gruppe ersetzt sein kann, wobei die Heterocyclyl-Gruppen über ein Kohlenstoff- oder Stickstoffatom an den jeweiligen Rest gebunden sind; R^{N} bezeichnet H, C₁₋₄-Alkyl-, C₁₋₄-Alkyl-C(O) oder C₁₋₄-Alkyl-O-C(O)-,
wobei die vorgenannten Bicycloalkyl- und Bicycloheterocyclyl-Gruppen und -Untergruppen kondensierte und verbrückte Ringsysteme umfassen,
wobei die vorgenannten Heteroaryl-Gruppen und -Untergruppen aus 5 bis 10 Atomen bestehen, enthaltend 1 bis 3 Heteroatome, ausgewählt aus N, O oder S(O)ᵣ, wobei r = 0, 1 oder 2, wobei in polycyclischen heteroaromatischen Gruppen der Ring oder die Ringe, der/die nicht mit dem jeweiligen Rest verknüpft ist/sind, teilweise oder vollständig gesättigt sein kann/können, wobei mindestens ein aromatischer Ring ein oder mehrere Hetereoatome umfasst,
oder ein Salz hiervon.

3. Verbindung nach Anspruch 1, wobei
R¹ ausgewählt ist aus der Gruppe, bestehend aus H, F, Cl, Br,
C₁₋₆-Alkyl, C₅₋₆-Alkenyl, C₃₋₆-Cycloalkyl, C₃₋₆-Cycloalkyl-C₁₋₄-alkyl, C₅₋₆-Cycloalkenyl, Tetrahydropyranyl-C₁₋₂-alkyl und C₁₋₆-Alkyloxy, wobei jede dieser Gruppen gegebenenfalls und unabhängig mit 1 bis 3 Fluoratomen und/oder 1 bis 3 R⁴-Gruppen substituiert ist,
Oxetanyl, Tetrahydrofuranyl, Dihydropyranyl, Tetrahydropyranyl, Morpholinyl, Tetrahydrofuran-3-yl-oxy, Tetrahydropyran-3-yloxy, Tetrahydropyran-4-yloxy, 8-Oxa-spiro[4.5]decenyl, 3-Oxa-spiro[5.5]undecenyl, 2-Oxa-bicyclo[3.1.1]heptyl, 2-Oxa-bicyclo[2.2.1]heptyl, 2-Oxa-bicyclo[3.1.1]hept-4-yloxy, 2-Oxa-bicyclo[3.1.1]hept-5-yloxy, 2-Oxa-bicyclo[3.1.1]hept-6-yloxy, 2-Oxa-bicyclo[2.2.1]hept-4-yloxy, 2-Oxa-bicyclo[2.2.1]hept-5-yloxy und 2-Oxa-bicyclo[2.2.1]hept-6-yloxy, wobei jede dieser Gruppen gegebenenfalls und unabhängig mit 1 bis 3 R⁴-Gruppen substituiert ist,
2-Oxo-1,2-dihydro-pyridyl, gegebenenfalls substituiert mit 1 bis 3 R⁴-Gruppen,
Phenyl und Heteroaryl, wobei jede dieser Gruppen gegebenenfalls und unabhängig mit 1 bis 3 R⁵-Gruppen substituiert ist,
Trifluormethylsulfanyl und Cyano,
wobei die vorgenannten Heteroaryl-Gruppen und -Untergruppen 5- und 6-gliedrige monocyclische Ringsysteme umfassen, enthaltend 1 oder 2 Heteroatome, ausgewählt aus N, NR^{N} und O, wobei R^{N} H und C₁₋₄-Alkyl bezeichnet, und
m und n bezeichnen beide die Zahl 1,
oder ein Salz hiervon.

4. Verbindung nach Anspruch 1 wobei
R¹ ausgewählt ist aus der Gruppe, bestehend aus H, F, Cl, Br,
C₁₋₆-Alkyl, C₅₋₆-Alkenyl, C₃₋₁₀-Cycloalkyl, C₃₋₁₀-Cycloalkyl-C₁₋₄-alkyl, C₅₋₆-Cycloalkenyl, C₁₋₆-Alkyloxy, C₃₋₆-Cycloalkyl-oxy und C₃₋₆-Cycloalkyl-C₁₋₄-alkyloxy, wobei jede dieser Gruppen gegebenenfalls und unabhängig mit 1 bis 3 Fluoratomen und/oder 1 bis 3 R⁴-Gruppen substituiert ist,
C₅₋₁₀-Heterocyclyl, C₅₋₁₀-Heterocyclyl-C₁₋₄-alkyl, Bicycloheterocyclyl-, Bicycloheterocyclyl-C₁₋₄-alkyl-, C₅₋₁₀-Heterocyclyl-C₁₋₄-alkyl-oxy und C₅₋₁₀-Heterocyclyloxy, wobei jede dieser Gruppen gegebenenfalls und unabhängig mit 1 bis 3 R⁴-Gruppen substituiert ist,
Phenyl, Phenyl-C₁₋₄-alkyl, Phenyl-C₁₋₄-alkyl-oxy, Phenyloxy, Heteroaryl, Heteroaryl-C₁₋₄-alkyl, Heteroaryl-C₁₋₄-alkyl-oxy und Heteroaryloxy, wobei jede dieser Gruppen gegebenenfalls und unabhängig mit 1 bis 3 R⁵-Gruppen substituiert ist,
C₁₋₂-Alkyl-sulfanyl, gegebenenfalls substituiert mit 1 bis 3 Fluoratomen, und
Cyano,
wobei die vorgenannten Heterocyclyl-Gruppen und -Untergruppen teilweise ungesättigt sein können und 1 bis 3 Heteroatome oder Gruppen umfassen, ausgewählt aus N, NR^{N}, O und S, mit der Maßgabe, dass nur bis zu zwei der Heteroatome O und S darstellen und keine O-O-, S-S- und S-O-Bindung gebildet wird, wobei eine Methylen-Gruppe, die an ein Heteroatom gebunden ist, durch eine Carbonyl-Gruppe ersetzt sein kann, wobei die Heterocyclyl-Gruppen über ein Kohlenstoff- oder ein Stickstoffatom an den jeweiligen Rest gebunden sind; R^{N} bezeichnet H, C₁₋₄-Alkyl-, C₁₋₄-Alkyl-C(O)- oder C₁₋₄-Alkyl-O-C(O)-,
wobei die vorgenannten Bicycloheterocyclyl-Gruppen und -Untergruppen teilweise ungesättigt sein können und 6 bis 10 Ringglieder und 1 bis 3 Heteroatome oder Gruppen umfassen, ausgewählt aus N, NR^{N}, O und S, mit der Maßgabe, dass nur bis zu zwei der Heteroatome O und S darstellen und keine O-O-, S-S- und S-O-Bindung gebildet wird, wobei eine Methylen-Gruppe, die an ein Heteroatom gebunden ist, durch eine Carbonyl-Gruppe ersetzt sein kann, wobei die Heterocyclyl-Gruppen über ein Kohlenstoff- oder Stickstoffatom an den jeweiligen Rest gebunden sind; R^{N} bezeichnet H, C₁₋₄-Alkyl-, C₁₋₄-Alkyl-C(O)- oder C₁₋₄-Alkyl-O-C(O)-,
wobei die vorgenannten Bicycloalkyl- und Bicycloheterocyclyl-Gruppen und -Untergruppen kondensierte und verbrückte Ringsysteme umfassen,
wobei die vorgenannten Heteroaryl-Gruppen und -Untergruppen aus 5 bis 10 Atomen bestehen, enthaltend 1 bis 3 Heteroatome, ausgewählt aus N, O oder S(O)ᵣ, wobei r = 0, 1 oder 2, wobei in polycyclischen heteroaromatischen Gruppen der Ring oder die Ringe, der/die nicht mit dem jeweiligen Rest verknüpft ist/sind, teilweise oder vollständig gesättigt sein kann/können, wobei mindestens ein aromatischer Ring ein oder mehrere Hetereoatome umfasst,
R² ist ausgewählt aus der Gruppe, bestehend aus F, Cl, Br, Methyl, Difluormethyl, Trifluormethyl, Cyclopropyl, Methoxy, Difluormethoxy und Trifluormethoxy,
R³ ist ausgewählt aus der Gruppe, bestehend aus F, Cl, Br, Methyl, Difluormethyl, Trifluormethyl, Methoxy, Difluormethoxy und Trifluormethoxy,
R⁴ ist ausgewählt aus der Gruppe, bestehend aus F, Cyano, Hydroxy, Methyl, Difluormethyl, Trifluormethyl, Methoxy, Difluormethoxy und Trifluormethoxy,
R⁵ ist ausgewählt aus der Gruppe, bestehend aus F, Cl, Cyano, C₁₋₃-Alkyl (bevorzugt Methyl), Difluormethyl, Trifluormethyl, Pentafluorethyl, Hydroxy, Methoxy, Difluormethoxy und Trifluormethoxy, und
m und n bezeichnen beide unabhängig die Zahl 0 oder 1,
oder ein Salz hiervon.

5. Verbindung nach Anspruch 1 wobei
R¹ ausgewählt ist aus der Gruppe, bestehend aus H, F, Cl, Br,
C₁₋₆-Alkyl, C₅₋₆-Alkenyl, C₃₋₁₀-Cycloalkyl, C₃₋₁₀-Cycloalkyl-C₁₋₄-alkyl, C₅₋₆-Cycloalkenyl, C₁₋₆-Alkyloxy, C₃₋₆-Cycloalkyl-oxy und C₃₋₆-Cycloalkyl-C₁₋₄-alkyloxy, wobei jede dieser Gruppen gegebenenfalls und unabhängig mit 1 bis 3 Fluoratomen und/oder 1 bis 3 R⁴-Gruppen substituiert ist,
C₅₋₁₀-Heterocyclyl, C₅₋₁₀-Heterocyclyl-C₁₋₄-alkyl, Bicycloheterocyclyl-, Bicycloheterocyclyl-C₁₋₄-alkyl-, C₅₋₁₀-Heterocyclyl-C₁₋₄-alkyl-oxy und C₅₋₁₀-Heterocyclyloxy, wobei jede dieser Gruppen gegebenenfalls und unabhängig mit 1 bis 3 R⁴-Gruppen substituiert ist,
Phenyl, Phenyl-C₁₋₄-alkyl, Phenyl-C₁₋₄-alkyl-oxy, Phenyloxy, Heteroaryl, Heteroaryl-C₁₋₄-alkyl, Heteroaryl-C₁₋₄-alkyl-oxy und Heteroaryloxy, wobei jede dieser Gruppen gegebenenfalls und unabhängig mit 1 bis 3 R⁵-Gruppen substituiert ist,
C₁₋₂-Alkyl-sulfanyl, gegebenenfalls substituiert mit 1 bis 3 Fluoratomen, und
Cyano,
wobei die vorgenannten Heterocyclyl-Gruppen und -Untergruppen teilweise ungesättigt sein können und 1 bis 3 Heteroatome oder Gruppen umfassen, ausgewählt aus N, NR^{N}, O und S, mit der Maßgabe, dass nur bis zu zwei der Heteroatome O und S darstellen und keine O-O-, S-S- und S-O-Bindung gebildet wird, wobei eine Methylen-Gruppe, die an ein Heteroatom gebunden ist, durch eine Carbonyl-Gruppe ersetzt sein kann, wobei die Heterocyclyl-Gruppen über ein Kohlenstoff- oder ein Stickstoffatom an den jeweiligen Rest gebunden sind; R^{N} bezeichnet H, C₁₋₄-Alkyl-, C₁₋₄-Alkyl-C(O)- oder C₁₋₄-Alkyl-O-C(O)-,
wobei die vorgenannten Bicycloheterocyclyl-Gruppen und -Untergruppen teilweise ungesättigt sein können und 6 bis 10 Ringglieder und 1 bis 3 Heteroatome oder Gruppen umfassen, ausgewählt aus N, NR^{N}, O und S, mit der Maßgabe, dass nur bis zu zwei der Heteroatome O und S darstellen und keine O-O-, S-S- und S-O-Bindung gebildet wird, wobei eine Methylen-Gruppe, die an ein Heteroatom gebunden ist, durch eine Carbonyl-Gruppe ersetzt sein kann, wobei die Heterocyclyl-Gruppen über ein Kohlenstoff- oder Stickstoffatom an den jeweiligen Rest gebunden sind; R^{N} bezeichnet H, C₁₋₄-Alkyl-, C₁₋₄-Alkyl-C(O)- oder C₁₋₄-Alkyl-O-C(O)-,
wobei die vorgenannten Bicycloalkyl- und Bicycloheterocyclyl-Gruppen und -Untergruppen kondensierte und verbrückte Ringsysteme umfassen,
wobei die vorgenannten Heteroaryl-Gruppen und -Untergruppen aus 5 bis 10 Atomen bestehen, enthaltend 1 bis 3 Heteroatome, ausgewählt aus N, O oder S(O)ᵣ, wobei r = 0, 1 oder 2, wobei in polycyclischen heteroaromatischen Gruppen der Ring oder die Ringe, der/die nicht mit dem jeweiligen Rest verknüpft ist/sind, teilweise oder vollständig gesättigt sein kann/können, wobei mindestens ein aromatischer Ring ein oder mehrere Hetereoatome umfasst,
R² ist ausgewählt aus der Gruppe, bestehend aus F, Cl, Br, Methyl, Difluormethyl, Trifluormethyl, Cyclopropyl, Methoxy, Difluormethoxy und Trifluormethoxy,
R³ ist ausgewählt aus der Gruppe, bestehend aus F, Cl, Br, Methyl, Difluormethyl, Trifluormethyl, Methoxy, Difluormethoxy und Trifluormethoxy,
R⁴ ist ausgewählt aus der Gruppe, bestehend aus F, Cyano, Hydroxy, Methyl, Difluormethyl, Trifluormethyl, Methoxy, Difluormethoxy und Trifluormethoxy,
R⁵ ist ausgewählt aus der Gruppe, bestehend aus F, Cl, Cyano, C₁₋₃-Alkyl (bevorzugt Methyl), Difluormethyl, Trifluormethyl, Pentafluorethyl, Hydroxy, Methoxy, Difluormethoxy und Trifluormethoxy,
m bezeichnet 0 und
n bezeichnet 0,
oder ein Salz hiervon.

6. Verbindung nach Anspruch 1 wobei
R¹ ausgewählt ist aus der Gruppe, bestehend aus H, F, Cl, Br,
C₁₋₆-Alkyl, C₅₋₆-Alkenyl, C₃₋₆-Cycloalkyl, C₃₋₆-Cycloalkyl-C₁₋₄-alkyl, C₅₋₆-Cycloalkenyl, Tetrahydropyranyl-C₁₋₂-alkyl und C₁₋₆-Alkyloxy, wobei jede dieser Gruppen gegebenenfalls und unabhängig mit 1 bis 3 Fluoratomen und/oder 1 bis 3 R⁴-Gruppen substituiert ist, Oxetanyl, Tetrahydrofuranyl, Dihydropyranyl, Tetrahydropyranyl, Morpholinyl, Tetrahydrofuran-3-yl-oxy, Tetrahydropyran-3-yloxy, Tetrahydropyran-4-yloxy, 8-Oxaspiro[4.5]decenyl, 3-Oxa-spiro[5.5]undecenyl, 2-Oxa-bicyclo[3.1.1]heptyl, 2-Oxabicyclo[2.2.1]heptyl, 2-Oxa-bicyclo[3.1.1]hept-4-yloxy, 2-Oxa-bicyclo[3.1.1]hept-5-yloxy, 2-Oxa-bicyclo[3.1.1]hept-6-yloxy, 2-Oxa-bicyclo[2.2.1]hept-4-yloxy, 2-Oxabicyclo[2.2.1]hept-5-yloxy und 2-Oxa-bicyclo[2.2.1]hept-6-yloxy, wobei jede dieser Gruppen gegebenenfalls und unabhängig mit 1 bis 3 R⁴-Gruppen substituiert ist,
2-Oxo-1,2-dihydro-pyridyl, gegebenenfalls substituiert mit 1 bis 3 R⁴-Gruppen,
Phenyl und Heteroaryl, wobei jede dieser Gruppen gegebenenfalls und unabhängig mit 1 bis 3 R⁵-Gruppen substituiert ist,
Trifluormethylsulfanyl und Cyano,
wobei die vorgenannten Heteroaryl-Gruppen und -Untergruppen 5- und 6-gliedrige monocyclische Ringsysteme umfassen, enthaltend 1 oder 2 Heteroatome, ausgewählt aus N, NR^{N} und O, wobei R^{N} H und C₁₋₄-Alkyl bezeichnet, und
R², R³, R⁴, R⁵, m und n sind wie in Anspruch 5 definiert,
oder ein Salz hiervon.

7. Pharmazeutisch akzeptables bzw. annehmbares Salz der Verbindung nach ein oder mehreren der Ansprüche 1 bis 6.

8. Pharmazeutische Zusammensetzung, umfassend ein oder mehrere Verbindungen nach ein oder mehreren der Ansprüche 1 bis 6 oder ein oder oder mehrere pharmazeutisch akzeptable bzw. annehmbare Salze hiervon, gegebenenfalls zusammen mit ein oder mehreren inerten Trägern und/oder Verdünnungsmitteln.

9. Verbindung nach ein oder mehreren der Ansprüche 1 bis 6 oder ein pharmazeutisch akzeptables bzw. annehmbares Salz hiervon zur Verwendung als Medikament.

10. Verbindung nach ein oder mehreren der Ansprüche 1 bis 6 oder ein pharmazeutisch akzeptables bzw. annehmbares Salz hiervon für die Behandlung von Erkrankungen oder Zuständen, die durch die Modulation der Funktion von GPR40 beeinflusst werden können, insbesondere für die Prophylaxe und/oder Therapie von metabolischen Erkrankungen, wie Diabetes, insbesondere Diabetes mellitus Typ 2, und Bedingungen, die mit der Krankheit in Zusammenhang stehen, einschließlich Insulinresistenz, Adipositas, kardiovaskuläre Erkrankungen und Dyslipidämie.

11. Pharmazeutische Zusammensetzung, umfassend ein oder mehrere Verbindungen nach ein oder mehreren der Ansprüche 1 bis 6 oder ein oder mehrere pharmazeutisch akzeptable bzw. annehmbare Salze hiervon und ein oder mehrere zusätzliche therapeutische Mittel, gegebenenfalls zusammen mit ein oder mehreren inerten Trägern und/oder Verdünnungsmitteln.

## Revendications

1. Composé de formule (I) 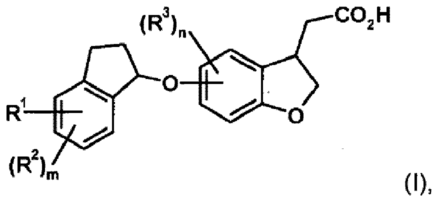 dans laquelle :
R¹ est sélectionné dans le groupe constitué de H, F, Cl, Br, I,
des groupes alkyle en C₁₋₈, alcényle en C₂₋₆, alcynyle en C₂₋₆, cycloalkyle en C₃₋₁₂, (cycloalkyle en C₃₋₁₂)alkyle en C₁₋₄, (bicycloalkyle en C₅-₁₂)-, (bicycloalkyle en C₅₋₁₂)-(alkyle en C₁₋₆), cycloalcényle en C₅₋₆, (cyclo-alcényle en C₅₋₆)-(alkyle en C₁₋₄), alkyloxy en C₁₋₈, (cycloalkyle en C₃₋₆)-oxy, et (cycloalkyle en C₃₋₆)-(alkyloxy en C₁₋₄), dans lequel l'un quelconque de ces groupes est facultativement et indépendamment substitué par 1 à 3 atomes de fluor et/ou 1 à 3 groupes R⁴,
des groupes hétérocyclyle en C₃₋₁₂, (hétérocyclyl en C₃₋₁₂)-(alkyle en C₁₋₄), bicyclohétérocyclyl-, bicyclohétérocyclyl (alkyle en C₁₋₆), hétérocyclyl (alkyle en C₁₋₄)-oxy, et hétérocyclyloxy en C₃₋₁₂, dans lequel l'un quelconque de ces groupes est facultativement et indépendamment substitué par 1 à 3 groupes R⁴,
des groupes aryle, aryl(alkyle en C₁₋₄), aryl(alkyle en C₁₋₄)-oxy, aryloxy, hétéroaryle, hétéroaryl(alkyle en C₁₋₄), hétéroaryl (alkyle en C₁₋₄)-oxy, et hétéroaryloxy, dans lequel l'un quelconque de ces groupes est facultativement et indépendamment substitué par 1 à 5 groupes R⁵,
des groupes (alkyle en C₁₋₄)-sulfanyle, (alkyle en C₁₋₄)-sulfinyle, et (alkyle en C₁₋₄)-sulfonyle, dans lequel l'un quelconque de ces groupes est facultativement substitué par 1 à 3 atomes de fluor, et
des groupes cyano, nitro, amino, alkylamino en C₁₋₄, et di-(alkyle en C₁₋₄)-amino ;
dans lequel les groupes hétérocyclyle et les sous-fractions susmentionnés peuvent être facultativement partiellement insaturés et comprennent de 1 à 3 hétéroatomes ou groupes sélectionnés parmi N, NR^{N}, 0 et S, à condition qu'uniquement au plus deux des hétéroatomes soient 0 et S et qu'aucune liaison O-O, S-S, et S-O ne soit formée, alors qu'un groupe méthylène lié à un hétéroatome peut être remplacé par un groupe carbonyle, tandis que les groupes hétérocyclyle sont liés au résidu respectif via un atome de carbone ou d'azote ; R^{N} représente H, un groupe (alkyle en C₁₋₄)-, (alkyle en C₁₋₄)-C(O)- ou (alkyle en C₁₋₄)-O-C(O)-,
dans lequel les groupes bicyclohétérocyclyle et les sous-fractions susmentionnés peuvent être partiellement insaturés et comprennent de 6 à 12 éléments cycliques et de 1 à 3 hétéroatomes ou groupes sélectionnés parmi N, NR^{N}, 0 et S, à condition qu'uniquement au plus deux des hétéroatomes soient 0 et S et qu'aucune liaison O-O, S-S, et S-O ne soit formée, alors qu'un groupe méthylène lié à un hétéroatome peut être remplacé par un groupe carbonyle, tandis que les groupes hétérocyclyle sont liés au résidu respectif via un atome de carbone ou d'azote ; R^{N} indique H, un groupe (alkyle en C₁₋₄)-, (alkyle en C₁₋₄)-C(O)-, ou (alkyle en C₁₋₄)-O-C(O)-,
dans lequel les groupes bicycloalkyle et bicyclohétérocyclyle et les sous-fractions susmentionnés comprennent des systèmes cycliques condensés, pontés et spiro-cycliques,
dans lequel les groupes aryle et les sous-fractions susmentionnés comprennent de 6 à 10 atomes de carbone, dans lequel dans les groupes aromatiques bicycliques le cycle qui n'est pas lié au résidu respectif peut être partiellement saturé, et
dans lequel les groupes hétéroaryle et les sous-fractions susmentionnés sont constitués de 5 à 14 atomes contenant de 1 à 3 hétéroatomes sélectionnés parmi N, 0 ou S(O)ᵣ, dans lequel r est 0, 1, ou 2, dans lequel dans les groupes hétéroaromatiques polycycliques les cycles qui ne sont pas liés au résidu respectif peuvent être partiellement ou totalement saturés, alors qu'au moins un cycle aromatique comprend un ou plusieurs hétéroatomes,
R² est sélectionné dans le groupe constitué de F, Cl, Br, I, des groupes cyano, alkyle en C₁₋₄, cycloalkyle en C₅₋₆, et alkyloxy en C₁₋₄, dans lequel tout groupe alkyle et cycloalkyle ou toute sous-fraction est facultativement substitué par 1 à 3 atomes de fluor,
R³ est sélectionné dans le groupe constitué de F, Cl, Br, I, des groupes alkyle en C₁₋₄, et alkyloxy en C₁₋₄, dans lequel tout groupe alkyle ou toute sous-fraction est facultativement substitué par 1 à 3 atomes de fluor,
R⁴ est sélectionné dans le groupe constitué de F, Cl, Br, I, des groupes cyano, alkyle en C₁₋₄, hydroxy, hydroxy-(alkyle en C₁₋₄), (alkyle en C₁₄)-oxy, (alkyloxy en C₁₋₄)-(alkyle en C₁₋₄), (alkyle en C₁₋₄)-sulfanyle, (alkyle en C₁₋₄)-sulfinyle, (alkyle en C₁₋₄)-sulfonyle, (cycloalkyle en C₃-C₆)-, (cycloalkyle en C₃₋₆)-oxy, dans lequel tout groupe alkyle et cycloalkyle ou toute sous-fraction est facultativement substitué par 1 à 3 atomes de fluor, et
R⁵ est sélectionné dans le groupe constitué de F, Cl, Br, I, des groupes cyano, nitro, amino, (alkyle en C₁₋₄)-amino, di-(alkyle en C₁₋₄)-amino, alkyle en C₁₋₄, alcényle en C₁₋₄, alcynyle en C₁₋₄, hydroxy, hydroxy-(alkyle en C₁₋₄), (alkyle en C₁₋₄)-oxy, (alkyloxy en C₁₋₄)-(alkyle en C₁₋₄), (alkyle en C₁₋₄)-sulfanyle, (alkyle en C₁₋₄)-sulfinyle, (alkyle en C₁₋₄)-sulfonyle, (cycloalkyle en C₃-C₆)-, (cycloalkyle en C₃₋₆)-oxy, dans lequel tout groupe alkyle et cycloalkyle ou toute sous-fraction est facultativement substitué par 1 à 5 atomes de fluor,
m est sélectionné parmi les nombres 0, 1, 2 et 3, de préférence parmi les nombres 0, 1 et 2 ou, de manière davantage préférée, parmi les nombres 0 et 1, le nombre 0 étant préféré entre tous et
n est sélectionné parmi les nombres 0, 1, 2 et 3, de préférence, parmi les nombres 0 et 1, le nombre 0 étant préféré entre tous,
dans lequel dans toute définition mentionnée ci-avant et sauf spécification contraire, toute fraction alkyle mentionnée ci-avant est à chaîne linéaire ou ramifiée,
ou un sel de celui-ci.

2. Composé selon la revendication 1, dans lequel
R¹ est sélectionné dans le groupe constitué de H, F, Cl, Br,
des groupes alkyle en C₁₋₆, alcényle en C₅₋₆, cycloalkyle en C₃₋₁₀, (cycloalkyle en C₃₋₁₀) alkyle en C₁₋₄, cyclo-alcényle en C₅₋₆, alkyloxy en C₁₋₆, (cycloalkyle en C₃₋₆)-oxy, et (cycloalkyle en C₃₋₆)-(alkyloxy en C₁₋₄), dans lequel l'un quelconque de ces groupes est facultativement et indépendamment substitué par 1 à 3 atomes de fluor et/ou 1 à 3 groupes R⁴,
des groupes hétérocyclyle en C₅₋₁₀, (hétérocyclyl en C₅₋₁₀)(alkyle en C₁₋₄), bicyclohétérocyclyl-, bicyclohétérocyclyl-(alkyle en C₁₋₄), (hétérocyclyl en C₅₋₁₀) (alkyle en C₁₋₄)-oxy, et hétérocyclyloxy en C₅₋₁₀, dans lequel l'un quelconque de ces groupes est facultati-vement et indépendamment substitué par 1 à 3 groupes R⁴,
des groupes phényle, phényl-(alkyle en C₁₋₄), phényl-(alkyle en C₁₋₄)-oxy, phényloxy, hétéroaryle, hétéro-aryl(alkyle en C₁₋₄), hétéroaryl (alkyle en C₁₋₄)-oxy, et hétéroaryloxy, dans lequel l'un quelconque de ces groupes est facultativement et indépendamment substitué par 1 à 3 groupes R⁵,
un groupe (alkyle en C₁₋₂)-sulfanyle facultativement substitué par 1 à 3 atomes de fluor, et
un groupe cyano,
dans lequel les groupes hétérocyclyle et les sous-fractions susmentionnés peuvent être partiellement insaturés et comprennent de 1 à 3 hétéroatomes ou groupes sélectionnés parmi N, NR^{N}, 0 et S, à condition qu'uniquement au plus deux des hétéroatomes soient 0 et S et qu'aucune liaison O-O, S-S, et S-O ne soit formée, alors qu'un groupe méthylène lié à un hétéroatome peut être remplacé par un groupe carbonyle, tandis que les groupes hétérocyclyle sont liés au résidu respectif via un atome de carbone ou d'azote ; R^{N} représente H, un groupe (alkyle en C₁₋₄)-, (alkyle en C₁₋₄)-C(O)-, ou (alkyle en C₁₋₄)-O-C(O)-,
dans lequel les groupes bicyclohétérocyclyle et les sous-fractions susmentionnés peuvent être partiellement insaturés et comprennent de 6 à 10 éléments cycliques et de 1 à 3 hétéroatomes ou groupes sélectionnés parmi N, NR^{N}, 0 et S, à condition qu'uniquement au plus deux des hétéroatomes soient 0 et S et qu'aucune liaison O-O, S-S, et S-O ne soit formée, alors qu'un groupe méthylène lié à un hétéroatome peut être remplacé par un groupe carbonyle, tandis que les groupes hétérocyclyle sont liés au résidu respectif via un atome de carbone ou d'azote ; R^{N} représente H, un groupe (alkyle en C₁₋₄)-, (alkyle en C₁₋₄)-C(O)-, ou (alkyle en C₁₋₄)-OC(O)-,
dans lequel les groupes bicycloalkyle et bicyclohétérocyclyle et les sous-fractions susmentionnés comprennent des systèmes cycliques condensés et pontés,
dans lequel les groupes hétéroaryle et les sous-fractions susmentionnés sont constitués de 5 à 10 atomes contenant de 1 à 3 hétéroatomes sélectionnés parmi N, 0, ou S(O)ᵣ, dans lequel r est 0, 1, ou 2, dans lequel dans les groupes hétéroaromatiques polycycliques le ou les cycles qui ne sont pas liés au résidu respectif peuvent être partiellement ou totalement saturés, alors qu'au moins un cycle aromatique comprend un ou plusieurs hétéroatomes,
ou un sel de celui-ci.

3. Composé selon la revendication 1, dans lequel R¹ est sélectionné dans le groupe constitué de H, F, Cl, Br,
des groupes alkyle en C₁₋₆, alcényle en C₅₋₆, cycloalkyle en C₃₋₆, (cycloalkyle en C₃₋₆) alkyle en C₁₋₄, cyclo-alcényle en C₅₋₆, tétrahydropyranyl-(alkyle en C₁₋₂), et alkyloxy en C₁₋₆, dans lequel l'un quelconque de ces groupes est facultativement et indépendamment substitué par 1 à 3 atomes de fluor et/ou 1 à 3 groupes R⁴,
des groupes oxétanyl, tétrahydrofuranyle, dihydro-pyranyle, tétrahydropyranyle, morpholinyle, tétrahydrofuran-3-yloxy, tétrahydropyran-3-yloxy, tétrahydropyran-4-yloxy, 8-oxa-spiro[4.5]décényle, 3-oxa-spiro-[5.5]undécényle, 2-oxa-bicyclo[3.1.1]heptyle, 2-oxa-bicyclo[2.2.1]heptyle, 2-oxa-bicyclo[3.1.1]hept-4-yloxy, 2-oxa-bicyclo[3.1.1]hept-5-yloxy, 2-oxa-bicyclo[3.1.1]-hept-6-yloxy, 2-oxa-bicyclo[2.2.1]hept-4-yloxy, 2-oxa-bicyclo[2.2.1]hept-5-yloxy, et 2-oxa-bicyclo[2.2.1]-hept-6-yloxy, dans lequel l'un quelconque de ces groupes est facultativement et indépendamment substitué par 1 à 3 groupes R⁴,
un groupe 2-oxo-1,2-dihydropyridyle facultativement substitué par 1 à 3 groupes R⁴,
des groupes phényle et hétéroaryle, dans lequel l'un quelconque de ces groupes est facultativement et indépendamment substitué par 1 à 3 groupes R⁵,
des groupes trifluorométhylsulfanyle et cyano,
dans lequel les groupes hétéroaryle et les sous-fractions susmentionnés comprennent des systèmes cycliques monocycliques à 5 et 6 chaînons contenant 1 ou 2 hétéroatomes sélectionnés parmi N, NR^{N}, et 0, dans lequel R^{N} représente H et un groupe alkyle en C₁₋₄, et
m et n indiquent tous les deux le nombre 1,
ou un sel de celui-ci.

4. Composé selon la revendication 1, dans lequel :
R¹ est sélectionné dans le groupe constitué de H, F, Cl, Br,
des groupes alkyle en C₁₋₆, alcényle en C₅₋₆, cycloalkyle en C₃₋₁₀, (cycloalkyle en C₃₋₁₀) alkyle en C₁₋₄, cycloalcényle en C₅₋₆, alkyloxy en C₁₋₆, (cycloalkyle en C₃₋₆)-oxy, et (cycloalkyle en C₃₋₆) - (alkyloxy en C₁₋₄), dans lequel l'un quelconque de ces groupes est facultativement et indépendamment substitué par 1 à 3 atomes de fluor et/ou 1 à 3 groupes R⁴,
des groupes hétérocyclyle en C₅₋₁₀, (hétérocyclyl en C₅₋₁₀)(alkyle en C₁₋₄) bicyclohétérocyclyl-, bicyclohétérocyclyl-(alkyle en C₁₋₄)-, (hétérocyclyl en C₅₋₁₀) (alkyle en C₁₋₄)-oxy, et hétérocyclyloxy en C₅₋₁₀, dans lequel l'un quelconque de ces groupes est facultati-vement et indépendamment substitué par 1 à 3 groupes R⁴,
des groupes phényle, phényl-(alkyle en C₁₋₄), phényl-(alkyle en C₁₋₄)-oxy, phényloxy, hétéroaryle, hétéroaryl(alkyle en C₁₋₄), hétéroaryl (alkyle en C₁₋₄)-oxy, et hétéroaryloxy, dans lequel l'un quelconque de ces groupes est facultativement et indépendamment substitué par 1 à 3 groupes R⁵,
un groupe (alkyle en C₁₋₂)-sulfanyle facultativement substitué par 1 à 3 atomes de fluor, et
un groupe cyano,
dans lequel les groupes hétérocyclyle et les sous-fractions susmentionnés peuvent être partiellement insaturés et comprennent de 1 à 3 hétéroatomes ou groupes sélectionnés parmi N, NR^{N}, 0 et S, à condition qu'uniquement au plus deux des hétéroatomes soient 0 et S et qu'aucune liaison O-O, S-S, et S-O ne soit formée, alors qu'un groupe méthylène lié à un hétéroatome peut être remplacé par un groupe carbonyle, tandis que les groupes hétérocyclyle sont liés au résidu respectif via un atome de carbone ou d'azote ; R^{N} indique H, un groupe (alkyle en C₁₋₄)-, (alkyle en C₁₋₄)-C(O)-, ou (alkyle en C₁₋₄)-O-C(O)-,
dans lequel les groupes bicyclohétérocyclyle et les sous-fractions susmentionnés peuvent être partiellement insaturés et comprennent de 6 à 10 éléments cycliques et de 1 à 3 hétéroatomes ou groupes sélectionnés parmi N, NR^{N}, 0 et S, à condition qu'uniquement au plus deux des hétéroatomes soient 0 et S et qu'aucune liaison O-O, S-S, et S-O ne soit formée, alors qu'un groupe méthylène lié à un hétéroatome peut être remplacé par un groupe carbonyle, tandis que les groupes hétéro-cyclyle sont liés au résidu respectif via un atome de carbone ou d'azote ; R^{N} représente H, un groupe (alkyle en C₁₋₄)-, (alkyle en C₁₋₄)-C(O)-, ou (alkyle en C₁₋₄)-OC(O)-,
dans lequel les groupes bicycloalkyle et bicyclohétéro-cyclyle et les sous-fractions susmentionnés comprennent des systèmes cycliques condensés et pontés,
dans lequel les groupes hétéroaryle et les sous-fractions susmentionnés sont constitués de 5 à 10 atomes contenant de 1 à 3 hétéroatomes sélectionnés parmi N, 0, ou S(O)ᵣ, dans lequel r est 0, 1, ou 2, dans lequel dans les groupes hétéroaromatiques polycycliques le ou les cycles qui ne sont pas liés au résidu respectif peuvent être partiellement ou totale-ment saturés, alors qu'au moins un cycle aromatique comprend un ou plusieurs hétéroatomes,
R² est sélectionné dans le groupe constitué de F, Cl, Br, des groupes méthyle, difluorométhyle, trifluoro-méthyle, cyclopropyle, méthoxy, difluorométhoxy, et trifluorométhoxy,
R³ est sélectionné dans le groupe constitué de F, Cl, Br, des groupes méthyle, difluorométhyle, trifluoro-méthyle, méthoxy, difluorométhoxy, et trifluorométhoxy,
R⁴ est sélectionné dans le groupe constitué de F, des groupes cyano, hydroxy, méthyle, difluorométhyle, tri-fluorométhyle, méthoxy, difluorométhoxy, et trifluorométhoxy,
R⁵ est sélectionné dans le groupe constitué de F, Cl, des groupes cyano, alkyle en C₁₋₃ (de préférence méthyle), difluorométhyle, trifluorométhyle, pentafluoroéthyle, hydroxy, méthoxy, difluorométhoxy, et trifluorométhoxy, et
m et n indiquent chacun indépendamment 0 ou 1,
ou un sel de celui-ci.

5. Composé selon la revendication 1, dans lequel
R¹ est sélectionné dans le groupe constitué de H, F, Cl, Br,
des groupes alkyle en C₁₋₆, alcényle en C₅₋₆, cycloalkyle en C₃₋₁₀, (cycloalkyle en C₃₋₁₀) alkyle en C₁₋₄, cycloalcényle en C₅₋₆, alkyloxy en C₁₋₆, (cycloalkyle en C₃₋₆)-oxy, et (cycloalkyle en C₃₋₆) - (alkyloxy en C₁₋₄), dans lequel l'un quelconque de ces groupes est facultativement et indépendamment substitué par 1 à 3 atomes de fluor et/ou 1 à 3 groupes R⁴,
des groupes hétérocyclyle en C₅₋₁₀, (hétérocyclyl en C₅₋₁₀)-(alkyle en C₁₋₄), bicyclohétérocyclyl-, bicyclohétérocyclyl- (alkyle en C₁₋₄)-, (hétérocyclyl en C₅₋₁₀)-(alkyle en C₁₋₄)-oxy, et hétérocyclyloxy en C₅₋₁₀, dans lequel l'un quelconque de ces groupes est facultativement et indépendamment substitué par 1 à 3 groupes R⁴,
des groupes phényle, phényl-(alkyle en C₁₋₄), phényl-(alkyle en C₁₋₄)-oxy, phényloxy, hétéroaryle, hétéro-aryl(alkyle en C₁₋₄), hétéroaryl (alkyle en C₀₋₄)-oxy, et hétéroaryloxy, dans lequel l'un quelconque de ces groupes est facultativement et indépendamment substitué par 1 à 3 groupes R⁵,
un groupe (alkyle en C₁₋₂)-sulfanyle facultativement substitué par 1 à 3 atomes de fluor, et
un groupe cyano,
dans lequel les groupes hétérocyclyle et les sous-fractions susmentionnés peuvent être partiellement insaturés et comprennent de 1 à 3 hétéroatomes ou groupes sélectionnés parmi N, NR^{N}, 0 et S, à condition qu'uniquement au plus deux des hétéroatomes soient 0 et S et qu'aucune liaison O-O, S-S, et S-O ne soit formée, alors qu'un groupe méthylène lié à un hétéroatome peut être remplacé par un groupe carbonyle, tandis que les groupes hétérocyclyle sont liés au résidu respectif via un atome de carbone ou d'azote ; R^{N} indique H, un groupe (alkyle en C₁₋₄)-, (alkyle en C₁₋₄)-C(O)-, ou (alkyle en C₁₋₄))-O-C(O)-,
dans lequel les groupes bicyclohétérocyclyle et les sous-fractions susmentionnés peuvent être partiellement insaturés et comprennent de 6 à 10 éléments cycliques et de 1 à 3 hétéroatomes ou groupes sélectionnés parmi N, NR^{N}, 0 et S, à condition qu'uniquement au plus deux des hétéroatomes soient 0 et S et qu'aucune liaison O-O, S-S, et S-O ne soit formée, alors qu'un groupe méthylène lié à un hétéroatome peut être remplacé par un groupe carbonyle, tandis que les groupes hétéro-cyclyle sont liés au résidu respectif via un atome de carbone ou d'azote ; R^{N} représente H, des groupes (alkyle en C₁₋₄)-, (alkyle en C₁₋₄)-C(O)- ou (alkyle en C₁₋₄)-O-C(O)-,
dans lequel les groupes bicycloalkyle et bicyclohétérocyclyle et les sous-fractions susmentionnés comprennent des systèmes cycliques condensés et pontés,
dans lequel les groupes hétéroaryle et les sous-fractions susmentionnés sont constitués de 5 à 10 atomes contenant de 1 à 3 hétéroatomes sélectionnés parmi N, 0, ou S(O)ᵣ, dans lequel r est 0, 1, ou 2, dans lequel dans les groupes hétéroaromatiques polycycliques le ou les cycles qui ne sont pas liés au résidu respectif peuvent être partiellement ou totalement saturés, alors qu'au moins un cycle aromatique comprend un ou plusieurs hétéroatomes,
R² est sélectionné dans le groupe constitué de F, Cl, Br, des groupes méthyle, difluorométhyle, trifluorométhyle, cyclopropyle, méthoxy, difluorométhoxy, et trifluorométhoxy,
R³ est sélectionné dans le groupe constitué de F, Cl, Br, des groupes méthyle, difluorométhyle, trifluorométhyle, méthoxy, difluorométhoxy, et trifluorométhoxy,
R⁴ est sélectionné dans le groupe constitué de F, des groupes cyano, hydroxy, méthyle, difluorométhyle, trifluorométhyle, méthoxy, difluorométhoxy, et trifluorométhoxy,
R⁵ est sélectionné dans le groupe constitué de F, Cl, des groupes cyano, alkyle en C₁₋₃ (de préférence méthyle), difluorométhyle, trifluorométhyle, pentafluoroéthyle, hydroxy, méthoxy, difluorométhoxy, et trifluorométhoxy,
m indique 0, et
n indique 0,
ou un sel de celui-ci.

6. Composé selon la revendication 1, dans lequel
R¹ est sélectionné dans le groupe constitué de H, F, Cl, Br,
des groupes alkyle en C₅₋₆, alcényle en C₅₋₆, cycloalkyle en C₃₋₆, (cycloalkyle en C₃₋₆) alkyle en C₁₋₄, cyclo-alcényle en C₅₋₆, tétrahydropyranyl-(alkyle en C₁₋₂), et alkyloxy en C₅₋₆, dans lequel l'un quelconque de ces groupes est facultativement et indépendamment substitué par 1 à 3 atomes de fluor et/ou 1 à 3 groupes R⁴,
des groupes oxétanyl, tétrahydrofuranyle, dihydropyranyle, tétrahydropyranyle, morpholinyle, tétrahydrofuran-3-yloxy, tétrahydropyran-3-yloxy, tétrahydropyran-4-yloxy, 8-oxa-spiro[4.5]décényle, 3-oxa-spiro-[5.5]undécényle, 2-oxa-bicyclo[3.1.1]heptyle, 2-oxabicyclo[2.2.1]heptyle, 2-oxa-bicyclo[3.1.1]hept-4-yloxy, 2-oxa-bicyclo[3.1.1]hept-5-yloxy, 2-oxa-bicyclo[3.1.1]-hept-6-yloxy, 2-oxa-bicyclo[2.2.1]hept-4-yloxy, 2-oxabicyclo[2.2.1]hept-5-yloxy, et 2-oxa-bicyclo[2.2.1]-hept-6-yloxy, dans lequel l'un quelconque de ces groupes est facultativement et indépendamment substitué par 1 à 3 groupes R⁴,
un groupe 2-oxo-1,2-dihydropyridyle, facultativement substitué par 1 à 3 groupes R⁴,
des groupes phényle et hétéroaryle, dans lequel l'un quelconque de ces groupes est facultativement et indépendamment substitué par 1 à 3 groupes R⁵,
des groupes trifluorométhylsulfanyle et cyano,
dans lequel les groupes hétéroaryle et les sous-fractions susmentionnés comprennent des systèmes cycliques monocycliques à 5 et 6 chaînons contenant 1 à 2 hétéroatomes sélectionnés parmi N, NR^{N}, et 0, dans lequel R^{N} indique H et un groupe alkyle en C₁₋₄, et
R², R³, R⁴, R⁵, m et n sont définis comme dans la revendication 5,
ou un sel de celui-ci.

7. Sel pharmaceutiquement acceptable d'un composé selon une ou plusieurs des revendications 1 à 6.

8. Composition pharmaceutique comprenant un ou plusieurs composés selon une ou plusieurs des revendications 1 à 6 ou un ou plusieurs sels pharmaceutiquement acceptables de ceux-ci, facultativement conjointement à un ou plusieurs véhicules et/ou diluants inertes.

9. Composé selon une ou plusieurs des revendications 1 à 6 ou un sel pharmaceutiquement acceptable de celui-ci, pour son utilisation en tant que médicament.

10. Composé selon une ou plusieurs des revendications 1 à 6, ou un sel pharmaceutiquement acceptable de celui-ci, pour le traitement de maladies ou d'affections qui peuvent être influencées par la modulation de la fonction de GPR40, en particulier pour la prophylaxie et/ou le traitement de maladies métaboliques, telles que le diabète, plus spécifiquement le diabète sucré de type 2, et d'affections associées à la maladie, dont l'insulinorésistance, l'obésité, une maladie cardiovasculaire et la dyslipidémie.

11. Composition pharmaceutique comprenant un ou plusieurs composés selon une ou plusieurs des revendications 1 à 6 ou un ou plusieurs sels pharmaceutiquement acceptables de ceux-ci, et un ou plusieurs agents thérapeutiques supplémentaires, facultativement conjointement à un ou plusieurs véhicules et/ou diluants inertes.
